# EUROPEAN PATENT APPLICATION

(11) **EP 4 553 068 A1**
(43) Date of publication of application: **14.05.2025**
(21) Application number: 23835546.5
(22) Date of filing: 05.07.2023
(51) Int. Cl.: C07D 239/36, A61K 31/192, A61K 31/513, A61K 31/517, A61P 9/10, A61P 13/00, A61P 13/04, A61P 13/08, A61P 13/10, A61P 17/00, A61P 19/02, A61P 25/00, A61P 25/04, A61P 25/28, A61P 27/02, A61P 27/06, A61P 29/00, A61P 35/00, A61P 37/06, A61P 43/00

(54) **PYRIMIDINE DERIVATIVES**

(30) Priority: 06.07.2022 JP 2022109093
(71) Applicant: ASKA Pharmaceutical Co., Ltd., Tokyo 108-8532 (JP)
(72) Inventor: MAEDA Satoshi, Fujisawa-shi, Kanagawa 251-8555 (JP); WATANABE Tomoaki, Fujisawa-shi, Kanagawa 251-8555 (JP); NOSE Takashi, Fujisawa-shi, Kanagawa 251-8555 (JP); TERAO Yoshito, Fujisawa-shi, Kanagawa 251-0012 (JP); TOKUHARA Hidekazu, Fujisawa-shi, Kanagawa 251-0012 (JP)
(74) Representative: Godemeyer Blum Lenze Patentanwälte Partnerschaft mbB - werkpatent
(86) International application number: PCT/JP2023/024847
(87) International publication number: WO 2024/010015

(57) **Abstract**

A compound represented by the following general formula (1), which has a potent inhibitory action against mPGES-1, and is useful as an active ingredient of a medicament for therapeutic treatment of inflammation and the like.

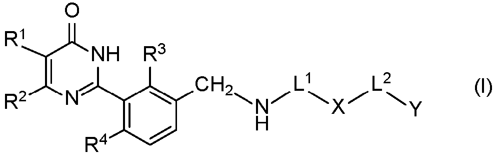

## Description

### Technical Field

The present invention relates to a novel pyrimidine derivative. More specifically, the present invention relates to a pyrimidine derivative having an mPGES-1 inhibitory action, and useful as an active ingredient of a medicament for prophylactic and/or therapeutic treatment of such diseases as inflammation, pain, and rheumatism.

### Background Art

Prostaglandin E2 (PGE2) is involved in inflammation, pain, pyrexia, and the like by means of PGE receptors, and can suppress the PGE2 production to suppress inflammation. Non-steroidal anti-inflammatory drugs (NSAIDs) inhibit cyclooxygenase (COX) in the upstream of the prostaglandin biosynthesis pathway, and thereby exhibit anti-inflammatory activity. However, they totally suppress the prostaglandin biosynthesis pathway downstream from the prostanoid production in which COX is involved, and therefore they cause gastric mucosal injury as side effects due to suppression of secretion of gastric mucus or blood flow in gastric mucosa.

There are two types of isozymes of COX, COX-1 and COX-2. Among them, COX-2 is expressed and induced in inflammatory tissues by various inflammation-promoting stimuli (for example, those of cytokines such as interleukin-1β). Medicaments that selectively inhibit this COX-2 suppress the production of PGI2, which has vasodilatation and platelet aggregation actions; however, since they do not inhibit the production of thromboxane A2 (TXA2) catalyzed by COX-1 (TXA2 causes vasoconstriction and platelet coagulation), they are considered to increase risk of thrombosis, and increase cardiovascular events, either.

In the downstream of the biosynthesis pathway of PGE2, PGE2 is biosynthesized from PGH2 by the prostaglandin E synthase (PGE synthase, PGES). As PGES, there are three kinds of enzymes, mPGES-1 (microsomal prostaglandin E2 synthase-1), mPGES-2 (microsomal prostaglandin E2 synthase-2), and cPGES (cytosolic PGE synthase). Among them, mPGES-1 is an inducible trimer enzyme, of which expression is increased by inflammatory stimuli (Proc. Natl. Acad. Sci. USA, 96, pp.7220-7225, 1999), and it is known to participate in cancer, inflammation, pain, pyrexia, tissue repair, and the like.

Since mPGES-1 inhibitors can selectively inhibit the final step of the PGE2 biosynthesis pathway in inflammation lesions (Pharmacol. Rev., 59, pp.207-224, 2007; J. Biol. Chem., 279, pp.33684-33695, 2004), they are expected as anti-inflammatory agents that do not cause gastric mucosal injuries, unlike the non-steroidal anti-inflammatory agents. There are also expected efficacies of mPGES-1 inhibitors for prophylactic and/or therapeutic treatment of pain, rheumatism, osteoarthritis, pyrexia, Alzheimer's disease, multiple sclerosis, arteriosclerosis, ocular hypertension such as glaucoma, ischemic retinopathy, systemic scleroderma, malignant tumors such as large intestine tumor, and diseases for which suppression of the PGE2 production exhibits efficacy (refer to International Patent Publication WO2015/125842 for PGE2, PGES, and mPGES-1, as well as uses of mPGES-1 inhibitors, and the like). In addition, it is also known that mPGES-1 inhibitors increase productions of other prostanoids in connection with the suppression of the PGE2 production (J. Biol. Chem., 280, pp.16579-16585, 2005).

As such mPGES-1 inhibitors, there are known the heterocyclic derivatives disclosed in Japanese Patent No. 5601422, the substituted pyrimidine compounds disclosed in International Patent Publication WO2015/59618, the triazine compounds disclosed in International Patent Publication WO2015/125842, and the like. International Patent Publication WO2015/59618 discloses a pyrimidine compound substituted with p-trifluoromethylphenyl group and 2-chloro-5-isobutyramidobenzyl group (Example 2), and International Patent Publication WO2015/125842 discloses triazine compounds substituted with p-trifluoromethylphenyl group and 2-chloro-5-isobutyramidobenzyl group (Examples 1 to 28). In addition, International Patent Publication WO2017/73709 discloses a pyrimidine derivative having mPGES-1 inhibitory activity which was substituted with m-phenylene group, and International Patent Publication WO2019/44868 discloses a pyrimidine derivative having mPGES-1 inhibitory activity which was substituted with heterocyclic ring.

### Prior art references

### Patent documents

Patent document 1: Japanese Patent No. 5601422
Patent document 2: International Patent Publication WO2015/59618
Patent document 3: International Patent Publication WO2015/125842
Patent document 4: International Patent Publication WO2017/073709
Patent document 5: International Patent Publication WO2019/44868

### Non-patent documents

Non-patent document 1: Proc. Natl. Acad. Sci. USA, 96, pp.7220-7225, 1999
Non-patent document 2: Pharmacol. Rev., 59, pp.207-224, 2007
Non-patent document 3: J. Biol. Chem., 279, pp.33684-33695, 2004
Non-patent document 4: J. Biol. Chem., 280, pp.16579-16585, 2005

### Summary of the Invention

### Object to be Achieved by the Invention

An object of the present invention is to provide a novel compound having an mPGES-1 inhibitory action, and useful as an active ingredient of a medicament for prophylactic and/or therapeutic treatment of such diseases as inflammation, pain, and rheumatism.

### Means for Achieving the Object

The inventors of the present invention conducted various researches in order to achieve the aforementioned object. As a result, they found that pyrimidine derivatives represented by the following general formula (1) have a potent mPGES-1 inhibitory action, and are useful as active ingredients of medicaments for prophylactic and/or therapeutic treatment of such diseases as inflammation, pain, and rheumatism, and accomplished the present invention.

The present invention thus provides:
[1] a compound represented by the following general formula (1): 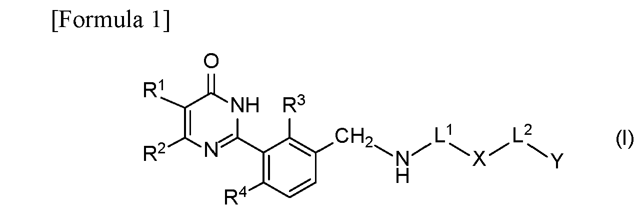
   (in the formula, R¹ and R² each independently represent a hydrogen atom, a halogen atom, a C₁₋₆ alkyl group which may have a substituent, or an aromatic heterocyclic group having 1 or 2 or more ring-constituting heteroatoms which may have a substituent, and when R¹ and R² both represent an alkyl group, they may be bonded to each other to form a C₃₋₆ alkylene group which may have a substituent;
   R³ and R⁴ each independently represent a hydrogen atom, a halogen atom, or a C₁₋₆ alkyl group which may have a substituent;
   L¹ represents -CO-, -SO₂-, or -CO-C(R⁵)(R⁶)- (R⁵ and R⁶ each independently represent a hydrogen atom or a C₁₋₆ alkyl group which may have a substituent, and when R⁵ and R⁶ both represent an alkyl group, they may be bonded to each other to form a C₂₋₅ alkylene group which may have a substituent);
   X represents a cyclic hydrocarbon group which may have a substituent or a heterocyclic group having 1 or 2 or more ring-constituting heteroatoms which may have a substituent;
   L² represents -A¹-R⁷-A²- (A¹ represents a single bond, -CO-, -NH-, or -O-; R⁷ represents a single bond or a C₁₋₆ alkylene group; and A² represents a single bond, -CO-, -NH-, or -O-); and
   Y represents a hydrogen atom, a cyclic hydrocarbon group which may have a substituent, or a heterocyclic group having 1 or 2 or more ring-constituting heteroatoms which may have a substituent)
   or a salt thereof.
   According to preferred embodiments of the aforementioned invention, there are provided the following compound or a salt thereof.
[2] The compound or a salt thereof according to the aforementioned [1],
   wherein R¹ and R² each independently is a hydrogen atom, a fluorine atom, a C₁₋₆ alkyl group (this alkyl group may be substituted with 1 or 2 or more halogen atoms), or an aromatic heterocyclic group having 1 or 2 or more ring-constituting heteroatoms which may have a substituent, and when R¹ and R² both represent alkyl groups, they may be bonded to each other to form a C₃₋₅ alkylene group;
   R³ and R⁴ each independently is a hydrogen atom, a fluorine atom, a chlorine atom, or a C₁₋₆ alkyl group (this alkyl group may be substituted with 1 or 2 or more fluorine atoms);
   L¹ is -CO-, -SO₂-, or -CO-C(R⁵)(R⁶)- (R⁵ and R⁶ each independently represent a hydrogen atom or a C₁₋₆ alkyl group (when R⁵ and R⁶ both represent an alkyl group, they may be bonded to each other to form a C₃₋₅ alkylene group));
   X is an aromatic hydrocarbon group which may have a substituent, a saturated or partially saturated cyclic hydrocarbon group which may have a substituent, an aromatic heterocyclic group having 1 or 2 or more ring-constituting heteroatoms which may have a substituent, or a saturated or partially saturated heterocyclic group having 1 or 2 or more ring-constituting heteroatoms which may have a substituent;
   in -A¹-R⁷-A²- represented by L², A¹, R⁷, and A² are all single bonds; A¹ is a single bond, R⁷ is -CO-, -NH-, or -O-, and A² is a single bond or a C₁₋₆ alkylene group; or A¹ is a C₁₋₆ alkylene group, R⁷ is -CO-, -NH-, or -O-, and A² is a single bond); and
   Y is a hydrogen atom, an aromatic hydrocarbon group which may have a substituent, a saturated or partially saturated cyclic hydrocarbon group which may have a substituent, an aromatic heterocyclic group having 1 or 2 or more ring-constituting heteroatoms which may have a substituent, or a saturated or partially saturated heterocyclic group having 1 or 2 or more ring-constituting heteroatoms which may have a substituent.
[3] The compound or a salt thereof according to the aforementioned [1],
   wherein R¹ is a hydrogen atom, a fluorine atom, or a C₁₋₆ alkyl group (this alkyl group may be substituted with 1 or 2 or more fluorine atoms);
   R² is a fluorine atom, a C₁₋₆ alkyl group (this alkyl group may be substituted with 1 or 2 or more halogen atoms), or an aromatic heterocyclic group having 1 or 2 or more ring-constituting heteroatoms which may have a substituent, and when R¹ and R² both represent alkyl groups, they may be bonded to each other to form a C₃₋₅ alkylene group;
   R³ and R⁴ each independently is a hydrogen atom, a fluorine atom, a chlorine atom, or a C₁₋₆ alkyl group (this alkyl group may be substituted with 1 or 2 or more fluorine atoms);
   L¹ is -CO-, -SO₂-, or -CO-C(R⁵)(R⁶)- (R⁵ and R⁶ each independently represent a hydrogen atom or a C₁₋₆ alkyl group (this alkyl group may be bonded to each other to form a C₃₋₄ alkylene group);
   X is a monocyclic or bicyclic aromatic hydrocarbon group which may have a substituent, a monocyclic or bicyclic, saturated or partially saturated cyclic hydrocarbon group which may have a substituent, a monocyclic or bicyclic aromatic heterocyclic group having 1 or 2 or more ring-constituting heteroatoms which may have a substituent, or a monocyclic or bicyclic, saturated or partially saturated heterocyclic group having 1 or 2 or more ring-constituting heteroatoms which may have a substituent;
   in -A¹-R⁷-A²- represented by L², A¹, R⁷, and A² are all single bonds; A¹ is a single bond, R⁷ is -CO-, -NH-, or -O-, and A² is a single bond or a C₁₋₄ alkylene group; or A¹ is a C₁₋₄ alkylene group, R⁷ is -CO-, -NH-, or -O-, and A² is a single bond); and
   Y is a hydrogen atom, a monocyclic or bicyclic aromatic hydrocarbon group which may have a substituent, a monocyclic or bicyclic, saturated or partially saturated cyclic hydrocarbon group which may have a substituent, a monocyclic or bicyclic aromatic heterocyclic group having 1 or 2 or more ring-constituting heteroatoms which may have a substituent, or a monocyclic or bicyclic, saturated or partially saturated heterocyclic group having 1 or 2 or more ring-constituting heteroatoms which may have a substituent.
[4] The compound or a salt thereof according to the aforementioned [1],
   wherein R¹ is a hydrogen atom, a fluorine atom, or a C₁₋₆ alkyl group (this alkyl group may be substituted with 1 or 2 or more fluorine atoms);
   R² is a fluorine atom or a C₁₋₆ alkyl group (this alkyl group may be substituted with 1 or 2 or more fluorine atoms), or a monocyclic aromatic heterocyclic group containing 1 or 2 or more ring-constituting nitrogen atoms which may have a substituent;
   R³ and R⁴ each independently is a hydrogen atom, a fluorine atom, a chlorine atom, or a C₁₋₆ alkyl group (this alkyl group may be substituted with 1 or 2 or more fluorine atoms);
   L¹ is -CO- or -SO₂-;
   X is a phenyl group which may have a substituent, a naphthyl group which may have a substituent, a monocyclic or bicyclic, 3- to 12-membered, saturated or partially saturated cyclic hydrocarbon group which may have a substituent, a monocyclic or bicyclic aromatic heterocyclic group containing 1 or 2 or more ring-constituting nitrogen atoms which may have a substituent, or a monocyclic or bicyclic, saturated or partially saturated heterocyclic group containing 1 or 2 or more ring-constituting nitrogen atoms which may have a substituent;
   in -A¹-R⁷-A²- represented by L², A¹, R⁷, and A² are all single bonds; A¹ is a single bond, R⁷ is -O-, and A² is a single bond or a C₁₋₄ alkylene group; or A¹ is a C₁₋₄ alkylene group, R⁷ is -O-, and A² is a single bond; and
   Y is a hydrogen atom, a monocyclic or bicyclic aromatic hydrocarbon group which may have a substituent, a monocyclic or bicyclic, 3- to 12-membered, saturated or partially saturated cyclic hydrocarbon group which may have a substituent, a monocyclic or bicyclic aromatic heterocyclic group containing 1 or 2 or more ring-constituting nitrogen atoms which may have a substituent, or a monocyclic or bicyclic, saturated or partially saturated heterocyclic group containing 1 or 2 or more ring-constituting nitrogen atoms which may have a substituent.
[5] The compound or a salt thereof according to the aforementioned [1],
   wherein R¹ is a hydrogen atom, a fluorine atom, or a methyl group;
   R² is a hydrogen atom, a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a pyridyl group, a pyridazinyl group, or a pyrazinyl group (provided that the aforementioned pyridyl group, pyridazinyl group, or pyrazinyl group may have 1 or 2 or more substituents selected from the following group: a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group);
   R³ and R⁴ each independently is a hydrogen atom, a fluorine atom, a chlorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
   L¹ is -CO-;
   X is a phenyl group which may have a substituent, a naphthyl group which may have a substituent, a monocyclic saturated hydrocarbon group which may have a substituent, a monocyclic aromatic heterocyclic group containing 1 or 2 or more ring-constituting nitrogen atoms which may have a substituent, or a monocyclic saturated or partially saturated heterocyclic group containing 1 or 2 or more ring-constituting nitrogen atoms which may have a substituent;
   in -A¹-R⁷-A²- represented by L², A¹, R⁷, and A² are all single bonds; A¹ is a single bond, R⁷ is -O-, and A² is a single bond, a methylene group, or an ethylene group; or A¹ is a methylene group or an ethylene group, R⁷ is -O-, and A² is a single bond; and
   Y is a hydrogen atom, a monocyclic or bicyclic aromatic hydrocarbon group which may have a substituent, a saturated, monocyclic, 3- to 7-membered cyclic hydrocarbon group which may have a substituent, a monocyclic or bicyclic aromatic heterocyclic group containing 1 or 2 or more ring-constituting nitrogen atoms which may have a substituent, or a saturated or partially saturated, monocyclic or bicyclic heterocyclic group containing 1 or 2 or more ring-constituting nitrogen atoms which may have a substituent.
[6] The compound or a salt thereof according to the aforementioned [1],
   wherein R¹ is a hydrogen atom or a fluorine atom;
   R² is a hydrogen atom, a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a 2-pyridyl group, a 3-pyridyl group, a 4-pyridyl group, a 3-pyridazinyl group, a 4-pyridazinyl group, or a 2-pyrazinyl group (provided that the aforementioned 2-pyridyl group, 3-pyridyl group, 4-pyridyl group, 3-pyridazinyl group, 4-pyridazinyl group, or 2-pyrazinyl group may have 1 or 2 or more substituents selected from the following group: a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group);
   R³ is a hydrogen atom or a fluorine atom;
   R⁴ is a fluorine atom, a chlorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
   L¹ is -CO-;
   X is a monocyclic saturated hydrocarbon group which may have a substituent or a monocyclic, saturated or partially saturated heterocyclic group containing 1 or 2 or more ring-constituting nitrogen atoms which may have a substituent;
   L² is a single bond, -O-, -CH₂-O-, or -O-CH₂-; and
   Y is a hydrogen atom, a phenyl group which may have a substituent, a naphthyl group which may have a substituent, or a monocyclic or bicyclic aromatic heterocyclic group containing 1 or 2 or more ring-constituting nitrogen atoms which may have a substituent.
[7] The compound or a salt thereof according to the aforementioned [1],
   wherein R¹ is a hydrogen atom or a fluorine atom;
   R² is a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a 2-pyridyl group, a 3-pyridyl group, a 4-pyridyl group, a 3-pyridazinyl group, a 4-pyridazinyl group, or a 2-pyrazinyl group (provided that the aforementioned 2-pyridyl group, 3-pyridyl group, 4-pyridyl group, 3-pyridazinyl group, 4-pyridazinyl group, or 2-pyrazinyl group may have 1 or 2 or more substituents selected from the following group: a methyl group, an ethyl group, a methoxy group, an ethoxy group, a fluorine atom, a chlorine atom, a difluoromethyl group, a trifluoromethyl group, a difluoromethyloxy group, a trifluoromethyloxy group, and a cyano group);
   R³ is a fluorine atom;
   R⁴ is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
   L¹ is -CO-;
   X is a monocyclic saturated hydrocarbon group which may have a substituent or a monocyclic saturated heterocyclic group containing 1 or 2 or more ring-constituting nitrogen atoms which may have a substituent;
   L² is a single bond, -O-, or -O-CH₂-; and
   Y is a hydrogen atom, a phenyl group which may have a substituent, a naphthyl group which may have a substituent, a pyridyl group which may have a substituent, or a quinolyl group which may have a substituent.
[8] The compound or a salt thereof according to the aforementioned [1],
   wherein R¹ is a hydrogen atom or a fluorine atom;
   R² is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
   R³ is a fluorine atom;
   R⁴ is a trifluoromethyl group;
   L¹ is -CO-;
   X is a 4- to 6-membered saturated hydrocarbon group which may have a substituent or a 4- to 6-membered saturated heterocyclic group containing one ring-constituting nitrogen atom which may have a substituent;
   L² is a single bond, -O-, or -O-CH₂-; and
   Y is a hydrogen atom, a phenyl group which may have a substituent, a naphthyl group which may have a substituent, a pyridyl group which may have a substituent, or a quinolyl group which may have a substituent.
[9] The compound or a salt thereof according to the aforementioned [1],
   wherein R¹ is a hydrogen atom or a fluorine atom;
   R² is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
   R³ is a fluorine atom;
   R⁴ is a trifluoromethyl group;
   L¹ is -CO-;
   X is a cyclobutanediyl group, a cyclopentanediyl group, a cyclohexanediyl group, an azetidinediyl group, a pyrrolidinediyl group, or a piperidinediyl group;
   L² is a single bond or -O-CH₂-; and
   Y is a hydrogen atom, a phenyl group, a naphthyl group, a pyridyl group, or a quinolyl group (provided that the aforementioned phenyl group, naphthyl group, pyridyl group, or quinolyl group may have 1 or 2 or more substituents selected from the following group: a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group).
[10] The compound or a salt thereof according to the aforementioned [1],
   wherein R¹ is a hydrogen atom or a fluorine atom;
   R² is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
   R³ is a fluorine atom;
   R⁴ is a trifluoromethyl group;
   L¹ is -CO-;
   X is a 1,3-cyclobutanediyl group, a 1,4-cyclohexanediyl group, a 1,3-azetidinediyl group, or a 1,4-piperidinediyl group;
   L² is a single bond or -O-CH₂-; and
   Y is a hydrogen atom, a phenyl group, a naphthyl group, a pyridyl group, or a quinolyl group (provided that the aforementioned phenyl group, naphthyl group, pyridyl group, or quinolyl group may have 1 or 2 or more substituents selected from the following group: a methyl group, an ethyl group, a methoxy group, an ethoxy group, a fluorine atom, a chlorine atom, a difluoromethyl group, a trifluoromethyl group, a difluoromethyloxy group, a trifluoromethyloxy group, and a cyano group).
[11] The compound or a salt thereof according to the aforementioned [1],
   wherein R¹ is a hydrogen atom or a fluorine atom;
   R² is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
   R³ is a fluorine atom;
   R⁴ is a trifluoromethyl group;
   L¹ is -CO-;
   X is a 1,3-cyclobutanediyl group;
   L² is -O-CH₂-; and
   Y is a phenyl group (provided that the aforementioned phenyl group may have 1 or 2 or more substituents selected from the following group: a methyl group, an ethyl group, a methoxy group, an ethoxy group, a fluorine atom, a chlorine atom, a difluoromethyl group, a trifluoromethyl group, a difluoromethyloxy group, a trifluoromethyloxy group, and a cyano group).

As other aspects, the present invention provides an mPGES-1 inhibitor containing a compound represented by the aforementioned general formula (1), or a salt thereof; and a PGE2 biosynthesis inhibitor containing a compound represented by the aforementioned general formula (1), or a salt thereof.

As still another aspect, the present invention provides a medicament containing a compound represented by the aforementioned general formula (1) or a physiologically acceptable salt thereof as an active ingredient. This medicament can be used for prophylactic and/or therapeutic treatment of, for example, inflammation, pain, rheumatism, osteoarthritis, pyrexia, Alzheimer's disease, multiple sclerosis, arteriosclerosis, ocular hypertension such as glaucoma, ischemic retinopathy, systemic scleroderma, malignant tumors such as large intestine tumor, and diseases for which suppression of the PGE2 production exhibits efficacy.

The present invention also provides use of a compound represented by the aforementioned general formula (1) or a salt thereof for manufacture of the aforementioned mPGES-1 inhibitor, the aforementioned PGE2 biosynthesis inhibitor, or the aforementioned medicament; a method for inhibiting mPGES-1 in a living body of a mammal including human, which comprises the step of administrating an effective amount of a compound represented by the aforementioned general formula (1) or a physiologically acceptable salt thereof to the mammal including human; a method for inhibiting biosynthesis of PGE2 in a living body of a mammal including human, which comprises the step of administrating an effective amount of a compound represented by the aforementioned general formula (1) or a physiologically acceptable salt thereof to the mammal including human; and a method for promoting production of a prostanoid other than PGE2 by inhibiting biosynthesis of PGE2 in a living body of a mammal including human, which comprises the step of administrating an effective amount of a compound represented by the aforementioned general formula (1) or a physiologically acceptable salt thereof to the mammal including human.

### Effect of the Invention

The compounds represented by the aforementioned general formula (1) and salts thereof provided by the present invention can exhibit a potent inhibitory action against mPGES-1 to inhibit the biosynthesis of PGE2. Therefore, the compounds represented by the aforementioned general formula (1) and salts thereof are useful as an active ingredient of a medicament for prophylactic and/or therapeutic treatment of, for example, pain, rheumatism, osteoarthritis, pyrexia, Alzheimer's disease, multiple sclerosis, arteriosclerosis, ocular hypertension such as glaucoma, ischemic retinopathy, systemic scleroderma, malignant tumors such as large intestine tumor, and diseases for which suppression of the PGE2 production exhibits efficacy.

### Modes for Carrying out the Invention

In this specification, the term halogen atom may mean fluorine atom, chlorine atom, bromine atom, or iodine atom. As the halogen atom, fluorine atom or chlorine atom is preferred.

In this specification, the term alkyl group may mean a straight, branched, or cyclic alkyl group, or an alkyl group consisting of a combination of the foregoing alkyl groups. Although carbon number of the alkyl group is not particularly limited, it is, for example, 1 to 6, preferably 1 to 4. The same shall apply to alkyl moieties of other substituents having an alkyl moiety (for example, alkoxy group).

In this specification, the term alkylene group may mean a straight or branched alkylene group. Although carbon number of the alkylen group is not particularly limited, it is, for example, 1 to 6, preferably 1 to 4. When two alkyl groups are bonded to form an alkylene group with a cyclic structure, carbon number of the alkylen group is not particularly limited, but is, for example, 2 to 6, preferably 3 to 4. Further, in some cases, for example, carbon number in a range of 3 to 6 or 2 to 5 may be preferred.

In this specification, examples of aromatic hydrocarbon groups include, for example, monocyclic or bicyclic aromatic hydrocarbon groups, preferably phenyl groups or naphthyl groups, and the like, but the examples are not limited to these.

In this specification, the cyclic hydrocarbon group may mean a non-aromatic hydrocarbon group, i.e., a saturated or partially saturated cyclic hydrocarbon group. Examples of the cyclic hydrocarbon group include, for example, monocyclic or polycyclic, preferably monocyclic or bicyclic, saturated or partially saturated cyclic hydrocarbon groups. Preferred examples include monocyclic or bicyclic, 3- to 12-membered, saturated or partially saturated cyclic hydrocarbon groups, more preferred examples include monocyclic, 3- to 7-membered, saturated cyclic hydrocarbon groups, and even more preferred examples include 4- to 6-membered, saturated hydrocarbon groups. For example, a cyclopropyl group, a cyclobutyl group, a cyclopentyl group, a cyclohexyl group, a cycloheptyl group, and the like are more preferable, but the cyclic hydrocarbon group is not limited to these.

In this specification, examples of ring-constituting heteroatom contained in the aromatic heterocyclic group or heterocyclic group include, for example, a nitrogen atom, an oxygen atom, a sulfur atom, or the like. When there are two or more ring-constituting heteroatoms, they may be the same or different.

In this specification, aromatic heterocyclic group may mean an aromatic heterocyclic group having 1 or 2 or more ring-constituting heteroatoms. Examples of the aromatic heterocyclic group include, for example, a monocyclic aromatic heterocyclic group or a condensed polycyclic aromatic heterocyclic group having 1 or 2 or more ring-constituting heteroatoms, and preferred examples include a monocyclic or bicyclic aromatic heterocyclic group having 1 or 2 or more ring-constituting heteroatoms.

Examples of monocyclic aromatic heterocyclic group having 1 or 2 or more ring-constituting heteroatoms include, for example, 2-furyl group, 3-furyl group, 2-thienyl group, 3-thienyl group, 1-pyrrolyl group, 2-pyrrolyl group, 3-pyrrolyl group, 2-oxazolyl group, 4-oxazolyl group, 5-oxazolyl group, 3-isoxazolyl group, 4-isoxazolyl group, 5-isoxazolyl group, 2-thiazolyl group, 4-thiazolyl group, 5-thiazolyl group, 3-isothiazolyl group, 4-isothiazolyl group, 5-isothiazolyl group, 1-imidazolyl group, 2-imidazolyl group, 4-imidazolyl group, 5-imidazolyl group, 1-pyrazolyl group, 3-pyrazolyl group, 4-pyrazolyl group, 5-pyrazolyl group, (1,2,3-oxadiazol)-4-yl group, (1,2,3-oxadiazol)-5-yl group, (1,2,4-oxadiazol)-3-yl group, (1,2,4-oxadiazol)-5-yl group, (1,2,5-oxadiazol)-3-yl group, (1,2,5-oxadiazol)-4-yl group, (1,3,4-oxadiazol)-2-yl group, (1,3,4-oxadiazol)-5-yl group, furazanyl group, (1,2,3-thiadiazol)-4-yl group, (1,2,3-thiadiazol)-5-yl group, (1,2,4-thiadiazol)-3-yl group, (1,2,4-thiadiazol)-5-yl group, (1,2,5-thiadiazol)-3-yl group, (1,2,5-thiadiazol)-4-yl group, (1,3,4-thiadiazolyl)-2-yl group, (1,3,4-thiadiazolyl)-5-yl group, (1H-1,2,3-triazol)-1-yl group, (1H-1,2,3-triazol)-4-yl group, (1H-1,2,3-triazol)-5-yl group, (2H-1,2,3-triazol)-2-yl group, (2H-1,2,3-triazol)-4-yl group, (1H-1,2,4-triazol)-1-yl group, (1H-1,2,4-triazol)-3-yl group, (1H-1,2,4-triazol)-5-yl group, (4H-1,2,4-triazol)-3-yl group, (4H-1,2,4-triazol)-4-yl group, (1H-tetrazol)-1-yl group, (1H-tetrazol)-5-yl group, (2H-tetrazol)-2-yl group, (2H-tetrazol)-5-yl group, 2-pyridyl group, 3-pyridyl group, 4-pyridyl group, 3-pyridazinyl group, 4-pyridazinyl group, 2-pyrimidinyl group, 4-pyrimidinyl group, 5-pyrimidinyl group, 2-pyrazinyl group, (1,2,3-triazin)-4-yl group, (1,2,3-triazin)-5-yl group, (1,2,4-triazin)-3-yl group, (1,2,4-triazin)-5-yl group, (1,2,4-triazin)-6-yl group, (1,3,5-triazin)-2-yl group, 1-azepinyl group, 2-azepinyl group, 3-azepinyl group, 4-azepinyl group, (1,4-oxazepin)-2-yl group, (1,4-oxazepin)-3-yl group, (1,4-oxazepin)-5-yl group, (1,4-oxazepin)-6-yl group, (1,4-oxazepin)-7-yl group, (1,4-thiazepin)-2-yl group, (1,4-thiazepin)-3-yl group, (1,4-thiazepin)-5-yl group, (1,4-thiazepin)-6-yl group, (1,4-thiazepin)-7-yl group, and the like, but the examples are not limited to these.

Examples of condensed polycyclic aromatic heterocyclic group having 1 or 2 or more ring-constituting heteroatoms include, for example, 2-benzofuranyl group, 3-benzofuranyl group, 4-benzofuranyl group, 5-benzofuranyl group, 6-benzofuranyl group, 7-benzofuranyl group, 1-isobenzofuranyl group, 4-isobenzofuranyl group, 5-isobenzofuranyl group, 2-benzo[b]thienyl group, 3-benzo[b]thienyl group, 4-benzo[b]thienyl group, 5-benzo[b]thienyl group, 6-benzo[b]thienyl group, 7-benzo[b]thienyl group, 1-benzo[c]thienyl group, 4-benzo[c]thienyl group, 5-benzo[c]thienyl group, 1-indolyl group, 2-indolyl group, 3-indolyl group, 4-indolyl group, 5-indolyl group, 6-indolyl group, 7-indolyl group, (2H-isoindol)-1-yl group, (2H-isoindol)-2-yl group, (2H-isoindol)-4-yl group, (2H-isoindol)-5-yl group, (1H-indazol)-1-yl group, (1H-indazol)-3-yl group, (1H-indazol)-4-yl group, (1H-indazol)-5-yl group, (1H-indazol)-6-yl group, (1H-indazol)-7-yl group, (2H-indazol)-1-yl group, (2H-indazol)-2-yl group, (2H-indazol)-4-yl group, (2H-indazol)-5-yl group, 2-benzoxazolyl group, 4-benzoxazolyl group, 5-benzoxazolyl group, 6-benzoxazolyl group, 7-benzoxazolyl group, (1,2-benzisoxazol)-3-yl group, (1,2-benzisoxazol)-4-yl group, (1,2-benzisoxazol)-5-yl group, (1,2-benzisoxazol)-6-yl group, (1,2-benzisoxazol)-7-yl group, (2,1-benzisoxazol)-3-yl group, (2,1-benzisoxazol)-4-yl group, (2,1-benzisoxazol)-5-yl group, (2,1-benzisoxazol)-6-yl group, (2,1-benzisoxazol)-7-yl group, 2-benzothiazolyl group, 4-benzothiazolyl group, 5-benzothiazolyl group, 6-benzothiazolyl group, 7-benzothiazolyl group, (1,2-benzisothiazol)-3-yl group, (1,2-benzisothiazol)-4-yl group, (1,2-benzisothiazol)-5-yl group, (1,2-benzisothiazol)-6-yl group, (1,2-benzisothiazol)-7-yl group, (2,1-benzisothiazol)-3-yl group, (2,1-benzisothiazol)-4-yl group, (2,1-benzisothiazol)-5-yl group, (2,1-benzisothiazol)-6-yl group, (2,1-benzisothiazol)-7-yl group, (1,2,3-benzoxadiazol)-4-yl group, (1,2,3-benzoxadiazol)-5-yl group, (1,2,3-benzoxadiazol)-6-yl group, (1,2,3-benzoxadiazol)-7-yl group, (2,1,3-benzoxadiazol)-4-yl group, (2,1,3-benzoxadiazol)-5-yl group, (1,2,3-benzothiadiazol)-4-yl group, (1,2,3-benzothiadiazol)-5-yl group, (1,2,3-benzothiadiazol)-6-yl group, (1,2,3-benzothiadiazol)-7-yl group, (2,1,3-benzothiadiazol)-4-yl group, (2,1,3-benzothiadiazol)-5-yl group, (1H-benzotriazol)-1-yl group, (1H-benzotriazol)-4-yl group, (1H-benzotriazol)-5-yl group, (1H-benzotriazol)-6-yl group, (1H-benzotriazol)-7-yl group, (2H-benzotriazol)-2-yl group, (2H-benzotriazol)-4-yl group, (2H-benzotriazol)-5-yl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 3-cinnolinyl group, 4-cinnolinyl group, 5-cinnolinyl group, 6-cinnolinyl group, 7-cinnolinyl group, 8-cinnolinyl group, 2-quinazolinyl group, 4-quinazolinyl group, 5-quinazolinyl group, 6-quinazolinyl group, 7-quinazolinyl group, 8-quinazolinyl group, 2-quinoxalinyl group, 5-quinoxalinyl group, 6-quinoxalinyl group, 1-phthalazinyl group, 5-phthalazinyl group, 6-phthalazinyl group, 2-naphthyridinyl group, 3-naphthyridinyl group, 4-naphthyridinyl group, 2-purinyl group, 6-purinyl group, 7-purinyl group, 8-purinyl group, 2-pteridinyl group, 4-pteridinyl group, 6-pteridinyl group, 7-pteridinyl group, 1-carbazolyl group, 2-carbazolyl group, 3-carbazolyl group, 4-carbazolyl group, 9-carbazolyl group, 2-(α-carbolinyl) group, 3-(α-carbolinyl) group, 4-(α-carbolinyl) group, 5-(α-carbolinyl) group, 6-(α-carbolinyl) group, 7-(α-carbolinyl) group, 8-(α-carbolinyl) group, 9-(α-carbolinyl) group, 1-(β-carbonylyl) group, 3-(β-carbonylyl) group, 4-(β-carbonylyl) group, 5-(β-carbonylyl) group, 6-(β-carbonylyl) group, 7-(β-carbonylyl) group, 8-(β-carbonylyl) group, 9-(β-carbonylyl) group, 1-(γ-carbolinyl) group, 2-(γ-carbolinyl) group, 4-(γ-carbolinyl) group, 5-(γ-carbolinyl) group, 6-(γ-carbolinyl) group, 7-(γ-carbolinyl) group, 8-(γ-carbolinyl) group, 9-(γ-carbolinyl) group, 1-acridinyl group, 2-acridinyl group, 3-acridinyl group, 4-acridinyl group, 9-acridinyl group, 1-phenoxazinyl group, 2-phenoxazinyl group, 3-phenoxazinyl group, 4-phenoxazinyl group, 10-phenoxazinyl group, 1-phenothiazinyl group, 2-phenothiazinyl group, 3-phenothiazinyl group, 4-phenothiazinyl group, 10-phenothiazinyl group, 1-phenazinyl group, 2-phenazinyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 2-phenanthrolinyl group, 3-phenanthrolinyl group, 4-phenanthrolinyl group, 5-phenanthrolinyl group, 6-phenanthrolinyl group, 7-phenanthrolinyl group, 8-phenanthrolinyl group, 9-phenanthrolinyl group, 10-phenanthrolinyl group, 1-thianthrenyl group, 2-thianthrenyl group, 1-indolizinyl group, 2-indolizinyl group, 3-indolizinyl group, 5-indolizinyl group, 6-indolizinyl group, 7-indolizinyl group, 8-indolizinyl group, 1-phenoxathiinyl group, 2-phenoxathiinyl group, 3-phenoxathiinyl group, 4-phenoxathiinyl group, thieno[2,3-b]furyl group, pyrrolo[1,2-b]pyridazinyl group, pyrazolo[1,5-a]pyridyl group, imidazo[11,2-a]pyridyl group, imidazo[1,5-a]pyridyl group, imidazo[1,2-b]pyridazinyl group, imidazo[1,2-a]pyrimidinyl group, 1,2,4-triazolo[4,3-a]pyridyl group, and 1,2,4-triazolo[4,3-a]pyridazinyl group, but the examples are not limited to these.

In this specification, the heterocyclic group may mean a non-aromatic heterocyclic group, i.e., a saturated or partially saturated heterocyclic group having 1 or 2 or more ring-constituting heteroatoms. Examples of the heterocyclic group include, for example, monocyclic or polycyclic, preferably monocyclic or bicyclic, saturated or partially saturated heterocyclic groups having 1 or 2 or more ring-constituting heteroatoms. Preferred examples include monocyclic saturated heterocyclic groups containing 1 or 2 or more ring-constituting nitrogen atoms, and more preferred examples include 4- to 6-membered saturated heterocyclic groups containing one ring-constituting nitrogen atom.

Examples of the saturated or partially saturated, monocyclic, 3- to 7-membered heterocyclic group include, for example, 1-aziridinyl group, 1-azetidinyl group, 1-pyrrolidinyl group, 2-pyrrolidinyl group, 3-pyrrolidinyl group, 2-tetrahydrofuryl group, 3-tetrahydrofuryl group, thiolanyl group, 1-imidazolidinyl group, 2-imidazolidinyl group, 4-imidazolidinyl group, 1-pyrazolidinyl group, 3-pyrazolidinyl group, 4-pyrazolidinyl group, 1-(2-pyrrolinyl) group, 1-(2-imidazolinyl) group, 2-(2-imidazolinyl) group, 1-(2-pyrazolinyl) group, 3-(2-pyrazolinyl) group, piperidino group, 2-piperidinyl group, 3-piperidinyl group, 4-piperidinyl group, 1-homopiperidinyl group, 2-tetrahydropyranyl group, morpholino group, (thiomorpholin)-4-yl group, 1-piperazinyl group, 1-homopiperazinyl group, and the like, but the examples are not limited to these.

Examples of the saturated or partially saturated, bicyclic, 8- to 12-membered heterocyclic group include, for example, 2-quinuclidinyl group, 2-cromanyl group, 3-cromanyl group, 4-cromanyl group, 5-cromanyl group, 6-cromanyl group, 7-cromanyl group, 8-cromanyl group, 1-isocromanyl group, 3-isocromanyl group, 4-isocromanyl group, 5-isocromanyl group, 6-isocromanyl group, 7-isocromanyl group, 8-isocromanyl group, 2-thiocromanyl group, 3-thiocromanyl group, 4-thiocromanyl group, 5-thiocromanyl group, 6-thiocromanyl group, 7-thiocromanyl group, 8-thiocromanyl group, 1-isothiocromanyl group, 3-isothiocromanyl group, 4-isothiocromanyl group, 5-isothiocromanyl group, 6-isothiocromanyl group, 7-isothiocromanyl group, 8-isothiocromanyl group, 1-indolinyl group, 2-indolinyl group, 3-indolinyl group, 4-indolinyl group, 5-indolinyl group, 6-indolinyl group, 7-indolinyl group, 1-isoindolinyl group, 2-isoindolinyl group, 4-isoindolinyl group, 5-isoindolinyl group, 2-(4H-chromenyl) group, 3-(4H-chromenyl) group, 4-(4H-chromenyl) group, 5-(4H-chromenyl) group, 6-(4H-chromenyl) group, 7-(4H-chromenyl) group, 8-(4H-chromenyl) group, 1-isochromenyl group, 3-isochromenyl group, 4-isochromenyl group, 5-isochromenyl group, 6-isochromenyl group, 7-isochromenyl group, 8-isochromenyl group, 1-(1H-pyrrolidinyl) group, 2-(1H-pyrrolidinyl) group, 3-(1H-pyrrolidinyl) group, 5-(1H-pyrrolidinyl) group, 6-(1H-pyrrolidinyl) group, 7-(1H-pyrrolidinyl) group, and the like, but the examples are not limited to these.

When the expression "which may have a substituent" is used for a certain functional group in this specification, it means that the functional group is unsubstituted, or the functional group has one or two or more substituents at chemically substitutable positions, unless otherwise indicated. Type, number, and substitution position of substituent existing on a functional group are not particularly limited, and when there are two or more substituents, they may be the same or different. Examples of the substituent existing on a functional group include, for example, an alkyl group, a halogen atom, oxo group, thioxo group, nitro group, nitroso group, cyano group, isocyano group, cyanato group, tiocyanato group, isocyanato group, isotiocyanato group, hydroxy group, sulfanyl group, carboxy group, sulfanylcarbonyl group, oxalo group, mesoxalo group, thiocarboxy group, dithiocarboxy group, carbamoyl group, thiocarbamoyl group, sulfo group, sulfamoyl group, sulfino group, sulfinamoyl group, sulfeno group, sulfenamoyl group, phosphono group, hydroxyphosphonyl group, a hydrocarbon group, a heterocyclic group, a hydrocarbon-oxy group, a (heterocyclic ring)-oxy group, a hydrocarbon-sulfanyl group, a (heterocyclic ring)-sulfanyl group, an acyl group, amino group, hydrazino group, hydrazono group, diazenyl group, ureido group, thioureido group, guanidino group, carbamoimidoyl group (amidino group), azido group, imino group, hydroxyamino group, hydroxyimino group, aminoxy group, diazo group, semicarbazino group, semicarbazono group, allophanyl group, hydantoyl group, phosphano group, phosphoroso group, phospho group, boryl group, silyl group, stanyl group, selanyl group, oxido group, and the like, but the examples are not limited to these examples.

The substituent in the aforementioned definitions may be substituted with another substituent at a chemically substitutable position on the substituent. Type, number, and substitution position of the substituent are not particularly limited, and when the substituent is substituted with two or more substituents, they may be the same or different. Examples of such a substituent include, for example, a halogenated alkyl group (for example, trifluoromethyl group and the like), a hydroxyalkyl group (for example, hydroxymethyl group and the like), a halogenated alkyl-carbonyl group (for example, trifluoroacetyl and the like), a halogenated alkyl-sulfonyl group (for example, trifluoromethanesulfonyl and the like), an acyl-oxy group, an acyl-sulfanyl group, an N-hydrocarbon-amino group, an N,N-di(hydrocarbon)-amino group, an N-(heterocyclic ring)-amino group, an N-hydrocarbon-N-(heterocyclic ring)-amino group, an acyl-amino group, a di(acyl)-amino group, and the like, but the examples are not limited to these examples.

Preferred examples of substituents that can exist on a functional group include, but are not limited to, 1 or 2 or more substituents selected from a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group.

R¹ and R² each independently represent a hydrogen atom, a halogen atom, a C₁₋₆ alkyl group which may have a substituent, or an aromatic heterocyclic group having 1 or 2 or more ring-constituting heteroatoms which may have a substituent. When R¹ and R² both represent an alkyl group, they may be bonded to each other to form a C₃₋₆ alkylene group which may have a substituent.

For example, R¹ and R² each independently represent a hydrogen atom, a fluorine atom, a C₁₋₆ alkyl group (this alkyl group may be substituted with 1 or 2 or more halogen atoms), or an aromatic heterocyclic group having 1 or 2 or more ring-constituting heteroatoms which may have a substituent, and when R¹ and R² both represent alkyl groups, they may be bonded to each other to form a C₃₋₅ alkylene group.

Preferably, R¹ is a hydrogen atom, a fluorine atom, or a C₁₋₆ alkyl group (this alkyl group may be substituted with 1 or 2 or more fluorine atoms), and R² is a fluorine atom, a C₁₋₆ alkyl group (this alkyl group may be substituted with 1 or 2 or more halogen atoms) or an aromatic heterocyclic group having 1 or 2 or more ring-constituting heteroatoms which may have a substituent, and when R¹ and R² both represent alkyl groups, they may be bonded to each other to form a C₃₋₅ alkylene group.

More preferably, R¹ is a hydrogen atom, a fluorine atom, or a C₁₋₆ alkyl group (this alkyl group may be substituted with 1 or 2 or more fluorine atoms), and R² is a fluorine atom, a C₁₋₆ alkyl group (this alkyl group may be substituted with 1 or 2 or more fluorine atoms), or a monocyclic aromatic heterocyclic group containing 1 or 2 or more ring-constituting nitrogen atoms which may have a substituent.

Even more preferably, R¹ is a hydrogen atom, a fluorine atom, or a methyl group, and R² is a hydrogen atom, a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a pyridyl group, a pyridazinyl group, or a pyrazinyl group, and the aforementioned pyridyl group, pyridazinyl group, or pyrazinyl group may have 1 or 2 or more substituents selected from the following group: aC₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group.

In a particularly preferred embodiment, R¹ is a hydrogen atom or a fluorine atom, and R² is a hydrogen atom, a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a 2-pyridyl group, a 3-pyridyl group, a 4-pyridyl group, a 3-pyridazinyl group, a 4-pyridazinyl group, or a 2-pyrazinyl group, and the aforementioned 2-pyridyl group, 3-pyridyl group, 4-pyridyl group, 3-pyridazinyl group, 4-pyridazinyl group, or 2-pyrazinyl group may have 1 or 2 or more substituents selected from the following group: a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group.

In another particularly preferred embodiment, R¹ is a hydrogen atom or a fluorine atom, and R² is a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a 2-pyridyl group, a 3-pyridyl group, a 4-pyridyl group, a 3-pyridazinyl group, a 4-pyridazinyl group, or a 2-pyrazinyl group, and the aforementioned 2-pyridyl group, 3-pyridyl group, 4-pyridyl group, 3-pyridazinyl group, 4-pyridazinyl group, or 2-pyrazinyl group may have 1 or 2 or more substituents selected from the following group: a methyl group, an ethyl group, a methoxy group, an ethoxy group, a fluorine atom, a chlorine atom, a difluoromethyl group, a trifluoromethyl group, a difluoromethyloxy group, a trifluoromethyloxy group, and a cyano group.

In yet another particularly preferred embodiment, R¹ is a hydrogen atom or a fluorine atom, and R² is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group.

R³ and R⁴ each independently represent a hydrogen atom, a halogen atom, or a C₁₋₆ alkyl group which may have a substituent.

Preferably, R³ and R⁴ are each independently a hydrogen atom, a fluorine atom, a chlorine atom, or a C₁₋₆ alkyl group (this alkyl group may be substituted with 1 or 2 or more fluorine atoms), and more preferably, R³ and R⁴ are each independently a hydrogen atom, a fluorine atom, a chlorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group.

In a further preferred embodiment, R³ is a hydrogen atom or a fluorine atom, and R⁴ is a fluorine atom, a chlorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group.

In yet another preferred embodiment, R³ is a fluorine atom, and R⁴ is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group.

Particularly preferably, R³ is a fluorine atom and R⁴ is a trifluoromethyl group.

L¹ represents -CO-, -SO₂-, or -CO-C(R⁵)(R⁶)- (R⁵ and R⁶ each independently represent a hydrogen atom or a C₁₋₆ alkyl group which may have a substituent, and when R⁵ and R⁶ both represent an alkyl group, they may be bonded to each other to form a C₂₋₅ alkylene group which may have a substituent).

Preferably, L¹ is -CO-, -SO₂-, or -CO-C(R⁵)(R⁶)- (R⁵ and R⁶ each independently represent a hydrogen atom or a C₁₋₆ alkyl group (when R⁵ and R⁶ both represent an alkyl group, they may be bonded to each other to form a C₃₋₅ alkylene group)). More preferably, L¹ is -CO-, -SO₂-, or -CO-C(R⁵)(R⁶)- (R⁵ and R⁶ each independently represent a hydrogen atom or a C₁₋₆ alkyl group (this alkyl group may be bonded to each other to form a C₃₋₄ alkylene group). Particularly preferably, L¹ is -CO- or -SO₂-, and most preferably L¹ is -CO-.

X represents a cyclic hydrocarbon group which may have a substituent or a heterocyclic group having 1 or 2 or more ring-constituting heteroatoms which may have a substituent.

Preferably, X is an aromatic hydrocarbon group which may have a substituent, a saturated or partially saturated cyclic hydrocarbon group which may have a substituent, an aromatic heterocyclic group having 1 or 2 or more ring-constituting heteroatoms which may have a substituent, or a saturated or partially saturated heterocyclic group having 1 or 2 or more ring-constituting heteroatoms which may have a substituent.

More preferably, X is a monocyclic or bicyclic aromatic hydrocarbon group which may have a substituent, a monocyclic or bicyclic, saturated or partially saturated cyclic hydrocarbon group which may have a substituent, a monocyclic or bicyclic aromatic heterocyclic group having 1 or 2 or more ring-constituting heteroatoms which may have a substituent, or a monocyclic or bicyclic, saturated or partially saturated heterocyclic group having 1 or 2 or more ring-constituting heteroatoms which may have a substituent.

In a further preferred embodiment, X is a phenyl group which may have a substituent, a naphthyl group which may have a substituent, a monocyclic or bicyclic, 3-to 12-membered, saturated or partially saturated cyclic hydrocarbon group which may have a substituent, a monocyclic or bicyclic aromatic heterocyclic group containing 1 or 2 or more ring-constituting nitrogen atoms which may have a substituent, or a monocyclic or bicyclic, saturated or partially saturated heterocyclic group containing 1 or 2 or more ring-constituting nitrogen atoms which may have a substituent.

In yet another preferred embodiment, X is a phenyl group which may have a substituent, a naphthyl group which may have a substituent, a monocyclic, saturated hydrocarbon group which may have a substituent, a monocyclic aromatic heterocyclic group containing 1 or 2 or more ring-constituting nitrogen atoms which may have a substituent, or a monocyclic saturated or partially saturated heterocyclic group containing 1 or 2 or more ring-constituting nitrogen atoms which may have a substituent; X is a monocyclic saturated hydrocarbon group which may have a substituent or a monocyclic, saturated or partially saturated heterocyclic group containing 1 or 2 or more ring-constituting nitrogen atoms which may have a substituent; or X is a 4- to 6-membered saturated hydrocarbon group which may have a substituent or a 4- to 6-membered saturated heterocyclic group containing one ring-constituting nitrogen atom which may have a substituent.

In a particularly preferred embodiment, X is a cyclobutanediyl group, a cyclopentanediyl group, a cyclohexanediyl group, an azetidinediyl group, a pyrrolidinediyl group, or a piperidinediyl group, or X is a 1,3-cyclobutanediyl group, a 1,4-cyclohexanediyl group, a 1,3-azetidinediyl group, or a 1,4-piperidinediyl group, and most preferably X is a 1,3-cyclobutanediyl group.

L² represents -A¹-R⁷-A²- (A¹ represents a single bond, -CO-, -NH-, or -O-, R⁷ represents a single bond or a C₁₋₆ alkylene group, and A² represents a single bond, -CO-, -NH-, or -O-).

It is preferable that in -A¹-R⁷-A²- represented by L², A¹, R⁷, and A² are all single bonds; A¹ is a single bond, R⁷ is -CO-, -NH-, or -O-, and A² is a single bond or a C₁₋₆ alkylene group; or A¹ is a C₁₋₆ alkylene group, R⁷ is -CO-, -NH-, or -O-, and A² is a single bond).

It is also preferable that in -A¹-R⁷-A²- represented by L², A¹, R⁷, and A² are all single bonds; A¹ is a single bond, R⁷ is -CO-, -NH-, or -O-, and A² is a single bond or a C₁₋₄ alkylene group; or A¹ is a C₁₋₄ alkylene group, R⁷ is -CO-, -NH-, or -O-, and A² is a single bond).

It is more preferable that in -A¹-R⁷-A²- represented by L², A¹, R⁷, and A² are all single bonds; A¹ is a single bond, R⁷ is -O-, and A² is a single bond or a C₁₋₄ alkylene group; or A¹ is a C₁₋₄ alkylene group, R⁷ is -O-, and A² is a single bond.

In yet another preferred embodiment, in -A¹-R⁷-A²- represented by L², A¹, R⁷, and A² are all single bonds; A¹ is a single bond, R⁷ is -O-, and A² is a single bond, a methylene group, or an ethylene group; or A¹ is a methylene group or an ethylene group, R⁷ is -O-, and A² is a single bond.

It is particularly preferable that L² is a single bond, -O-, -CH₂-O-, or -O-CH₂-; L² is a single bond, -O-, or -O-CH₂-; L² is a single bond or -O-CH₂-; or L² is -O-CH₂-.

Y represents a hydrogen atom, a cyclic hydrocarbon group which may have a substituent, or a heterocyclic group having 1 or 2 or more ring-constituting heteroatoms which may have a substituent.

For example, Y is a hydrogen atom, an aromatic hydrocarbon group which may have a substituent, a saturated or partially saturated cyclic hydrocarbon group which may have a substituent, an aromatic heterocyclic group having 1 or 2 or more ring-constituting heteroatoms which may have a substituent, or a saturated or partially saturated heterocyclic group having 1 or 2 or more ring-constituting heteroatoms which may have a substituent. In another embodiment, for example, Y is a hydrogen atom, a monocyclic or bicyclic aromatic hydrocarbon group which may have a substituent, a monocyclic or bicyclic, saturated or partially saturated cyclic hydrocarbon group which may have a substituent, a monocyclic or bicyclic aromatic heterocyclic group having 1 or 2 or more ring-constituting heteroatoms which may have a substituent, or a monocyclic or bicyclic, saturated or partially saturated heterocyclic group having 1 or 2 or more ring-constituting heteroatoms which may have a substituent.

In a preferred embodiment, Y is a hydrogen atom, a monocyclic or bicyclic aromatic hydrocarbon group which may have a substituent, a monocyclic or bicyclic, 3-to 12-membered, saturated or partially saturated cyclic hydrocarbon group which may have a substituent, a monocyclic or bicyclic aromatic heterocyclic group containing 1 or 2 or more ring-constituting nitrogen atoms which may have a substituent, or a monocyclic or bicyclic, saturated or partially saturated heterocyclic group containing 1 or 2 or more ring-constituting nitrogen atoms which may have a substituent.

In another preferred embodiment, Y is a hydrogen atom, a monocyclic or bicyclic aromatic hydrocarbon group which may have a substituent, a saturated, monocyclic, 3- to 7-membered cyclic hydrocarbon group which may have a substituent, a monocyclic or bicyclic aromatic heterocyclic group containing 1 or 2 or more ring-constituting nitrogen atoms which may have a substituent, or a saturated or partially saturated, monocyclic or bicyclic heterocyclic group containing 1 or 2 or more ring-constituting nitrogen atoms which may have a substituent.

More preferably, Y is a hydrogen atom, a phenyl group which may have a substituent, a naphthyl group which may have a substituent, or a monocyclic or bicyclic aromatic heterocyclic group containing 1 or 2 or more ring-constituting nitrogen atoms which may have a substituent.

Particularly preferably, Y is a hydrogen atom, a phenyl group which may have a substituent, a naphthyl group which may have a substituent, a pyridyl group which may have a substituent, or a quinolyl group which may have a substituent.

In another particularly preferred embodiment, Y is a hydrogen atom, a phenyl group, a naphthyl group, a pyridyl group, or a quinolyl group (provided that the aforementioned phenyl group, naphthyl group, pyridyl group, or quinolyl group may have 1 or 2 or more substituents selected from the following group: a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group).

In yet another particularly preferred embodiment, Y is a hydrogen atom, a phenyl group, a naphthyl group, a pyridyl group, or a quinolyl group, and the aforementioned phenyl group, naphthyl group, pyridyl group, or quinolyl group may have 1 or 2 or more substituents selected from the following group: a methyl group, an ethyl group, a methoxy group, an ethoxy group, a fluorine atom, a chlorine atom, a difluoromethyl group, a trifluoromethyl group, a difluoromethyloxy group, a trifluoromethyloxy group, and a cyano group.

In a most preferred embodiment, Y is a phenyl group, and the aforementioned phenyl group may have 1 or 2 or more substituents selected from the following group: a methyl group, an ethyl group, a methoxy group, an ethoxy group, a fluorine atom, a chlorine atom, a difluoromethyl group, a trifluoromethyl group, a difluoromethyloxy group, a trifluoromethyloxy group, and a cyano group.

Examples of the compounds of the present invention falling within the scope of the general formula (1) include, for example;
N-{2-fluoro-3-[6-oxo-4-(trifluoromethyl)-1,6-dihydropyrimidin-2-yl]-4-(trifluoromethyl)benzyl}-1-(8-methylquinolin-2-yl)piperidine-4-carboxamide;
N-{2-fluoro-3-[6-oxo-4-(trifluoromethyl)-1,6-dihydropyrimidin-2-yl]-4-(trifluoromethyl)benzyl}-1-[5-(trifluoromethyl)pyridin-2-yl]piperidine-4-carboxamide;
1-(4-chlorophenyl)-N-{2-fluoro-3-[6-oxo-4-(trifluoromethyl)-1,6-dihydropyrimidin-2-yl]-4-(trifluoromethyl)benzyl}piperidine-4-carboxamide;
trans-3-[(3-chiorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide;
trans-3-[(3-chlorobenzyl)oxy]-N-[2-fluoro-3-(4-methyl-6-oxo-1,6-dihydropyrimidin-2-yl)-4-(trifluoromethyl)benzyl]cyclobutane-1-carboxamide;
trans-4-[(2-chlorobenzyl)oxy]-N-{2-fluoro-3-[6-oxo-4-(trifluoromethyl)-1,6-dihydropyrimidin-2-yl]-4-(trifluoromethyl)benzyl}cyclohexane-1-carboxamide;
trans-3-[(3-chlorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide;
trans-4-[(2-chlorobenzyl)oxy]-N-[2-fluoro-3-(5-fluoro-4-methyl-6-oxo-1,6-dihydropyrimidin-2-yl)-4-(trifluoromethyl)benzyl]cyclohexane-1-carboxamide;
trans-4-[(2,4-difluorobenzyl)oxy]-N-[3-(4-ethyl-6-oxo-1,6-dihydropyrimidin-2-yl)-2-fluoro-4-(trifluoromethyl)benzyl]cyclohexane-1-carboxamide,
and the like, but the examples are not limited to these.

The compounds represented by the general formula (1) may be in the form of salt. The salt is not particularly limited, and appropriately selected depending on the purpose. Examples include, for example, salts with alkali metals such as sodium and potassium; salts with alkaline earth metals such as calcium and magnesium; salts with organic amines such as methylamine, ethylamine, and diethanolamine, mineral acid salts such as hydrochlorides, sulfates, and nitrates, organic acid salts such as p-toluenesulfonates, maleates, and tartrates, and the like.

The compounds represented by the general formula (1) and salts thereof may exist in the form of hydrate or solvate. Type of solvent that forms the solvate is not particularly limited, and examples include, for example, ethanol, ethyl acetate, acetone, and the like. The compounds represented by the general formula (1) may exist as an enantiomer, diastereoisomer, or geometrical isomer depending on type of substituent, and besides arbitrary isomers in a pure form, mixtures of arbitrary isomers also fall within the scope of the present invention.

The compounds represented by the general formula (1) and salts thereof can be easily synthesized by performing common chemical reactions widely used by those skilled in the art with starting compounds easily obtainable for those skilled in the art. Specific preparation methods of the compounds of the present invention are shown in the examples mentioned in this specification. By referring to those synthesis methods, those skilled in the art can easily prepare the compounds of the present invention falling within the scope of the general formula (1).

### Synthesis scheme-1

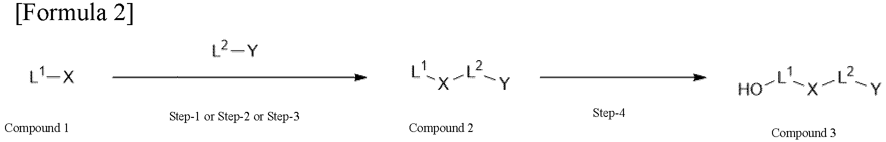

### Step-1

A compound 2 can be produced by reacting a compound 1 with L²-Y via an aromatic nucleophilic substitution reaction.

Examples of base used for this reaction include organic bases (amines, for example, mono- to trialkylamines such as methylamine, ethylamine, diethylamine, triethylamine, propylamine, isopropylamine, diisopropylethylamine, and 1,8-diazabicyclo[5.4.0]-7-undecene; alkanolamines such as ethanolamine; alkylenepolyamines such as ethylenediamine, and diethylenetriamine, and the like), inorganic bases (metal hydroxides such as sodium hydroxide, potassium hydroxide, calcium hydroxide, iron hydroxide, and aluminum hydroxide; alkali metal carbonates such as sodium carbonate, sodium hydrogencarbonate, potassium carbonate, rubidium carbonate, and cesium carbonate; sodium hydride, sodium tert-butoxide, potassium tert-butoxide, potassium fluoride, cesium fluoride, tripotassium phosphate), and the like. Particularly preferred examples include potassium carbonate, cesium carbonate, and tripotassium phosphate. A salt may be formed with one or two or more kinds of these bases.

Reaction time, reaction temperature, and the like may be selected from conventionally used ranges. Reaction temperature is preferably 20 to 200°C, particularly preferably 80 to 150°C. Reaction time is preferably 0.25 to 24 hours, particularly preferably 1 to 12 hours.

Solvent used for this reaction may be selected from conventionally used solvents. N,N-Dimethylformamide, N,N-dimethylsulfoxide, N,N-dimethylacetamide, sulfolane, and the like are preferred.

### Step-2

The compound 2 can be produced by performing a Buchwald-Hartwig cross-coupling reaction using L²-Y on the compound 1.

Examples of solvent, ligand used for this reaction include acetic acid palladium, bis(dibenzylideneacetone)palladium(0), 2,2'-bis(diphenylphosphino)-1,1'-binaphthyl, 1,1'-bis(diphenylphosphino)ferrocene, 2-dicyclohexylphosphino-2',6'-dimethoxybiphenyl, 2-dicyclohexylphosphino-2',4',6'-triisopropylbiphenyl, 2-dicyclohexylphosphino-2',6'-diisopropoxybiphenyl, (2-dicyclohexylphosphino-2',6'-diisopropoxy-1,1'-biphenyl)[2-(2'-amino-1,1'-biphenyl)]palladium(II) methanesulfonic acid, and the like.

Examples of base used for this reaction include organic bases (amines, for example, mono- to trialkylamines such as methylamine, ethylamine, diethylamine, triethylamine, propylamine, isopropylamine, diisopropylethylamine, and 1,8-diazabicyclo[5.4.0]-7-undecene; alkanolamines such as ethanolamine; alkylenepolyamines such as ethylenediamine, and diethylenetriamine, and the like), inorganic bases (metal hydroxides such as sodium hydroxide, potassium hydroxide, calcium hydroxide, iron hydroxide, and aluminum hydroxide; alkali metal carbonates such as sodium carbonate, sodium hydrogencarbonate, potassium carbonate, rubidium carbonate, and cesium carbonate; sodium hydride, sodium tert-butoxide, potassium tert-butoxide, potassium fluoride, cesium fluoride, tripotassium phosphate), and the like. Particularly preferred examples include potassium carbonate, cesium carbonate, sodium tert-butoxide, and potassium tert-butoxide. A salt may be formed with one or two or more kinds of these bases.

Reaction time, reaction temperature, and the like may be selected from conventionally used ranges. Reaction temperature is preferably 20 to 200°C, particularly preferably 80 to 150°C. Reaction time is preferably 0.25 to 24 hours, particularly preferably 1 to 12 hours.

Solvent used for this reaction may be selected from conventionally used solvents. 1,2-Dimethoxyethane, N,N-dimethylformamide, N,N-dimethylsulfoxide, N,N-dimethylacetamide, and the like are preferred.

### Step-3

The compound 2 can be produced by reacting the compound 1 with L²-Y in the presence of a base.

Examples of base used for this reaction include organic bases (amines, for example, mono- to trialkylamines such as methylamine, ethylamine, diethylamine, triethylamine, propylamine, isopropylamine, diisopropylethylamine, and 1,8-diazabicyclo[5.4.0]-7-undecene; alkanolamines such as ethanolamine; alkylenepolyamines such as ethylenediamine, and diethylenetriamine, and the like), inorganic bases (metal hydroxides such as sodium hydroxide, potassium hydroxide, calcium hydroxide, iron hydroxide, and aluminum hydroxide; alkali metal carbonates such as sodium carbonate, sodium hydrogencarbonate, potassium carbonate, rubidium carbonate, and cesium carbonate; sodium hydride, sodium tert-butoxide, potassium tert-butoxide, potassium fluoride, cesium fluoride, tripotassium phosphate), and the like. Particularly preferred examples include sodium hydride and diisopropylethylamine. A salt may be formed with one or two or more kinds of these bases.

Reaction time, reaction temperature, and the like may be selected from conventionally used ranges. Reaction temperature is preferably 0 to 200°C. Reaction time is preferably 0.25 to 24 hours, particularly preferably 8 to 24 hours.

Solvent used for this reaction may be selected from conventionally used solvents. Tetrahydrofuran, N,N-dimethylformamide, and the like are preferred. It is also preferable to perform the reaction without solvent.

### Step-4

The compound 2 obtained in steps 1 to 3 above can be hydrolyzed under basic conditions to produce a compound 3.

Examples of base used for this reaction include inorganic bases (metal hydroxides such as sodium hydroxide, potassium hydroxide, calcium hydroxide, lithium hydroxide, iron hydroxide, and aluminum hydroxide; alkali metal carbonates such as sodium carbonate, sodium hydrogencarbonate, potassium carbonate, rubidium carbonate, and cesium carbonate; sodium hydride, sodium tert-butoxide, potassium tert-butoxide, potassium fluoride, cesium fluoride, tripotassium phosphate), and the like. Particularly preferred examples include sodium hydroxide, potassium hydroxide, calcium hydroxide, and lithium hydroxide. A salt may be formed with one or two or more kinds of these bases.

Reaction time, reaction temperature, and the like may be selected from conventionally used ranges. Reaction temperature is preferably 0 to 140°C, particularly preferably 20 to 80°C. Reaction time is preferably 0.25 to 48 hours, particularly preferably 1 to 24 hours.

Solvent used for this reaction may be selected from conventionally used solvents. Water, methanol, ethanol, 1-propanol, 2-propanol, tetrahydrofuran, and the like are preferred.

### Synthesis scheme-2

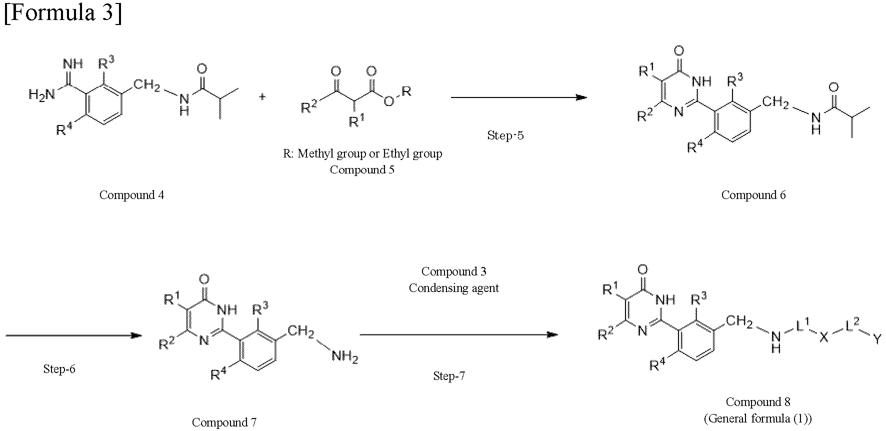

### Step-5

A compound 6 can be produced by reacting the compound 4 with the compound 5 in the presence of a base in a manner similar to the production method disclosed in International Patent Publication WO2017/073709.

Examples of base used for this reaction include organic bases (amines, for example, mono- to trialkylamines such as methylamine, ethylamine, diethylamine, triethylamine, propylamine, isopropylamine, diisopropylethylamine, and 1,8-diazabicyclo[5.4.0]-7-undecene; alkanolamines such as ethanolamine; alkylenepolyamines such as ethylenediamine, and diethylenetriamine, and the like), inorganic bases (metal hydroxides such as sodium hydroxide, potassium hydroxide, calcium hydroxide, iron hydroxide, and aluminum hydroxide; alkali metal carbonates such as sodium carbonate, sodium hydrogencarbonate, potassium carbonate, rubidium carbonate, and cesium carbonate; sodium hydride, sodium tert-butoxide, potassium tert-butoxide, potassium fluoride, cesium fluoride, tripotassium phosphate), and the like. Particularly preferred examples include potassium carbonate, sodium hydride, and potassium tert-butoxide. A salt may be formed with one or two or more kinds of these bases.

Reaction time, reaction temperature, and the like may be selected from conventionally used ranges. Reaction temperature is preferably 0 to 140°C, particularly preferably 20 to 80°C. Reaction time is preferably 0.25 to 48 hours, particularly preferably 1 to 24 hours.

Solvent used for this reaction may be selected from conventionally used solvents. Water, tetrahydrofuran, N,N-dimethylformamide, methanol, ethanol, 1-propanol, 2-propanol, and the like are preferred.

### Step-6

A compound 7 can be produced by hydrolyzing the compound 6 under acidic conditions.

Examples of reactant include, for example, inorganic acids (such as hydrochloric acid, sulfuric acid, and nitric acid), organic acids (methanesulfonic acid, trifluoromethanesulfonic acid, benzenesulfonic acid, toluenesulfonic acid, trifluoroacetic acid, and mono-, di- or trichloroacetic acid), and the like. Among these strong acids, hydrochloric acid is particularly preferable.

Solvent used for this reaction may be selected from conventionally used solvents. It is preferable to perform the reaction without solvent.

Reaction time, reaction temperature, and the like of the deprotection reaction may be selected from conventionally used ranges. Reaction temperature is preferably 0 to 150°C, particularly preferably 50 to 130°C. Reaction time is preferably 1 to 48 hours, particularly preferably 12 to 24 hours.

### Step-7

The compound 3 can be condensed with the compound 7 in the presence of a condensing agent and a base to produce a compound 8 (a compound represented by the general formula (1)).

The condensing agent may be selected from conventionally used condensing agents. Examples of the condensing agent include N,N'-dicyclohexylcarbodiimide, 1-ethyl-3-(3-dimethylaminopropyl)carbodiimide, N,N'-carbonyldiimidazole, 1-[bis(dimethylamino)methylene]-1H-1,2,3-triazolo[4,5-b]pyridinium 3-oxide hexafluorophosphate, propylphosphonic anhydride (cyclic trimer), and the like.

Examples of base used for this reaction include organic bases (amines, for example, mono- to trialkylamines such as methylamine, ethylamine, diethylamine, triethylamine, propylamine, isopropylamine, diisopropylethylamine, and 1,8-diazabicyclo[5.4.0]-7-undecene; alkanolamines such as ethanolamine; alkylenepolyamines such as ethylenediamine, and diethylenetriamine, and the like), inorganic bases (metal hydroxides such as sodium hydroxide, potassium hydroxide, calcium hydroxide, iron hydroxide, and aluminum hydroxide; alkali metal carbonates such as sodium carbonate, sodium hydrogencarbonate, potassium carbonate, rubidium carbonate, and cesium carbonate; sodium hydride, sodium tert-butoxide, potassium tert-butoxide, potassium fluoride, cesium fluoride, tripotassium phosphate), and the like. Preferred examples include alkali metal bases and organic bases. Particularly preferred examples include triethylamine, diisopropylethylamine, and the like. A salt may be formed with one or two or more kinds of these bases.

Solvent used may be selected from conventionally used solvents. Dichloromethane, N,N-dimethylformamide, tetrahydrofuran, ethyl acetate, and the like are preferred. Reaction temperature is preferably -10 to 60°C. Reaction time is preferably 1 to 48 hours.

The compounds of the present invention represented by the general formula (1) have an mPGES-1 inhibitory action, and can inhibit the PGE2 biosynthesis on the basis of the inhibitory action. Therefore, on the basis of the mPGES-1 inhibitory action, the medicament of the present invention containing a compound represented by the general formula (1) or a physiologically acceptable salt thereof of the present invention as an active ingredient can be used for prophylactic and/or therapeutic treatment of, for example, inflammation, pain, rheumatism, osteoarthritis, pyrexia, Alzheimer's disease, multiple sclerosis, arteriosclerosis, ocular hypertension such as glaucoma, ischemic retinopathy, systemic scleroderma, malignant tumors such as large intestine tumor, and diseases for which suppression of the PGE2 production exhibits efficacy.

More specifically, the medicament of the present invention can be used as a medicament for prophylactic and/or therapeutic treatment of, for example, inflammatory colitis, irritable bowel syndrome, migraine, headache, low back pain, lumbar spinal canal stenosis, intervertebral disc herniation, temporomandibular arthrosis, neck-shoulder-arm syndrome, cervical spondylosis, endometriosis, adenomyosis uteri, premature delivery, threatened premature delivery, dysmenorrhea, overactive bladder, bladder outlet obstruction associated with benign prostatic hyperplasia, nocturia, urinary incontinence, neurogenic bladder, interstitial cystitis, bladder pain syndrome, urinary calculus, benign prostatic hyperplasia, chronic prostatitis, intrapelvic pain syndrome, erectile dysfunction, cognitive disorder, neurodegenerative disease, Alzheimer's disease, pulmonary hypertension, psoriasis, rheumatoid arthritis, rheumatic fever, fibromyalgia, neuralgia, complex regional pain syndrome, fascia dyscrasia, ischemic heart disease, hypertension, angina pectoris, viral infectious disorders, bacterial infection, fungal infectious disorders, burn, inflammation and pain after operation, trauma, or extraction of a tooth, malignant tumor, myocardial infarction, atherosclerosis, thrombosis, embolism, type I diabetes mellitus, type II diabetes mellitus, cerebral apoplexy, gout, arthritis, osteoarthritis, juvenile arthritis, ankylosing spondilitis, tenosynovitis, ligamentum osteosis, systemic erythematodes, vasculitis, pancreatitis, nephritis, conjunctivitis, iritis, scleritis, uveitis, wound treatment, dermatitis, eczema, osteoporosis, asthma, chronic obstructive pulmonary disease, fibroid lung, allergic conditions, familial adenomatous polyposis, pachydermia, bursitis, hysteromyoma, or pain in cancer. As for the relation of mPGES-1 inhibitory action and use as medicament, for example, International Patent Publication WO2015/125842 can be referred to. The entire disclosures of this international patent publication and all the references cited therein are incorporated into the disclosure of this specification by reference.

Although a compound represented by the aforementioned general formula (1) or a physiologically acceptable salt thereof as the active ingredient of the medicament of the present invention may be administered as the medicament of the present invention, a pharmaceutical composition for oral or parenteral administration can be preferably prepared by a method well known to those skilled in the art, and administered. Examples of pharmaceutical composition suitable for oral administration include, for example, tablets, powders, capsules, subtilized granules, solutions, granules, syrups, and the like, and pharmaceutical composition suitable for parenteral administration include, for example, injections such as injections for intravenous injection and intramuscular injection, fusion drips, inhalants, eye drops, nose drops, suppositories, transdermal preparations, transmucosal preparations, and the like, but the pharmaceutical composition is not limited to these.

The aforementioned pharmaceutical composition can be produced by a method well known to those skilled in the art using pharmaceutical additives commonly used for preparation of pharmaceutical compositions in this industry. Such pharmaceutical additives are not particularly limited, and can be appropriately chosen depending on form of the pharmaceutical composition, purpose thereof such as impartation of properties for sustained release, and the like. Examples of the pharmaceutical additives include, for example, excipients, binders, fillers, disintegrating agents, surfactants, lubricants, dispersing agents, buffering agents, preservatives, corrigents, perfumes, coating agents, diluents, and the like, but the pharmaceutical additives are not limited to these.

Dose of the medicament of the present invention is not particularly limited, and can be appropriately chosen depending on type of disease to be prevented or treated, purpose of administration such as prevention or treatment, type of active ingredient, weight, age, conditions of patient, administration route, and the like. In the case of oral administration, for example, it can be used at a dose in the range of about 0.01 to 500 mg in terms of weight of the active ingredient as the daily dose for adults. However, the dose can be appropriately chosen by those skilled in the art, and is not limited to the aforementioned range.

### Examples

Hereafter, the present invention will be explained in more detail with reference to examples. However, the present invention is not limited by these examples.

### Reference Examples

### Reference Example 1: N-{2-fluoro-3-[6-oxo-4-(trifluoromethyl)-1,6-dihydropyrimidin-2-yl]-4-(trifluoromethyl)benzyl}isobutyramide

N-[3-carbamimidoyl-2-fluoro-4-(trifluoromethyl)benzyl]isobutyramide formate (5.00 g), ethyl 4,4,4-trifluoroacetoacetate (5.24 g), potassium carbonate (3.93 g), and ethanol (50 mL) were stirred at 55°C for 2 days. 1N hydrochloric acid and water were added, the resulting mixture was extracted twice with ethyl acetate, and the solvent was evaporated under reduced pressure. The residue was purified by silica gel column chromatography to obtain the title compound (5.58 g).

### Reference Example 2: 2-[3-(aminomethyl)-2-fluoro-6-(trifluoromethyl)phenyl]-6-(trifluoromethyl)pyrimidin-4(3H)-one

A solution of N-{2-fluoro-3-[6-oxo-4-(trifluoromethyl)-1,6-dihydropyrimidin-2-yl]-4-(trifluoromethyl)benzyl}isobutyramide (860 mg) in concentrated hydrochloric acid (8.6 mL) and acetic acid (0.86 mL) was stirred at 110°C for 5.5 hours. The reaction mixture was left to cool, a 6N sodium hydroxide solution was added thereto, and the resulting mixture was extracted twice with ethyl acetate. The resulting organic layer was dried over sodium sulfate, and then the solvent was evaporated under reduced pressure to obtain the title compound (309 mg).

### Reference Example 3: N-[2-fluoro-3-(4-methyl-6-oxo-1,6-dihydropyrimidin-2-yl)-4-(trifluoromethyl)benzyl]isobutyramide

By performing operations similar to those of Reference Example 1 using N-[3-carbamimidoyl-2-fluoro-4-(trifluoromethyl)benzyl]isobutyramide·formate and ethyl 3-oxobutanoate, the title compound was obtained.

### Reference Example 4: 2-[3-(aminomethyl)-2-fluoro-6-(trifluoromethyl)phenyl]-6-methylpyrimidin-4(3H)-one

By performing operations similar to those of Reference Example 2 using N-[2-fluoro-3-(4-methyl-6-oxo-1,6-dihydropyrimidin-2-yl)-4-(trifluoromethyl)benzyl]isobutyramide, the title compound was obtained.

### Reference Example 5: N-[3-(4-ethyl-6-oxo-1,6-dihydropyrimidin-2-yl)-2-fluoro-4-(trifluoromethyl)benzyl]isobutyramide

By performing operations similar to those of Reference Example 1 using N-[3-carbamimidoyl-2-fluoro-4-(trifluoromethyl)benzyl]isobutyramide·formate and ethyl 3-oxovalerate, the title compound was obtained.

### Reference Example 6: 2-[3-(aminomethyl)-2-fluoro-6-(trifluoromethyl)phenyl]-6-ethylpyrimidin-4(3H)-one

By performing operations similar to those of Reference Example 2 using N-[3-(4-ethyl-6-oxo-1,6-dihydropyrimidin-2-yl)-2-fluoro-4-(trifluoromethyl)benzyl]isobutyramide, the title compound was obtained.

### Reference Example 7: N-[3-(4,5-dimethyl-6-oxo-1,6-dihydropyrimidin-2-yl)-2-fluoro-4-(trifluoromethyl)benzyl]isobutyramide

By performing operations similar to those of Reference Example 1 using N-[3-carbamimidoyl-2-fluoro-4-(trifluoromethyl)benzyl]isobutyramide·formate and ethyl 2-methylacetoacetate, the title compound was obtained.

### Reference Example 8: 2-[3-(aminomethyl)-2-fluoro-6-(trifluoromethyl)phenyl]-5,6-dimethylpyrimidin-4(3H)-one

By performing operations similar to those of Reference Example 2 using N-[3-(4,5-dimethyl-6-oxo-1,6-dihydropyrimidin-2-yl)-2-fluoro-4-(trifluoromethyl)benzyl]isobutyramide, the title compound was obtained.

### Reference Example 9: N-[2-fluoro-3-(4-oxo-4,5,6,7-tetrahydro-3H-cyclopenta[d]pyrimidin-2-yl)-4-(trifluoromethyl)benzyl]isobutyramide

By performing operations similar to those of Reference Example 1 using N-[3-carbamimidoyl-2-fluoro-4-(trifluoromethyl)benzyl]isobutyramide·formate and ethyl 2-oxocyclopentanecarboxylate, the title compound was obtained.

### Reference Example 10: 2-[3-(aminomethyl)-2-fluoro-6-(trifluoromethyl)phenyl]-3,5,6,7-tetrahydro-4H-cyclopenta[d]pyrimidin-4-one

By performing operations similar to those of Reference Example 2 using N-[2-fluoro-3-(4-oxo-4,5,6,7-tetrahydro-3H-cyclopenta[d]pyrimidin-2-yl)-4-(trifluoromethyl)benzyl]isobutyramide, the title compound was obtained.

### Reference Example 11: N-[2-fluoro-3-(5-fluoro-4-methyl-6-oxo-1,6-dihydropyrimidin-2-yl)-4-(trifluoromethyl)benzyl]isobutyramide

By performing operations similar to those of Reference Example 1 using N-[3-carbamimidoyl-2-fluoro-4-(trifluoromethyl)benzyl]isobutyramide·formate and ethyl 2-fluoroacetoacetate, the title compound was obtained.

### Reference Example 12: 2-[3-(aminomethyl)-2-fluoro-6-(trifluoromethyl)phenyl]-5-fluoro-6-methylpyrimidin-4(3H)-one

By performing operations similar to those of Reference Example 2 using N-[2-fluoro-3-(5-fluoro-4-methyl-6-oxo-1,6-dihydropyrimidin-2-yl)-4-(trifluoromethyl)benzyl]isobutyramide, the title compound was obtained.

### Reference Example 13: N-[3-(4-ethyl-5-fluoro-6-oxo-1,6-dihydropyrimidin-2-yl)-2-fluoro-4-(trifluoromethyl)benzyl]isobutyramide

By performing operations similar to those of Reference Example 1 using N-[3-carbamimidoyl-2-fluoro-4-(trifluoromethyl)benzyl]isobutyramide·formate and ethyl 2-fluoro-3-oxopentanoate, the title compound was obtained.

### Reference Example 14: 2-[3-(aminomethyl)-2-fluoro-6-(trifluoromethyl)phenyl]-6-ethyl-5-fluoropyrimidin-4(3H)-one

By performing operations similar to those of Reference Example 2 using N-[3-(4-ethyl-5-fluoro-6-oxo-1,6-dihydropyrimidin-2-yl)-2-fluoro-4-(trifluoromethyl)benzyl]isobutyramide, the title compound was obtained.

### Reference Example 15: N-{3-[4-(difluoromethyl)-6-oxo-1,6-dihydropyrimidin-2-yl]-2-fluoro-4-(trifluoromethyl)benzyl}isobutyramide

By performing operations similar to those of Reference Example 1 using N-[3-carbamimidoyl-2-fluoro-4-(trifluoromethyl)benzyl]isobutyramide·formate and ethyl 4,4-difluoroacetoacetate, the title compound was obtained.

### Reference Example 16: 2-[3-(aminomethyl)-2-fluoro-6-(trifluoromethyl)phenyl]-6-(difluoromethyl)pyrimidin-4(3H)-one

By performing operations similar to those of Reference Example 2 using N-{3-[4-(difluoromethyl)-6-oxo-1,6-dihydropyrimidin-2-yl]-2-fluoro-4-(trifluoromethyl)benzyl}isobutyramide, the title compound was obtained.

### Reference Example 17: N-[2-fluoro-3-(6-oxo-1,6-dihydropyrimidin-2-yl)-4-(trifluoromethyl)benzyl]isobutyramide

By performing operations similar to those of Reference Example 1 using N-[3-carbamimidoyl-2-fluoro-4-(trifluoromethyl)benzyl]isobutyramide·formate and ethyl propiolate, the title compound was obtained.

### Reference Example 18: 2-[3-(aminomethyl)-2-fluoro-6-(trifluoromethyl)phenyl]pyrimidin-4(3H)-one

By performing operations similar to those of Reference Example 2 using N-[2-fluoro-3-(6-oxo-1,6-dihydropyrimidin-2-yl)-4-(trifluoromethyl)benzyl]isobutyramide, the title compound was obtained.

### Reference Example 19: N-{4-chloro-2-fluoro-3-[6-oxo-4-(trifluoromethyl)-1,6-dihydropyrimidin-2-yl]benzyl}isobutyramide

By performing operations similar to those of Reference Example 1 using N-(3-carbamimidoyl-4-chloro-2-fluorobenzyl)isobutyramide·hydrochloride and ethyl 4,4,4-trifluoroacetoacetate, the title compound was obtained.

### Reference Example 20: 2-[3-(aminomethyl)-6-chloro-2-fluorophenyl]-6-(trifluoromethyl)pyrimidin-4(3H)-one

By performing operations similar to those of Reference Example 2 using N-{4-chloro-2-fluoro-3-[6-oxo-4-(trifluoromethyl)-1,6-dihydropyrimidin-2-yl]benzyl}isobutyramide, the title compound was obtained.

### Reference Example 21: N-{3-[6-oxo-4-(trifluoromethyl)-1,6-dihydropyrimidin-2-yl]-4-(trifluoromethyl)benzyl }isobutyramide

By performing operations similar to those of Reference Example 1 using N-[3-carbamimidoyl-4-(trifluoromethyl)benzyl]isobutyramide·formate and ethyl 4,4,4-trifluoroacetoacetate, the title compound was obtained.

### Reference Example 22: 2-[5-(aminomethyl)-2-(trifluoromethyl)phenyl]-6-(trifluoromethyl)pyrimidin-4(3H)-one

By performing operations similar to those of Reference Example 2 using N-{3-[6-oxo-4-(trifluoromethyl)-1,6-dihydropyrimidin-2-yl]-4-(trifluoromethyl)benzyl}isobutyramide, the title compound was obtained.

### Reference Example 23: methyl 1-(8-methylquinolin-2-yl)azetidine-3-carboxylate

A solution of 2-chloro-8-methylquinoline (533 mg), methyl azetidine-3-carboxylate· hydrochloride (910 mg), and potassium carbonate (1.66 g) in dimethyl sulfoxide (6 mL) was stirred at 110°C for 2 days. Water was added to the reaction mixture, the mixture was extracted three times with ethyl acetate, and the combined organic layers were dried over sodium sulfate. The solvent was evaporated under reduced pressure. The residue was purified by silica gel column chromatography to obtain the title compound (398 mg).

### Reference Example 24: 1-(8-methylquinolin-2-yl)azetidine-3-carboxylic acid

To a solution of methyl 1-(8-methylquinolin-2-yl)azetidine-3-carboxylate (394 mg) in methanol (4 mL), 1N aqueous sodium hydroxide (4.6 mL) was added. The reaction mixture was stirred at 50°C for 3 hours. 1N Hydrochloric acid was added to the reaction mixture, the mixture was extracted twice with ethyl acetate, and the combined organic layers were dried over sodium sulfate. The solvent was evaporated under reduced pressure to obtain the title compound (204 mg).

### Reference Example 25: ethyl 1-(imidazo[1,2-b]pyridazin-6-yl)piperidine-4-carboxylate

By performing operations similar to those of Reference Example 23 using 6-chloroimidazo[1,2-b]pyridazine and ethyl 4-piperidinecarboxylate, the title compound was obtained. The obtained compound was used for the following reaction without purification.

### Reference Example 26: 1-(imidazo[1,2-b]pyridazin-6-yl)piperidine-4-carboxylic acid

By performing operations similar to those of Reference Example 24 using ethyl 1-(imidazo[1,2-b]pyridazin-6-yl)piperidine-4-carboxylate, the title compound was obtained.

### Reference Example 27: ethyl 1-(3-methylimidazo[1,2-b]pyridazin-6-yl)piperidine-4-carboxylate

By performing operations similar to those of Reference Example 23 using 6-chloro-3-methylimidazo[1,2-b]pyridazine and ethyl 4-piperidinecarboxylate, the title compound was obtained. The obtained compound was used for the following reaction without purification.

### Reference Example 28: 1-(3-methylimidazo[1,2-b]pyridazin-6-yl)piperidine-4-carboxylic acid

By performing operations similar to those of Reference Example 24 using ethyl 1-(3-methylimidazo[1,2-b]pyridazin-6-yl)piperidine-4-carboxylate, the title compound was obtained.

### Reference Example 29: ethyl 4-fluoro-1-(8-methylquinolin-2-yl)piperidine-4-carboxylate

By performing operations similar to those of Reference Example 23 using 2-chloro-8-methylquinoline and ethyl 4-fluoropiperidine-4-carboxylate·hydrochloride, the title compound was obtained.

### Reference Example 30: 4-fluoro-1-(8-methylquinolin-2-yl)piperidine-4-carboxylic acid

By performing operations similar to those of Reference Example 24 using ethyl 4-fluoro-1-(8-methylquinolin-2-yl)piperidine-4-carboxylate, the title compound was obtained.

### Reference Example 31: 4-fluoro-1-[5-(trifluoromethyl)pyridin-2-yl]piperidine-4-carboxylic acid

By performing operations similar to those of Reference Examples 23 and 24 using 2-chloro-5-(trifluoromethyl)pyridine and ethyl 4-fluoropiperidine-4-carboxylate·hydrochloride, the title compound was obtained.

### Reference Example 32: ethyl 1-[3-fluoro-5-(trifluoromethyl)pyridin-2-yl]piperidine-4-carboxylate

By performing operations similar to those of Reference Example 23 using 2,3-difluoro-5-(trifluoromethyl)pyridine and ethyl 4-piperidinecarboxylate, the title compound was obtained.

### Reference Example 33: 1-[3-fluoro-5-(trifluoromethyl)pyridin-2-yl]piperidine-4-carboxylic acid

By performing operations similar to those of Reference Example 24 using ethyl 1-[3-fluoro-5-(trifluoromethyl)pyridin-2-yl]piperidine-4-carboxylate, the title compound was obtained.

### Reference Example 34: 1-[5-(trifluoromethoxy)pyridin-2-yl]piperidine-4-carboxylic acid

By performing operations similar to those of Reference Example 24 using ethyl 1-[5-(trifluoromethoxy)pyridin-2-yl]piperidine-4-carboxylate, the title compound was obtained.

### Reference Example 35: ethyl 1-(5-chloro-6-methylpyridin-2-yl)piperidine-4-carboxylate

By performing operations similar to those of Reference Example 23 using 3,6-dichloropicoline and ethyl 4-piperidinecarboxylate, the title compound was obtained. The obtained compound was used for the following reaction without purification.

### Reference Example 36: 1-(5-chloro-6-methylpyridin-2-yl)piperidine-4-carboxylic acid

By performing operations similar to those of Reference Example 24 using ethyl 1-(5-chloro-6-methylpyridin-2-yl)piperidine-4-carboxylate, the title compound was obtained.

### Reference Example 37: ethyl 1-[5-(trifluoromethyl)pyrazin-2-yl]piperidine-4-carboxylate

By performing operations similar to those of Reference Example 23 using 2-chloro-5-(trifluoromethyl)pyrazine and ethyl 4-piperidinecarboxylate, the title compound was obtained.

### Reference Example 38: 1-[5-(trifluoromethyl)pyrazin-2-yl]piperidine-4-carboxylic acid

By performing operations similar to those of Reference Example 24 using ethyl 1-[5-(trifluoromethyl)pyrazin-2-yl]piperidine-4-carboxylate, the title compound was obtained.

### Reference Example 39: ethyl 1-(4-chloro-3-fluorophenyl)piperidine-4-carboxylate

To a solution of 4-bromo-1-chloro-2-fluorobenzene (234 µL) in 1,2-dimethoxyethane (13 mL), ethyl 4-piperidinecarboxylate (447 µL), cesium carbonate (1.89 g), and (2-dicyclohexylphosphino-2',6'-diisopropoxy-1,1'-biphenyl)[2-(2'-amino-1,1'-biphenyl)]palladium(II) methanesulfonate (99 mg) were added. Microwaves were irradiated on the mixture at 130°C for 60 minutes. The reaction mixture was filtered through a Celite layer, and the solvent was evaporated under reduced pressure. The resulting residue was purified by silica gel column chromatography, and the solvent was evaporated under reduced pressure to obtain the title compound (195 mg).

### Reference Example 40: 1-(4-chloro-3-fluorophenyl)piperidine-4-carboxylic acid

To a solution of ethyl 1-(4-chloro-3-fluorophenyl)piperidine-4-carboxylate (195 mg) in methanol (2 mL), 1N sodium hydroxide (2.0 mL) was added. The reaction mixture was stirred at 50°C for 2 hours. The reaction mixture was neutralized with the addition of 1N hydrochloric acid (2.0 mL). The precipitated solid was washed with hexane and collected by filtration to obtain the title compound (150 mg).

### Reference Example 41: ethyl 1-[3-(difluoromethyl)phenyl]piperidine-4-carboxylate

By performing operations similar to those of Reference Example 39 using 1-bromo-3-(difluoromethyl)benzene and ethyl 4-piperidinecarboxylate, the title compound was obtained.

### Reference Example 42: 1-[3-(difluoromethyl)phenyl]piperidine-4-carboxylic acid

By performing operations similar to those of Reference Example 40 using ethyl 1-[3-(difluoromethyl)phenyl]piperidine-4-carboxylate, the title compound was obtained.

### Reference Example 43: 1-[4-(difluoromethoxy)phenyl]piperidine-4-carboxylic acid

By performing operations similar to those of Reference Example 40 using ethyl 1-[4-(difluoromethoxy)phenyl]piperidine-4-carboxylate, the title compound was obtained.

### Reference Example 44: ethyl trans-4-[(2-chlorobenzyl)oxy]cyclohexane-1-carboxylate

To ethyl trans-4-hydroxycyclohexane-1-carboxylate (244 µL), 1-(bromomethyl)-2-chlorobenzene (215 µL) and N,N-diisopropylethylamine (638 µL) were added. The reaction mixture was stirred at 150°C for 2 hours. After adding 1N hydrochloric acid (8 mL), the mixture was extracted with ethyl acetate, washed once with water and saturated brine in that order, and dried over magnesium sulfate. The solvent was evaporated under reduced pressure to obtain the title compound (533 mg). The obtained compound was used for the following reaction without purification.

### Reference Example 45: trans-4-[(2-chlorobenzyl)oxy]cyclohexane-1-carboxylic acid

To a solution of ethyl trans-4-[(2-chlorobenzyl)oxy]cyclohexane-1-carboxylate (533 mg) in methanol (4 mL), 1N sodium hydroxide (5.39 mL) was added. The reaction mixture was stirred at room temperature overnight. The reaction mixture was neutralized with the addition of 1N hydrochloric acid (5.39 mL). The precipitated solid was washed with hexane and collected by filtration to obtain the title compound (177 mg).

### Reference Example 46: ethyl trans-4-[(3-chlorobenzyl)oxy]cyclohexane-1-carboxylate

By performing operations similar to those of Reference Example 44 using ethyl trans-4-hydroxycyclohexane-1-carboxylate and 1-(bromomethyl)-3-chlorobenzene, the title compound was obtained. The obtained compound was used for the following reaction without purification.

### Reference Example 47: trans-4-[(3-chlorobenzyl)oxy]cyclohexane-1-carboxylic acid

By performing operations similar to those of Reference Example 45 using ethyl trans-4-[(3-chlorobenzyl)oxy]cyclohexane-1-carboxylate, the title compound was obtained. The obtained compound was used for the following reaction without purification.

### Reference Example 48: ethyl trans-4-{[3-(trifluoromethyl)benzyl]oxy}cyclohexane-1-carboxylate

By performing operations similar to those of Reference Example 44 using ethyl trans-4-hydroxycyclohexane-1-carboxylate and 1-(bromomethyl)-3-(trifluoromethyl)benzene, the title compound was obtained.

### Reference Example 49: trans-4-{[3-(trifluoromethyl)benzyl]oxy}cyclohexane-1-carboxylic acid

By performing operations similar to those of Reference Example 45 using ethyl trans-4-[(3-{trifluoromethyl}benzyl)oxy]cyclohexane-1-carboxylate, the title compound was obtained.

### Reference Example 50: ethyl trans-4-{[4-(trifluoromethyl)benzyl]oxy}cyclohexane-1-carboxylate

By performing operations similar to those of Reference Example 44 using ethyl trans-4-hydroxycyclohexane-1-carboxylate and 1-(bromomethyl)-4-(trifluoromethyl)benzene, the title compound was obtained.

### Reference Example 51: trans-4-{[4-(trifluoromethyl)benzyl]oxy}cyclohexane-1-carboxylic acid

By performing operations similar to those of Reference Example 45 using ethyl trans-4-{[4-(trifluoromethyl)benzyl]oxy}cyclohexane-1-carboxylate, the title compound was obtained.

### Reference Example 52: ethyl trans-4-[(2,4-difluorobenzyl)oxy]cyclohexane-1-carboxylate

By performing operations similar to those of Reference Example 44 using ethyl trans-4-hydroxycyclohexane-1-carboxylate and 1-(bromomethyl)-2,4-difluorobenzene, the title compound was obtained.

### Reference Example 53: trans-4-[(2,4-difluorobenzyl)oxy]cyclohexane-1-carboxylic acid

By performing operations similar to those of Reference Example 45 using ethyl trans-4-[(2,4-difluorobenzyl)oxy]cyclohexane-1-carboxylate, the title compound was obtained.

### Reference Example 54: ethyl trans-4-[(3,4-difluorobenzyl)oxy]cyclohexane-1-carboxylate

By performing operations similar to those of Reference Example 44 using ethyl trans-4-hydroxycyclohexane-1-carboxylate and 1-(bromomethyl)-3,4-difluorobenzene, the title compound was obtained.

### Reference Example 55: trans-4-[(3,4-difluorobenzyl)oxy]cyclohexane-1-carboxylic acid

By performing operations similar to those of Reference Example 45 using ethyl trans-4-[(3,4-difluorobenzyl)oxy]cyclohexane-1-carboxylate, the title compound was obtained.

### Reference Example 56: ethyl trans-4-[(3,5-difluorobenzyl)oxy]cyclohexane-1-carboxylate

By performing operations similar to those of Reference Example 44 using ethyl trans-4-hydroxycyclohexane-1-carboxylate and 1-(bromomethyl)-3,5-difluorobenzene, the title compound was obtained.

### Reference Example 57: trans-4-[(3,5-difluorobenzyl)oxy]cyclohexane-1-carboxylic acid

By performing operations similar to those of Reference Example 45 using ethyl trans-4-[(3,5-difluorobenzyl)oxy]cyclohexane-1-carboxylate, the title compound was obtained.

### Reference Example 58: ethyl trans-4-[(4-chloro-2-fluorobenzyl)oxy]cyclohexane-1-carboxylate

By performing operations similar to those of Reference Example 44 using ethyl trans-4-hydroxycyclohexane-1-carboxylate and 1-(bromomethyl)-4-chloro-2-fluorobenzene, the title compound was obtained.

### Reference Example 59: trans-4-[(4-chloro-2-fluorobenzyl)oxy]cyclohexane-1-carboxylic acid

By performing operations similar to those of Reference Example 45 using ethyl trans-4-[(4-chloro-2-fluorobenzyl)oxy]cyclohexane-1-carboxylate, the title compound was obtained.

### Reference Example 60: ethyl trans-4-[(3-chloro-5-fluorobenzyl)oxy]cyclohexane-1-carboxylate

By performing operations similar to those of Reference Example 44 using ethyl trans-4-hydroxycyclohexane-1-carboxylate and 1-(bromomethyl)-3-chloro-5-fluorobenzene, the title compound was obtained.

### Reference Example 61: trans-4-[(3-chloro-5-fluorobenzyl)oxy]cyclohexane-1-carboxylic acid

By performing operations similar to those of Reference Example 45 using ethyl trans-4-[(3-chloro-5-fluorobenzyl)oxy]cyclohexane-1-carboxylate, the title compound was obtained.

### Reference Example 62: methyl 3-[(2-chlorobenzyl)oxy]cyclobutane-1-carboxylate

To a solution of methyl trans-3-hydroxycyclobutanecarboxylate (700 mg) in N,N-dimethylformamide (5 mL), sodium hydride (236 mg) was added under ice cooling. The reaction mixture was stirred at the same temperature for 1 hour. 2-Chlorobenzyl chloride (1.30 g) was added to the mixture, and the resulting mixture was stirred at room temperature for 11 hours. 2N Hydrochloric acid and water were added, and the resulting mixture was extracted three times with ethyl acetate. The combined organic layers were washed with brine and dried over sodium sulfate. The solvent was evaporated under reduced pressure. The resulting residue was purified by silica gel column chromatography to obtain the title compound (588 mg). The obtained compound was used as it was for the following reaction.

### Reference Example 63: trans-3-[(2-chlorobenzyl)oxy]cyclobutane-1-carboxylic acid

### Reference Example 64: cis-3-[(2-chlorobenzyl)oxy]cyclobutane-1-carboxylic acid

To a solution of methyl 3-[(2-chlorobenzyl)oxy]cyclobutane-1-carboxylate (588 mg) in tetrahydrofuran (6 mL) and water (2 mL), lithium hydroxide (166 mg) was added. The reaction mixture was stirred at room temperature for 12 hours. 2N Hydrochloric acid and water were added, and the resulting mixture was extracted three times with ethyl acetate. The combined organic layers were washed with brine and dried over sodium sulfate. The solvent was evaporated under reduced pressure. The resulting residue was purified by silica gel column chromatography, and further purified by preparative HPLC and chiral SFC to obtain trans-3-[(2-chlorobenzyl)oxy]cyclobutane-1-carboxylic acid (44 mg) and cis-3-[(2-chlorobenzyl)oxy]cyclobutane-1-carboxylic acid (247 mg).

### Reference Example 65: trans-3-[(3-chlorobenzyl)oxy]cyclobutane-1-carboxylic acid

### Reference Example 66: cis-3-[(3-chlorobenzyl)oxy]cyclobutane-1-carboxylic acid

By performing operations similar to those of Reference Examples 62 to 64 using methyl trans-3-hydroxycyclobutanecarboxylate and 3-chlorobenzyl chloride, the title compound was obtained.

### Reference Example 67: trans-3-[(4-chlorobenzyl)oxy]cyclobutane-1-carboxylic acid

### Reference Example 68: cis-3-[(4-chlorobenzyl)oxy]cyclobutane-1-carboxylic acid

By performing operations similar to those of Reference Examples 62 to 64 using methyl trans-3-hydroxycyclobutanecarboxylate and 4-chlorobenzyl chloride, the title compound was obtained.

### Reference Example 69: trans-3-{[2-(trifluoromethyl)benzyl]oxy}cyclobutane-1-carboxylic acid

### Reference Example 70: cis-3-{[2-(trifluoromethyl)benzyl]oxy}cyclobutane-1-carboxylic acid

By performing operations similar to those of Reference Examples 62 to 64 using methyl trans-3-hydroxycyclobutanecarboxylate and 2-(trifluoromethyl)benzyl chloride, the title compound was obtained.

### Reference Example 71: trans-3-{[3-(trifluoromethyl)benzyl]oxy}cyclobutane-1-carboxylic acid

### Reference Example 72: cis-3-{[3-(trifluoromethyl)benzyl]oxy}cyclobutane-1-carboxylic acid

By performing operations similar to those of Reference Examples 62 to 64 using methyl trans-3-hydroxycyclobutanecarboxylate and 3-(trifluoromethyl)benzyl chloride, the title compound was obtained.

### Reference Example 73: trans-3-{[4-(trifluoromethyl)benzyl]oxy }cyclobutane-1-carboxylic acid

### Reference Example 74: cis-3-{[4-(trifluoromethyl)benzyl]oxy}cyclobutane-1-carboxylic acid

By performing operations similar to those of Reference Examples 62 to 64 using methyl trans-3-hydroxycyclobutanecarboxylate and 4-(trifluoromethyl)benzyl chloride, the title compound was obtained.

### Reference Example 75: trans-3-[(2,4-difluorobenzyl)oxy]cyclobutane-1-carboxylic acid

By performing operations similar to those of Reference Examples 62 to 64 using methyl trans-3-hydroxycyclobutanecarboxylate and 2,4-difluorobenzyl bromide, the title compound was obtained.

### Reference Example 76: trans-3-[(4-chloro-2-fluorobenzyl)oxy]cyclobutane-1-carboxylic acid

By performing operations similar to those of Reference Examples 62 to 64 using methyl trans-3-hydroxycyclobutanecarboxylate and 4-chloro-2-fluorobenzyl bromide, the title compound was obtained.

### Reference Example 77: trans-3-[(3,4-difluorobenzyl)oxy]cyclobutane-1-carboxylic acid

By performing operations similar to those of Reference Examples 62 to 64 using methyl trans-3-hydroxycyclobutanecarboxylate and 3,4-difluorobenzyl bromide, the title compound was obtained.

### Reference Example 78: trans-3-[(3,5-difluorobenzyl)oxy]cyclobutane-1-carboxylic acid

By performing operations similar to those of Reference Examples 62 to 64 using methyl trans-3-hydroxycyclobutanecarboxylate and 3,5-difluorobenzyl bromide, the title compound was obtained.

### Reference Example 79: trans-3-[(3-fluorobenzyl)oxy]cyclobutane-1-carboxylic acid

By performing operations similar to those of Reference Examples 62 to 64 using methyl trans-3-hydroxycyclobutanecarboxylate and 3-fluorobenzyl bromide, the title compound was obtained.

### Reference Example 80: trans-3-[(2,3-difluorobenzyl)oxy]cyclobutane-1-carboxylic acid

By performing operations similar to those of Reference Examples 62 to 64 using methyl trans-3-hydroxycyclobutanecarboxylate and 2,3-difluorobenzyl bromide, the title compound was obtained.

### Reference Example 81: trans-3-[(2-chloro-4-fluorobenzyl)oxy]cyclobutane-1-carboxylic acid

By performing operations similar to those of Reference Examples 62 to 64 using methyl trans-3-hydroxycyclobutanecarboxylate and 2-chloro-4-fluorobenzyl bromide, the title compound was obtained.

### Reference Example 82: trans-3-[(3-chloro-2-fluorobenzyl)oxy]cyclobutane-1-carboxylic acid

By performing operations similar to those of Reference Examples 62 to 64 using methyl trans-3-hydroxycyclobutanecarboxylate and 3-chloro-2-fluorobenzyl bromide, the title compound was obtained.

### Reference Example 83: trans-3-[(5-chloro-2-fluorobenzyl)oxy]cyclobutane-1-carboxylic acid

By performing operations similar to those of Reference Examples 62 to 64 using methyl trans-3-hydroxycyclobutanecarboxylate and 5-chloro-2-fluorobenzyl bromide, the title compound was obtained.

### Reference Example 84: trans-3-[(3-chloro-4-fluorobenzyl)oxy]cyclobutane-1-carboxylic acid

### Reference Example 85: cis-3-[(3-chloro-4-fluorobenzyl)oxy]cyclobutane-1-carboxylic acid

By performing operations similar to those of Reference Examples 62 to 64 using ethyl 3-hydroxycyclobutanecarboxylate and 3-chloro-4-fluorobenzyl bromide, the title compound was obtained.

### Reference Example 86: trans-3-[(3-chloro-5-fluorobenzyl)oxy]cyclobutane-1-carboxylic acid

### Reference Example 87: cis-3-[(3-chloro-5-fluorobenzyl)oxy]cyclobutane-1-carboxylic acid

By performing operations similar to those of Reference Examples 62 to 64 using ethyl 3-hydroxycyclobutanecarboxylate and 3-chloro-5-fluorobenzyl bromide, the title compound was obtained.

### Reference Example 88: ethyl 4-{[5-(trifluoromethyl)pyridin-2-yl]oxy}cyclohexane-1-carboxylate

By performing operations similar to those of Reference Example 62 using 2-chloro-5-(trifluoromethyl)pyridine and ethyl 4-hydroxycyclohexane-1-carboxylate, the title compound was obtained.

### Reference Example 89: 4-{[5-(trifluoromethyl)pyridin-2-yl]oxy}cyclohexane-1-carboxylic acid

By performing operations similar to those of Reference Examples 63 and 64 using ethyl 4-{[5-(trifluoromethyl)pyridin-2-yl]oxy}cyclohexane-1-carboxylate, the title compound was obtained.

### Reference Example 90: Ethyl trans-3-{[5-(trifluoromethyl)pyridin-2-yl]oxy}cyclobutane-1-carboxylate

By performing operations similar to those of Reference Example 62 using 2-chloro-5-(trifluoromethyl)pyridine and ethyl trans-3-hydroxycyclobutane-1-carboxylate, the title compound was obtained.

### Reference Example 91: trans-3-{[5-(trifluoromethyl)pyridin-2-yl]oxy}cyclobutane-1-carboxylic acid

By performing operations similar to those of Reference Examples 63 and 64 using ethyl trans-3-{[5-(trifluoromethyl)pyridin-2-yl]oxy }cyclobutane-1-carboxylate, the title compound was obtained.

### Reference Example 92: ethyl cis-3-{[5-(trifluoromethyl)pyridin-2-yl]oxy}cyclobutane-1-carboxylate

By performing operations similar to those of Reference Example 62 using 2-chloro-5-(trifluoromethyl)pyridine and ethyl cis-3-hydroxycyclobutane-1-carboxylate, the title compound was obtained.

### Reference Example 93: cis-3-{[5-(trifluoromethyl)pyridin-2-yl]oxy}cyclobutane-1-carboxylic acid

By performing operations similar to those of Reference Examples 63 and 64 using ethyl cis-3-{[5-(trifluoromethyl)pyridin-2-yl]oxy }cyclobutane-1-carboxylate, the title compound was obtained.

### Reference Example 94: trans-3-(4-chlorophenoxy)cyclobutane-1-carboxylic acid

By performing operations similar to those of Reference Examples 63 and 64 using ethyl trans-3-(4-chlorophenoxy)cyclobutane-1-carboxylate, the title compound was obtained.

### Reference Example 95: cis-3-(4-chlorophenoxy)cyclobutane-1-carboxylic acid

By performing operations similar to those of Reference Examples 63 and 64 using ethyl cis-3-(4-chlorophenoxy)cyclobutane-1-carboxylate, the title compound was obtained.

### Examples

### Example 1: N-{2-fluoro-3-[6-oxo-4-(trifluoromethyl)-1,6-dihydropyrimidin-2-yl]-4-(trifluoromethyl)benzyl}-1-(imidazo[1,2-b]pyridazin-6-yl)piperidine-4-carboxamide

To a solution of 2-[3-(aminomethyl)-2-fluoro-6-(trifluoromethyl)phenyl]-6-(trifluoromethyl)pyrimidin-4(3H)-one·hydrochloride (144 mg) in N,N-dimethylformamide (5 mL), 1-(imidazo[1,2-b]pyridazin-6-yl)piperidine-4-carboxylic acid (108 mg), triethylamine (205 µL), and O-(7-azabenzotriazole-1-yl)-N,N,N',N'-tetramethyluronium hexafluorophosphate (167 mg) were added. The reaction mixture was stirred at room temperature overnight. The mixture was extracted with ethyl acetate, and then washed once with saturated aqueous ammonium chloride, water, and saturated brine in that order, and dried over magnesium sulfate. The solvent was evaporated under reduced pressure. The resulting residue was purified by silica gel column chromatography and dried under reduced pressure to obtain the title compound (30 mg).

### Example 2: N-{2-fluoro-3-[6-oxo-4-(trifluoromethyl)-1,6-dihydropyrimidin-2-yl]-4-(trifluoromethyl)benzyl}-4-(imidazo[1,2-b]pyridazin-6-yl)benzamide

By performing operations similar to those of Example 1 using 2-[3-(aminomethyl)-2-fluoro-6-(trifluoromethyl)phenyl]-6-(trifluoromethyl)pyrimidin-4(3H)-one·2,2,2-trifluoroacetate and 4-(imidazo[1,2-b]pyridazin-6-yl)benzoic acid, the title compound was obtained.

### Example 3: N-{2-fluoro-3-[6-oxo-4-(trifluoromethyl)-1,6-dihydropyrimidin-2-yl]-4-(trifluoromethyl)benzyl}-1-(3-methylimidazo[1,2-b]pyridazin-6-yl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(3-methylimidazo[1,2-b]pyridazin-6-yl)piperidine-4-carboxylic acid, the title compound was obtained.

### Example 4: N-{2-fluoro-3-[6-oxo-4-(trifluoromethyl)-1,6-dihydropyrimidin-2-yl]-4-(trifluoromethyl)benzyl}-1-(8-methylquinolin-2-yl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(8-methylquinolin-2-yl)piperidine-4-carboxylic acid, the title compound was obtained.

### Example 5: N-{2-fluoro-3-[6-oxo-4-(trifluoromethyl)-1,6-dihydropyrimidin-2-yl]-4-(trifluoromethyl)benzyl}-1-(quinolin-2-yl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(quinolin-2-yl)piperidine-4-carboxylic acid, the title compound was obtained.

### Example 6: N-{2-fluoro-3-[6-oxo-4-(trifluoromethyl)-1,6-dihydropyrimidin-2-yl]-4-(trifluoromethyl)benzyl}-1-(8-methylquinolin-2-yl)azetidine-3-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(8-methylquinolin-2-yl)azetidine-3-carboxylic acid, the title compound was obtained.

### Example 7: N-[2-fluoro-3-(6-oxo-1,6-dihydropyrimidin-2-yl)-4-(trifluoromethyl)benzyl]-1-(8-methylquinolin-2-yl)piperidine-4-carboxamide

By performing operations similar to those of Example 1 using 2-[3-(aminomethyl)-2-fluoro-6-(trifluoromethyl)phenyl]pyrimidin-4(3H)-one and 1-(8-methylquinolin-2-yl)piperidine-4-carboxylic acid, the title compound was obtained.

### Example 8: N-[4-chloro-3-(6-oxo-1,6-dihydropyrimidin-2-yl)benzyl]-1-(8-methylquinolin-2-yl)piperidine-4-carboxamide

By performing operations similar to those of Example 1 using 2-[5-(aminomethyl)-2-chlorophenyl]pyrimidin-4(3H)-one and 1-(8-methylquinolin-2-yl)piperidine-4-carboxylic acid, the title compound was obtained.

### Example 9: N-[2-fluoro-3-(4-methyl-6-oxo-1,6-dihydropyrimidin-2-yl)-4-(trifluoromethyl)benzyl]-1-(8-methylquinolin-2-yl)piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-(8-methylquinolin-2-yl)piperidine-4-carboxylic acid, the title compound was obtained.

### Example 10: N-[3-(4-ethyl-6-oxo-1,6-dihydropyrimidin-2-yl)-2-fluoro-4-(trifluoromethyl)benzyl]-1-(8-methylquinolin-2-yl)piperidine-4-carboxamide

By performing operations similar to those of Example 1 using 2-[3-(aminomethyl)-2-fluoro-6-(trifluoromethyl)phenyl]-6-ethylpyrimidin-4(3H)-one , 1-(8-methylquinolin-2-yl)piperidine-4-carboxylic acid, the title compound was obtained.

### Example 11: N-[2-fluoro-3-(4-isopropyl-6-oxo-1,6-dihydropyrimidin-2-yl)-4-(trifluoromethyl)benzyl]-1-(8-methylquinolin-2-yl)piperidine-4-carboxamide

By performing operations similar to those of Example 1 using 2-[3-(aminomethyl)-2-fluoro-6-(trifluoromethyl)phenyl]-6-isopropylpyrimidin-4(3H)-one and 1-(8-methylquinolin-2-yl)piperidine-4-carboxylic acid, the title compound was obtained.

### Example 12: N-{3-[4-(difluoromethyl)-6-oxo-1,6-dihydropyrimidin-2-yl]-2-fluoro-4-(trifluoromethyl)benzyl}-1-(8-methylquinolin-2-yl)piperidine-4-carboxamide

By performing operations similar to those of Example 1 using 2-[3-(aminomethyl)-2-fluoro-6-(trifluoromethyl)phenyl]-6-(difluoromethyl)pyrimidin-4(3H)-one and 1-(8-methylquinolin-2-yl)piperidine-4-carboxylic acid, the title compound was obtained.

### Example 13: N-[3-(4,5-dimethyl-6-oxo-1,6-dihydropyrimidin-2-yl)-2-fluoro-4-(trifluoromethyl)benzyl]-1-(8-methylquinolin-2-yl)piperidine-4-carboxamide

By performing operations similar to those of Example 1 using 2-[3-(aminomethyl)-2-fluoro-6-(trifluoromethyl)phenyl]-5,6-dimethylpyrimidin-4(3H)-one and 1-(8-methylquinolin-2-yl)piperidine-4-carboxylic acid, the title compound was obtained.

### Example 14: N-[2-fluoro-3-(5-fluoro-4-methyl-6-oxo-1,6-dihydropyrimidin-2-yl)-4-(trifluoromethyl)benzyl]-1-(8-methylquinolin-2-yl)piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-(8-methylquinolin-2-yl)piperidine-4-carboxylic acid, the title compound was obtained.

### Example 15: 4-fluoro-N-{2-fluoro-3-[6-oxo-4-(trifluoromethyl)-1,6-dihydropyrimidin-2-yl]-4-(trifluoromethyl)benzyl}-1-(8-methylquinolin-2-yl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 4-fluoro-1-(8-methylquinolin-2-yl)piperidine-4-carboxylic acid, the title compound was obtained.

### Example 16: N-{2-fluoro-3-[6-oxo-4-(trifluoromethyl)-1,6-dihydropyrimidin-2-yl]-4-(trifluoromethyl)benzyl}-4-(pyridazin-3-yl)benzamide

By performing operations similar to those of Example 1 using 2-[3-(aminomethyl)-2-fluoro-6-(trifluoromethyl)phenyl]-6-(trifluoromethyl)pyrimidin-4(3H)-one·2,2,2-trifluoroacetate and 4-(pyridazin-3-yl)benzoic acid, the title compound was obtained.

### Example 17: N-{2-fluoro-3-[6-oxo-4-(trifluoromethyl)-1,6-dihydropyrimidin-2-yl]-4-(trifluoromethyl)benzyl}-1-(6-methylpyridin-2-yl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(6-methylpyridin-2-yl)piperidine-4-carboxylic acid, the title compound was obtained.

### Example 18: N-{2-fluoro-3-[6-oxo-4-(trifluoromethyl)-1,6-dihydropyrimidin-2-yl]-4-(trifluoromethyl)benzyl}-1-(5-methylpyridin-2-yl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(5-methylpyridin-2-yl)piperidine-4-carboxylic acid, the title compound was obtained.

### Example 19: N-{2-fluoro-3-[6-oxo-4-(trifluoromethyl)-1,6-dihydropyrimidin-2-yl]-4-(trifluoromethyl)benzyl}-1-(pyridin-2-yl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(pyridin-2-yl)piperidine-4-carboxylic acid, the title compound was obtained.

### Example 20: N-{2-fluoro-3-[6-oxo-4-(trifluoromethyl)-1,6-dihydropyrimidin-2-yl]-4-(trifluoromethyl)benzyl}-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[5-(trifluoromethyl)pyridin-2-yl]piperidine-4-carboxylic acid, the title compound was obtained.

### Example 21: N-{2-fluoro-3-[6-oxo-4-(trifluoromethyl)-1,6-dihydropyrimidin-2-yl]-4-(trifluoromethyl)benzyl}-1-[6-(trifluoromethyl)pyridin-3-yl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[6-(trifluoromethyl)pyridin-3-yl]piperidine-4-carboxylic acid, the title compound was obtained.

### Example 22: N-{2-fluoro-3-[6-oxo-4-(trifluoromethyl)-1,6-dihydropyrimidin-2-yl]-4-(trifluoromethyl)benzyl}-1-[6-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[6-(trifluoromethyl)pyridin-2-yl]piperidine-4-carboxylic acid, the title compound was obtained.

### Example 23: N-[3-(4-ethyl-6-oxo-1,6-dihydropyrimidin-2-yl)-2-fluoro-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-ethylpyrimidin-4(3H)-one and 1-[5-(trifluoromethyl)pyridin-2-yl]piperidine-4-carboxylic acid, the title compound was obtained.

### Example 24: N-[3-(4-ethyl-6-oxo-1,6-dihydropyrimidin-2-yl)-2-fluoro-4-(trifluoromethyl)benzyl]-1-(6-methylpyridin-2-yl)piperidine-4-carboxamide

By performing operations similar to those of Example 1 using 2-[3-(aminomethyl)-2-fluoro-6-(trifluoromethyl)phenyl]-6-ethylpyrimidin-4(3H)-one and 1-(6-methylpyridin-2-yl)piperidine-4-carboxylic acid, the title compound was obtained.

### Example 25: N-{2-fluoro-3-[6-oxo-4-(trifluoromethyl)-1,6-dihydropyrimidin-2-yl]-4-(trifluoromethyl)benzyl}-1-[5-(trifluoromethyl)pyridin-2-yl]azetidine-3-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[5-(trifluoromethyl)pyridin-2-yl]azetidine-3-carboxylic acid, the title compound was obtained.

### Example 26: N-{2-fluoro-3-[6-oxo-4-(trifluoromethyl)-1,6-dihydropyrimidin-2-yl]-4-(trifluoromethyl)benzyl}-1-[6-(trifluoromethyl)pyridin-2-yl]azetidine-3-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[6-(trifluoromethyl)pyridin-2-yl]azetidine-3-carboxylic acid, the title compound was obtained.

### Example 27: 1-(5-chloropyridin-2-yl)-N-{2-fluoro-3-[6-oxo-4-(trifluoromethyl)-1,6-dihydropyrimidin-2-yl]-4-(trifluoromethyl)benzyl}piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(5-chloropyridin-2-yl)piperidine-4-carboxylic acid, the title compound was obtained.

### Example 28: N-[2-fluoro-3-(6-oxo-1,6-dihydropyrimidin-2-yl)-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]pyrimidin-4(3H)-one and 1-[5-(trifluoromethyl)pyridin-2-yl]piperidine-4-carboxylic acid, the title compound was obtained.

### Example 29: N-{3-[4-(difluoromethyl)-6-oxo-1,6-dihydropyrimidin-2-yl]-2-fluoro-4-(trifluoromethyl)benzyl}-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-(difluoromethyl)pyrimidin-4(3H)-one and 1-[5-(trifluoromethyl)pyridin-2-yl]piperidine-4-carboxylic acid, the title compound was obtained.

### Example 30: 1-(3,5-dichloropyridin-2-yl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(3,5-dichloropyridin-2-yl)piperidine-4-carboxylic acid, the title compound was obtained.

### Example 31: N-{4-chloro-2-fluoro-3-[6-oxo-4-(trifluoromethyl)-1,6-dihydropyrimidin-2-yl]benzyl}-1-(8-methylquinolin-2-yl)piperidine-4-carboxamide

By performing operations similar to those of Example 1 using 2-[3-(aminomethyl)-6-chloro-2-fluorophenyl]-6-(trifluoromethyl)pyrimidin-4(3H)-one and 1-(8-methylquinolin-2-yl)piperidine-4-carboxylic acid, the title compound was obtained.

### Example 32: N-{4-chloro-2-fluoro-3-[6-oxo-4-(trifluoromethyl)-1,6-dihydropyrimidin-2-yl]benzyl}-1-[5-(trifluoromethyl)pyridin-2-yl]piperidine-4-carboxamide

By performing operations similar to those of Example 1 using 2-[3-(aminomethyl)-6-chloro-2-fluorophenyl]-6-(trifluoromethyl)pyrimidin-4(3H)-one and 1-[5-(trifluoromethyl)pyridin-2-yl]piperidine-4-carboxylic acid, the title compound was obtained.

### Example 33: N-[2-fluoro-3-(5-fluoro-4-methyl-6-oxo-1,6-dihydropyrimidin-2-yl)-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-[5-(trifluoromethyl)pyridin-2-yl]piperidine-4-carboxylic acid, the title compound was obtained.

### Example 34: N-[2-fluoro-3-(4-methyl-6-oxo-1,6-dihydropyrimidin-2-yl)-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-methylpyrimidin-4(3H)-one 1-[5-(trifluoromethyl)pyridin-2-yl]piperidine-4-carboxylic acid, the title compound was obtained.

### Example 35: N-[3-(4,5-dimethyl-6-oxo-1,6-dihydropyrimidin-2-yl)-2-fluoro-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-5,6-dimethylpyrimidin-4(3H)-one and 1-[5-(trifluoromethyl)pyridin-2-yl]piperidine-4-carboxylic acid, the title compound was obtained.

### Example 36: N-{2-fluoro-3-[6-oxo-4-(trifluoromethyl)-1,6-dihydropyrimidin-2-yl]-4-(trifluoromethyl)benzyl}-1-[5-(trifluoromethyl)pyrazin-2-yl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[5-(trifluoromethyl)pyrazin-2-yl]piperidine-4-carboxylic acid, the title compound was obtained.

### Example 37: 1-(5-chloro-3-fluoropyridin-2-yl)-N-{2-fluoro-3-[6-oxo-4-(trifluoromethyl)-1,6-dihydropyrimidin-2-yl]-4-(trifluoromethyl)benzyl}piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(5-chloro-3-fluoropyridin-2-yl)piperidine-4-carboxylic acid, the title compound was obtained.

### Example 38: 4-fluoro-N-{2-fluoro-3-[6-oxo-4-(trifluoromethyl)-1,6-dihydropyrimidin-2-yl]-4-(trifluoromethyl)benzyl}-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 4-fluoro-1-[5-(trifluoromethyl)pyridin-2-yl]piperidine-4-carboxylic acid, the title compound was obtained.

### Example 39: N-{2-fluoro-3-[6-oxo-4-(trifluoromethyl)-1,6-dihydropyrimidin-2-yl]-4-(trifluoromethyl)benzyl}-1-[3-fluoro-5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[3-fluoro-5-(trifluoromethyl)pyridin-2-yl]piperidine-4-carboxylic acid, the title compound was obtained.

### Example 40: N-{2-fluoro-3-[6-oxo-4-(trifluoromethyl)-1,6-dihydropyrimidin-2-yl]-4-(trifluoromethyl)benzyl}-1-[5-(trifluoromethoxy)pyridin-2-yl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[5-(trifluoromethoxy)pyridin-2-yl]piperidine-4-carboxylic acid, the title compound was obtained.

### Example 41: N-[2-fluoro-3-(4-oxo-4,5,6,7-tetrahydro-3H-cyclopenta[d]pyrimidin-2-yl)-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-3,5,6,7-tetrahydro-4H-cyclopenta[d]pyrimidin-4-one and 1-[5-(trifluoromethyl)pyridin-2-yl]piperidine-4-carboxylic acid, the title compound was obtained.

### Example 42: N-[3-(4-ethyl-5-fluoro-6-oxo-1,6-dihydropyrimidin-2-yl)-2-fluoro-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and 1-[5-(trifluoromethyl)pyridin-2-yl]piperidine-4-carboxylic acid, the title compound was obtained.

### Example 43: 1-(5-chloropyridin-2-yl)-N-[3-(4,5-dimethyl-6-oxo-1,6-dihydropyrimidin-2-yl)-2-fluoro-4-(trifluoromethyl)benzyl]piperidine-4-carboxamide

By performing operations similar to those of Example 1 using 2-[3-(aminomethyl)-2-fluoro-6-(trifluoromethyl)phenyl]-5,6-dimethylpyrimidin-4(3H)-one 1-(5-chloropyridin-2-yl)piperidine-4-carboxylic acid, the title compound was obtained.

### Example 44: 1-(3,5-dichloropyridin-2-yl)-N-[2-fluoro-3-(5-fluoro-4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-(3,5-dichloropyridin-2-yl)piperidine-4-carboxylic acid, the title compound was obtained.

### Example 45: 1-(3,5-dichloropyridin-2-yl)-N-{3-[4-(difluoromethyl)-6-oxo-1,6-dihydropyrimidin-2-yl]-2-fluoro-4-(trifluoromethyl)benzyl}piperidine-4-carboxamide

By performing operations similar to those of Example 1 using 2-[3-(aminomethyl)-2-fluoro-6-(trifluoromethyl)phenyl]-6-(difluoromethyl)pyrimidin-4(3H)-one and 1-(3,5-dichloropyridin-2-yl)piperidine-4-carboxylic acid, the title compound was obtained.

### Example 46: 1-(3,5-dichloropyridin-2-yl)-N-[2-fluoro-3-(4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-(3,5-dichloropyridin-2-yl)piperidine-4-carboxylic acid, the title compound was obtained.

### Example 47: N-[3-(4-ethyl-5-fluoro-6-oxo-1,6-dihydropyrimidin-2-yl)-2-fluoro-4-(trifluoromethyl)benzyl]-1-[5-(trifluoromethoxy)pyridin-2-yl]piperidine-4-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and 1-[5-(trifluoromethoxy)pyridin-2-yl]piperidine-4-carboxylic acid, the title compound was obtained.

### Example 48: N-[2-fluoro-3-(5-fluoro-4-methyl-6-oxo-1,6-dihydropyrimidin-2-yl)-4-(trifluoromethyl)benzyl]-1-[3-fluoro-5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-[3-fluoro-5-(trifluoromethyl)pyridin-2-yl]piperidine-4-carboxylic acid, the title compound was obtained.

### Example 49: N-[2-fluoro-3-(4-methyl-6-oxo-1,6-dihydropyrimidin-2-yl)-4-(trifluoromethyl)benzyl]-1-[3-fluoro-5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-[3-fluoro-5-(trifluoromethyl)pyridin-2-yl]piperidine-4-carboxylic acid, the title compound was obtained.

### Example 50: N-{3-[6-oxo-4-(trifluoromethyl)-1,6-dihydropyrimidin-2-yl]-4-(trifluoromethyl)benzyl}-1-[5-(trifluoromethyl)pyridin-2-yl]piperidine-4-carboxamide

By performing operations similar to those of Example 1 using 2-[5-(aminomethyl)-2-(trifluoromethyl)phenyl]-6-(trifluoromethyl)pyrimidin-4(3H)-one and 1-[5-(trifluoromethyl)pyridin-2-yl]piperidine-4-carboxylic acid, the title compound was obtained.

### Example 51: 4-fluoro-N-[2-fluoro-3-(5-fluoro-4-methyl-6-oxo-1,6-dihydropyrimidin-2-yl)-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 4-fluoro-1-[5-(trifluoromethyl)pyridin-2-yl]piperidine-4-carboxylic acid, the title compound was obtained.

### Example 52: 1-(5-chloro-3-fluoropyridin-2-yl)-N-[2-fluoro-3-(5-fluoro-4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-(5-chloro-3-fluoropyridin-2-yl)piperidine-4-carboxylic acid, the title compound was obtained.

### Example 53: N-[2-fluoro-3-(4-methyl-6-oxo-1,6-dihydropyrimidin-2-yl)-4-(trifluoromethyl)benzyl]-1-[5-(trifluoromethoxy)pyridin-2-yl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-[5-(trifluoromethoxy)pyridin-2-yl]piperidine-4-carboxylic acid, the title compound was obtained.

### Example 54: 1-(5-chloro-6-methylpyridin-2-yl)-N-{2-fluoro-3-[6-oxo-4-(trifluoromethyl)-1,6-dihydropyrimidin-2-yl]-4-(trifluoromethyl)benzyl}piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(5-chloro-6-methylpyridin-2-yl)piperidine-4-carboxylic acid, the title compound was obtained.

### Example 55: N-{3-[4-(difluoromethyl)-6-oxo-1,6-dihydropyrimidin-2-yl]-2-fluoro-4-(trifluoromethyl)benzyl}-1-[3-fluoro-5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-(difluoromethyl)pyrimidin-4(3H)-one and 1-[3-fluoro-5-(trifluoromethyl)pyridin-2-yl]piperidine-4-carboxylic acid, the title compound was obtained.

### Example 56: 4-fluoro-N-[2-fluoro-3-(4-methyl-6-oxo-1,6-dihydropyrimidin-2-yl)-4-(trifluoromethyl)benzyl]-1-[5-(trifluoromethyl)pyridin-2-yl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 4-fluoro-1-[5-(trifluoromethyl)pyridin-2-yl]piperidine-4-carboxylic acid, the title compound was obtained.

### Example 57: 1-(5-chloropyridin-2-yl)-N-[3-(4-ethyl-5-fluoro-6-oxo-1,6-dihydropyrimidin-2-yl)-2-fluoro-4-(trifluoromethyl)benzyl]piperidine-4-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and 1-(5-chloropyridin-2-yl)piperidine-4-carboxylic acid, the title compound was obtained.

### Example 58: N-{2-fluoro-3-[6-oxo-4-(trifluoromethyl)-1,6-dihydropyrimidin-2-yl]-4-(trifluoromethyl)benzyl}-1-(4-fluorophenyl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(4-fluorophenyl)piperidine-4-carboxylic acid, the title compound was obtained.

### Example 59: N-{2-fluoro-3-[6-oxo-4-(trifluoromethyl)-1,6-dihydropyrimidin-2-yl]-4-(trifluoromethyl)benzyl}-1-(3-methylphenyl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(3-methylphenyl)piperidine-4-carboxylic acid, the title compound was obtained.

### Example 60: N-{2-fluoro-3-[6-oxo-4-(trifluoromethyl)-1,6-dihydropyrimidin-2-yl]-4-(trifluoromethyl)benzyl}-1-(4-fluoro-3-methylphenyl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(4-fluoro-3-methylphenyl)piperidine-4-carboxylic acid, the title compound was obtained.

### Example 61: N-{2-fluoro-3-[6-oxo-4-(trifluoromethyl)-1,6-dihydropyrimidin-2-yl]-4-(trifluoromethyl)benzyl}-1-(4-methylphenyl)piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(4-methylphenyl)piperidine-4-carboxylic acid, the title compound was obtained.

### Example 62: 1-(3-chlorophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(3-chlorophenyl)piperidine-4-carboxylic acid, the title compound was obtained.

### Example 63: N-{2-fluoro-3-[6-oxo-4-(trifluoromethyl)-1,6-dihydropyrimidin-2-yl]-4-(trifluoromethyl)benzyl}-1-[4-(trifluoromethyl)phenyl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[4-(trifluoromethyl)phenyl]piperidine-4-carboxylic acid, the title compound was obtained.

### Example 64: 1-(4-chlorophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(4-chlorophenyl)piperidine-4-carboxylic acid, the title compound was obtained.

### Example 65: 1-[3-(difluoromethyl)phenyl]-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[3-(difluoromethyl)phenyl]piperidine-4-carboxylic acid, the title compound was obtained.

### Example 66: 1-(3,4-dichlorophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(3,4-dichlorophenyl)piperidine-4-carboxylic acid, the title compound was obtained.

### Example 67: N-{2-fluoro-3-[6-oxo-4-(trifluoromethyl)-1,6-dihydropyrimidin-2-yl]-4-(trifluoromethyl)benzyl}-1-[3-(trifluoromethyl)phenyl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[3-(trifluoromethyl)phenyl]piperidine-4-carboxylic acid, the title compound was obtained.

### Example 68: N-{2-fluoro-3-[6-oxo-4-(trifluoromethyl)-1,6-dihydropyrimidin-2-yl]-4-(trifluoromethyl)benzyl }-1-[4-(trifluoromethoxy)phenyl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[4-(trifluoromethoxy)phenyl]piperidine-4-carboxylic acid, the title compound was obtained.

### Example 69: 1-(2,3-dichlorophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(2,3-dichlorophenyl)piperidine-4-carboxylic acid, the title compound was obtained.

### Example 70: 1-(4-chloro-2-fluorophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(4-chloro-2-fluorophenyl)piperidine-4-carboxylic acid, the title compound was obtained.

### Example 71: 1-[4-(difluoromethoxy)phenyl]-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[4-(difluoromethoxy)phenyl]piperidine-4-carboxylic acid, the title compound was obtained.

### Example 72: N-[2-fluoro-3-(5-fluoro-4-methyl-6-oxo-1,6-dihydropyrimidin-2-yl)-4-(trifluoromethyl)benzyl]-1-[4-(trifluoromethoxy)phenyl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-[4-(trifluoromethoxy)phenyl]piperidine-4-carboxylic acid, the title compound was obtained.

### Example 73: 1-(3,4-dichlorophenyl)-N-[2-fluoro-3-(5-fluoro-4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-(3,4-dichlorophenyl)piperidine-4-carboxylic acid, the title compound was obtained.

### Example 74: N-{2-fluoro-3-[6-oxo-4-(trifluoromethyl)-1,6-dihydropyrimidin-2-yl]-4-(trifluoromethyl)benzyl}-1-[3-(trifluoromethoxy)phenyl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[3-(trifluoromethoxy)phenyl]piperidine-4-carboxylic acid, the title compound was obtained.

### Example 75: 1-(2-chloro-4-cyanophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(2-chloro-4-cyanophenyl)piperidine-4-carboxylic acid, the title compound was obtained.

### Example 76: 1-(4-chlorophenyl)-N-[2-fluoro-3-(4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-(4-chlorophenyl)piperidine-4-carboxylic acid, the title compound was obtained.

### Example 77: 1-(4-chlorophenyl)-N-[2-fluoro-3-(5-fluoro-4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-(4-chlorophenyl)piperidine-4-carboxylic acid, the title compound was obtained.

### Example 78: 1-(4-chlorophenyl)-N-[3-(4,5-dimethyl-6-oxo-1,6-dihydropyrimidin-2-yl)-2-fluoro-4-(trifluoromethyl)benzyl]piperidine-4-carboxamide

By performing operations similar to those of Example 1 using 2-[3-(aminomethyl)-2-fluoro-6-(trifluoromethyl)phenyl]-5,6-dimethylpyrimidin-4(3H)-one and 1-(4-chlorophenyl)piperidine-4-carboxylic acid, the title compound was obtained.

### Example 79: 1-(4-chlorophenyl)-N-[3-(4-ethyl-5-fluoro-6-oxo-1,6-dihydropyrimidin-2-yl)-2-fluoro-4-(trifluoromethyl)benzyl]piperidine-4-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and 1-(4-chlorophenyl)piperidine-4-carboxylic acid, the title compound was obtained.

### Example 80: N-[3-(4-ethyl-5-fluoro-6-oxo-1,6-dihydropyrimidin-2-yl)-2-fluoro-4-(trifluoromethyl)benzyl]-1-[4-(trifluoromethoxy)phenyl]piperidine-4-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and 1-[4-(trifluoromethoxy)phenyl]piperidine-4-carboxylic acid, the title compound was obtained.

### Example 81: 1-(4-chloro-3-fluorophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(4-chloro-3-fluorophenyl)piperidine-4-carboxylic acid, the title compound was obtained.

### Example 82: 1-(3,5-dichlorophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(3,5-dichlorophenyl)piperidine-4-carboxylic acid, the title compound was obtained.

### Example 83: 1-(4-chloro-3-methoxyphenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(4-chloro-3-methoxyphenyl)piperidine-4-carboxylic acid, the title compound was obtained.

### Example 84: 1-(3,4-dichlorophenyl)-N-{3-[4-(difluoromethyl)-6-oxo-1,6-dihydropyrimidin-2-yl]-2-fluoro-4-(trifluoromethyl)benzyl}piperidine-4-carboxamide

By performing operations similar to those of Example 1 using 2-[3-(aminomethyl)-2-fluoro-6-(trifluoromethyl)phenyl]-6-(difluoromethyl)pyrimidin-4(3H)-one and 1-(3,4-dichlorophenyl)piperidine-4-carboxylic acid, the title compound was obtained.

### Example 85: N-{3-[4-(difluoromethyl)-6-oxo-1,6-dihydropyrimidin-2-yl]-2-fluoro-4-(trifluoromethyl)benzyl }-1-[4-(trifluoromethoxy)phenyl]piperidine-4-carboxamide

By performing operations similar to those of Example 1 using 2-[3-(aminomethyl)-2-fluoro-6-(trifluoromethyl)phenyl]-6-(difluoromethyl)pyrimidin-4(3H)-one and 1-[4-(trifluoromethoxy)phenyl]piperidine-4-carboxylic acid, the title compound was obtained.

### Example 86: 1-(3,4-dichlorophenyl)-N-[2-fluoro-3-(4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-(3,4-dichlorophenyl)piperidine-4-carboxylic acid, the title compound was obtained.

### Example 87: N-[2-fluoro-3-(4-methyl-6-oxo-1,6-dihydropyrimidin-2-yl)-4-(trifluoromethyl)benzyl]-1-[4-(trifluoromethoxy)phenyl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-[4-(trifluoromethoxy)phenyl]piperidine-4-carboxylic acid, the title compound was obtained.

### Example 88: 1-(3-chloro-4-fluorophenyl)-N-{2-fluoro-3-[6-oxo-4-(trifluoromethyl)-1,6-dihydropyrimidin-2-yl]-4-(trifluoromethyl)benzyl } piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(3-chloro-4-fluorophenyl)piperidine-4-carboxylic acid, the title compound was obtained.

### Example 89: 1-(4-chlorophenyl)-N-[2-fluoro-3-(4-oxo-4,5,6,7-tetrahydro-3H-cyclopenta[d]pyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-3,5,6,7-tetrahydro-4H-cyclopenta[d]pyrimidin-4-one and 1-(4-chlorophenyl)piperidine-4-carboxylic acid, the title compound was obtained.

### Example 90: 1-(2-chloro-4-cyanophenyl)-N-[3-(4-ethyl-5-fluoro-6-oxo-1,6-dihydropyrimidin-2-yl)-2-fluoro-4-(trifluoromethyl)benzyl]piperidine-4-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and 1-(2-chloro-4-cyanophenyl)piperidine-4-carboxylic acid, the title compound was obtained.

### Example 91: 1-(4-chloro-3-fluorophenyl)-N-[2-fluoro-3-(5-fluoro-4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-(4-chloro-3-fluorophenyl)piperidine-4-carboxylic acid, the title compound was obtained.

### Example 92: 1-(4-chloro-3-fluorophenyl)-N-{3-[4-(difluoromethyl)-6-oxo-1,6-dihydropyrimidin-2-yl]-2-fluoro-4-(trifluoromethyl)benzyl}piperidine-4-carboxamide

By performing operations similar to those of Example 1 using 2-[3-(aminomethyl)-2-fluoro-6-(trifluoromethyl)phenyl]-6-(difluoromethyl)pyrimidin-4(3H)-one and 1-(4-chloro-3-fluorophenyl)piperidine-4-carboxylic acid, the title compound was obtained.

### Example 93: 1-(4-chloro-3-methoxyphenyl)-N-[2-fluoro-3-(4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-(4-chloro-3-methoxyphenyl)piperidine-4-carboxylic acid, the title compound was obtained.

### Example 94: N-[2-fluoro-3-(4-methyl-6-oxo-1,6-dihydropyrimidin-2-yl)-4-(trifluoromethyl)benzyl]-1-[3-(trifluoromethyl)phenyl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-[3-(trifluoromethyl)phenyl]piperidine-4-carboxylic acid, the title compound was obtained.

### Example 95: 1-(4-chloro-3-fluorophenyl)-N-[2-fluoro-3-(4-methyl-6-oxo-1,6-dihydropyrimidin-2-yl)-4-(trifluoromethyl)benzyl]piperidine-4-carboxamide

By performing operations similar to those of Example 1 using 2-[3-(aminomethyl)-2-fluoro-6-(trifluoromethyl)phenyl]-6-methylpyrimidin-4(3H)-one and 1-(4-chloro-3-fluorophenyl)piperidine-4-carboxylic acid, the title compound was obtained.

### Example 96: 1-(4-chloro-3-methoxyphenyl)-N-[3-(4-ethyl-5-fluoro-6-oxo-1,6-dihydropyrimidin-2-yl)-2-fluoro-4-(trifluoromethyl)benzyl]piperidine-4-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and 1-(4-chloro-3-methoxyphenyl)piperidine-4-carboxylic acid, the title compound was obtained.

### Example 97: N-[2-fluoro-3-(5-fluoro-4-methyl-6-oxo-1,6-dihydropyrimidin-2-yl)-4-(trifluoromethyl)benzyl]-1-[3-(trifluoromethyl)phenyl]piperidine-4-carboxamide

By performing operations similar to those of Example 1 using 2-[3-(aminomethyl)-2-fluoro-6-(trifluoromethyl)phenyl]-5-fluoro-6-methylpyrimidin-4(3H)-one and 1-[3-(trifluoromethyl)phenyl]piperidine-4-carboxylic acid, the title compound was obtained.

### Example 98: 1-(4-chloro-3-methoxyphenyl)-N-[3-(4-ethyl-6-oxo-1,6-dihydropyrimidin-2-yl)-2-fluoro-4-(trifluoromethyl)benzyl]piperidine-4-carboxamide

By performing operations similar to those of Example 1 using 2-[3-(aminomethyl)-2-fluoro-6-(trifluoromethyl)phenyl]-6-ethylpyrimidin-4(3H)-one and 1-(4-chloro-3-methoxyphenyl)piperidine-4-carboxylic acid, the title compound was obtained.

### Example 99: trans-4-[(2-chlorobenzyl)oxy]-N-{2-fluoro-3-[6-oxo-4-(trifluoromethyl)-1,6-dihydropyrimidin-2-yl]-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-[(2-chlorobenzyl)oxy]cyclohexane-1-carboxylic acid, the title compound was obtained.

### Example 100: trans-4-[(3-chlorobenzyl)oxy]-N-{2-fluoro-3-[6-oxo-4-(trifluoromethyl)-1,6-dihydropyrimidin-2-yl]-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-[(3-chlorobenzyl)oxy]cyclohexane-1-carboxylic acid, the title compound was obtained.

### Example 101: trans-4-[(4-chlorobenzyl)oxy]-N-{2-fluoro-3-[6-oxo-4-(trifluoromethyl)-1,6-dihydropyrimidin-2-yl]-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-[(4-chlorobenzyl)oxy]cyclohexane-1-carboxylic acid, the title compound was obtained.

### Example 102: trans-4-[(2-chlorobenzyl)oxy]-N-[2-fluoro-3-(5-fluoro-4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-4-[(2-chlorobenzyl)oxy]cyclohexane-1-carboxylic acid, the title compound was obtained.

### Example 103: trans-4-[(2-chlorobenzyl)oxy]-N-{3-[4-(difluoromethyl)-6-oxo-1,6-dihydropyrimidin-2-yl]-2-fluoro-4-(trifluoromethyl)benzyl}cyclohexane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-4-[(2-chlorobenzyl)oxy]cyclohexane-1-carboxylic acid, the title compound was obtained.

### Example 104: trans-4-[(4-chlorobenzyl)oxy]-N-[2-fluoro-3-(5-fluoro-4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-4-[(4-chlorobenzyl)oxy]cyclohexane-1-carboxylic acid, the title compound was obtained.

### Example 105: trans-4-[(4-chlorobenzyl)oxy]-N-[2-fluoro-3-(4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-[(4-chlorobenzyl)oxy]cyclohexane-1-carboxylic acid, the title compound was obtained.

### Example 106: trans-4-[(2,4-difluorobenzyl)oxy]-N-[2-fluoro-3-(4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-[(2,4-difluorobenzyl)oxy]cyclohexane-1-carboxylic acid, the title compound was obtained.

### Example 107: trans-4-[(3,4-difluorobenzyl)oxy]-N-[2-fluoro-3-(4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-[(3,4-difluorobenzyl)oxy]cyclohexane-1-carboxylic acid, the title compound was obtained.

### Example 108: trans-4-[(2-chlorobenzyl)oxy]-N-[2-fluoro-3-(4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-[(2-chlorobenzyl)oxy]cyclohexane-1-carboxylic acid, the title compound was obtained.

### Example 109: trans-N-[2-fluoro-3-(4-methyl-6-oxo-1,6-dihydropyrimidin-2-yl)-4-(trifluoromethyl)benzyl]-4-{[3-(trifluoromethyl)benzyl]oxy}cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-{[3-(trifluoromethyl)benzyl]oxy}cyclohexane-1-carboxylic acid, the title compound was obtained.

### Example 110: trans-N-[2-fluoro-3-(4-methyl-6-oxo-1,6-dihydropyrimidin-2-yl)-4-(trifluoromethyl)benzyl]-4-{[4-(trifluoromethyl)benzyl]oxy}cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-{[4-(trifluoromethyl)benzyl]oxy}cyclohexane-1-carboxylic acid, the title compound was obtained.

### Example 111: trans-4-[(3-chlorobenzyl)oxy]-N-[2-fluoro-3-(4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-[(3-chlorobenzyl)oxy]cyclohexane-1-carboxylic acid, the title compound was obtained.

### Example 112: trans-N-{2-fluoro-3-[6-oxo-4-(trifluoromethyl)-1,6-dihydropyrimidin-2-yl]-4-(trifluoromethyl)benzyl}-4-{[3-(trifluoromethyl)benzyl]oxy}cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-{[3-(trifluoromethyl)benzyl]oxy}cyclohexane-1-carboxylic acid, the title compound was obtained.

### Example 113: trans-N-{2-fluoro-3-[6-oxo-4-(trifluoromethyl)-1,6-dihydropyrimidin-2-yl]-4-(trifluoromethyl)benzyl}-4-{[4-(trifluoromethyl)benzyl Joxy } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-{[4-(trifluoromethyl)benzyl]oxy}cyclohexane-1-carboxylic acid, the title compound was obtained.

### Example 114: trans-4-[(2,4-difluorobenzyl)oxy]-N-{2-fluoro-3-[6-oxo-4-(trifluoromethyl)-1,6-dihydropyrimidin-2-yl]-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-[(2,4-difluorobenzyl)oxy]cyclohexane-1-carboxylic acid, the title compound was obtained.

### Example 115: trans-4-[(3,4-difluorobenzyl)oxy]-N-{2-fluoro-3-[6-oxo-4-(trifluoromethyl)-1,6-dihydropyrimidin-2-yl]-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-[(3,4-difluorobenzyl)oxy]cyclohexane-1-carboxylic acid, the title compound was obtained.

### Example 116: trans-4-[(4-chlorobenzyl)oxy]-N-{3-[4-(difluoromethyl)-6-oxo-1,6-dihydropyrimidin-2-yl]-2-fluoro-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-4-[(4-chlorobenzyl)oxy]cyclohexane-1-carboxylic acid, the title compound was obtained.

### Example 117: trans-4-[(3,5-difluorobenzyl)oxy]-N-{2-fluoro-3-[6-oxo-4-(trifluoromethyl)-1,6-dihydropyrimidin-2-yl]-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-[(3,5-difluorobenzyl)oxy]cyclohexane-1-carboxylic acid, the title compound was obtained.

### Example 118: trans-4-[(4-chloro-2-fluorobenzyl)oxy]-N-{2-fluoro-3-[6-oxo-4-(trifluoromethyl)-1,6-dihydropyrimidin-2-yl]-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-[(4-chloro-2-fluorobenzyl)oxy]cyclohexane-1-carboxylic acid, the title compound was obtained.

### Example 119: trans-4-[(3-chloro-5-fluorobenzyl)oxy]-N-{2-fluoro-3-[6-oxo-4-(trifluoromethyl)-1,6-dihydropyrimidin-2-yl]-4-(trifluoromethyl)benzyl } cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-[(3-chloro-5-fluorobenzyl)oxy]cyclohexane-1-carboxylic acid, the title compound was obtained.

### Example 120: trans-4-[(3,5-difluorobenzyl)oxy]-N-[2-fluoro-3-(4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-[(3,5-difluorobenzyl)oxy]cyclohexane-1-carboxylic acid, the title compound was obtained.

### Example 121: trans-4-[(4-chloro-2-fluorobenzyl)oxy]-N-[2-fluoro-3-(4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-[(4-chloro-2-fluorobenzyl)oxy]cyclohexane-1-carboxylic acid, the title compound was obtained.

### Example 122: trans-4-[(3-chloro-5-fluorobenzyl)oxy]-N-[2-fluoro-3-(4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-methylpyrimidin-4(3H)-one and trans-4-[(3-chloro-5-fluorobenzyl)oxy]cyclohexane-1-carboxylic acid, the title compound was obtained.

### Example 123: trans-4-[(3-chlorobenzyl)oxy]-N-[2-fluoro-3-(5-fluoro-4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-4-[(3-chlorobenzyl)oxy]cyclohexane-1-carboxylic acid, the title compound was obtained.

### Example 124: trans-4-[(2-chlorobenzyl)oxy]-N-[3-(4-ethyl-5-fluoro-6-oxo-1,6-dihydropyrimidin-2-yl)-2-fluoro-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-4-[(2-chlorobenzyl)oxy]cyclohexane-1-carboxylic acid, the title compound was obtained.

### Example 125: trans-4-[(4-chlorobenzyl)oxy]-N-[3-(4-ethyl-5-fluoro-6-oxo-1,6-dihydropyrimidin-2-yl)-2-fluoro-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-4-[(4-chlorobenzyl)oxy]cyclohexane-1-carboxylic acid, the title compound was obtained.

### Example 126: trans-4-[(2,4-difluorobenzyl)oxy]-N-[2-fluoro-3-(5-fluoro-4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-4-[(2,4-difluorobenzyl)oxy]cyclohexane-1-carboxylic acid, the title compound was obtained.

### Example 127: trans-4-[(3,4-difluorobenzyl)oxy]-N-[2-fluoro-3-(5-fluoro-4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-4-[(3,4-difluorobenzyl)oxy]cyclohexane-1-carboxylic acid, the title compound was obtained.

### Example 128: trans-4-[(4-chloro-2-fluorobenzyl)oxy]-N-[2-fluoro-3-(5-fluoro-4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-4-[(4-chloro-2-fluorobenzyl)oxy]cyclohexane-1-carboxylic acid, the title compound was obtained.

### Example 129: trans-4-[(2,4-difluorobenzyl)oxy]-N-[3-(4-ethyl-6-oxo-1,6-dihydropyrimidin-2-yl)-2-fluoro-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-ethylpyrimidin-4(3H)-one and trans-4-[(3-chlorobenzyl)oxy]cyclohexane-1-carboxylic acid, the title compound was obtained.

### Example 130: trans-N-[2-fluoro-3-(5-fluoro-4-methyl-6-oxo-1,6-dihydropyrimidin-2-yl)-4-(trifluoromethyl)benzyl]-4-{[3-(trifluoromethyl)benzyl]oxy}cyclohexane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-4-{[3-(trifluoromethyl)benzyl]oxy}cyclohexane-1-carboxylic acid, the title compound was obtained.

### Example 131: trans-4-[(3-chloro-5-fluorobenzyl)oxy]-N-[2-fluoro-3-(5-fluoro-4-methyl-6-oxo-1,6-dihydropyrimidin-2-yl)-4-(trifluoromethyl)benzyl]cyclohexane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-4-[(3-chloro-5-fluorobenzyl)oxy]cyclohexane-1-carboxylic acid, the title compound was obtained.

### Example 132: trans-4-[(2,4-difluorobenzyl)oxy]-N-[3-(4-ethyl-5-fluoro-6-oxo-1,6-dihydropyrimidin-2-yl)-2-fluoro-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-4-[(2,4-difluorobenzyl)oxy]cyclohexane-1-carboxylic acid, the title compound was obtained.

### Example 133: trans-4-[(3,4-difluorobenzyl)oxy]-N-[3-(4-ethyl-5-fluoro-6-oxo-1,6-dihydropyrimidin-2-yl)-2-fluoro-4-(trifluoromethyl)benzyl]cyclohexane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-4-[(3,4-difluorobenzyl)oxy]cyclohexane-1-carboxylic acid, the title compound was obtained.

### Example 134: trans-3-(benzyloxy)-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-(benzyloxy)cyclobutane-1-carboxylic acid, the title compound was obtained.

### Example 135: cis-3-(benzyloxy)-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-(benzyloxy)cyclobutane-1-carboxylic acid, the title compound was obtained.

### Example 136: trans-3-[(2-chlorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(2-chlorobenzyl)oxy]cyclobutane-1-carboxylic acid, the title compound was obtained.

### Example 137: cis-3-[(2-chlorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-[(2-chlorobenzyl)oxy]cyclobutane-1-carboxylic acid, the title compound was obtained.

### Example 138: trans-3-[(3-chlorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(3-chlorobenzyl)oxy]cyclobutane-1-carboxylic acid, the title compound was obtained.

### Example 139: cis-3-[(3-chlorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-[(3-chlorobenzyl)oxy]cyclobutane-1-carboxylic acid, the title compound was obtained.

### Example 140: trans-3-[(4-chlorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(4-chlorobenzyl)oxy]cyclobutane-1-carboxylic acid, the title compound was obtained.

### Example 141: cis-3-[(4-chlorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-[(4-chlorobenzyl)oxy]cyclobutane-1-carboxylic acid, the title compound was obtained.

### Example 142: trans-N-{2-fluoro-3-[6-oxo-4-(trifluoromethyl)-1,6-dihydropyrimidin-2-yl]-4-(trifluoromethyl)benzyl}-3-{[2-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-{[2-(trifluoromethyl)benzyl]oxy}cyclobutane-1-carboxylic acid, the title compound was obtained.

### Example 143: cis-N-{2-fluoro-3-[6-oxo-4-(trifluoromethyl)-1,6-dihydropyrimidin-2-yl]-4-(trifluoromethyl)benzyl}-3-{[2-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-{[2-(trifluoromethyl)benzyl]oxy}cyclobutane-1-carboxylic acid, the title compound was obtained.

### Example 144: trans-N-{2-fluoro-3-[6-oxo-4-(trifluoromethyl)-1,6-dihydropyrimidin-2-yl]-4-(trifluoromethyl)benzyl}-3-{[3-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-{[3-(trifluoromethyl)benzyl]oxy}cyclobutane-1-carboxylic acid, the title compound was obtained.

### Example 145: cis-N-{2-fluoro-3-[6-oxo-4-(trifluoromethyl)-1,6-dihydropyrimidin-2-yl]-4-(trifluoromethyl)benzyl}-3-{[3-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-{[3-(trifluoromethyl)benzyl]oxy}cyclobutane-1-carboxylic acid, the title compound was obtained.

### Example 146: trans-N-{2-fluoro-3-[6-oxo-4-(trifluoromethyl)-1,6-dihydropyrimidin-2-yl]-4-(trifluoromethyl)benzyl}-3-{[4-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-{[4-(trifluoromethyl)benzyl]oxy}cyclobutane-1-carboxylic acid, the title compound was obtained.

### Example 147: cis-N-{2-fluoro-3-[6-oxo-4-(trifluoromethyl)-1,6-dihydropyrimidin-2-yl]-4-(trifluoromethyl)benzyl}-3-{[4-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-{[4-(trifluoromethyl)benzyl]oxy}cyclobutane-1-carboxylic acid, the title compound was obtained.

### Example 148: trans-3-[(2,4-difluorobenzyl)oxy]-N-{2-fluoro-3-[6-oxo-4-(trifluoromethyl)-1,6-dihydropyrimidin-2-yl]-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(2,4-difluorobenzyl)oxy]cyclobutane-1-carboxylic acid, the title compound was obtained.

### Example 149: trans-3-[(4-chloro-2-fluorobenzyl)oxy]-N-{2-fluoro-3-[6-oxo-4-(trifluoromethyl)-1,6-dihydropyrimidin-2-yl]-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(4-chloro-2-fluorobenzyl)oxy]cyclobutane-1-carboxylic acid, the title compound was obtained.

### Example 150: trans-3-[(3,4-difluorobenzyl)oxy]-N-{2-fluoro-3-[6-oxo-4-(trifluoromethyl)-1,6-dihydropyrimidin-2-yl]-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(3,4-difluorobenzyl)oxy]cyclobutane-1-carboxylic acid, the title compound was obtained.

### Example 151: trans-3-[(3,5-difluorobenzyl)oxy]-N-{2-fluoro-3-[6-oxo-4-(trifluoromethyl)-1,6-dihydropyrimidin-2-yl]-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(3,5-difluorobenzyl)oxy]cyclobutane-1-carboxylic acid, the title compound was obtained.

### Example 152: trans-N-{2-fluoro-3-[6-oxo-4-(trifluoromethyl)-1,6-dihydropyrimidin-2-yl]-4-(trifluoromethyl)benzyl}-3-[(3-fluorobenzyl)oxy]cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(3-fluorobenzyl)oxy]cyclobutane-1-carboxylic acid, the title compound was obtained.

### Example 153: trans-3-[(2,3-difluorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(2,3-difluorobenzyl)oxy]cyclobutane-1-carboxylic acid, the title compound was obtained.

### Example 154: trans-3-[(2-chloro-4-fluorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(2-chloro-4-fluorobenzyl)oxy]cyclobutane-1-carboxylic acid, the title compound was obtained.

### Example 155: trans-3-[(3-chloro-2-fluorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(3-chloro-2-fluorobenzyl)oxy]cyclobutane-1-carboxylic acid, the title compound was obtained.

### Example 156: trans-3-[(5-chloro-2-fluorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(5-chloro-2-fluorobenzyl)oxy]cyclobutane-1-carboxylic acid, the title compound was obtained.

### Example 157: trans-3-[(3-chloro-4-fluorobenzyl)oxy]-N-{2-fluoro-3-[6-oxo-4-(trifluoromethyl)-1,6-dihydropyrimidin-2-yl]-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(3-chloro-4-fluorobenzyl)oxy]cyclobutane-1-carboxylic acid, the title compound was obtained.

### Example 158: cis-3-[(3-chloro-4-fluorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-[(3-chloro-4-fluorobenzyl)oxy]cyclobutane-1-carboxylic acid, the title compound was obtained.

### Example 159: trans-3-[(3-chloro-5-fluorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-[(3-chloro-5-fluorobenzyl)oxy]cyclobutane-1-carboxylic acid, the title compound was obtained.

### Example 160: cis-3-[(3-chloro-5-fluorobenzyl)oxy]-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-[(3-chloro-5-fluorobenzyl)oxy]cyclobutane-1-carboxylic acid, the title compound was obtained.

### Example 161: trans-3-[(2-chlorobenzyl)oxy]-N-{3-[4-(difluoromethyl)-6-oxo-1,6-dihydropyrimidin-2-yl]-2-fluoro-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(2-chlorobenzyl)oxy]cyclobutane-1-carboxylic acid, the title compound was obtained.

### Example 162: trans-3-[(3-chlorobenzyl)oxy]-N-{3-[4-(difluoromethyl)-6-oxo-1,6-dihydropyrimidin-2-yl]-2-fluoro-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(3-chlorobenzyl)oxy]cyclobutane-1-carboxylic acid, the title compound was obtained.

### Example 163: trans-3-[(4-chlorobenzyl)oxy]-N-{3-[4-(difluoromethyl)-6-oxo-1,6-dihydropyrimidin-2-yl]-2-fluoro-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(4-chlorobenzyl)oxy]cyclobutane-1-carboxylic acid, the title compound was obtained.

### Example 164: trans-N-{3-[4-(difluoromethyl)-6-oxo-1,6-dihydropyrimidin-2-yl]-2-fluoro-4-(trifluoromethyl)benzyl}-3-{[2-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-{[2-(trifluoromethyl)benzyl]oxy}cyclobutane-1-carboxylic acid, the title compound was obtained.

### Example 165: trans-N-{3-[4-(difluoromethyl)-6-oxo-1,6-dihydropyrimidin-2-yl]-2-fluoro-4-(trifluoromethyl)benzyl}-3-{[3-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-{[3-(trifluoromethyl)benzyl]oxy}cyclobutane-1-carboxylic acid, the title compound was obtained.

### Example 166: trans-N-{3-[4-(difluoromethyl)-6-oxo-1,6-dihydropyrimidin-2-yl]-2-fluoro-4-(trifluoromethyl)benzyl}-3-{[4-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-{[4-(trifluoromethyl)benzyl]oxy}cyclobutane-1-carboxylic acid, the title compound was obtained.

### Example 167: trans-3-[(2,4-difluorobenzyl)oxy]-N-{3-[4-(difluoromethyl)-6-oxo-1,6-dihydropyrimidin-2-yl]-2-fluoro-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(2,4-difluorobenzyl)oxy]cyclobutane-1-carboxylic acid, the title compound was obtained.

### Example 168: trans-3-[(4-chloro-2-fluorobenzyl)oxy]-N-{3-[4-(difluoromethyl)-6-oxo-1,6-dihydropyrimidin-2-yl]-2-fluoro-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(4-chloro-2-fluorobenzyl)oxy]cyclobutane-1-carboxylic acid, the title compound was obtained.

### Example 169: trans-3-[(3,4-difluorobenzyl)oxy]-N-{3-[4-(difluoromethyl)-6-oxo-1,6-dihydropyrimidin-2-yl]-2-fluoro-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(3,4-difluorobenzyl)oxy]cyclobutane-1-carboxylic acid, the title compound was obtained.

### Example 170: trans-3-[(3,5-difluorobenzyl)oxy]-N-{3-[4-(difluoromethyl)-6-oxo-1,6-dihydropyrimidin-2-yl]-2-fluoro-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(3,5-difluorobenzyl)oxy]cyclobutane-1-carboxylic acid, the title compound was obtained.

### Example 171: trans-N-{3-[4-(difluoromethyl)-6-oxo-1,6-dihydropyrimidin-2-yl]-2-fluoro-4-(trifluoromethyl)benzyl}-3-[(3-fluorobenzyl)oxy]cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(3-fluorobenzyl)oxy]cyclobutane-1-carboxylic acid, the title compound was obtained.

### Example 172: trans-3-[(2,3-difluorobenzyl)oxy]-N-{3-[4-(difluoromethyl)-6-oxo-1,6-dihydropyrimidin-2-yl]-2-fluoro-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(2,3-difluorobenzyl)oxy]cyclobutane-1-carboxylic acid, the title compound was obtained.

### Example 173: trans-3-[(2-chloro-4-fluorobenzyl)oxy]-N-{3-[4-(difluoromethyl)-6-oxo-1,6-dihydropyrimidin-2-yl]-2-fluoro-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(2-chloro-4-fluorobenzyl)oxy]cyclobutane-1-carboxylic acid, the title compound was obtained.

### Example 174: trans-3-[(3-chloro-2-fluorobenzyl)oxy]-N-{3-[4-(difluoromethyl)-6-oxo-1,6-dihydropyrimidin-2-yl]-2-fluoro-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(3-chloro-2-fluorobenzyl)oxy]cyclobutane-1-carboxylic acid, the title compound was obtained.

### Example 175: trans-3-[(5-chloro-2-fluorobenzyl)oxy]-N-{3-[4-(difluoromethyl)-6-oxo-1,6-dihydropyrimidin-2-yl]-2-fluoro-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(5-chloro-2-fluorobenzyl)oxy]cyclobutane-1-carboxylic acid, the title compound was obtained.

### Example 176: trans-3-[(3-chloro-4-fluorobenzyl)oxy]-N-{3-[4-(difluoromethyl)-6-oxo-1,6-dihydropyrimidin-2-yl]-2-fluoro-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(3-chloro-4-fluorobenzyl)oxy]cyclobutane-1-carboxylic acid, the title compound was obtained.

### Example 177: trans-3-[(3-chloro-5-fluorobenzyl)oxy]-N-{3-[4-(difluoromethyl)-6-oxo-1,6-dihydropyrimidin-2-yl]-2-fluoro-4-(trifluoromethyl)benzyl } cyclobutane-1-carboxamide

By performing operations similar to those of Example 1 using 2-[3-(aminomethyl)-2-fluoro-6-(trifluoromethyl)phenyl]-6-(difluoromethyl)pyrimidin-4(3H)-one and trans-3-[(3-chloro-5-fluorobenzyl)oxy]cyclobutane-1-carboxylic acid, the title compound was obtained.

### Example 178: trans-3-[(2-chlorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(2-chlorobenzyl)oxy]cyclobutane-1-carboxylic acid, the title compound was obtained.

### Example 179: trans-3-[(3-chlorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(3-chlorobenzyl)oxy]cyclobutane-1-carboxylic acid, the title compound was obtained.

### Example 180: trans-3-[(4-chlorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(4-chlorobenzyl)oxy]cyclobutane-1-carboxylic acid, the title compound was obtained.

### Example 181: trans-N-[2-fluoro-3-(4-methyl-6-oxo-1,6-dihydropyrimidin-2-yl)-4-(trifluoromethyl)benzyl]-3-{[2-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-{[2-(trifluoromethyl)benzyl]oxy}cyclobutane-1-carboxylic acid, the title compound was obtained.

### Example 182: trans-N-[2-fluoro-3-(4-methyl-6-oxo-1,6-dihydropyrimidin-2-yl)-4-(trifluoromethyl)benzyl]-3-{[3-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-{[3-(trifluoromethyl)benzyl]oxy}cyclobutane-1-carboxylic acid, the title compound was obtained.

### Example 183: trans-N-[2-fluoro-3-(4-methyl-6-oxo-1,6-dihydropyrimidin-2-yl)-4-(trifluoromethyl)benzyl]-3-{[4-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-{[4-(trifluoromethyl)benzyl]oxy}cyclobutane-1-carboxylic acid, the title compound was obtained.

### Example 184: trans-3-[(2,4-difluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(2,4-difluorobenzyl)oxy]cyclobutane-1-carboxylic acid, the title compound was obtained.

### Example 185: trans-3-[(4-chloro-2-fluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(4-chloro-2-fluorobenzyl)oxy]cyclobutane-1-carboxylic acid, the title compound was obtained.

### Example 186: trans-3-[(3,4-difluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(3,4-difluorobenzyl)oxy]cyclobutane-1-carboxylic acid, the title compound was obtained.

### Example 187: trans-3-[(3,5-difluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(3,5-difluorobenzyl)oxy]cyclobutane-1-carboxylic acid, the title compound was obtained.

### Example 188: trans-N-[2-fluoro-3-(4-methyl-6-oxo-1,6-dihydropyrimidin-2-yl)-4-(trifluoromethyl)benzyl]-3-[(3-fluorobenzyl)oxy]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(3-fluorobenzyl)oxy]cyclobutane-1-carboxylic acid, the title compound was obtained.

### Example 189: trans-3-[(2,3-difluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(2,3-difluorobenzyl)oxy]cyclobutane-1-carboxylic acid, the title compound was obtained.

### Example 190: trans-3-[(2-chloro-4-fluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(2-chloro-4-fluorobenzyl)oxy]cyclobutane-1-carboxylic acid, the title compound was obtained.

### Example 191: trans-3-[(3-chloro-2-fluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(3-chloro-2-fluorobenzyl)oxy]cyclobutane-1-carboxylic acid, the title compound was obtained.

### Example 192: trans-3-[(5-chloro-2-fluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(5-chloro-2-fluorobenzyl)oxy]cyclobutane-1-carboxylic acid, the title compound was obtained.

### Example 193: trans-3-[(3-chloro-4-fluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(3-chloro-4-fluorobenzyl)oxy]cyclobutane-1-carboxylic acid, the title compound was obtained.

### Example 194: trans-3-[(3-chloro-5-fluorobenzyl)oxy]-N-[2-fluoro-3-(4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-methylpyrimidin-4(3H)-one and trans-3-[(3-chloro-5-fluorobenzyl)oxy]cyclobutane-1-carboxylic acid, the title compound was obtained.

### Example 195: trans-3-[(2-chlorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(2-chlorobenzyl)oxy]cyclobutane-1-carboxylic acid, the title compound was obtained.

### Example 196: trans-3-[(3-chlorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(3-chlorobenzyl)oxy]cyclobutane-1-carboxylic acid, the title compound was obtained.

### Example 197: trans-3-[(4-chlorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(4-chlorobenzyl)oxy]cyclobutane-1-carboxylic acid, the title compound was obtained.

### Example 198: trans-N-[3-(4-ethyl-6-oxo-1,6-dihydropyrimidin-2-yl)-2-fluoro-4-(trifluoromethyl)benzyl]-3-{[2-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-{[2-(trifluoromethyl)benzyl]oxy}cyclobutane-1-carboxylic acid, the title compound was obtained.

### Example 199: trans-N-[3-(4-ethyl-6-oxo-1,6-dihydropyrimidin-2-yl)-2-fluoro-4-(trifluoromethyl)benzyl]-3-{[3-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-{[3-(trifluoromethyl)benzyl]oxy}cyclobutane-1-carboxylic acid, the title compound was obtained.

### Example 200: trans-N-[3-(4-ethyl-6-oxo-1,6-dihydropyrimidin-2-yl)-2-fluoro-4-(trifluoromethyl)benzyl]-3-{[4-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-{[4-(trifluoromethyl)benzyl]oxy}cyclobutane-1-carboxylic acid, the title compound was obtained.

### Example 201: trans-3-[(2,4-difluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(2,4-difluorobenzyl)oxy]cyclobutane-1-carboxylic acid, the title compound was obtained.

### Example 202: trans-3-[(4-chloro-2-fluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(4-chloro-2-fluorobenzyl)oxy]cyclobutane-1-carboxylic acid, the title compound was obtained.

### Example 203: trans-3-[(3,4-difluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(3,4-difluorobenzyl)oxy]cyclobutane-1-carboxylic acid, the title compound was obtained.

### Example 204: trans-3-[(3,5-difluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(3,5-difluorobenzyl)oxy]cyclobutane-1-carboxylic acid, the title compound was obtained.

### Example 205: trans-N-[3-(4-ethyl-6-oxo-1,6-dihydropyrimidin-2-yl)-2-fluoro-4-(trifluoromethyl)benzyl]-3-[(3-fluorobenzyl)oxy]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(3-fluorobenzyl)oxy]cyclobutane-1-carboxylic acid, the title compound was obtained.

### Example 206: trans-3-[(2,3-difluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(2,3-difluorobenzyl)oxy]cyclobutane-1-carboxylic acid, the title compound was obtained.

### Example 207: trans-3-[(2-chloro-4-fluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(2-chloro-4-fluorobenzyl)oxy]cyclobutane-1-carboxylic acid, the title compound was obtained.

### Example 208: trans-3-[(3-chloro-2-fluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(3-chloro-2-fluorobenzyl)oxy]cyclobutane-1-carboxylic acid, the title compound was obtained.

### Example 209: trans-3-[(5-chloro-2-fluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(5-chloro-2-fluorobenzyl)oxy]cyclobutane-1-carboxylic acid, the title compound was obtained.

### Example 210: trans-3-[(3-chloro-4-fluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(3-chloro-4-fluorobenzyl)oxy]cyclobutane-1-carboxylic acid, the title compound was obtained.

### Example 211: trans-3-[(3-chloro-5-fluorobenzyl)oxy]-N-[3-(4-ethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethylpyrimidin-4(3H)-one and trans-3-[(3-chloro-5-fluorobenzyl)oxy]cyclobutane-1-carboxylic acid, the title compound was obtained.

### Example 212: trans-3-[(2-chlorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(2-chlorobenzyl)oxy]cyclobutane-1-carboxylic acid, the title compound was obtained.

### Example 213: trans-3-[(3-chlorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(3-chlorobenzyl)oxy]cyclobutane-1-carboxylic acid, the title compound was obtained.

### Example 214: trans-3-[(4-chlorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(4-chlorobenzyl)oxy]cyclobutane-1-carboxylic acid, the title compound was obtained.

### Example 215: trans-N-[2-fluoro-3-(5-fluoro-4-methyl-6-oxo-1,6-dihydropyrimidin-2-yl)-4-(trifluoromethyl)benzyl]-3-{[2-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-{[2-(trifluoromethyl)benzyl]oxy}cyclobutane-1-carboxylic acid, the title compound was obtained.

### Example 216: trans-N-[2-fluoro-3-(5-fluoro-4-methyl-6-oxo-1,6-dihydropyrimidin-2-yl)-4-(trifluoromethyl)benzyl]-3-{[3-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-{ [3-(trifluoromethyl)benzyl]oxy}cyclobutane-1-carboxylic acid, the title compound was obtained.

### Example 217: trans-N-[2-fluoro-3-(5-fluoro-4-methyl-6-oxo-1,6-dihydropyrimidin-2-yl)-4-(trifluoromethyl)benzyl]-3-{[4-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-{ [4-(trifluoromethyl)benzyl]oxy}cyclobutane-1-carboxylic acid, the title compound was obtained.

### Example 218: trans-3-[(2,4-difluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(2,4-difluorobenzyl)oxy]cyclobutane-1-carboxylic acid, the title compound was obtained.

### Example 219: trans-3-[(4-chloro-2-fluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(4-chloro-2-fluorobenzyl)oxy]cyclobutane-1-carboxylic acid, the title compound was obtained.

### Example 220: trans-3-[(3,4-difluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(3,4-difluorobenzyl)oxy]cyclobutane-1-carboxylic acid, the title compound was obtained.

### Example 221: trans-3-[(3,5-difluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(3,5-difluorobenzyl)oxy]cyclobutane-1-carboxylic acid, the title compound was obtained.

### Example 222: trans-N-[2-fluoro-3-(5-fluoro-4-methyl-6-oxo-1,6-dihydropyrimidin-2-yl)-4-(trifluoromethyl)benzyl]-3-[(3-fluorobenzyl)oxy]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(3-fluorobenzyl)oxy]cyclobutane-1-carboxylic acid, the title compound was obtained.

### Example 223: trans-3-[(2,3-difluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(2,3-difluorobenzyl)oxy]cyclobutane-1-carboxylic acid, the title compound was obtained.

### Example 224: trans-3-[(2-chloro-4-fluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(2-chloro-4-fluorobenzyl)oxy]cyclobutane-1-carboxylic acid, the title compound was obtained.

### Example 225: trans-3-[(3-chloro-2-fluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(3-chloro-2-fluorobenzyl)oxy]cyclobutane-1-carboxylic acid, the title compound was obtained.

### Example 226: trans-3-[(5-chloro-2-fluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(5-chloro-2-fluorobenzyl)oxy]cyclobutane-1-carboxylic acid, the title compound was obtained.

### Example 227: trans-3-[(3-chloro-4-fluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(3-chloro-4-fluorobenzyl)oxy]cyclobutane-1-carboxylic acid, the title compound was obtained.

### Example 228: trans-3-[(3-chloro-5-fluorobenzyl)oxy]-N-[2-fluoro-3-(5-fluoro-4-methyl-6-oxo-1,6-dihydropyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5-fluoro-6-methylpyrimidin-4(3H)-one and trans-3-[(3-chloro-5-fluorobenzyl)oxy]cyclobutane-1-carboxylic acid, the title compound was obtained.

### Example 229: trans-3-[(2-chlorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(2-chlorobenzyl)oxy]cyclobutane-1-carboxylic acid, the title compound was obtained.

### Example 230: trans-3-[(3-chlorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(3-chlorobenzyl)oxy]cyclobutane-1-carboxylic acid, the title compound was obtained.

### Example 231: trans-3-[(4-chlorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(4-chlorobenzyl)oxy]cyclobutane-1-carboxylic acid, the title compound was obtained.

### Example 232: trans-N-[3-(4-ethyl-5-fluoro-6-oxo-1,6-dihydropyrimidin-2-yl)-2-fluoro-4-(trifluoromethyl)benzyl]-3-{[2-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-{[2-(trifluoromethyl)benzyl]oxy}cyclobutane-1-carboxylic acid, the title compound was obtained.

### Example 233: trans-N-[3-(4-ethyl-5-fluoro-6-oxo-1,6-dihydropyrimidin-2-yl)-2-fluoro-4-(trifluoromethyl)benzyl]-3-{[3-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-{[3-(trifluoromethyl)benzyl]oxy}cyclobutane-1-carboxylic acid, the title compound was obtained.

### Example 234: trans-N-[3-(4-ethyl-5-fluoro-6-oxo-1,6-dihydropyrimidin-2-yl)-2-fluoro-4-(trifluoromethyl)benzyl]-3-{[4-(trifluoromethyl)benzyl]oxy}cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-{[4-(trifluoromethyl)benzyl]oxy}cyclobutane-1-carboxylic acid, the title compound was obtained.

### Example 235: trans-3-[(2,4-difluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(2,4-difluorobenzyl)oxy]cyclobutane-1-carboxylic acid, the title compound was obtained.

### Example 236: trans-3-[(4-chloro-2-fluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(4-chloro-2-fluorobenzyl)oxy]cyclobutane-1-carboxylic acid, the title compound was obtained.

### Example 237: trans-3-[(3,4-difluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(3,4-difluorobenzyl)oxy]cyclobutane-1-carboxylic acid, the title compound was obtained.

### Example 238: trans-3-[(3,5-difluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(3,5-difluorobenzyl)oxy]cyclobutane-1-carboxylic acid, the title compound was obtained.

### Example 239: trans-N-[3-(4-ethyl-5-fluoro-6-oxo-1,6-dihydropyrimidin-2-yl)-2-fluoro-4-(trifluoromethyl)benzyl]-3-[(3-fluorobenzyl)oxy]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(3-fluorobenzyl)oxy]cyclobutane-1-carboxylic acid, the title compound was obtained.

### Example 240: trans-3-[(2,3-difluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(2,3-difluorobenzyl)oxy]cyclobutane-1-carboxylic acid, the title compound was obtained.

### Example 241: trans-3-[(2-chloro-4-fluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(2-chloro-4-fluorobenzyl)oxy]cyclobutane-1-carboxylic acid, the title compound was obtained.

### Example 242: trans-3-[(3-chloro-2-fluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(3-chloro-2-fluorobenzyl)oxy]cyclobutane-1-carboxylic acid, the title compound was obtained.

### Example 243: trans-3-[(5-chloro-2-fluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(5-chloro-2-fluorobenzyl)oxy]cyclobutane-1-carboxylic acid, the title compound was obtained.

### Example 244: trans-3-[(3-chloro-4-fluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-6-ethyl-5-fluoropyrimidin-4(3H)-one and trans-3-[(3-chloro-4-fluorobenzyl)oxy]cyclobutane-1-carboxylic acid, the title compound was obtained.

### Example 245: trans-3-[(3-chloro-5-fluorobenzyl)oxy]-N-[3-(4-ethyl-5-fluoro-6-oxo-1,6-dihydropyrimidin-2-yl)-2-fluoro-4-(trifluoromethyl)benzyl]cyclobutane-1-carboxamide

By performing operations similar to those of Example 1 using2-[3-(aminomethyl)-2-fluoro-6-(trifluoromethyl)phenyl]-6-ethyl-5-fluoropyrimidin-4(3H)-one and trans-3-[(3-chloro-5-fluorobenzyl)oxy]cyclobutane-1-carboxylic acid, the title compound was obtained.

### Example 246: trans-3-[(3-chlorobenzyl)oxy]-N-[3-(4,5-dimethyl-6-oxo-1,6-dihydropyrimidin-2-yl)-2-fluoro-4-(trifluoromethyl)benzyl]cyclobutane-1-carboxamide

By performing operations similar to those of Example 1 using 2-[3-(aminomethyl)-2-fluoro-6-(trifluoromethyl)phenyl]-5,6-dimethylpyrimidin-4(3H)-one and trans-3-[(3-chlorobenzyl)oxy]cyclobutane-1-carboxylic acid, the title compound was obtained.

### Example 247: trans-3-[(3-chlorobenzyl)oxy]-N-[2-fluoro-3-(4-oxo-4,5,6,7-tetrahydro-3H-cyclopenta[d]pyrimidin-2-yl)-4-(trifluoromethyl)benzyl]cyclobutane-1-carboxamide

By performing operations similar to those of Example 1 using 2-[3-(aminomethyl)-2-fluoro-6-(trifluoromethyl)phenyl]-3,5,6,7-tetrahydro-4H-cyclopenta[d]pyrimidin-4-one and trans-3-[(3-chlorobenzyl)oxy]cyclobutane-1-carboxylic acid, the title compound was obtained.

### Example 248: N-{2-fluoro-3-[6-oxo-4-(trifluoromethyl)-1,6-dihydropyrimidin-2-yl]-4-(trifluoromethyl)benzyl}-4-{[5-(trifluoromethyl)pyridin-2-yl]oxy}cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 4-{[5-(trifluoromethyl)pyridin-2-yl]oxy}cyclohexane-1-carboxylic acid (Reference Example 89), the title compound was obtained.

### Example 249: trans-N-{2-fluoro-3-[6-oxo-4-(trifluoromethyl)-1,6-dihydropyrimidin-2-yl]-4-(trifluoromethyl)benzyl}-3-{[5-(trifluoromethyl)pyridin-2-yl]oxy}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-{[5-(trifluoromethyl)pyridin-2-yl]oxy}cyclobutane-1-carboxylic acid (Reference Example 91), the title compound was obtained.

### Example 250: cis-N-{2-fluoro-3-[6-oxo-4-(trifluoromethyl)-1,6-dihydropyrimidin-2-yl]-4-(trifluoromethyl)benzyl}-3-{[5-(trifluoromethyl)pyridin-2-yl]oxy}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-{[5-(trifluoromethyl)pyridin-2-yl]oxy}cyclobutane-1-carboxylic acid (Reference Example 93), the title compound was obtained.

### Example 251: trans-3-(2-chlorophenoxy)-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-(2-chlorophenoxy)cyclobutane-1-carboxylic acid, the title compound was obtained.

### Example 252: cis-3-(2-chlorophenoxy)-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-(2-chlorophenoxy)cyclobutane-1-carboxylic acid, the title compound was obtained.

### Example 253: trans-3-(3-chlorophenoxy)-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-(3-chlorophenoxy)cyclobutane-1-carboxylic acid, the title compound was obtained.

### Example 254: cis-3-(3-chlorophenoxy)-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-(3-chlorophenoxy)cyclobutane-1-carboxylic acid, the title compound was obtained.

### Example 255: trans-3-(4-chlorophenoxy)-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-3-(4-chlorophenoxy)cyclobutane-1-carboxylic acid (Reference Example 94), the title compound was obtained.

### Example 256: cis-3-(4-chlorophenoxy)-N-{2-fluoro-3-[6-oxo-4-(trifluoromethyl)-1,6-dihydropyrimidin-2-yl]-4-(trifluoromethyl)benzyl}cyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-(4-chlorophenoxy)cyclobutane-1-carboxylic acid (Reference Example 95), the title compound was obtained.

### Example 257: cis-N-{2-fluoro-3-[6-oxo-4-(trifluoromethyl)-1,6-dihydropyrimidin-2-yl]-4-(trifluoromethyl)benzyl}-3-isobutoxycyclobutane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and cis-3-isobutoxycyclobutane-1-carboxylic acid, the title compound was obtained.

### Example 258: 4-(3,4-dichlorophenoxy)-N-{2-fluoro-3-[6-oxo-4-(trifluoromethyl)-1,6-dihydropyrimidin-2-yl]-4-(trifluoromethyl)benzyl}piperidine-1-carboxamide

To a solution of 4-(3,4-dichlorophenoxy)piperidine·hydrochloride (20 mg) and triethylamine (39 µL) in tetrahydrofuran (5.7 µL), triphosgene (7.3 mg) was added. The reaction mixture was stirred at room temperature for 1 hour, and then 2-[3-(Aminomethyl)-2-fluoro-6-(trifluoromethyl)phenyl]-6-(trifluoromethyl)pyrimidin-4(3H)-one (50 mg) was added to the reaction mixture. The resulting mixture was stirred at room temperature overnight. Aqueous sodium hydrogencarbonate was added to the resulting mixture. The mixture was extracted with ethyl acetate and dried over magnesium sulfate. The solvent was evaporated. The resulting residue was purified by preparative HPLC to obtain the title compound (8 mg).

### Example 259: N-{2-fluoro-3-[6-oxo-4-(trifluoromethyl)-1,6-dihydropyrimidin-2-yl]-4-(trifluoromethyl)benzyl}-4-[2-(trifluoromethyl)phenoxy]piperidine-1-carboxamide

By performing operations similar to those of Example 258 using 2-[3-(aminomethyl)-2-fluoro-6-(trifluoromethyl)phenyl]-6-(trifluoromethyl)pyrimidin-4(3H)-one and 4-[2-(trifluoromethyl)phenoxy]piperidine-1-carboxylic acid, the title compound was obtained.

### Example 260: N-{2-fluoro-3-[6-oxo-4-(trifluoromethyl)-1,6-dihydropyrimidin-2-yl]-4-(trifluoromethyl)benzyl}-4-[4-(trifluoromethyl)phenoxy]piperidine-1-carboxamide

By performing operations similar to those of Example 258 using 2-[3-(aminomethyl)-2-fluoro-6-(trifluoromethyl)phenyl]-6-(trifluoromethyl)pyrimidin-4(3H)-one and 4-[4-(trifluoromethyl)phenoxy]piperidine-1-carboxylic acid, the title compound was obtained.

### Example 261: N-{2-fluoro-3-[6-oxo-4-(trifluoromethyl)-1,6-dihydropyrimidin-2-yl]-4-(trifluoromethyl)benzyl}-3-{[3-(trifluoromethyl)benzyl]oxy}azetidine-1-carboxamide

By performing operations similar to those of Example 258 using 2-[3-(aminomethyl)-2-fluoro-6-(trifluoromethyl)phenyl]-6-(trifluoromethyl)pyrimidin-4(3H)-one and 3-{[3-(trifluoromethyl)benzyl]oxy}azetidine-1-carboxylic acid, the title compound was obtained.

### Example 262: N-{2-fluoro-3-[6-oxo-4-(trifluoromethyl)-1,6-dihydropyrimidin-2-yl]-4-(trifluoromethyl)benzyl}-3-{[3-(trifluoromethyl)phenoxy]methyl}azetidine-1-carboxamide

By performing operations similar to those of Example 258 using 2-[3-(aminomethyl)-2-fluoro-6-(trifluoromethyl)phenyl]-6-(trifluoromethyl)pyrimidin-4(3H)-one and 3-{[3-(trifluoromethyl)phenoxy]methyl}azetidine-1-carboxylic acid, the title compound was obtained.

### Example 263: N-(2-fluoro-3-{6-oxo-4-[5-(trifluoromethyl)pyridin-2-yl]-1,6-dihydropyrimidin-2-yl}-4-(trifluoromethyl)benzyl)benzamide

By performing operations similar to those of Example 1 using 2-[3-(aminomethyl)-2-fluoro-6-(trifluoromethyl)phenyl]-6-[5-(trifluoromethyl)pyridin-2-yl]pyrimidin-4(3H)-one and benzoic acid, the title compound was obtained.

### Example 264: N-(2-fluoro-3-{6-oxo-4-[5-(trifluoromethyl)pyridin-2-yl]-1,6-dihydropyrimidin-2-yl}-4-(trifluoromethyl)benzyl)picolinamide

By performing operations similar to those of Example 1 using 2-[3-(aminomethyl)-2-fluoro-6-(trifluoromethyl)phenyl]-6-[5-(trifluoromethyl)pyridin-2-yl]pyrimidin-4(3H)-one and 2-pyridine carboxylic acid, the title compound was obtained.

### Example 265: N-(2-fluoro-3-{6-oxo-4-[5-(trifluoromethyl)pyridin-2-yl]-1,6-dihydropyrimidin-2-yl}-4-(trifluoromethyl)benzyl)-[1,1'-biphenyl]-4-carboxamide

By performing operations similar to those of Example 1 using 2-[3-(aminomethyl)-2-fluoro-6-(trifluoromethyl)phenyl]-6-[5-(trifluoromethyl)pyridin-2-yl]pyrimidin-4(3H)-one and 4-biphenylcarboxylic acid, the title compound was obtained.

### Example 266: 3-(benzyloxy)-N-(2-fluoro-3-{6-oxo-4-[5-(trifluoromethyl)pyridin-2-yl]-1,6-dihydropyrimidin-2-yl }-4-(trifluoromethyl)benzyl)cyclobutane-1-carboxamide

By performing operations similar to those of Example 1 using 2-[3-(aminomethyl)-2-fluoro-6-(trifluoromethyl)phenyl]-6-[5-(trifluoromethyl)pyridin-2-yl]pyrimidin-4(3H)-one and 3-(benzyloxy)cyclobutane-1-carboxylic acid, the title compound was obtained.

### Example 267: N-(2-fluoro-3-{6-oxo-4-[5-(trifluoromethyl)pyridin-2-yl]-1,6-dihydropyrimidin-2-yl}-4-(trifluoromethyl)benzyl)-1-(imidazo[1,2-b]pyridazin-6-yl)piperidine-4-carboxamide

By performing operations similar to those of Example 1 using 2-[3-(aminomethyl)-2-fluoro-6-(trifluoromethyl)phenyl]-6-[5-(trifluoromethyl)pyridin-2-yl]pyrimidin-4(3H)-one and 1-(imidazo[1,2-b]pyridazin-6-yl)piperidine-4-carboxylic acid, the title compound was obtained.

### Example 268: N-(2-fluoro-3-{6-oxo-4-[5-(trifluoromethyl)pyridin-2-yl]-1,6-dihydropyrimidin-2-yl}-4-(trifluoromethyl)benzyl)benzenesulfonamide

By performing operations similar to those of Example 272 using 2-[3-(aminomethyl)-2-fluoro-6-(trifluoromethyl)phenyl]-6-[5-(trifluoromethyl)pyridin-2-yl]pyrimidin-4(3H)-one and benzenesulfonyl chloride, the title compound was obtained.

### Example 269: N-(2-fluoro-3-{6-oxo-4-[5-(trifluoromethyl)pyridin-2-yl]-1,6-dihydropyrimidin-2-yl}-4-(trifluoromethyl)benzyl)-2-(trifluoromethyl)benzenesulfonamide

By performing operations similar to those of Example 272 using 2-[3-(aminomethyl)-2-fluoro-6-(trifluoromethyl)phenyl]-6-[5-(trifluoromethyl)pyridin-2-yl]pyrimidin-4(3H)-one and 2-(trifluoromethyl)benzenesulfonyl chloride, the title compound was obtained.

### Example 270: N-(2-fluoro-3-{6-oxo-4-[5-(trifluoromethyl)pyridin-2-yl]-1,6-dihydropyrimidin-2-yl}-4-(trifluoromethyl)benzyl)-4-(trifluoromethyl)benzenesulfonamide

By performing operations similar to those of Example 272 using 2-[3-(aminomethyl)-2-fluoro-6-(trifluoromethyl)phenyl]-6-[5-(trifluoromethyl)pyridin-2-yl]pyrimidin-4(3H)-one and 4-(trifluoromethyl)benzenesulfonyl chloride, the title compound was obtained.

### Example 271: N-(2-fluoro-3-{6-oxo-4-[5-(trifluoromethyl)pyridin-2-yl]-1,6-dihydropyrimidin-2-yl}-4-(trifluoromethyl)benzyl)-[1,1'-biphenyl]-4-sulfonamide

By performing operations similar to those of Example 272 using 2-[3-(aminomethyl)-2-fluoro-6-(trifluoromethyl)phenyl]-6-[5-(trifluoromethyl)pyridin-2-yl]pyrimidin-4(3H)-one and 4-biphenylsulfonyl chloride, the title compound was obtained.

### Example 272: N-{2-fluoro-3-[6-oxo-4-(trifluoromethyl)-1,6-dihydropyrimidin-2-yl]-4-(trifluoromethyl)benzyl}-2-(trifluoromethyl)benzenesulfonamide

To a solution of 2-[3-(aminomethyl)-2-fluoro-6-(trifluoromethyl)phenyl]-6-(trifluoromethyl)pyrimidin-4(3H)-one·2,2,2-trifluoroacetate (99.7 mg) in N,N-dimethylformamide (2 mL), triethylamine (237 µL) and 2-(trifluoromethyl)benzenesulfonyl chloride (49 µL) were added under ice cooling. The reaction mixture was stirred at room temperature overnight. Saturated aqueous ammonium chloride was added to the resulting mixture. The mixture was extracted three times with ethyl acetate. The combined organic layers were washed with water and saturated brine. The organic layer was dried over magnesium sulfate, and the solvent was evaporated under reduced pressure. The resulting residue was purified by silica gel column chromatography to obtain the title compound (5.8 mg).

### Example 273: N-{2-fluoro-3-[6-oxo-4-(trifluoromethyl)-1,6-dihydropyrimidin-2-yl]-4-(trifluoromethyl)benzyl}-2-(trifluoromethyl)benzamide

By performing operations similar to those of Example 272 using 2-[3-(aminomethyl)-2-fluoro-6-(trifluoromethyl)phenyl]-6-(trifluoromethyl)pyrimidin-4(3H)-one·2,2,2-trifluoroacetate and 2-(trifluoromethyl)benzoyl chloride, the title compound was obtained.

### Example 274: 2,3-dichloro-N-{2-fluoro-3-[6-oxo-4-(trifluoromethyl)-1,6-dihydropyrimidin-2-yl]-4-(trifluoromethyl)benzyl}benzamide

By performing operations similar to those of Example 1 using 2-[3-(aminomethyl)-2-fluoro-6-(trifluoromethyl)phenyl]-6-(trifluoromethyl)pyrimidin-4(3H)-one and 2,3-dichlorobenzoic acid, the title compound was obtained.

### Example 275: N-{2-fluoro-3-[6-oxo-4-(trifluoromethyl)-1,6-dihydropyrimidin-2-yl]-4-(trifluoromethyl)benzyl }-1-[2-(4-fluorophenyl)acetyl]piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[2-(4-fluorophenyl)acetyl]piperidine-4-carboxylic acid, the title compound was obtained.

### Example 276: N-{2-fluoro-3-[6-oxo-4-(trifluoromethyl)-1,6-dihydropyrimidin-2-yl]-4-(trifluoromethyl)benzyl }-1-[2-(4-fluorophenyl)acetyl]azetidine-3-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-[2-(4-fluorophenyl)acetyl]azetidine-3-carboxylic acid, the title compound was obtained.

### Example 277: 1-benzyl-N-{2-fluoro-3-[6-oxo-4-(trifluoromethyl)-1,6-dihydropyrimidin-2-yl]-4-(trifluoromethyl)benzyl}piperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-benzylpiperidine-4-carboxylic acid, the title compound was obtained.

### Example 278: N-{2-fluoro-3-[6-oxo-4-(trifluoromethyl)-1,6-dihydropyrimidin-2-yl]-4-(trifluoromethyl)benzyl }-1-phenethylpiperidine-4-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-phenethylpiperidine-4-carboxylic acid, the title compound was obtained.

### Example 279: trans-4-(4-chlorophenyl)-N-{2-fluoro-3-[6-oxo-4-(trifluoromethyl)-1,6-dihydropyrimidin-2-yl]-4-(trifluoromethyl)benzyl}cyclohexane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and trans-4-(4-chlorophenyl)cyclohexane-1-carboxylic acid, the title compound was obtained.

### Example 280: N-{2-fluoro-3-[6-oxo-4-(trifluoromethyl)-1,6-dihydropyrimidin-2-yl]-4-(trifluoromethyl)benzyl}-2,3-dihydro-1H-indene-2-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and indan-2-carboxylic acid, the title compound was obtained.

### Example 281: 1-(4-chlorophenyl)-N-{2-fluoro-3-[6-oxo-4-(trifluoromethyl)-1,6-dihydropyrimidin-2-yl]-4-(trifluoromethyl)benzyl}cyclopentane-1-carboxamide

By performing operations similar to those of Example 1 using 2-[3-(aminomethyl)-2-fluoro-6-(trifluoromethyl)phenyl]-6-(trifluoromethyl)pyrimidin-4(3H)-one and 1-(4-chlorophenyl)cyclopentane-1-carboxylic acid, the title compound was obtained.

### Example 282: 2-(4-chlorophenyl)-N-{2-fluoro-3-[6-oxo-4-(trifluoromethyl)-1,6-dihydropyrimidin-2-yl]-4-(trifluoromethyl)benzyl}-2-methylpropanamide

By performing operations similar to those of Example 1 using 2-[3-(aminomethyl)-2-fluoro-6-(trifluoromethyl)phenyl]-6-(trifluoromethyl)pyrimidin-4(3H)-one and 2-(4-chlorophenyl)-2-methylpropionic acid, the title compound was obtained.

### Example 283: N-{2-fluoro-3-[6-oxo-4-(trifluoromethyl)-1,6-dihydropyrimidin-2-yl]-4-(trifluoromethyl)benzyl 1-2-[4-(trifluoromethyl)phenyl]acetamide

By performing operations similar to those of Example 1 using 2-[3-(aminomethyl)-2-fluoro-6-(trifluoromethyl)phenyl]-6-(trifluoromethyl)pyrimidin-4(3H)-one and 4-(trifluoromethyl)phenylacetic acid, the title compound was obtained.

The chemical structural formulas and NMR spectrum data of the compounds obtained in the Reference Examples and Examples are shown below.

**[Table 1]**

| **Reference example No.** | **Chemical Structure** | **NMR (¹H-NMR)** | **MS (m/z)** |
|---|---|---|---|
| 1 | 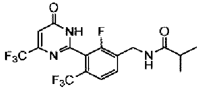 | DMSO-d₆,δ: 1.05 (6H, d. J = 7.2 Hz), 2.42-2.54 (1H, m), 4.40 (2H, d, J = 5.6 Hz), 7.06 (1H, s), 7.67 (1H, t, J = 8.0 Hz), 7.79 (1H, d, J = 8.0 Hz), 8.47 (1H, t, J = 5.6 Hz), 13.90 (1H, brs) | 425(M⁺) |
| 2 | 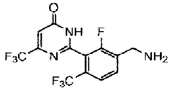 | DMSO-d₆,δ:4.06 (2H, s), 6.18 (1H, s), 7.26 (2H, brs), 7.61-7.70 (2H, m) | 355(M⁺) |
| 3 | 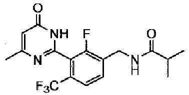 | DMSO-d₆,δ: 1.05 (6H, d, J = 6.8 Hz), 2.23 (3H, s), 2.42-2.51 (1H, m), 4.38 (2H, d, J = 6.0 Hz), 6.28 (1H, brs), 7.61 (1H, t, J = 8.0 Hz), 7.73 (1H, d, J = 8.0 Hz), 8.46 (1H, t, J = 6.0 Hz), 12.98 (1H, brs) | 371(M⁺) |
| 4 | 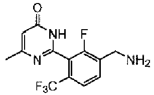 | DMSO-d₆,δ:2.19 (3H, s), 3.84 (2H, s), 6.20 (1H, s), 7.70 (1H, d, J = 8.4 Hz), 7.85 (1H, t, J = 8.0 Hz) | 301(M⁺) |
| 5 | 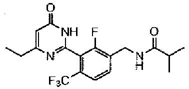 | DMSO-d₆,δ:1.05 (6H, d, J = 6.8 Hz), 1.13 (3H, t, J = 7.6 Hz), 2.41-2.54 (3H, m), 4.38 (2H, d, J = 6.0 Hz), 6.24 (1H, brs), 7.61 (1H, t, J = 7.6 Hz), 7.74 (1H, d, J = 8.4 Hz), 8.46 (1H, t, J = 6.0 Hz), 12.97 (1H, brs) | 385(M⁺) |
| 6 | 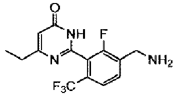 | DMSO-d₆,δ: 1.13 (3H, t, J = 7.6 Hz), 2.44-2.53 (2H, m), 3.86 (2H, s), 5.67 (2H, brs), 8.24 (1H, s), 7.72 (1H, d, J = 8.0 Hz), 7.88 (1H, t, J = 7.6 Hz) | 315(M⁺) |
| 7 | 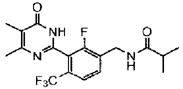 | DMSO-d₆,δ:1.05 (6H, d, J = 6.8 Hz), 1.98 (3H, s), 2.24 (3H, s), 2.42-2.54 (1H, m), 4.37 (2H, d, J = 6.0 Hz), 7.60 (1H, t, J = 8.0 Hz), 7.72 (1H, d, J = 8.4 Hz), 8.45 (1H, t, J = 6.0 Hz), 12.90 (1H, brs) | 385(M⁺) |
| 8 | 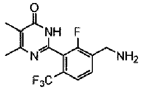 | DMSO-d₆,δ: 1.98 (3H, s), 2.24 (3H, s), 3.85 (2H, s), 5.74 (2H, brs), 7.71 (1H, d, J = 8.4 Hz), 7.87 (1H, t, J = 8.0 Hz) | 315(M⁺) |
| 9 | 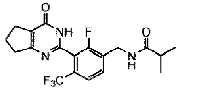 | DMSO-d₆,δ:1.05 (6H, d, J = 6.8 Hz), 2.02 (2H, quin, J = 7.6 Hz), 2.47 (1H, sept, J = 6.8 Hz), 2.71 (2H, t, J = 7.6 Hz), 2.81 (2H, t, J = 7.6 Hz), 4.37 (2H, d, J = 5.6 Hz), 7.60 (1H, t, J = 8.0 Hz), 7.73 (1H, d, J = 8.0 Hz), 8.45 (1H, t, J = 5.6 Hz), 12.89 (1H, brs) | 397(M⁺) |
| 10 | 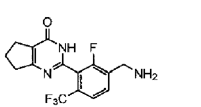 | DMSO-d₆,δ: 2.01 (2H, quin, J = 7.6 Hz), 2.70 (2H, t, J = 7.6 Hz), 2.80 (2H, t, J = 7.6 Hz), 3.84 (2H, s), 7.71 (1H, d, J = 7.6 Hz), 7.87 (1H, t, J = 7.6 Hz) | 327(M⁺) |

**[Table 2]**

| **Reference example No.** | **Chemical Structure** | **NMR (¹H-NMR)** | **MS (m/z)** |
|---|---|---|---|
| 11 | 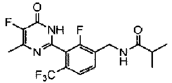 | DMSO-d₆,δ: 1.05 (6H, d, J = 7.2 Hz), 2.26 (3H, d, J = 3.6 Hz), 2.42-2.52 (1H, m), 4.38 (2H, d, J = 6.0 Hz), 7.63 (1H, t, J = 8.0 Hz), 7.75 (1H, d, J = 8.4 Hz), 8.46 (1H, t, J = 6.0 Hz), 13.61 (1H, brs) | 389(M⁺) |
| 12 | 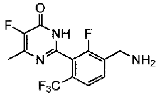 | DMSO-d₆,δ:2.13 (3H, d, J = 2.8 Hz), 4.04 (2H, s), 7.61 (1H, d, J = B.0 Hz), 7.67 (1H, t, J = 8.0 Hz) | 319(M⁺) |
| 13 | 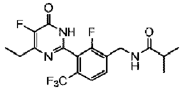 | DMSO-d₆,δ: 1.05 (6H, d, J = 6.8 Hz), 1.14 (3H, t,. J = 7.2 Hz), 2.42-2.53 (1H, m), 2.56-2.64 (2H, m), 4.38 (2H, d, J = 6.0 Hz), 7.63 (1H, t, J = 7.6 Hz), 7.75 (1H, d, J = 7.6 Hz), 8.46 (tH, t, J = 6.0 Hz), 13.62 (1H, brs) | 403(M⁺) |
| 14 | 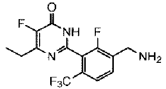 | DMSO-d₆,δ: 1.11 (3H, t, J = 8.0 Hz), 2.49-2.54 (2H, m), 4.15 (2H, s), 7.63-7.68 (2H, m), 8.18 (2H, brs) | 333(M⁺) |
| 15 | 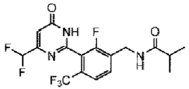 | DMSO-d₆,δ: 1.05 (6H, d, J = 6.8 Hz), 2.42-2.52 (1H, m), 4.39 (2H, d, J = 5.2 Hz), 6.75 (1H, brs), 8.80 (1H, t, J = 54.4 Hz), 7.65 (1H, t, J = 7.6 Hz), 7.77 (1H, d, J = 8.4 Hz), 8.48 (1H, t, J = 5.6 Hz), 13.57 (1H, brs) | 407(M⁺) |
| 16 | 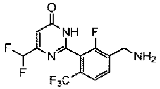 | DMSO-d₆,δ:4.08 (2H, s), 6.32 (1H, s), 8.80 (1H, t, J = 54.4 Hz), 7.66-7.73 (2H, m) | 337(M⁺) |
| 17 | 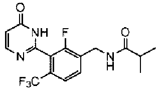 | DMSO-d₆,δ: 1.05 (6H, d, J = 6.8 Hz), 2.44-2.54 (1H, m), 4.38 (2H, d, J = 5.6 Hz), 6.44 (1H, brs), 7.62 (1H, t, J = 8.0 Hz), 7.74 (1H, d, J = 8.0 Hz), 8.07 (1H, brs), 8.46 (1H, t, J = 5.6 Hz), 13.16 (1H, brs) | 357(M⁺) |
| 18 | 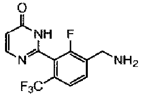 | DMSO-d₆,δ:3.85 (2H, s), 5.68 (2H, brs), 6.26 (1H, d, J = 6.4 Hz), 7.68 (1 H, d, J = 8.4 Hz), 7.83 (1H, t, J = 8.0 Hz), 7.96 (1H, d, J = 6.4 Hz) | 287(M⁺) |
| 19 | 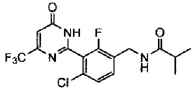 | DMSO-d₆,δ: 1.03 (6H, d, J = 6.8 Hz), 2.39-2.49 (1H, m), 4.31 (2H, d, J = 6.0 Hz), 7.04 (1H, s), 7.48-7.52 (2H, m), 8.39 (1H, t, J = 6.0 Hz), 13.83 (1H, brs) | 391(M⁺) |
| 20 | 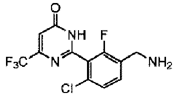 | DMSO-d₆,δ:3.84 (2H, s), 6.09 (1H, s), 6.54 (2H, brs), 7.22-7.33 (1H, m), 7.44-7.52 (1H, m) | 320(M⁺) |

**[Table 3]**

| **Reference example No.** | **Chemical Structure** | **NMR** (**¹H-NMR)** | **MS (m/z)** |
|---|---|---|---|
| 21 | 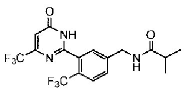 | DMSO-d₆,δ: 1.05 (6H, d, J = 7.2 Hz), 2.40-2.52 (1H, m), 4.39 (2H, d, J = 6.0 Hz), 3.96 (1H, s), 7.60-7.63 (2H, m), 7.88 (1H, d₁ J = 8.8 Hz), 8.42 (1H, t, J = 5.6 Hz), 13.62 (1H, brs) | 407(M⁺) |
| 23 | 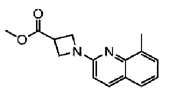 | DMSO-d₆,δ:2.54 (3H, s), 3.65-3.73 (1H, m), 3.69 (3H, s), 4.16 (2H, dd, J = 8.4, 6.0 Hz), 4.29 (2H, t, J = 8.4 Hz), 8.77 (1H, d, J - 8.8 Hz), 7.13 (1H, t, J = 7.6 Hz), 7.42 1H, d, J = 8.4 Hz), 7.56 (1H, d, J = 8.0 Hz), 8.03 (1H, d, J = 8.8 Hz) | 256(M⁺) |
| 24 | 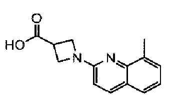 | DMSO-d₆,δ:2.55 (3H, s), 3.54-3.61 (1H, m), 4.14 (2H, dd, J = 8.4, 6.4 Hz), 4.27 (2H, t, J = 8.4 Hz), 6.76 (1H, d, J = 8.8 Hz), 7.13 (1H, t, J = 8.0 Hz), 7.42 (1H, d, J = 6.8 Hz), 7.55 (1H, d, J = 8.0 Hz), 8.02 (1H, d, J = 8.8 Hz), 12.73 (1H, brs) | 242(M⁺) |
| 26 | 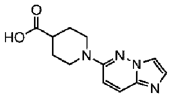 | DMSO-d₆,δ:1.55-1.65 (2H, m), 1.90 (2H, id, J = 13.2, 3.2 Hz), 2.50-2.55 (1H, m), 2.97-3.04 (2H, m), 4.05 (2H, dt, J = 13.2, 3.2 Hz), 7.16 (1H, d, J = 10.0 Hz), 7.47 (1H, d, J = 3.2 Hz), 7.81 (1H, d, J = 10.0 Hz), 7.89 (1H, s), 12.30 (1H, br) | 246(M⁺) |
| 28 | 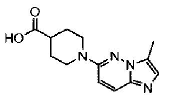 | DMSO-d₆,δ:1.56-1.66 (2H, m), 1.90-1.94 2H, m), 2.38 (3H, s), 2.50-2.55 (1H, m), 2.97-3.04 (2H, m), 4.08-4.12 (2H, m), 7.11 (1H, d, J = 10.0 Hz), 7.30 (1H, s), 7.77 (1H, 1H, d, J = 10.0 Hz), 12.32 (1H, br) | 260(M⁺) |
| 29 | 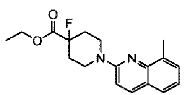 | CDCI₃,δ:1.30 (3H, t, J = 7.2 Hz), 2.01-2.28 (4H, m), 2.65 (3H, s), 3.42 (2H, td, J = 12.4, 2.8 Hz), 4.24 (2H, q, J = 7.2 Hz), 4.47 (2H, dt, J = 13.2, 2.4 Hz), 7.02 (1H, d, J = 9.6 Hz), 7.15 (1H, t, J = 8.0 Hz), 7.42 (1H, d, J = 6.8 Hz), 7.47 (1H, d, J = 7.6 Hz), 7.90 (1H, d, J = 8.8 Hz) | 316(M⁺) |
| 30 | 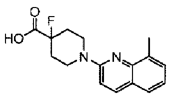 | DMSO-d₆,δ: 1.93-2.12 (4H, m), 2.56 (3H, s), 3.24-3.32 (2H, m), 4.46 (2H, d, J = 13.2 Hz), 7.14 (1H, t, J = 7.6 Hz), 7.31 (1H, d, J = 9.6 Hz), 7.42 (1H, d, J = 6.8 Hz), 7.55 (1H, d, J = 7.6 Hz), 8.04 (1H, d, J = 9.2 Hz), 13.43 (1H, brs) | 288(M⁺) |
| 31 | 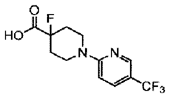 | DMSO-d₆,δ: 1.84-2.05 (4H, m), 3.21-3.28 2H, m), 4.28-4.36 (2H, m), 7.05 (1H, d, J = 9.2 Hz), 7.82 (1H, dd, J = 9.2, 2.8 Hz), 8.43 (1H, s), 13.47 (1H, brs) | 292(M⁺) |
| 32 | 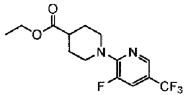 | CDCl₃,δ:1.27 (3H, t, J = 7.6 Hz), 1.82 (2H, qd, J = 11.2, 4.0 Hz), 2.00 (2H, dd, J = 13.2, 4.0 Hz), 2.58 (1H, tt, J = 11.2, 4.0 Hz), 3.09 (2H, td, J = 13.2, 2.4 Hz), 4.16 2H, q, J = 7.2 Hz), 4.23 (2H, dt, J = 13.6, 3.2 Hz), 7.37 (1H, dd, J = 13.2, 2.0 Hz), 8.22 (1H, s) | 320(M⁺) |
| 33 | 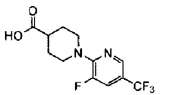 | DMSO-d₆,δ:1.60 (2H, qd, J = 11.2, 4.0 Hz), 1.90 (2H, dd, J = 13.2, 3.2 Hz), 2.52-2.60 (1H, m), 3.13 (2H, td, J = 11.2, 2.4 Hz), 4.13 (2H, dt, J = 13.2, 3.2 Hz), 7.90 1H, dd, J = 13.6, 2.0 Hz), 8.33 (1H, t, J = 1.2 Hz), 12.29 (1H, brs) | 292(M⁺) |

**[Table 4]**

| **Reference example No.** | **Chemical Structure** | **NMR** (**¹H-NMR)** | **MS (m/z)** |
|---|---|---|---|
| 34 | 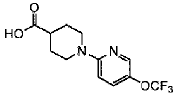 | DMSO-d₆,δ:1.45-1.57 (2H, m), 1.82-1.90 (2H, m), 2.46-2.55 (1H, m), 2.97 (2H, td, J = 13.2, 2.4 Hz), 4.17 (2H, dt, J = 13.2, 3.6 Hz), 8.91 (1H, d, J = 9.2 Hz), 7.55-7.59 (1H, m), 8.13 (1H, d, J = 2.8 Hz), 1227 (1H, brs) | 290(M⁺) |
| 36 | 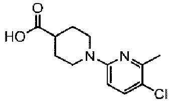 | DMSO-d₆,δ:1.44-1.56 (2H, m), 1.85 (2H, dd, J = 13.6, 3.6 Hz), 2.36 (3H, s), 2.44-2.52 (1H, m), 2.86-2.94 (2H, m), 4.15 (2H, dt, J = 13.6, 3.6 Hz), 6.69 (1H, d, J = 8.8 Hz), 7.48 (1H, d, J = 8.4 Hz), 12.24 (1H, brs) | 254(M⁺) |
| 37 | 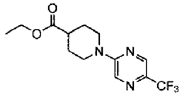 | DMDMSO-d₆,δ:1.19 (3H, t, J = 7.1 Hz), 1.51-1.82 (2H, m), 1.89-1.99 (2H, m), 2.66-2.74 (1H, m), 3.12-3.31 (2H, m), 4.02-4.11 (2H, m), 4.36 (2H, dt, J = 13.6, 3.5 Hz), 8.44 (1H, d, J = 1.5 Hz), 8.47 (1H, d, J = 0.7 Hz) | 304 [M+H]⁺ |
| 38 | 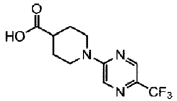 | DMSO-d₆,δ: 1.49-1.62 (2H, m), 1.88-1.97 (2H, m), 2.60 (1H, tt, J = 10.8, 4.0 Hz), 3.12-3.22 (2H, m), 4.34 (2H, dt, J = 13.5, 3.5 Hz), 8.43 (1H, s), 8.46 (1H, s), 12.32 (1H, brs) | 276 [M+H]⁺ |
| 39 | 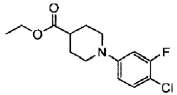 | CDCI₃,δ:1.27 (3H, t, J = 7.2 Hz), 1.84 (2H, qd, J = 11.2, 3.6 Hz), 1.98-2.05 (2H, m), 2.45 (1H, tt, J = 11.6, 4.0 Hz), 2.81 (2H, td, J = 12.4, 2.8 Hz), 3.59 (2H, dt, J = 13.2, 3.6 Hz), 4.16 (2H, q, J = 6.8 Hz), 6.61 (1H, dd, J = 8.8. 2.8 Hz), 6.66 (1H, dd, J = 12.4, 2.8 Hz), 7.20 (1H, t, J = 8.8 Hz) | 285(M⁺) |
| 40 | 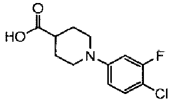 | DMSO-d₆,δ: 1.58 (2H, qd, J = 11.6, 4.0 Hz), 1.86 (2H, dd, J = 13.2, 3.6 Hz), 2.43 (1H, tt, J = 11.2, 4.0 Hz), 2.81 (2H, td, J = 12.8, 2.8 Hz), 3.67 (2H, dt, J = 12.8, 3.2 Hz), 8.77 (1H, dd, J = 8.8, 2.4 Hz), 6.94 (1H, dd, J = 13.2, 2.8 Hz), 7.30 (1H, t, J = 8.8 Hz), 12.26 (1H, brs) | 257(M⁺) |
| 41 | 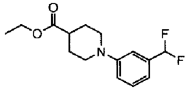 | CDCI₃,δ:1.27 (3H, t, J = 7.2 Hz), 1.86 (2H, qd, J = 11.2, 4.0 Hz), 2.04 (2H, dd, J = 13.2, 2.8 Hz), 2.45 (1H, tt, J = 11.2, 4.0 Hz), 2.83 (2H, td, J = 12.0, 2.8 Hz), 3.68 (2H, dt, J = 12.4, 3.2 Hz), 4.16 (2H, q, J = 7.2 Hz), 8.58 (1H, t, J = 58.8 Hz), 8.94 (1H, d, J = 7.6 Hz), 7.00-7.04 (2H, m), 7.31 (1H, t, J = 8.0 Hz) | 283(M⁺) |
| 42 | 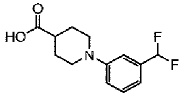 | DMSO-d₆,δ:1.62 (2H, qd, J = 12.0, 4.0 Hz), 1.90 (2H, dd, J = 13.2, 3.2 Hz), 2.38-2.46 (1H, m), 2.80 (2H, td, J = 12.0, 2.4 Hz), 3.68 (2H, dt, J = 12.8, 3.2 Hz), 6.91 (1H, t, J = 56.0 Hz), 6,92 (1H, d, J = 8.8 Hz), 7.08-7.11 (2H, m), 7.32 (1H, t, J = 8.0 Hz), 12.27 (1H, brs) | 255(M⁺) |
| 43 | 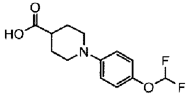 | DMSO-d₆,δ:1.62 (2H, qd, J = 11.2, 3.6 Hz), 1.89 (2H, dd, J = 13.8, 3.2 Hz), 2.38 (1H, tt, J = 11.2, 4.0 Hz), 2.72 (2H, td, J = 12.0, 2.4 Hz), 3.57 (2H, dt, J = 12.4, 3.2 Hz), 6.95 (2H, d, J = 9.8 Hz), 7.02 (2H, d, J = 9.6 Hz), 7.05 (1H, t, J = 75.2 Hz), 12.26 (1H, brs) | 271(M⁺) |
| 45 | 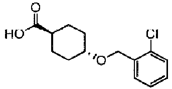 | DMSO-d₆,δ: 1.21-1.42 (4H, m), 1.91 (2H, dd, J = 13.6, 2.8 Hz), 2.05 (2H, dd, J = 13.2, 3.2 Hz), 2.19 (1H, tt, J = 11.2, 4.0 Hz), 3.32-3.41 (1H, m), 4.56 (2H, s), 7.29-7.37 (2H, m), 7.43 (1H, dd, J = 7.6, 1.6 Hz), 7.50 (1H, dd, J = 7.2, 2.0 Hz), 12.08 (1H, brs) | 268(M⁺) |

**[Table 5]**

| **Reference example No.** | **Chemical Structure** | **NMR** (**¹H-NMR)** | **MS (m/z)** |
|---|---|---|---|
| 49 | 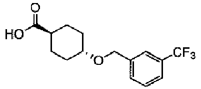 | DMSO-d₆,δ:1.20-1.41 (4H, m), 1.91 (2H, dd, J = 13.2, 2.0 Hz), 2.04 (2H, dd, J = 12.8, 2.4 Hz), 2.19 (1H, tt, J = 11.2, 3.6 Hz), 3.31-3.38 (1H, m), 4.60 (2H, s), 7.55-7.66 (4H, m), 12.08 (1H, brs) | 302(M⁺) |
| 51 | 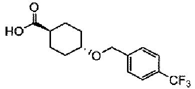 | DMSO-d₆,δ:1.19-1.41 (4H, m), 1.91 (2H, dd, J = 12.8, 3.2 Hz), 2.04 (2H, dd, J = 12.4, 2.4 Hz), 2.18 (1H, tt, J = 11.2, 4.0 Hz), 3.30-3.38 (1H, m), 4.61 (2H, s), 7.53 (2H, d, J = 8.0 Hz), 7.70 (2H, d, J = 8.0 Hz), 12.07 (1H, brs) | 302(M⁺) |
| 53 | 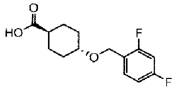 | DMSO-d₆,δ:1.21 (2H, qd, J = 12.8, 3.2 Hz), 1.34 (2H, qd, J = 13.2, 3.2 Hz), 1.89 (2H, dd, J = 13.6, 2.4 Hz), 2.01 (2H, dd, J = 12.8, 3.6 Hz), 2.17 (1H, tt, J = 11.6, 3.6 Hz), 3.29-3.36 (1H, m), 4.50 (2H, s), 7.07 (1H, tdd, J = 8.8, 2.4, 1.2 Hz), 7.21 (1H, td, J = 9.8, 2.4 Hz), 7.47 (1H, td, J = 8.8, 6.8 Hz), 12.08 (1H, brs) | 270(M⁺) |
| 55 | 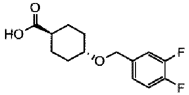 | DMSO-d₆,δ:1.23 (2H, qd, J = 12.8, 2.8 Hz), 1.34 (2H, qd, J = 13.2, 3.2 Hz), 1.90 (2H, dd, J = 12.8, 3.2 Hz), 2.01 (2H, dd, J = 12.8, 2.8 Hz), 2.17 (1H, tt, J = 12.0, 4.0 Hz), 3.27-3.33 (1H, m), 4.48 (2H, s), 7.14-7.18 (1H, m), 7.31-7.43 (2H, m), 12.08 (1H, brs) | 270(M⁺) |
| 57 | 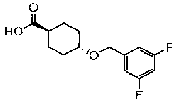 | DMSO-d₆,δ:1.24 (2H, qd, J = 12.8, 2.8 Hz), 1.35 (2H, qd, J = 12.8, 2.8 Hz), 1.90 (2H, dd, J = 13.6, 3.2 Hz), 2.02 (2H, dd, J = 12.8, 3.2 Hz), 2.18 (1H, tt, J = 11.2, 3.6 Hz), 3.25-3.35 (1H, m), 4.53 (2H, s), 7.01 (2H, dd, J=8.8, 2.4 Hz), 7.10 (1H, tt, J = 13.2, 2.4 Hz), 12.03 (1H, brs) | 270(M⁺) |
| 59 | 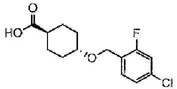 | DMSO-d₆,δ: 1.23 (2H, qd, J = 12.8, 2.8 Hz), 1.35 (2H, qd, J = 12.8, 2.4 Hz), 1.90 (2H, dd, J = 13.2, 2.4 Hz), 2.01 (2H, dd, J = 12.4, 3.6 Hz), 2.17 (1H, tt, J = 11.6, 3.6 Hz), 3.30-3.50 (1H, m), 4.52 (2H, s), 7.28 (1H, dd, J = 8.0, 1.6 Hz), 7.39 (1H, dd, J = 9.6, 2.0 Hz), 7.46 (1H, t, J = 8.0 Hz) | 286(M⁺) |
| 61 | 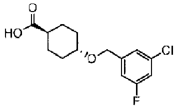 | DMSO-d₆,δ:1.25 (2H, qd, J = 12.8, 3.2 Hz), 1.35 (2H, qd, J = 13.2, 2.8 Hz), 1.90 (2H, dd, J = 13.6, 3.2 Hz), 2.02 (2H, dd, J = 12.8, 2.8 Hz), 2.18 (1H, tt, J = 11.2, 4.0 Hz), 3.28-3.36 (1H, m), 4.52 (2H, s), 7.14 (1H, dd, J = 9.2, 0.8 Hz), 7.24 (1H, s), 7.32 (1H, dt, J = 8.8, 2.0 Hz), 12.05 (1H, brs) | 286(M⁺) |
| 63 | 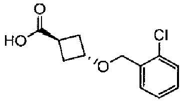 | DMSO-d₆,δ:2.13-2.25 (2H, m), 2.33-2.42 (2H, m), 2.86-3.03 (1H, m), 4.09-4.30 (1H, m), 4.44 (2H, s), 7.06-7.77 (4H, m), 12.19 (1H, brs) | 263 [M+Na]⁺ |
| 64 | 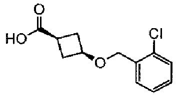 | DMSO-d₆,δ:1.91-2.07 (2H, m), 2.38 (2H, d, J = 7.2 Hz), 2.51-2.60 (1H, m), 3,90-4.00 (1H, m), 4.43 (2H, s), 7.25-7.55 (4H, m) | 241 [M+H]⁺ |
| 65 | 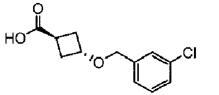 | DMSO-d₆,δ:2.13-2.21 (2H, m), 2.32-2.40 (2H, m), 2.88-2.97 (1H, m), 4.10-4.20 (1H, m), 4.38 (2H, s), 7.25-7.42 (4H, m) | 263 [M+Na]⁺ |

**[Table 6]**

| **Reference example No.** | **Chemical Structure** | **NMR** (**¹H-NMR)** | **MS (m/z)** |
|---|---|---|---|
| 66 | 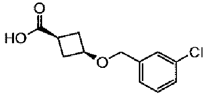 | DMSO-d₆,δ: 1.91-2.04 (2H, m), 2.32-2.40 (2H, m), 2.51-2.58 (1H, m), 3.86-3.93 (1H, m), 4.37 (2H, s), 7.21-7.47 (4H, m) | 263 [M+Na]⁺ |
| 67 | 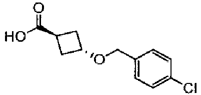 | DMSO-d₆,δ:2.08-2.17 (2H, m), 2.30-2.38 (2H, m), 2.83-2.92 (1H, m), 4.12-4.18 (1H, m), 4.35 (2H, s), 7.30-7.44 (4H, m) | 239 [M-H]⁺ |
| 68 | 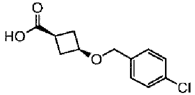 | DMSO-d₆,δ:1.93-2.04 (2H, m), 2.36-2.43 (2H, m), 2.53-2.83 (1H, m), 3.81-3.91 (1H, m), 4.38 (2H, s), 7.20-7.49 (4H, m), 12.16 (1H, brs) | 263 [M+Na]⁺ |
| 69 | 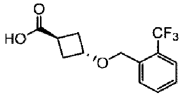 | DMSO-d₆,δ:2.12-2.23 (2H, m), 2.36-2.39 (2H, m), 2.86-3.01 (1H, m), 4.17-4.23 (1H, m), 4.52 (2H, s), 7.52 (1H, dt, J = 8.0, 4.03 Hz), 7.64- 7.76 (3H, m), 12.23 (1H, brs) | 297 [M+Na]⁺ |
| 70 | 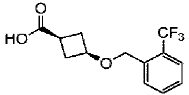 | DMSO-d₆,δ:1.96-2.08 (2H, m), 2.39-2.47 (2H, m), 2.55-2.64 (1H, m), 3.89-4.03 (1H, m), 4.52 (2H, s), 7.48-7.58 (1H, m), 7.62-7.77 (3H, m), 12.19 (1H, brs) | 297 [M+Na]⁺ |
| 71 | 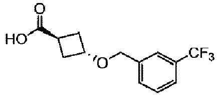 | DMSO-d₆,δ:2.14-2.24 (2H, m), 2.33-2.41 (2H, m), 2.89-2.98 (1H, m), 4.17-4.20 (1H, m), 4.47 (2H, s), 7.53-7.69 (4H, m), 12.20 (1H, brs) | 297 [M+Na]⁺ |
| 72 | 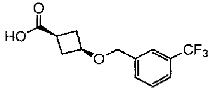 | DMSO-d₆,δ:1.95-2.06 (2H, m), 2.33-2.42 (2H, m), 2.51-2.61 (1H, m), 3.86-3.99 (1H, m), 4.46 (2H, d, J = 1.6 Hz), 7.55-7.69 (4H, m) | 275 [M+H]⁺ |
| 73 | 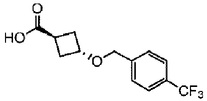 | DMSO-d₆,δ:2.14-2.24 (2H, m), 2.33-2.42 (2H, m), 2.88-2.99 (1H, m), 4.16-4.19 (1H, m), 4.47 (2H, s), 7.54 (2H, d, J = 8.0 Hz), 7.71 (2H, d, J = 8.0 Hz), 12.21 (1H, brs) | 297 [M+Na]⁺ |
| 74 | 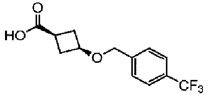 | DMSO-d₆,δ: 1.96-2.06 (2H, m), 2.37-2.46 (2H, m), 2.54-2.61 (1H, m), 3.89-4.01 (1H, m), 4.47 (2H, s), 7.54 (2H, d, J = 8.0 Hz), 7.70 (2H, d, J = 8.0 Hz), 12.10 (1H, brs) | 275 [M+H]⁺ |
| 75 | 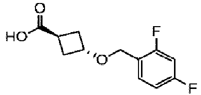 | CDCl₃,δ:2.28-2.36 (2H, m), 2.53-2.58 (2H, m), 3.07-3.09 (1H, m), 4.28-4.32 (1H, m), 4.42 (2H, s), 6.77-6.89 (2H, m), 7.33-7.39 (1H, m) | 241 [M-H]⁻ |

**[Table 7]**

| **Reference example No.** | **Chemical Structure** | **NMR (¹H-NMR)** | **MS (m/z)** |
|---|---|---|---|
| 76 | 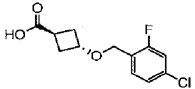 | CDCl₃,δ:2.30-2.37 (2H, m), 2.53-2.60 (2H, m), 3.06-3.12 (1H, m), 4.28-4.32 (1H, m), 4.44 (2H, s), 7.08 (1H, dd, J = 10.0, 2.0 Hz), 7.13 (1H, dd, J = 8.0, 1.2 Hz), 7.34 (1H, t, J = 8.0 Hz) | 257[M-H]⁻ |
| 77 | 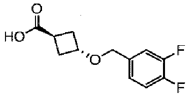 | CDCl₃,δ:2.29-2.37 (2H, m), 2.52-2.58 (2H, m), 3.06-3.12 (1H, m), 4.27-4.32 (1H, m), 4.36 (2H, s), 7.03-7.27 (3H, m) | 243 [M+H]⁺ |
| 78 | 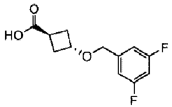 | CDCl₃,δ: 2.30-2.38 (2H, m), 2.53-2.59 (2H, m), 3.12-3.16 (1H, m), 4.28-4.31 (1H, m), 4.39 (2H, s), 8.68-6.74 (1H, m), 6.83-6.88 (2H, m) | 241 [M-H]⁻ |
| 79 | 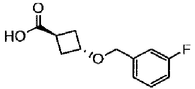 | CDCl₃,δ:2.20-2.38 (2H, m), 2.52-2.58 (2H, m), 3.06-3.11 (1H, m), 4.26-4.33 (1H, m), 4.41 (2H, s), 6.95-7.00 (1H, m), 7.04-7.09 (2H, m), 7.26-7.32 (1H, m) | 223 [M-H]⁻ |
| 80 | 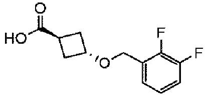 | CDCl₃,δ:2.33-2.43 (2H, m), 2.57-2.65 (2H, m), 3.10-3.17 (1H, m), 4.34-4.38 (1H, m), 4.54 (2H. s), 7.09-7.22 (3H, m) | 241 [M-H]⁻ |
| 81 | 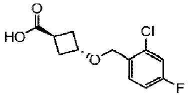 | CDCl₃,δ:2.32-2.40 (2H, m), 2.56-2.61 (2H, m), 3.08-3.13 (1H, m), 4.32-4.35 (1H, m), 4.46 (2H, s), 6.96-7.01 (2H, m), 7.12 (1H, dd, J = 6.4, 2.4 Hz), 7.40-7.50 (1H, m) | 259 [M+H]⁺ |
| 82 | 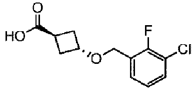 | CDCl₃,δ: 2.31-2.38 (2H, m), 2.55-2.61 (2H, m), 3.08-3.13 (1H, m), 4.31-4.33 (1H, m), 4.49 (2H, s), 7.05-7.09 (1H, m), 7.28-7.35 (2H, m) | 257 [M-H]⁻ |
| 83 | 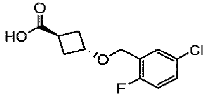 | CDCl₃,δ:2.39-2.62 (2H, m), 2.56-2.62 (2H, m), 3.07-3.14 (1H, m), 4.28-4.35 (1H, m), 4.45 (2H, s), 6.98 (1H, t, J = 8.8 Hz), 7.20-7.26 (1H, m), 7.35-7.45 (1H, m) | 257 [M-H]⁻ |
| 84 | 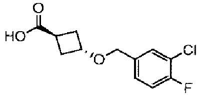 | DMSO-d₆,δ:2.12-2.22 (2H, m), 2.32-2.40 (2H, m), 2.88-2.99 (1H, m), 4.11-4.20 (1H, m), 4.36 (2H, s), 7.31-7.41 (2H, m), 7.53 (1H, dd, J = 7.3, 2.0 Hz), 12.16 (1H, s) | 257 [M-H]⁻ |
| 85 | 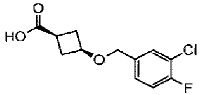 | DMSO-d₆,δ:1.93-2.08 (2H, m), 2.36-2.45 (2H, m), 2.53-2.64 (1H, m), 3.88-3.97 (1H, m), 4.36 (2H, s), 7.31-7.41 (2H, m), 7.53 (1H, dd, J = 7.5, 1.8 Hz), 12.13 (1H, s) | 257 [M-H]⁻ |

**[Table 8]**

| **Reference example No.** | **Chemical Structure** | **NMR (¹H-NMR)** | **MS (m/z)** |
|---|---|---|---|
| 89 | 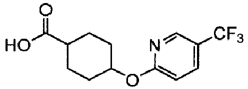 | DMSO-d₆,δ: 1.36-1.58 (3H, m), 1.62-1.88 (2H, m), 1.91-2.17 (3H, m), 2.20-2.42 (1H, m), 4.93-5.23 (1H, m), 6.91-7.02 (1H, m), 8.03 (1H, dd, J = 8.4, 2.3 Hz), 8.51-8.63 (1H, m), 12.08 (1H, s) | 288 [M-H]⁺ |
| 90 | 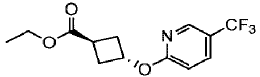 | DMSO-d₆,δ:1.21 (3H, t, J = 7.1 Hz), 2.37-2.47 (2H, m), 2.58-2.69 (2H, m), 3.16 (1H, ttd, J = 10.1, 4.0, 1.0 Hz), 4.12 (2H, q, J = 7.1 Hz), 5.29-5.43 (1H, m), 7.01 (1H, d, J = 8.8 Hz), 8.07 (1H, dd, J = 8.8, 2.7 Hz), 8.57 (1H, td, J = 1.7, 0.7 Hz) | 290 [M+H]⁺ |
| 91 | 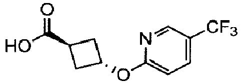 | DMSO-d₆,δ:2.32-2.44 (2H, m), 2.63 (2H, ddd, J = 13.4, 7.2, 3.9 Hz), 3.07 (1H, ttd, J = 10.1, 3.9, 1.0 Hz), 5.28-5.42 (1H, m), 7.00 (1H, d, J = 8.8 Hz), 8.06 (1H, dd, J = 8.7, 2.6 Hz), 8.53-8.60 (1H, m), 12.30 (1H, s) | 262 [M+H]⁺ |
| 92 | 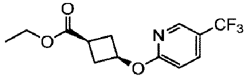 | DMSO-d₆,δ: 1.19 (3H, t, J = 7.1 Hz), 2.19-2.29 (2H, m), 2.67-2.77 (2H, m), 2.82-2.97 (1H, m), 4.08 (2H, q, J = 7.1 Hz), 5.17 (1H, quin, J = 7.4 Hz), 7.01 (1H, d, J = 8.6 Hz), 8.07 (1H, dd, J = 8.7, 2.6 Hz), 8.55 (1H, dt, J = 2.3, 1.0 Hz) | 290 [M+H]⁺ |
| 93 | 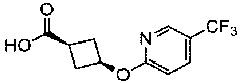 | DMSO-d₆,δ:2.11-2.29 (2H, m), 2.63-2.73 (2H, m), 2.74-2.88 (1H, m), 5.07-5.23 (1H, m), 7.00 (1H, d, J = 8.8 Hz), 8.06 (1H, dd, J = 8.8, 2,4 Hz), 8.47-8.59 (1H, m), 12.26 (1H, s) | 262 [M+H]⁺ |
| 94 | 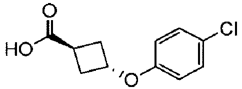 | DMSO-d₆,δ:2.27-2.35 (2H, m), 2.59-2.68 (2H, m), 3.02-3.11 (1H, m), 4.79 (1H, quin, J = 6.4 Hz), 6.84 (2H, d, J = 8.8 Hz), 7.30 (2H, d, J = 9.2 Hz), 12.30 (1H, brs) | 227 [M+H]⁺ |
| 95 | 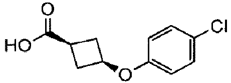 | DMSO-d₆,δ:2.13-2.16 (2H, m), 2.67-2.74 (3H, m), 4.58-4.66 (1H, m), 6.85-6.89 (2H, m), 7.29-7.33 (2H, m), 12.29 (1H, s) | 249 [M+Na]⁺ |

**[Table 9]**

| **Example No.** | **Chemical Structure** | **NMR (¹H-NMR)** | **MS (m/z)** |
|---|---|---|---|
| 1 | 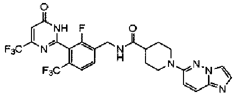 | DMSO-d₆,δ:1.65 (2H, qd, J = 12.4, 3.2 Hz), 1.84 (2H, d, J = 10.8 Hz), 2.47-2.56 (1H, m), 2.94 (2H, td, J = 12.8, 2.0 Hz), 4.17 (2H, d, J = 13.2 Hz), 4.41 (2H, d, J = 5.6 Hz), 7.01 (1H, s), 7.18 (1H, d, J = 10.4 Hz), 7.48 (1H, s), 7.68 (1H, t, J = 8.0 Hz), 7.77 (1 H, d, J = 8.0 Hz), 7.82 (1H, d, J = 10.4 Hz), 7.89 (1H, s), 8.58 (1H, t, J = 5.6 Hz) | 583(M⁺) |
| 2 | 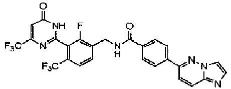 | DMSO-d₆,δ: 4.66 (2H, d, J = 5.6 Hz), 7.05 (1H, s), 7.79-7.91 (4H, m), 8.10 (2H, d, J = 8.0 Hz), 8.21-8.32 (3H, m), 8.40 (1H, s), 9.38 (1H, t, J = 6.0 Hz), 13.92 (1H, brs) | 576(M⁺) |
| 3 | 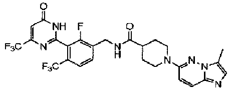 | DMSO-d₆,δ: 1.66 (2H, qd, J = 12.4, 3.2 Hz), 1.85 (2H, dd, J = 12.8, 2.4 Hz), 2.38 (3H, s), 2.48-2.56 (1H, m), 2.95 (2H, td, J = 12.8, 2.0 Hz), 4.23 (2H, d, J = 13.2 Hz), 4.41 (2H, d, J = 6.0 Hz), 7.01 (1H, s), 7.12 (1H, d, J = 9.6 Hz), 7.31 (1H, s), 7.68 (1H, t, J = 8.0 Hz), 7.77 (1H, d, J = 8.0 Hz), 7.78 (1H, d, J = 9.6 Hz), 8.59 (1H, t, J = 6.0 Hz), 13.90 (1H, brs) | 597(M⁺) |
| 4 | 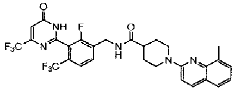 | DMSO-d₆,δ:1.54-1.70 (2H, m), 1.60-1.91 (2H, m), 2.55 (4H, s), 2.92-3.04 (2H, m), 3.29 (1H, s), 4.32-4.46 (2H, m), 4.51-4.66 (2H, m), 7.06-7.15 (1H, m), 7.25 (1H, d, J = 9.3 Hz), 7.40 (1H, d, J = 6.8 Hz), 7.52 (1H, d, J = 8.8 Hz), 7.56-7.66 (1H, m), 7.66-7.77 (1H, m), 7.99 (1H, d, J = 9.3 Hz), 8.56 (1H, t, J = 6.2 Hz), 13.88 (1H, s) | 608 [M+H]⁺ |
| 5 | 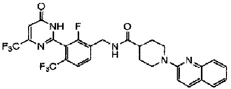 | DMSO-d₆,δ:1.55-1.65 (2H, m), 1.85 (2H, dd, J = 12.8, 2.4 Hz), 2.46-2.54 (1H, m), 2.93-3.01 (2H, m), 4.41 (2H, d, J = 5.2 Hz), 4.57 (2H, d, J = 13.2 Hz), 7.02 (1H, s), 7.18-7.23 (1H, m), 7.26 (1H, d, J = 9.6 Hz), 7.47-7.56 (2H, m), 7.68 (2H, t, J = 7.2 Hz), 7.77 (1H, d, J = 8.4 Hz), 8.02 (1H, d, J = 9.2 Hz), 8.59 (1H, t, J = 5.6 Hz), 13.89 (1H, brs) | 593(M⁺) |
| 6 | 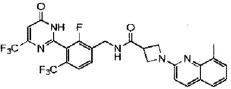 | DMSO-d₆,δ:2.54 (3H, s), 3.58-3.67 (1H, m), 4.14 (2H, dd, J = 8.1, 6.1 Hz), 4.20-4.29 (2H, m), 4.43 (2H, d, J = 5.6 Hz), 6.28 (1H, brs), 6.75 (1H, d, J = 8.8 Hz), 7.08-7.16 (1H, m), 7.35-7.46 (1H, m), 7.50-7.63 (3H, m), 8.01 (1H, d, J = 9.0 Hz), 8.73 (1H, t, J = 5.9 Hz), 13.92 (1H, brs) | 580 [M+H]⁺ |

**[Table 10]**

| **Example No.** | **Chemical Structure** | **NMR** (**¹H-NMR)** | **MS (m/z)** |
|---|---|---|---|
| 7 | 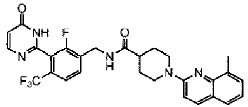 | DMSO-d₆,δ:1.63 (2H, qd, J = 12.4, 3.2 Hz), 1.83-1.89 (2H, m), 2.46-2.61 (1H, m), 2.55 (3H, s), 2.99 (2H, t, J = 12.4 Hz), 4.40 (2H, d, J = 6.0 Hz), 4.60 (2H, d, J = 13.2 Hz), 6.43 (1H, brs), 7.11 (1H, t, J = 7.8 Hz), 7.25 (1H, d, J = 9.2 Hz), 7.40 (1H, d, J = 6.8 Hz), 7.53 (1H, d, J = 8.0 Hz), 7.63 (1H, t, J = 7.6 Hz), 7.73 (1H, d, J = 8.4 Hz), 8.00 (1H, d, J = 9.6 Hz), 8.05 (1H, brs), 8.58 (1H, t, J = 6.0 Hz), 13.16 (1H, brs) | 539(M⁺) |
| 8 | 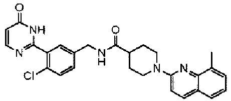 | DMSO-d₆,δ:1.62 (2H, qd, J = 12.4, 4.2 Hz), 1.82 (2H, dd, J = 13.1, 2.6 Hz), 2.52-2.57 (4H, m), 2.92-3.03 (2H, m), 4.30 (2H, d, J = 6.1 Hz), 4.57 (2H, d, J = 13.4 Hz), 6.34 (1H, d, J = 6.6 Hz), 7.06-7.15 (1H, m), 7.23 (1H, d, J = 9.3 Hz), 7.36-7.42 (2H, m), 7.44 (1H, d, J = 2.2 Hz), 7.50-7.56 (2H, m), 7.95-8.05 (2H, m), 8.33-8.55 (1H, m), 12.80 (1H, brs) | 488 [M+H]⁺ |
| 9 | 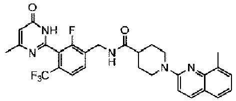 | DMSO-d₆,δ:1.57-1.69 (2H, m), 1.79-1.91 (2H. m), 2.22 (3H, s), 2.55 (3H, s), 2.56-2.63 (1H, m), 2.93-3.11 (2H, m), 4.40 (2H, d, J = 5.6 Hz), 4.60 (2H, d, J = 13.0 Hz), 6.27 (1H, brs), 7.01-7.16 (1H, m), 7.25 (1H, d, J = 9.3 Hz), 7.40 (1H, d, J = 6.6 Hz), 7.52(1H, d, J = 8.1 Hz), 7.57-7.67 (1H, m), 7.73 (1H, d, J = 8.3 Hz), 8.00 (1H, d, J = 9.3 Hz), 8.58 (1H, t, J = 6.0 Hz), 12.98 (1H, brs) | 554 [M+H]⁺ |
| 10 | 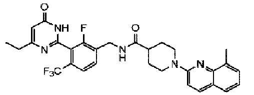 | DMSO-d₆,δ:1.13 (3H, t, J = 7.2 Hz), 1.62 (2H, qd, J = 12.0, 3.6 Hz), 1.86 (2H, dd, J = 12.8, 2.4 Hz), 2.45-2.62 (3H, m), 2.55 (3H, s), 2.99 (2H, td, J = 12.4, 2.4 Hz), 4.40 (2H, d, J = 6.0 Hz), 4.60 (2H, d, J = 13.2 Hz), 6.25 (1H, brs), 7.10 (1H, t, J = 8.0 Hz), 7.25 (1H, d, J = 9.2 Hz), 7.40 (1H, d, J = 6.8 Hz), 7.52 (1H, d, J = 8.4 Hz), 7.62 (1H, t. J = 8.0 Hz), 7.72 (1H, d, J = 8.4 Hz), 7.99 (1H, d, J = 8.8 Hz), 8.58 (1H, t, J = 6.0 Hz), 12,93 (1H, brs) | 567(M⁺) |
| 11 | 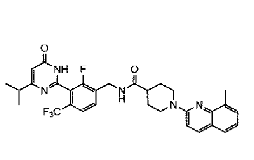 | DMSO-d₆,δ:1.13 (6H, d, J = 6.8 Hz), 1.63 (2H, qd, J = 12.0, 3.6 Hz), 1.86 (2H, dd, J = 12.8, 2.0 Hz), 2.55 (3H, s), 2.69-2.77 (1H, m), 2.99 (2H, td, J = 12.4, 2.0 Hz), 4.40 (2H, d, J = 6.0 Hz), 4.60 (2H, d, J = 13.2 Hz), 6.23 (1H, s), 7.11 (1H, t, J = 7.2 Hz), 7.25 (1H, d, J = 9.2 Hz), 7.40 (1H, d, J = 7.2 Hz), 7.52 (1H, d, J = 7.6 Hz), 7.62 (1H, t, J = 7.2 Hz), 7.72 (1H, d, J = 8.0 Hz), 7.99 (1H, d, J = 9.6 Hz), 8.58 (1H, t, J = 6.4 Hz), 12.90 (1H, brs) | 581(M⁺) |
| 12 | 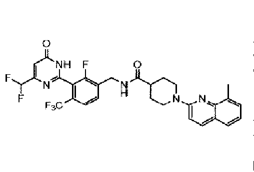 | DMSO-d₆,δ: 1.63 (2H, qd, J = 12.4, 3.6 Hz), 1.82-1.88 (2H, m), 2.45-2.61 (1H, m), 2.55 (3H, s), 2.94-3.03 (2H, m), 4.41 (2H, d, J = 5.6 Hz), 4.60 (2H, d, J = 13.2 Hz), 8.74 (1H, s), 6.79 (1H, t, J = 54.4 Hz), 7.11 (1H, t, J = 7.6 Hz), 7.25 (1H, d, J = 9.2 Hz), 7.40 (1H, d, J = 6.8 Hz), 7.53 (1H, d, J = 8.0 Hz), 7.67 (1H, t, J = 7.6 Hz), 7.76 (1H, d, J = 8.4 Hz), 8.00 (1H, d, J = 9.6 Hz), 8.59 (1H, t, J = 5.6 Hz), 13.58 (1H, brs) | 589(M⁺) |

**[Table 11]**

| **Example No.** | **Chemical Structure** | **NMR** (**¹H-NMR)** | **MS (m/z)** |
|---|---|---|---|
| 13 | 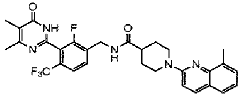 | DMSO-d₆,δ: 1.57-1.68 (2H, m), 1.84-1.88 (2H, m), 1.98 (3H, s), 2.24 (3H, s), 2.45-2.57 (1H, m), 2.55 (3H, s), 2.95-3.02 (2H, m), 4.39 (2H, d, J = 6.4 Hz), 4.60 (2H, d, J = 13.2 Hz), 7.11 (1H, t, J = 7.6 Hz), 7.25 (1H, d, J = 9.2 Hz), 7.40 (1H, d, J = 6.8 Hz), 7.53 (1H, d, J = 8.0 Hz), 7.58-7.63 (1H, m), 7.70-7.73 (1H, m), 8.00 (1H, d, J = 9.6 Hz), 8.57 (1H, t, J = 6.0 Hz), 12.90 (1H, brs) | 567(M⁺) |
| 14 | 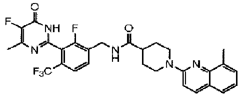 | DMSO-d₆,δ: 1.63 (2H, qd, J = 12.4, 3.6 Hz), 1.86 (2H, dd, J = 12.8, 2.4 Hz), 2.26 (3H, d, J = 3.6 Hz), 2.47-2.58 (1H, m), 2.55 (3H, s), 2.99 (2H, td, J = 12.8, 2.0 Hz), 4.40 (2H, d, J = 5.2 Hz), 4.60 (2H, d, J = 13.2 Hz), 7.11 (1H, t, J = 8.0 Hz), 7.25 (1H, d, J = 9.2 Hz), 7.40 (1H, d, J = 6.8 Hz), 7.53 (1H, d, J = 8.0 Hz), 7.64 (1H, t, J = 8.0 Hz), 7.74 (1H, d, J = 8.4 Hz), 8.00 (1H, d, J = 9.2 Hz), 8.58 (1H, t, J = 6.0 Hz), 13.62 (1H, brs) | 571(M⁺) |
| 15 | 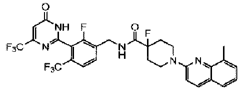 | DMSO-d₆,δ: 1.89-2.18 (4H, m), 2.56 (3H, s), 3.24-3.32 (2H, m), 4.46 (2H, d, J = 5.6 Hz), 4.54 (2H, d, J = 14.0 Hz), 7.04 (1H, brs), 7.14 (1H, t, J = 7.6 Hz), 7.31 (1H, d, J = 9.2 Hz), 7.42 (1H, d, J = 6.8 Hz), 7.55 (1H, d, J = 8.0 Hz), 7.68 (1H, t, J = 7.6 Hz), 7.78 (1H, d, J = 8.0 Hz), 8.04 (1H, d, J = 9.6 Hz), 9.03 (1H, td, J = 6.0, 2.4Hz), 13.89 (1H, brs) | 625(M⁺) |
| 16 | 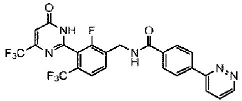 | DMSO-d₆,δ:4.66 (2H, d, J = 4.4 Hz), 7.06 (1H, s), 7.76-7.87 (3H, m), 8.10 (2H, d, J = 8.4 Hz), 8.29-8.35 (3H, m), 9.27 (1H, d, J = 4.0 Hz), 9.38 (1H, t, J = 5.2 Hz), 13.92 (1H, brs) | 537(M⁺) |
| 17 | 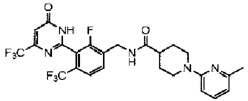 | DMSO-d₆,δ:1.55 (2H, qd, J = 12.4, 2.0 Hz), 1.79 (2H, dd, J = 12.8, 2.4 Hz), 2.28 (3H, s), 2.44-2.53 (1H, m), 2.78 (2H, td, J = 12.8, 2.0 Hz), 4.32 (2H, d, J = 13.2 Hz), 4.41 (2H, d, J = 5.6 Hz), 6.46 (1H, d, J = 6.8 Hz), 6.61 (1H, d, J = 8.8 Hz), 7.04 (1H, s), 7.39 (1H, dd, J = 8.4, 7.6 Hz), 7.67 (1H, t, J = 7.6 Hz), 7.78 (1H, d, J = 8.4 Hz), 8.57 (1H, t, J = 6.0 Hz), 13.89 (1H, brs) | 557(M⁺) |
| 18 | 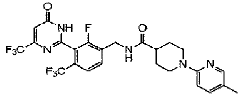 | DMSO-d₆,δ:1.55 (2H, qd, J = 12.4, 3.2 Hz), 1.77 (2H, d, J = 11.2 Hz), 2.13 (3H, s), 2.43-2.55 (1H, m), 2.78 (2H, t, J = 12.0 Hz), 4.25 (2H, d, J = 13.2 Hz), 4.40 (2H, d, J = 5.6 Hz), 6.76 (1H, d, J = 8.8 Hz), 7.04 (1H, s), 7.35 (1H, dd, J = 8.0, 1.6 Hz), 7.67 (1H, t, J = 8.0 Hz), 7.77 (1H, d, J = 8.0 Hz), 7.93 (1H, d, J = 1.6 Hz), 8.56 (1H, t, J = 6.0 Hz), 13.90 (1H, brs) | 557(M⁺) |

**[Table 12]**

| **Example No.** | **Chemical Structure** | **NMR** (**¹H-NMR)** | **MS (m/z)** |
|---|---|---|---|
| 19 | 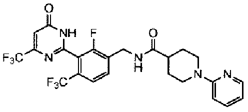 | DMSO-d₆,δ:1.55 (2H, qd, J = 12.4, 4.0 Hz), 1.79 (2H, dd, J = 13.6, 2.8 Hz), 2.43-2.55 (1H, m), 2.82 (2H, td, J = 12.0, 2.4 Hz), 4.32 (2H, d, J = 12.8 Hz), 4.41 (2H, d, J = 5.2 Hz), 6.60 (1H, dd, J = 6.8, 4.8 Hz), 6.83 (1H, d, J = 8.8 Hz), 7.02 (1H, s), 7.51 (1H, ddd, J = 8.8, 6.8, 2.0 Hz), 7.67 (1H, t, J = 7.6 Hz), 7.77 (1H, dd, J = 8.0, 2.8 Hz), 8.09 (1H, dd, J = 4.8, 1.2 Hz), 8.57 (1H, t, J = 6.0 Hz), 13.90 (1H, brs) | 543(M⁺) |
| 20 | 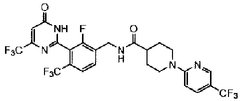 | DMSO-d₆,δ:1.49-1.60 (2H, m), 1.82 (2H, dd, J = 13.2, 2.4 Hz), 2.47-2.83 (1H, m), 2.95-3.03 (2H, m), 4.38-4.48 (4H, m), 6.97 (1H, d, J = 9.2 Hz), 7.03 (1H, s), 7.87 (1H, t, J = 7.6 Hz). 7.75-7.79 (2H, m), 8.38-8.41 (1H, m), 8.59 (1H, t, J = 6.0 Hz), 13.89 (1H, brs) | 611(M⁺) |
| 21 | 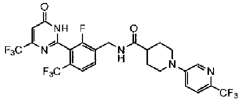 | DMSO-d₆,δ:1.59-1.72 (2H, m), 1.84 (2H, dd, J = 12.8, 2.4 Hz), 2.46-2.54 (1H, m), 2.93 (2H, td, J = 12.8, 2.4 Hz), 3.98 (2H, d, J = 12.8 Hz), 4.42 (2H, d, J = 6.0 Hz), 7.04 (1H, s), 7.44 (1H, dd, J = 8.8, 2.8 Hz), 7.62 (1H, d, J = 8.8 Hz), 7.68 (1H, t, J = 7.6 Hz), 7.78 (1H, d, J = 8.0 Hz), 8.43 (1H, d, J = 2.8 Hz), 8.59 (1H, t, J = 6.0 Hz), 13.90 (1H, brs) | 611(M⁺) |
| 22 | 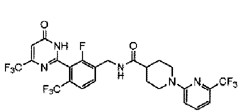 | DMSO-d₆,δ: 1.50-1.61 (2H, m), 1.83 (2H, dd, J = 12.4, 2.0 Hz), 2.47-2.54 (1H, m), 2.93 (2H, td, J = 13.2, 2.0 Hz), 4.36 (2H, d, J = 13.6 Hz), 4.41 (2H, d, J = 6.0 Hz), 7.01 (2H, d, J = 7.2 Hz), 7.14 (1H, d, J = 8.8 Hz), 7.65-7.79 (3H, m), 8.59 (1H, t, J = 6.0 Hz), 13.90 (1H, brs) | 611(M⁺) |
| 23 | 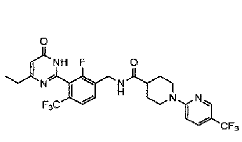 | DMSO-d₆,δ:1.13 (3H, t, J = 7.2 Hz), 1.48-1.60 (2H, m), 1.82 (2H, dd, J = 13.2, 2.4 Hz), 2.46-2.62 (3H, m), 2.95-3.03 (2H, m), 4.39 (2H, d, J = 6.0 Hz), 4.44 (2H, d, J = 13.6 Hz), 6.25 (1H, brs), 6.97 (1H, d, J = 9.2 Hz), 7.61 (1H, t, J = 8.0 Hz), 7.72 (1H, d, J = 8.0 Hz), 7.77 (1H, dd, J = 9.2, 2.4 Hz), 8.38-8.42 (1H, m), 8.57 (1H, t, J = 8.0 Hz), 12.95 (1H, brs) | 571(M⁺) |
| 24 | 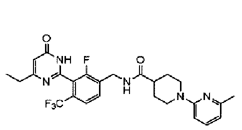 | DMSO-d₆,δ:1.13 (3H, t, J = 7.2 Hz), 1.55 (2H, qd, J = 12.0, 3.6 Hz), 1.78 (2H, dd, J = 12.0, 2.0 Hz), 2.29 (3H, s), 2.46-2.54 (3H, m), 2.78 (2H, td, J = 12.4, 2.4 Hz), 4.32 (2H, d, J = 13.2 Hz), 4.39 (2H, d, J = 5.6 Hz), 6.26 (1H, brs), 6.47 (1H, d, J = 7.2 Hz), 6.61 (1H, d, J = 8.4 Hz), 7.39 (1H, dd, J = 8.4, 7.6 Hz), 7.62 (1H, t, J = 7.6 Hz), 7.73 (1H, d, J = 8.0 Hz), 8.56 (1H, t, J = 6.0 Hz), 12.94 (1H, brs) | 517(M⁺) |

**[Table 13]**

| **Example No.** | **Chemical Structure** | **NMR (¹H-NMR)** | **MS (m/z)** |
|---|---|---|---|
| 25 | 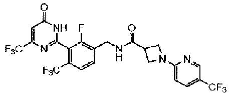 | DMSO-d₆, δ: 3.56-3.65 (1H, m), 4.08 (2H, dd, J = 8.0, 6.4 Hz), 4.20 (2H, 1, J = 8.0 Hz), 4.47 (2H, d, J = 6.0 Hz), 6.50 (1H, d, J = 8.8 Hz), 7.07 (1H, s), 7.72-7.81 (3H, m), 8.38 (1H, d, J = 1.2 Hz), 8.80 (1H, t, J = 6.0 Hz), 13.90 (1H, brs) | 583(M⁺) |
| 26 | 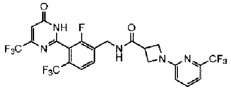 | DMSO-d₆, δ:3.56-3.64 (1H, m), 4.04 (2H, dd, J = 8.0, 6.0 Hz), 4.17 (2H, t, J = 8.0 Hz), 4.46 (2H, d, J = 6.0 Hz), 6.88 (1H, d, J = 8.8 Hz), 7.05-7.07 (2H, m), 7.71-7.80 (3H, *m),* 8.78 (1H, t, J = 6.0 Hz), 13.90 (1H, brs) | 583(M⁺) |
| 27 | 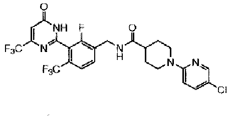 | DMSO-d₆,δ: 1.54 (2H, qd, J = **12.4,** 3.6 Hz), 1.79 (2H, dd, J = 13.2, 2.4 Hz), 2.46-2.55 (1H, m), 2.87 (2H, td, J = 13.2, 2.4 Hz), 4.28 (2H, d, J = 13.2 Hz), 4.41 (2H, d, J = 5.8 Hz), 6.88 (1H, d, J = 8.8 Hz), 7.05 (1H, s), 7.57 (1H, dd, J = 9.2, 2.8 Hz), 7.67 (1H, **t,** J = 8.0 Hz), 7.77 (1H, d, J = 7.6 Hz), 8.09 (1H, d, J = 2.4 Hz), 8.58 (1H, t, J = 6.0 Hz), 13.90 (1H, brs) | 577(M⁺) |
| 28 | 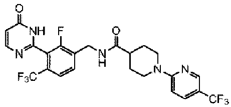 | DMSO-d₆,δ: 1.54 (2H, qd, J = 12.8, 2.8 Hz), 1.82 (2H, dd, J = 12.8, 2.8 Hz), 2.46-2.62 (1H, m), 2.94-3.03 (2H, m), 4.39 (2H, d, J = 6.0 Hz), 4.43 (2H, d, J = 13.2 Hz), 6.44 (1H, brs), 6.98 (1H, d, J = 8.8 Hz), 7.62 (1H, t, J = 8.0 Hz), 7.73 (1H, d, J = 8.4 Hz), 7.77 (1H, dd, J = 9.2, 2.4 Hz), 8.06 (1H, brs), 8.39-8.40 (1H, m), 8.57 (1H, t, J = 6.0 Hz), 13.16 (1 H, brs) | 543(M⁺) |
| 29 | 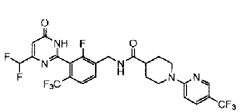 | DMSO-d₆,δ:1.54 (2H, qd, J = 12.4, 3.6 Hz), 1.82 (2H, dd, J = 12.4, 3.2 Hz), 2.47-2.63 (1H, m), 2.99 (2H, td, J = 13.2, 2.0 Hz), 4.38-4.48 (4H, m), 6.74 (1H, brs), 5.79 (1H, t, J = 53.6 Hz), 6.97 (1H, d, J = 9.2 Hz), 7.66 (1H, t, J = 8.0 Hz), 7.74-7.80 (2H, m), 8.38-8.41 (1H, m), 8.59 (1H, t, J = 5.6 Hz), 13.58 (1H, brs) | 593(M⁺) |
| 30 | 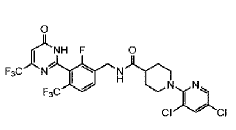 | DMSO-d₆,0:1.67-1.80 (2H, m), 1.81-1.89 (2H, m), 2.38-2.48 (1H, m), 2.76-2.87 (2H, m), 3.75 (2H, d, J = 13.0 Hz), 4.42 (2H, d, J = 5.6 Hz), 7.04 (1H, s), 7.63-7.73 (1H, m), 7.76-7.83 (1H, m), 8.04 (1H, d, J = 2.4 Hz), 8.28 (1H, d, J = 2.4 Hz), 8.59 (1H, t, J = 5.9 Hz), 13.90 (1H, brs) | 612 [M+H]⁺ |

**[Table 14]**

| **Example No.** | **Chemical Structure** | **NMR (¹H-NMR)** | **MS (m/z)** |
|---|---|---|---|
| 31 | 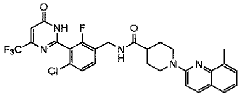 | DMSO-d₆,δ:1.61 (2H, qd, J = 12.3, 3.5 Hz), 1.84 (2H, dd, J = 13.1, 2.6 Hz), 2.52-2.59 (4H, m), 2.97 (2H, td, J = 12.6, 2.2 Hz), 4.33 (2H, d, J = 5.9 Hz), 4.59 (2H, d, J = 13.4 Hz), 7.01 (1H, s), 7.08-7.14 (1H, m), 7.24 (1H, d, J = 9.0 Hz), 7.38-7.42 (1H, m), 7.45-7.56 (3H, m), 7.99 (1H, d, J = 9.3 Hz), 8.51 (1H, t, J = 5.9 Hz), 13.83 (1H, brs) | 574 [M+H]⁺ |
| 32 | 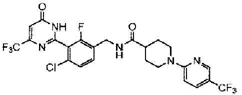 | DMSO-d₆,δ: 1.53 (2H, qd, J = 12.8, 3.6 Hz), 1.80 (2H, dd, J = 12.8, 3.6 Hz), 2.46-2.60 (1H, m), 2.92-3.02 (2H, m), 4.32 (2H, d, J = 6.0 Hz), 4.43 (2H, d, J = 13.2 Hz), 6.97 (1H, d, J = 9.2 Hz), 7.01 (1H, brs), 7.47-7.50 (2H, m), 7.77 (1H, dd, J = 9.2, 2.4 Hz), 8.38-8.41 (1H, m), 8.51 (1H, t, J = 6.0 Hz), 13.83 (1H, brs) | 577(M⁺) |
| 33 | 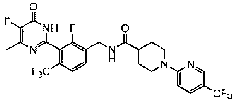 | DMSO-d₆,δ: 1.54 (2H, qd, J = 12.4, 3.6 Hz), 1.82 (2H, dd, J = 13.2, 2.4 Hz), 2.26 (3H, d, J = 3.2 Hz), 2.53-2.61 (1H, m), 2.99 (2H, td, J = 12.4, 2.0 Hz), 4.39 (2H, d, J = 5.6 Hz), 4.43 (2H, d, J = 13.2 Hz), 6.97 (1H, d, J = 8.8 Hz), 7.63 (1H, t, J = 8.0 Hz), 7.73 (1H, d, J = 8.4 Hz), 7.77 (1H, dd, J = 8.8, 2.8 Hz), 8.40 (1H, d, J = 0.8 Hz), 8.58 (1H, t, J = 6.0 Hz), 13.62 (1H, brs) | 575(M⁺) |
| 34 | 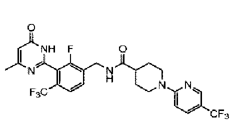 | DMSO-d₆,δ: 1.54 (2H, qd, J = 12.4, 3.6 Hz), 1.82 (2H, dd, J = 12.8, 2.4 Hz), 2.23 (3H, s), 2.52-2.62 (1H, m), 2.99 (2H, td, J = 12.0, 2.0 Hz), 4.39 (2H, d, J = 5.6 Hz), 4.43 (2H, d, J = 13.6 Hz), 6.30 (1H, brs), 6.97 (1H, d, J = 9.2 Hz), 7.61 (1H, t, J = 8.0 Hz), 7.72 (1H, d, J = 8.4 Hz), 7.77 (1H, dd, J = 8.8, 2.4 Hz), 8.39 (1 H, d, J = 2.0 Hz), 8.57 (1H, t, J = 6.0 Hz), 12.94 (1H, brs) | 557(M') |
| 35 | 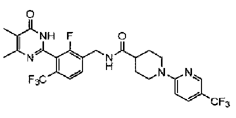 | DMSO-d₆,δ:1.55 (2H, qd, J = 12.3, 3.9 Hz), 1.82 (2H, dd, J = 13.1, 2.8 Hz), 1.98 (3H, s). 2.24 (3H, s), 2.53-2.82 (1H, m), 2.92-3.06 (2H, m), 4.32-4.52 (4H, m), 6.97 (1H, d, J = 9.0 Hz), 7.56-7.64 (1H, m), 7.70 (1H, d, J = 8.3 Hz), 7.77 (1H, dd, J = 9.0, 2.4 Hz), 8.39 (1H, s), 8.56 (1H, t, J = 5.9 Hz), 12.88 (1H, brs) | 572 [M+H]⁺ |
| 36 | 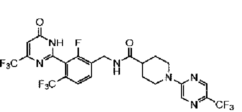 | DMSO-d₆,δ; 1.53-165 (2H, m), 1.63-1.91 (2H, m), 2.52-2.68 (1H, m), 3.03-3.13 (2H, m), 4.41 (2H, d, J = 5.6 Hz), 4.48 (2H, d, J = 13.4 Hz), 7.02 (1H, s), 7.67 (1H, t, J = 7.6 Hz), 7.77 (1H, d, J = 8.1 Hz), 8.45 (2H, d, J = 9.6 Hz), 8.59 (1H, t, J = 5.9 Hz), 13.89 (1H, brs) | 613 [M+H]⁺ |

**[Table 15]**

| **Example** No. | **Chemical Structure** | **NMR (¹H-NMR)** | **MS (m/z)** |
|---|---|---|---|
| 37 | 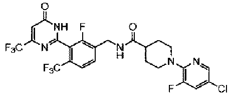 | DMSO-d₅,δ: 167 (2H, qd, J = 12.0, 3.6 Hz), 1.82 (2H, dd, J = 12.8, 2.4 Hz), 2.45-2.55 (1H, m), 2.92 (2H, td, J = 12.8, 2.0 Hz), 3.98 (2H, d, J = 13.2 Hz), 4.41 (2H, d, J = 6:0 Hz), 7.05 (1H, brs), 7.68 (1H, t, J = 8.0 Hz), 7.77 (1H, d, J = 8.0 Hz), 7.80 (1H, dd, J = 12.4, 1.6 Hz), 8.07 (1H, d, J = 1.6 Hz), 8.57 (1H, t, J = 6.0 Hz), 13.89 (1H, brs) | 595(M⁺) |
| 38 | 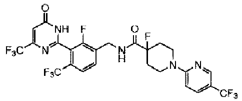 | DMSO-d₆,δ; 1.86-2.12 (4H, m), 3.20-3.30 (2H, m), 4.40 (2H, d, J = 13.6 Hz), 4.46 (2H, d, J = 5.2 Hz), 7.03-7.08 (2H, m), 7.68 (1H, t, J = 7.6 Hz), 7.78 (1H, d, J = 8.4 Hz), 7.82 (1H, dd, J = 9.2, 2.4 Hz), 8.43 (1H, d, J = 0.8 Hz), 9.00-9.06 (1H, m), 13.89 (1H, brs) | 629(M⁺) |
| 39 | 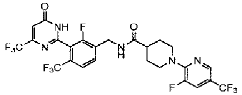 | DMSO-d₆,δ: 1.66 (2H, qd, J = 12.0, 3.6 Hz), 1.85 (2H, dd, J = 13.2, 2.4 Hz), 2.46-2.61 (1H, m), 3.06 (2H, td, J = 11.6, 2.0 Hz), 4.26 (2H, d, J = 13.2 Hz), 4.41 (2H, d, J = 5.6 Hz), 7.04 (1H, s), 7.68 (1H, t, J = 8.0 Hz), 7.77 (1H, d, J = 8.4 Hz), 7.91 (1H, dd, J = 13.6, 2.0 Hz), 8.34 (1H, s), 8.58 (1H, t, J = 6.0 Hz), 13.89 (1H, brs) | 629(M⁺) |
| 40 | 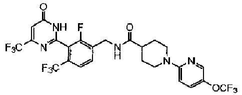 | DMSO-d₆,δ:1.56 (2H, qd, J = 12.4, 3.6 Hz), 1.81 (2H, dd, J = 12.4, 2.4 Hz), 2.47-2.55 (1H, m), 2.90 (2H, td, J = 12.4, 2.4 Hz), 4.31 (2H, d, J = 13.6 Hz), 4.41 (2H, d, J = 5.2 Hz), 6.92 (1H, d, J = 9.2 Hz), 7.04 (1H, brs), 7.57 (1H, dd, J = 9.6, 2.8 Hz), 7.68 (1H, t, J = 7.8 Hz), 7.77 (1H, d, J = 8.4 Hz), 8.14 (1H, d, J = 3.2 Hz), 8.57 (1H, t, J = 6.0 Hz), 13.89 (1H, brs) | 627(M⁺) |
| 41 | 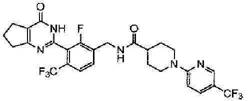 | DMSO-d₆,δ:1.54 (2H, qd, J = 12.4, 3.2 Hz), 1.82 (2H, dd, J = 12.4, 2.4 Hz), 2.02 (2H, quin, J = 7.6 Hz), 2.52-2.62 (1H, m), 2.70 (2H, t, J = 7.6 Hz), 2.80 (2H, t, J = 7.6 Hz), 2.99 (2H, td, J = 13.2, 2.4 Hz), 4.39 (2H, d, J = 6.0 Hz), 4.43 (2H, d, J = 13.2 *Hz),* 8.97 (1H, d, J = 9.2 Hz), 7.61 (1H, t, J = 8.0 Hz), 7.71 (1 H, d, J = 8.4 Hz), 7.76 (1H, dd, J = 9.2, 2.4 Hz), 8.39 (1H, d, J = 1.6 Hz), 8.56 (1H, t, J = 6.0 Hz), 12.89 (1H, brs) | 583(M⁺) |
| 42 | 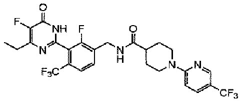 | DMSO-d₆,δ: 1.14 (3H, t, J = 8.0 Hz), 1.54 (2H, qd, J = 12.4, 3.6 Hz), 1.82 (2H, dd, J = 13.2, 2.4 Hz), 2.47-2.56 (1H, m), 2.57-2.62 (2H, m), 2.99 (2H, td, J = 12.4, 2.0 Hz), 4.39 (2H, d, J = 6.0 Hz), 4.43 (2H, d, J = 14.0 Hz), 6.97 (1H, d, J = 9.6 Hz), 7.63 (1H, t, J = 7.6 Hz), 7.73 (1H, d, J = 8.4 Hz), 7.76 (1H, dd, J = 9.6, 2.4 Hz), 8.40 (1H, s), 8.57 (1H, t, J = 6.0 Hz), 13.60 (1H, brs) | 589(M⁺) |

**[Table 16]**

| **Example No.** | **Chemical Structure** | **NMR (¹H-NMR)** | **MS (m/z)** |
|---|---|---|---|
| 43 | 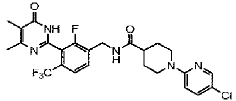 | DMSO-d₆,δ: 1.55 (2H, qd, J = 12.4, 3.6 Hz), 1.79 (2H, dd, J = 13.2, 2.4 Hz), 1.98 (3H, s), 2.24 (3H, s), 2.47-2.55 (1H, m), 2.87 (2H, td, J = 12.4, 2.4 Hz), 4.28 (2H, d, J = 13.2 Hz), 4.38 (2H, d, J = 6.0 Hz), 6.88 (1H, d, J = 9.6 Hz), 7.54-7.64 (2H, m), 7.71 (1H, d, J = 8.4 Hz), 8.09 (1H, d, J = 2.4 Hz), 8.54 (1H, t, J = 6.0 Hz), 12.89 (1H, brs) | 537(M⁺) |
| 44 | 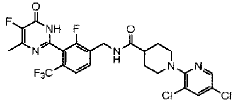 | DMSO-d₆,δ:1.74 (2H, qd, J = 12.8, 4.0 Hz), 1.85 (2H, dd, J = 12.8, 2.4 Hz), 2.26 (3H, d, J = 3.6 Hz), 2.41-2.52 (1H, m), 2.81 (2H, td, J = 12.4, 2.4 Hz), 3.75 (2H, d, J = 13.2 Hz), 4.40 (2H, d, J = 5.6 Hz), 7.65 (1H, t, J = 8.0 Hz), 7.74 (1H, d, J = 8.0 Hz), 8.03 (1H, d, J = 2.0 Hz), 8.26 (1H, d, J = 2.4 Hz), 8.57 (1H, t, J = 6.0 Hz), 13.62 (1H, brs) | 575(M⁺) |
| 45 | 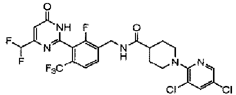 | DMSO-d₆,δ : 1.66-1.79 (2H, m), 1.81-1.88 (2H, m), 236-2.46 (1H, m), 2.76-2.86 (2H, m), 3.75 (2H, d, J = 13.4 Hz), 4.41 (2H, d, J = 5.4 Hz), 6.61-6.95 (2H, m), 7.59-7.71 (1H, m), 7.76 (1H, d, J = 9.0 Hz), 8.02 (1H, d, J = 2.2 Hz), 8.26 (1H, d, J = 2.2 Hz), 8.58 (1H, t, J = 5.9 Hz), 13.57 (1H, brs), | 594 [M+H]⁺ |
| 46 | 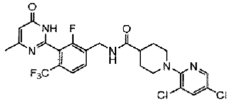 | DMSO-d₆,δ: 1.74 (2H, qd, J = 12.0, 3.6 Hz), 1.85 (2H, dd, J = 12.8, 2.8 Hz), 2.23 (3H, s), 2.45 (1H, tt, J = 11.2, 4.0 Hz), 2.81 (2H, td, J = 12.4, 2.4 Hz), 3.75 (2H, d, J = 12.8 Hz), 4.40 (2H, d, J = 5.2 Hz), 6.28 (1H, brs), 7.63 (1H, t, J = 7.6 Hz), 7.72 (1H, d, J = 8.4 Hz), 8.03 (1H, d, J = 2.4 Hz), 8.26 (1H, d, J = 2.4 Hz), 8.57 (1H, t, J = 6.0 Hz), 12.96 (1H, brs) | 557(M⁺) |
| 47 | 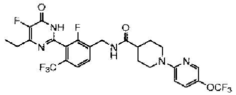 | DMSO-d₆,δ: 1.14 (3H, t, J = 7.2 Hz), 1.56 (2H, qd, J = 12.4, 3.6 Hz), 1.81 (2H, dd, J = 12.8, 2.4 Hz), 2.48-2.64 (3H, m), 2.90 (2H, td, J = 13.2, 2.4 Hz), 4.31 (2H, d, J = 13.6 Hz), 4.39 (2H, d, J = 5.6 Hz), 6.92 (1H, d, J = 9.2 Hz), 7.55-7.60 (1H, m), 7.84 (1H, t, J = 8.0 Hz), 7.74 (1H, d, J = 8.0 Hz), 8.14 (1H, d, J = 3.2 Hz), 8.56 (1H, t, J = 6.0 Hz), 13.60 (1H, brs) | 605(M⁺) |
| 48 | 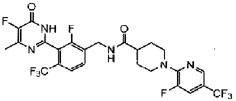 | DMSO-d₆,δ: 1.65 (2H, qd, J = 12.4, 3.6 Hz), 1.84 (2H, dd, J = 12.8, 2.4 Hz), 2.26 (3H, d, J = 3.6 Hz), 2.57 (1H, tt, J = 11.6, 4.0 Hz), 3.06 (2H, t, J = 12.0 Hz), 4.26 (2H, d, J = 13.2 Hz), 4.39 (2H, d, J = 6.0 Hz), 7.63 (1H, t, J = 7.6 Hz), 7.73 (1H, d, J = 8.0 Hz), 7.91 (1H, dd, J = 13.6, 2.0 Hz), 8.34 (1H, s), 8.57 (1H, t, J = 6.0 Hz), 13.61 (1H, brs) | 593(M⁺) |

**[Table 17]**

| **Example No.** | **Chemical Structure** | **NMR (¹H-NMR)** | **MS (m/z)** |
|---|---|---|---|
| 49 | 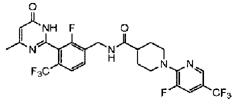 | DMSO-d₆,δ: 1.66 (2H, qd, J = 12.4, 3.2 Hz), 1.84 (2H, dd, J = 12.8, 2.4 Hz), 2.22 (3H, s), 2.57 (1H, tt, J = 11.2, 4.0 Hz), 3.06 (2H, t, J = 12.0 Hz), 4.26 (2H, d, J = 13.2 Hz), 4.39 (2H, d, J = 8.0 Hz), 6.29 (1H, brs), 7.62 (1H, t, J = 8.0 Hz), 7.72 (1H, d, J = 8.4 Hz), 7.91 (1H, dd, J = 14.4, 2.0 Hz), 8.34 (1H, s), 8.57 (1H, t, J = 6.0 Hz), 12.93 (1H, brs) | 575(M⁺) |
| 50 | 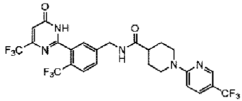 | DMSO-d₆,δ: 1.55 (2H, qd, J = 12.4, 3.6 Hz), 1.82 (2H, dd, J = 12.4, 2.4 Hz), 2.52-2.60 (1H, m), 2.93-3.04 (2H, m), 4.37-4.46 (4H, m), 6.94-6.99 (2H, m), 7.60-7.64 (2H, m), 7.76 (1H, dd, J = 8.8, 2.4 Hz), 7.87 (1H, d, J = 8.8 Hz), 8.38-8.41 (1H, m), 8.54 (1H, 1, J = 6.0 Hz), 13.61 (1H, brs) | 593(M⁺) |
| 51 | 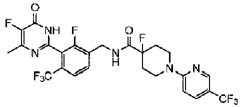 | DMSO-d₆,δ: 1.85-2.11 (4H, m), 2.25 (3H, d, J = 3.2 Hz), 3.20-3.30 (2H, m), 4.34-4.46 (4H, m), 7.05 (1H, d, J = 8.8 Hz), 7.59-7.67 (1H, m), 7.73 (1H, d, J = 8.4 Hz), 7.82 (1H, dd, J = 9.2, 2.4 Hz), 8.43 (1H, d, J = 2.8 Hz), 9.01 (1H, td, J = 6.0, 2.0 Hz), 13.61 (1H, brs) | 593(M⁺) |
| 52 | 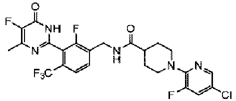 | DMSO-d₆,δ:1.67 (2H, qd, J = 12.4, 3.2 Hz), 1.82 (2H, dd, J = 13.2, 3.2 Hz), 2.26 (3H, d, J = 3.6 Hz), 2.45-2.55 (1H, m), 2.92 (2H, td, J = 12.8, 2.0 Hz), 3.98 (2H, d, J = 13.2 Hz), 4.39 (2H, d, J = 5.6 Hz), 7.63 (1H, t, J = 8.0 Hz), 7.73 (1H, d, J = 8.4 Hz), 7.80 (1H, dd, J = 12.8, 2.0 Hz), 8.07 (1H, d, J = 1.6 Hz), 8.56 (1H, t, J = 5.6 Hz), 13.61 (1H, brs) | 559(M⁺) |
| 53 | 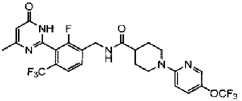 | DMSO-d₆,δ:1.56 (2H, qd, J = 12.4, 3.6 Hz), 1.80 (2H, dd, J = 13.2, 2.4 Hz), 2.22 (3H, s), 2.47-2.56 (1H, m), 2.90 (2H, td, J = 12.4, 2.4 Hz), 4.31 (2H, d, J = 13.2 Hz), 4.39 (2H, d, J = 6.0 Hz), 6.28 (1H, brs), 6.92 (1H, d, J = 10.0 Hz), 7.54-7.64 (2H, m), 7.71 (1H, d, J = 8.4 Hz), 8.14 (1H, d, J = 2.8 Hz), 8.55 (1H, t, J = 5.6 Hz), 12.96 (1H, brs) | 573(M⁺) |
| 54 | 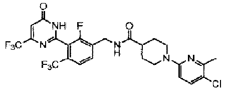 | CDCl₃,δ: 1.63 (2H, qd, J = 12.3, 3.9 Hz), 1.85 (2H, d, J = 10.8 Hz), 2.20-2.38 (1H, m), 2.45 (3H, s), 2.79 (2H, td, J = 12.7, 2.3 Hz), 4.28 (2H, d, J = 13.2 Hz), 4.48 (2H. d, J = 5.9 Hz), 6.10-6.26 (1H, m), 6.43 (1H, d, J = 9.0 Hz), 6.82 (1H, s), 7.36 (1H, d, J = 8.8 Hz), 7.48-7.61 (2H, m). | 592 [M+H]**⁺** |

**[Table 18]**

| **Example No.** | **Chemical Structure** | **NMR (¹H-NMR)** | **MS (m/z)** |
|---|---|---|---|
| 55 | 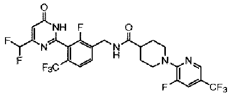 | DMSO-d₆,δ:1.57-1.75 (2H, m), 1.85 (2H, dd, J = 13.0, 2.7 Hz), 2.52-2.63 (1H, m), 3.06 (2H, t, J = 11.7 Hz), 4.28 (2H, d, J = 13.4 Hz), 4.41 (2H, d, J = 5.6 Hz), 6.59-7.00 (2H, m), 7.59-7.71 (1H, m), 7.71-7.79 (1H, m), 7.90 (1H, dd, J = 13.9, 2.0 Hz), 8.33 (1H, s), 8.56 (1H, t, J = 6.0 Hz), 13.55 (1H, brs) | 612 [M+H]**⁺** |
| 56 | 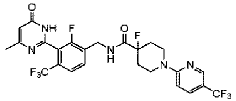 | DMSO-d₆,δ:1.86-1.98 (2H, m), 1.98-2.11 (2H, m), 2.23 (3H, s), 3.25 (2H, td, J = 12.4, 2.4 Hz), 4.39 (2H, d, J = 14.0 Hz), 4.44 (2H, d, J = 6.0 Hz), 6.30 (1H, brs), 7.05 (1H, d, J = 9.6 Hz), 7.62 (1H, t, J = 8.0 Hz), 7.72 (1H, d, J = 8.4 Hz), 7.82 (1H, dd, J = 8.8, 2.4 Hz), 8.43 (1 H, d, J = 1.6 Hz), 9.01 (1H, td, J = 6.0, 1.6 Hz), 12.94 (1H, brs) | 575(M⁺) |
| 57 | 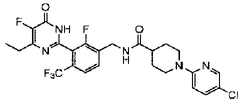 | DMSO-d₆,δ: 1.14 (3H, t, J = 8.0 Hz), 1.54 (2H, qd, J = 12.4, 3.6 Hz), 1.79 (2H, dd, J = 12.4, 2.4 Hz), 2.47-2.55 (1H, m), 2.56-2.64 (2H, m), 2.87 (2H, td, J = 12.4, 2.8 Hz), 4.28 (2H, d, J = 13.2 Hz), 4.39 (2H, d, J = 5.2 Hz), 6.88 (1H, d, J = 9.6 Hz), 7.57 (1H, dd, J = 9.2, 2.8 Hz), 7.63 (1H, t, J = 7.6 Hz), 7.73 (1H, d, J = 8.4 Hz), 8.09 (1H, d, J = 2.8 Hz), 8.56 (1H, t, J = 5.6 Hz), 13.60 (1H, brs) | 555(M⁺) |
| 58 | 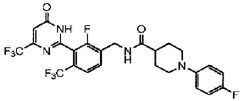 | DMSO-d₆,δ: 1.69 (2H, qd, J = 12.4, 3.6 Hz), 1.82 (2H, dd, J = 12.8, 2.4 Hz), 2.32-2.40 (1H, m), 2.64 (2H, td, J = 12.4, 2.4 Hz), 3.62 (2H, d, J = 12.4 Hz), **4.41** (2H, d, J = 5.8 Hz), 6.88 (1H, brs), 6.93-6.98 (2H, m), 700-706 (2H, m), 7.64 (1H, t, J = 7.6 Hz), 7.74 (1H, d, J = 8.0 Hz), 8.58 (1H, t, J = 6.0 Hz), 13.91 (1H, brs) | 560(M⁺) |
| 59 | 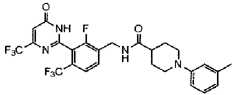 | DMSO-d₆,δ:1.67 (2H, qd, J = 12.4, 3.6 Hz), 1.81 (2H, dd, J = 12.0, 2.0 Hz), 2.24 (3H, s), 2.34-2.43 (1H, m), 2.67 (2H, td, J = 12.0, 2.4 Hz), 3.71 (2H, d, J = 12.8 Hz), 4.41 (2H, d, J = 5.6 Hz), 6.57 (1H, d, J = 7.2 Hz), 6.71-6.76 (2H, m), 7.05-7.10 (2H, m), 7.68 (1H, t, J = 8.0 Hz), 7.78 (1H, d, J = 8.0 Hz), 8.57 (1H, t, J = 6.0 Hz), 13.90 (1H, brs) | 556(M⁺) |
| 60 | 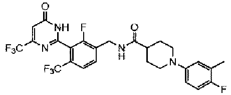 | DMSO-d₆,δ: 1.69 (2H, qd, J = 12.4, 3.6 Hz), 1.82 (2H, dd, J = 12.8, 2.4 Hz), 2.18 (3H, d, J = 1.6 Hz), 2.32-2.40 (1H, m), 2.82 (2H, td, J = 12.4, 2.0 Hz), 3.81 (2H, d, J = 12.8 Hz), 4.42 (2H, d, J = 5.6 Hz), 6.73-6.78 (1H, m), 8.85 (1H, dd, J = 6.0, 3.2 Hz), 6.95 (1 H, t, J = 9.2 Hz), 7.05 (1H, s), 7.69 (1H, t, J = 7.6 Hz), 7.78 (1H, d, J = 8.4 Hz), 8.57 (1 H, t, J = 6.0 Hz), 13.90 (1H, brs) | 574(M⁺) |

**[Table 19]**

| **Example No.** | **Chemical Structure** | **NMR (¹H-NMR)** | **MS (m/z)** |
|---|---|---|---|
| 61 | 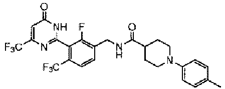 | DMSO-d₆,δ: 1.68 (2H, qd, J = 12.0, 3.6 Hz), 1.81 (2H, dd, J = 12.8, 2.4 Hz), 2.19 (3H, s), 2.32-2.40 (1H, m), 2.62 (2H, td, J = 12.4, 2.8 Hz), 3.64 (2H, d, J = 12.4 Hz), 4.41 (2H, d, J = 5.6 Hz), 6.83 (2H, d, J = 8.4 Hz), 7.01 (2H, d, J = 8.4 Hz), 7.05 (1H, s), 7.68 (1H, t, J = 8.0 Hz), 7.78 (1H, d, J = 8.4 Hz), 8.57 (1H, t, J = 6.0 Hz), 13.90 (1H, brs) | 556(M⁺) |
| 62 | 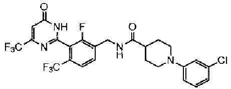 | DMSO-d₆,δ: 1.65 (2H, qd, J = 12.4, 3.6 Hz), 1.81 (2H, dd, J = 13.2, 2.0 Hz), 2.39-2.48 (1H, m), 2.75 (2H, td, J = 12.4, 2.4 Hz), 3.78 (2H, d, J = 12.8 Hz), 4.42 (2H, d, J = 5.6 Hz), 6.75 (1H, dd, J = 8.0, 1.6 Hz), 6.90 (1H, dd, J = 8.4, 1.6 Hz), 6.94 (1H, t, J = 2.4 Hz), 7.06 (1H, s), 7,19 (1H, t, J = 8.4 Hz), 7.69 (1H, t, J = 7.6 Hz), 7.78 (1H, d, J = 8.4 Hz), 8.58 (1H, t, J = 6.0 Hz), 13.90 (1H, brs) | 576(M⁺) |
| 63 | 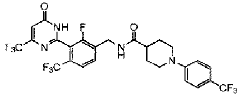 | DMSO-d₆,δ: 1.52-1.70 (2H, m), 1.82 (2H, d, J = 10.5 Hz), 2.42-2.48 (1H, m), 2.86 (2H, t, J = 11.6 Hz), 3.90 (2H, d, J = 12.7 Hz), 4.41 (2H, d, J = 5.6 Hz), 6.99 (1H, brs), 7.08 (2H, d, J = 8.8 Hz), 7.48 (2H, d, J = 8.8 Hz), 7.62-7.71 (1H, m), 7.71-7.83 (1H, m), 8.55 (1H, t, J = 5.7 Hz), 13.86 (1H, brs) | 611 [M+H]⁺ |
| 64 | 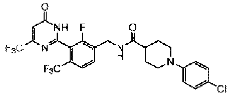 | DMSO-d₆,δ:1.66 (2H, qd, J = 12.4, 4.0 Hz), 1.81 (2H, dd, J = 12.8, 2.0 Hz), 2.36-2.45 (1H, m), 2.70 (2H, td, J = 12.0, 2.4 Hz), 3.72 (2H, d, J = 12.8 Hz), 4.41 (2H, d, J = 6.0 Hz), 6.95 (2H, d, J = 8.8 Hz), 7.06 (1H, s), 7.21 (2H, d, J = 9.2 Hz), 7.68 (1H, t, J = 8.0 Hz), 7.78 (1H, d, J = 8.0 Hz), 8.57 (1H, t, J = 6.0 Hz), 13.89 (1H, brs) | 576(M⁺) |
| 65 | 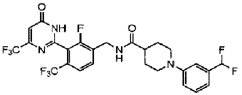 | DMSO-d₆,δ: 1.67 (2H, qd, J = 12.0, 3.2 Hz), 1.82 (2H, dd, J = 12.8, 2.4 Hz), 2.39-2.47 (1H, m), 2.75 (2H, td, J = 12.4, 2.4 Hz), 3.79 (2H, d, J = 12.4 Hz), 4.42 (2H, d, J = 5.2 Hz), 6.91 (1H, t, J = 56.0 Hz), 6.92 (1H, d, J = 8.8 Hz), 7.05-7.12 (3H, m), 7.33 (1H, t, J = 8.0 Hz), 7.69 (1H, t, J = 8.0 Hz), 7.78 (1H, d, J = 8.4 Hz), 8.58 (1H, t, J = 6.0 Hz), 13.90 (1H, brs) | 592(M⁺) |
| 66 | 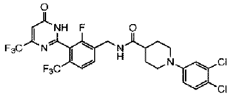 | DMSO-d₆,δ: 1.64 (2H, qd, J = 12.4, 3.2 Hz), 1.80 (2H, dd, J = 12.4, 2.0 Hz), 2.39-2.48 (1H, m), 2.76 (2H, td, J = 12.0, 2.4 Hz), 3.78 (2H, d, J = 12.4 Hz), 4.41 (2H, d, J = 6.0 Hz), 6.94 (1H, dd, J = 9.2, 2.8 Hz), 7.07 (1H, s), 7.13 (1H, d, J = 2.8 Hz), 7.37 (1H, d, J = 9.2 Hz), 7.69 (1H, t. J = 8.0 Hz), 7.78 (1H, d, J = 8.4 Hz), 8.58 (1H, t, J = 5.6 Hz), 13.90 (1H, brs) | 610(M⁺) |

**[Table 20]**

| **Example No.** | **Chemical Structure** | **NMR (¹H-NMR)** | **MS (m/z)** |
|---|---|---|---|
| 67 | 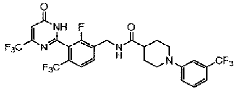 | DMSO-d₆,δ:1.87 (2H, qd, J = 12.3, 3.9 Hz), 1.78-1.89 (2H, m), 2.37-2.48 (1H, m), 2.79 (2H, td, J = 12.3, 2.4 Hz), 3.84 (2H, d, J = 12.7 Hz), 4.42 (2H, d, J = 5.9 Hz), 6.98 (1H, brs), 7.04 (1H, d, J = 7.6 Hz), 7.16 (1H, s), 7.20-7.28 (1H, m), 7.36-7.47 (1H, m), 7.62-7.71 (1H, m), 7.73-7.80 (1H, m), 8.59 (1H, t, J = 5.9 Hz), 13.90 (1H, brs) | 611 [M+H]⁺ |
| 68 | 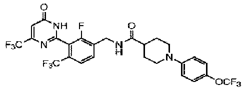 | DMSO-d₆,δ: 1.68 (2H, qd, J = 12.3, 3.8 Hz), 1.79-1.88 (2H, m), 2.41 (1H, tt, J = 11.5, 3.9 Hz), 2.73 (2H, td, J = 12.2, 2.4 Hz), 3.74 (2H, d, J = 12.7 Hz). 4.42 (2H, d, J = 5.6 Hz), 6.92-707 (3H, m), 7.17 (2H, d, J = 8.3 Hz), 7.63-7.72 (1H, m), 7.74-7.81 (1H, m), 8.58 (1H, t, J = 5.9 Hz), 13.90 (1H, brs) | 627 [M+H]⁺ |
| 69 | 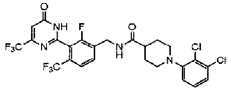 | DMSO-d₆,δ: 1.73-1.90 (4H, m), 2.35-2.45 (1H, m), 2.65-2.73 (2H, m), 3.28-3.32 (2H, m), 4.43 (2H, d, J = 5.6 Hz), 7.05 (1H, brs), 7.15 (1H, dd, J = 7.2, 3.2 Hz), 7.27-7.33 (2H, m), 7.70 (1H, t, J = 8.0 Hz), 7.79 (1H, d, J = 8.0 Hz), 8.80 (1H, t, J = 6.0 Hz), 13.90 (1H, brs) | 610(M⁺) |
| 70 | 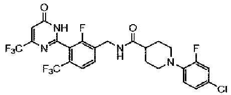 | DMSO-d₆,δ:1.74 (2H, qd, J = 12.0, 3.6 Hz), 1.85 (2H, dd, J = 12.4, 2.4 Hz), 2.32-2.42 (1H, m), 2.69 (2H, td, J = 12.0, 2.4 Hz), 3.36 (2H, d, J = 12.0 Hz), 4.42 (2H, d, J = 5.2 Hz), 7.04 (1H, s), 7.07 (1 H, d, J = 9.2 Hz), 7.17 (1H, ddd, J = 8.8, 2.4, 0.8 Hz), 7.32 (1H, dd, J = 12.4, 2.4 Hz), 7.69 (1H, t, J = 8,0 Hz), 7.78 (1H, d, J = 8.4 Hz), 8.57 (1H, t, J = 5.6 Hz), 13.89 (1H, brs) | 594(M⁺) |
| 71 | 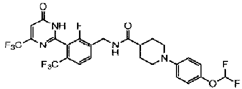 | DMSO-d₆,δ: 1.68 (2H, qd, J = 12.4, 3.6 Hz), 1.82 (2H, dd, J = 12.8, 2.4 Hz), 2.34-2.44 (1H, m), 2.88 (2H, td, J = 12.0, 2.4 Hz), 3.68 (2H, d, J = 12.4 Hz), 4.42 (2H, d, J = 5.6 Hz), 6.96 (2H, d, J = 9.6 Hz), 7.03 (2H, d, J = 9.6 Hz), 7.04 (1H, t, J = 74.8 Hz), 7.05 (1H, brs), 7.69 (1H, t, J = 7.6 Hz), 7.78 (1H, d, J = 8.0 Hz), 8.57 (1H, t, J = 6.0 Hz), 13.89 (1H, brs) | 608(M⁺) |
| 72 | 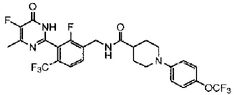 | DMSO-d₆,δ:1.67 (2H, qd, J = 12.0, 3.6 Hz), 1.82 (2H, dd, J = 12.8, 2.0 Hz), 2.26 (3H, d, J = 4.0 Hz), 2.37-2.45 (1H, m), 2.73 (2H, td, J = 12.4, 2.0 Hz), 3.74 (2H, d, J = 12.4 Hz), 4.40 (2H, d, J = 6.0 Hz), 7.00 (2H, d, J = 9.2 Hz), 7.17 (2H, d, J = 8.8 Hz), 7.65 (1H, t, J = 80 Hz), 7.74 (1H, d, J = 8.4 Hz), 8.58 (1H, t, J = 6.0 Hz), 13.61 (1H, brs) | 590(M⁺) |

**[Table 21]**

| **Example No.** | **Chemical Structure** | **NMR (¹H-NMR)** | **MS (m/z)** |
|---|---|---|---|
| 73 | 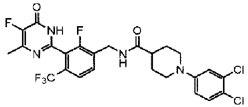 | DMSO-6₆,δ:1.63 (2H, qd, J = 12.0, 3.6 Hz), 1.80 (2H, dd, J = 12.8, 2.0 Hz), 2.26 (3H, d, J = 3.2 Hz), 2.39-2.47 (1H, m), 2.76 (2H, td, J = 12.8, 2.4 Hz), 3.78 (2H, d, J = 13.2 Hz), 4.39 (2H, d, J = 6.0 Hz), 6.94 (1H, dd, J = 8.8, 2.8 Hz), 7.13 (1H, d, J = 2.8 Hz), 7.37 (1H, d, J = 8.8 Hz), 7.64 (1H, t, J = 8.0 Hz), 7.73 (1H, d, J = 8.4 Hz), 8.56 (1H, t, J = 6.0 Hz), 13.61 (1H, brs) | 574(M⁺) |
| 74 | 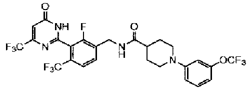 | DMSO-d₆,δ: 1.65 (2H, qd, J = 12.4, 3.6 Hz), 1.82 (2H, dd, J = 12.8, 2.0 Hz), 2.44 (1H, tt, J = 11.6, 4.0 Hz), 2.77 (2H, td, J = 12.4, 2.0 Hz), 3.80 (2H, d, J = 12.8 Hz), 4.41 (2H, d, J = 6.0 Hz), 6.66 (1H, d, J = 8.4 Hz), 6.84 (1H, s), 6.95 (1H, dd, J = 8.4, 2.0 Hz), 7.05 (1H, s), 7.29 (1H, t, J = 8.4 Hz), 7.69 (1H, t, J = 7.6 Hz), 7.78 (1H, d, J = 8.4 Hz), 8,58 (1H, t, J = 6.0 Hz), 13.89 (1H, brs) | 626(M⁺) |
| 75 | 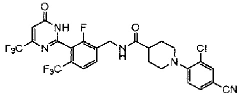 | DMSO-d₆,δ:1.77 (2H, qd, J = 11.6, 3.2 Hz), 1.88 (2H, dd, J = 11.6, 2.0 Hz), 2.39-2.48 (1H, m), 2.78 (2H, td, J = 12.4, 2.0 Hz), 3.46 (2H, d, J = 12.4 Hz), 4.43 (2H, d, J = 5.2 Hz), 7.04 (1H, s), 7.25 (1H, d, J = 8.8 Hz), 7.69 (1H, t, J = 8.0 Hz), 7.74 (1H, dd, J = 8.4, 2.0 Hz), 7.78 (1H, d, J = 8.0 Hz), 7.94 (1H, d, J = 2.0 Hz), 8.60 (1H, t, J = 5.6 Hz), 13.89 (1H, brs) | 601(M⁺) |
| 76 | 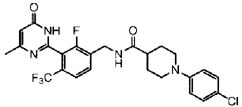 | DMSO-d₆,δ; 1.66 (2H, qd, J = 12.4, 3.6 Hz), 1.81 (2H, dd, J = 13.2, 2.4 Hz), 2.23 (3H, s), 2.40 (1H, tt, J = 11.6, 4.0 Hz), 2.70 (2H, td, J = 12.4, 2.4 Hz), 3.72 (2H, d, J = 12.8 Hz), 4.39 (2H, d, J = 6.0 Hz), 6.29 (1H, brs), 6.95 (2H, d, J = 8.8 Hz), 7.21 (2H, d, J = 9.2 Hz), 7.62 (1H, t, J = 7.6 Hz), 7.72 (1H, d, J = 8.0 Hz), 8.55 (1H, t, J = 6.0 Hz), 12.95 (1H, brs) | 522(M⁺) |
| 77 | 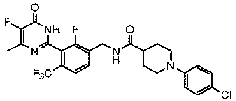 | DMSO-d₆,δ:1.66 (2H, qd, J = 12.4, 3.6 Hz), 1.81 (2H, dd, J = 12.4, 2.4 Hz), 2.26 (3H, d, J = 3.2 Hz), 2.40 (1H, tt, J = 11.6, 3.6 Hz), 2.70 (2H, td, J = 12.4, 2.4 Hz), 3.72 (2H, d, J = 12.8 Hz), 4.39 (2H, d, J = 5.6 Hz), 6.95 (2H, d, J = 9.2 Hz), 7.21 (2H, d, J = 9.2 Hz), 7.64 (1H, t, J = 8.0 Hz), 7.73 (1H, d, J = 8.4 Hz), 8.56 (1H, t, J = 5.6 Hz), 13.61 (1H, brs) | 540(M⁺) |
| 78 | 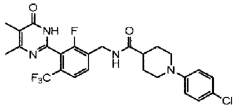 | DMSO-d₆,δ:1.66 (2H, qd, J = 12.4, 3.6 Hz), 1.82 (2H, d, J = 12.0 Hz), 1.98 (3H, s), 2.24 (3H, s), 2.40 (1H, tt, J = 11.6, 4.0 Hz), 2.70 (2H, td, J = 12.0, 2.0 Hz), 3.72 (2H, d, J = 12.8 Hz), 4.39 (2H, d, J = 6.0 Hz), 8.95 (2H, d, J = 9.2 Hz), 7.21 (2H, d, J = 9.2 Hz), 7.81 (1 H, t, J = 8.0 Hz), 7.71 (1H, d, J = 8.4 Hz), 8.55 (1H, t, J = 6.0 Hz), 12.89 (1H, brs) | 536(M⁺) |

**[Table 22]**

| **Example No.** | **Chemical Structure** | **NMR (¹H-NMR)** | **MS (m/z)** |
|---|---|---|---|
| 79 | 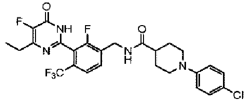 | DMSO-d₆,δ:1.14 (3H, t, J = 8.0 Hz), 1.66 (2H, qd, J = 12.4, 3.6 Hz), 1.81 (2H, dd, J = 12.8, 2.4 Hz), 2.40 (1H, tt, J = 11.6, 4.0 Hz), 2.57-2.63 (2H, m), 2.71 (2H, td, J = 12.0, 2.4 Hz), 3.72 (2H, d, J = 12.8 Hz), 4.40 (2H, d, J = 5.6 Hz), 6.95 (2H, d, J = 9.2 Hz), 7.21 (2H, d, J = 9.2 Hz), 7.64 (1H, t, J = 8.0 Hz), 7.74 (1H, d, J = 8.0 Hz), 8.58 (1H, t, J = 5.6 Hz), 13.61 (1H, brs) | 554(M⁺) |
| 80 | 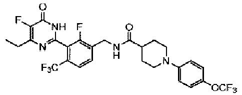 | DMSO-d₆,δ:1.14 (3H, t, J = 7.6 Hz), 1.67 (2H, qd, J = 12.4, 4.0 Hz), 1.83 (2H, dd, J = 12.4, 2.4 Hz), 2.41 (1H, tt, J = 11.6, 3.6 Hz), 2.56-2.64 (2H, m), 2.73 (2H, td, J = 12.4, 2.4 Hz), 3.74 (2H, d, J = 12.4 Hz), 4.40 (2H, d, J = 6.0 Hz), 7.01 (2H, d, J = 8.8 Hz), 7.17 (2H, d, J = 8.4 Hz), 7,64 (1H, t, J = 8.0 Hz), 7.74 (1H, d, J = 8.4 Hz), 8.56 (1H, t, J = 6.0 Hz), 13.61 (1H, brs) | 604(M+) |
| 81 | 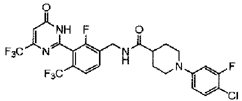 | DMSO-d₆,δ:1.63 (2H, qd, J = 12.4, 3.6 Hz), 1.80 (2H, d, J = 10.8 Hz), 2.43 (1H, tt, J = 12.0, 4.0 Hz), 2.76 (2H, td, J = 12.4, 2.4 Hz), 3.78 (2H, d, J = 13.2 Hz), 4.41 (2H, d, J = 6.0 Hz), 6.78 (1H, dd, J = 8.8, 2.4 Hz), 6.95 (1H, dd, J = 13.6, 2.8 Hz), 7.04 (1H, s), 7.31 (1H, t, J = 8.8 Hz), 7.68 (1H, t, J = 8.0 Hz), 7.77 (1H, d, J = 8.4 Hz), 8.57 (1H, t, J = 6.0 Hz), 13.89 (1H, brs) | 594(M⁺) |
| 82 | 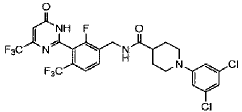 | DMSO-d₆,δ:1.61 (2H, qd, J = 12.4, 3.2 Hz), 1.79 (2H, dd, J = 12.8, 2.4 Hz), 2.45 (1H, tt, J = 11.2, 4.0 Hz), 2.80 (2H, td, J = 12.8, 2.0 Hz), 3.83 (2H, d, J = 13.2 Hz), 4.41 (2H, d, J = 5.6 Hz), 6.82 (1H, t, J = 2.0 Hz), 6.94 (2H, d, J = 2.0 Hz), 7.05 (1H, s), 7.68 (1H, t, J = 7.6 Hz), 7.78 (1H, d, J = 8.4 Hz), 8.57 (1H, t, J = 5.6 Hz), 13.89 (1H, brs) | 610(M⁺) |
| 83 | 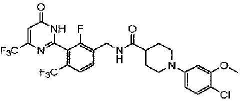 | DMSO-d₆,δ:1.67 (2H, qd, J = 12.4, 3.6 Hz), 1.82 (2H, d, J = 10.8 Hz), 2.41 (1H, tt, J = 11.6, 4.0 Hz), 2.72 (2H, td, J = 12.4, 2.0 Hz), 3.76 (2H, d, J = 12.8 Hz), 3.82 (3H, s), 4.42 (2H, d, J = 5.6 Hz), 6.50 (1H, dd, J = 8.8, 2.8 Hz), 6.64 (1H, d, J = 3.2 Hz), 7.05 (1H, s), 7.16 (1H, d, J = 8.4 Hz), 7.69 (1H, t, J = 8.0 Hz), 7.78 (1H, d, J = 8.4 Hz), 8.57 (1H, t, J = 5.6 Hz), 13.89 (1H, brs) | 606(M⁺) |
| 84 | 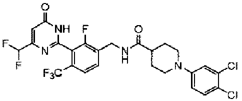 | DMSO-d₆,δ: 1.64 (2H, qd, J = 12.0, 3.6 Hz), 1.80 (2H, dd, J = 12.8, 2.0 Hz), 2.43 (1H, tt, J = 11.6, 4.0 Hz), 2.76 (2H, td, J = 12.4, 2.0 Hz), 3.78 (2H, d, J = 13.2 Hz), 4.41 (2H, d, J = 5.6 Hz), 6.74 (1H, brs), 6.79 (1H, t, J = 54.4 Hz), 6.94 (1H, dd, J = 9.2, 2.8 Hz), 7.13 (1H, d, J = 2.8 Hz), 7.37 (1H, d, J = 9.2 Hz), 7.66 (1H, t, J = 7.6 Hz), 7.76 (1H, d, J = 8.4 Hz), 8.57 (1H, t, J = 5.6 Hz), 13.56 (1H, brs) | 592(M⁺) |

**[Table 23]**

| **Example No.** | **Chemical Structure** | **NMR (¹H-NMR)** | **MS (m/z)** |
|---|---|---|---|
| 85 | 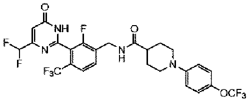 | DMSO-d₆,δ: 1.67 (2H, qd, J = 12.4, 3.6 Hz), 1.83 (2H, dd, J = 12.8, 2.0 Hz), 2.41 (1H, tt, J = 12.0, 4.0 Hz), 2.73 (2H, td, J = 12.4, 2.4 Hz), 3.74 (2H, d, J = 12.4 Hz), 4.41 (2H, d, J = 6.0 Hz), 6.74 (1H, brs), 6.79 (1H, t, J = 54.0 Hz), 7.01 (2H, d, J = 9.2 Hz), 7.17 (2H, d, J = 8.4 Hz), 7.67 (1H, t, J = 8.0 Hz), 7.76 (1H, d, J = 8.0 Hz), 8.57 (1H, t, J = 6.0 Hz), 13.57 (1H, brs) | 608(M⁺) |
| 86 | 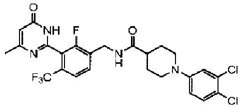 | DMSO-d₆,δ:1.64 (2H, qd, J = 12.4, 3.6 Hz), 1.80 (2H, dd, J = 12.8, 2.4 Hz), 2.23 (3H, s), 2.43 (1H, tt, J = 12.0, 4.0 Hz), 2.76 (2H, td, J = 12.4, 2.4 Hz), 3.78 (2H, d, J = 12.8 Hz), 4.39 (2H, d, J = 6.0 Hz), 6.29 (1H, brs), 6.94 (1H, dd, J = 9.2, 2.8 Hz), 7.13 (1H, d, J = 2.8 Hz), 7.37 (1H, d, J = 8.8 Hz), 7.62 (1H, t, J = 8.0 Hz), 7.72 (1H, d, J = 8.4 Hz), 8.55 (1H, t, J = 6.0 Hz), 12.95 (1H, brs) | 556(M⁺) |
| 87 | 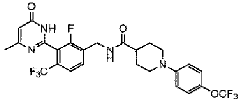 | DMSO-d₆,δ:1.67 (2H, qd, J = 12.4, 3.2 Hz), 1.82 (2H, d, J = 11.2 Hz), 2.23 (3H, s), 2.41 (1H, tt, J = 11.6, 4.0 Hz), 2.70-2.76 (2H, m), 3.74 (2H, d, J = 12.0 Hz), 4.40 (2H, d, J = 5.2 Hz), 8.29 (1H, brs), 7.01 (2H, d, J = 9.2 Hz), 7.17 (2H, d, J = 8.8 Hz), 7.62 (1H, t, J = 8.0 Hz), 7.72 (1H, d, J = 8.4 Hz), 8.56 (1H, t, J = 5.6 Hz), 12.94 (1H, brs) | 572(M⁺) |
| 88 | 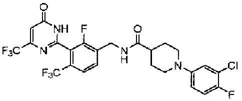 | DMSO-d₆,δ:1.67 (2H, qd, J = 12.4, 4.0 Hz), 1.82 (2H, dd, J = 12.8, 2.8 Hz), 2.39 (1H, tt, J = 12.0, 4.0 Hz), 2.69 (2H, td, J = 12.4, 2.4 Hz), 3.69 (2H, d, J = 12.8 Hz), 4.41 (2H, d, J = 6.0 Hz), 6.91-6.96 (1H, m), 7.05 (1H, s), 7.07 (1H, dd, J = 6.4, 2.8 Hz), 7.22 (1H, t, J = 9.2 Hz), 7.69 (1 H, t, J = 8.0 Hz), 7.78 (1H, d, J = 8.4 Hz), 8.57 (1H, t, J = 6.0 Hz), 13.89 (1H, brs) | 594(M⁺) |
| 89 | 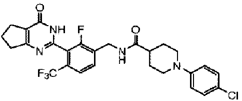 | DMSO-d₆,δ:1.66 (2H, qd, J = 12.4, 3.6 Hz), 1.81 (2H, dd, J = 13.2, 2.4 Hz), 2.02 (2H, quin, J = 7.6 Hz), 2.40 (1H, tt, J = 11.6, 4.0 Hz), 2.67-2.74 (4H, m), 2.80 (2H, t, J = 8.0 Hz), 3.72 (2H, d, J = 12.4 Hz), 4.39 (2H, d, J = 6.0 Hz), 6.95 (2H, d, J = 9.6 Hz), 7.21 (2H, d, J = 8.8 Hz), 7.62 (1H, t, J = 8.0 Hz), 7.72 (1H, d, J = 8.4 Hz), 8.55 (1H, t, J = 6.0 Hz), 12.90 (1H, brs) | 548(M⁺) |
| 90 | 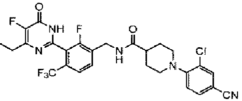 | DMSO-d₆,δ:1.14 (3H, t, J = 8.0 Hz), 1.77 (2H, qd, J = 12.0, 3.6 Hz), 1.88 (2H, dd, J = 12.0, 2.8 Hz), 2.39-2.49 (1H, m), 2.56-2.64 (2H, m), 2.78 (2H, td, J = 12.0, 2.0 Hz), 3.46 (2H, d, J = 12.4 Hz), 4.41 (2H, d, J = 6.0 Hz), 7.25 (1H, d, J = 8.4 Hz), 7.65 (1H, t, J = 8.0 Hz), 7.72-7.76 (2H, m), 7.94 (1H, d, J = 2.0 Hz), 8.59 (1H, t, J = 6.0 Hz), 13.61 (1H, brs) | 579(M⁺) |

**[Table 24]**

| **Example No.** | **Chemical Structure** | **NMR (¹H-NMR)** | **MS (m/z)** |
|---|---|---|---|
| 91 | 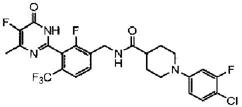 | DMSO-d₆,δ:1.63 (2H, qd, J = 12.4, 3.6 Hz), 1.80 (2H, dd, J = 13.2, 2.4 Hz), 2.26 (3H, d, J = 3.6 Hz), 2.43 (1H, tt, J = 11.6, 4.0 Hz), 2.76 (2H, td, J = 12.4, 2.8 Hz), 3.78 (2H, d, J = 13.2 Hz), 4.39 (2H, d, J = 6.0 Hz), 6.78 (1H, dd, J = 8.4, 2.4 Hz), 6.95 (1H, dd, J = 13.2, 2.4 Hz), 7.31 (1H, t, J = 9.2 Hz), 7.64 (1H, t, J = 8.0 Hz), 7.73 (1H, d, J = 8.4 Hz), 8.58 (1H, t, J = 5.6 Hz), 13.61 (1H, brs) | 558(M⁺) |
| 92 | 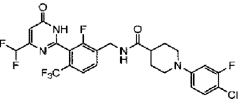 | DMSO-d₆,δ:1.63 (2H, qd, J = 12.0, 3.6 Hz), 1.77-1.83 (2H, m), 2.43 (1H, tt, J = 12.0, 4.0 Hz), 2.76 (2H, td, J = 12.4, 2.4 Hz), 3.78 (2H, d, J = 12.4 Hz), 4.41 (2H, d, J = 5.2 H_{z}), 6.74 (1H, brs), 6.78 (1H, dd, J = 8.4, 2.8 Hz), 6.79 (1H, t, J = 54.0 Hz), 6.95 (1H, dd, J = 13.6, 2.8 Hz), 7.31 (1H, t, J = 8.8 Hz), 7.66 (1H, t, J = 8.0 Hz), 7.76 (1H, d, J = 8.0 Hz), 8.56 (1H, t, J = 5.2 Hz), 13.57 (1H, brs) | 576(M⁺) |
| 93 | 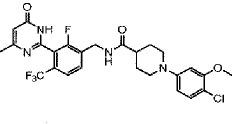 | DMSO-d₆,δ:1.67 (2H, qd, J = 12.4, 3.6 Hz), 1.82 (2H, dd, J = 13.2, 2.4 Hz), 2.23 (3H, s), 2.41 (1H, tt, J = 11.2, 4.0 Hz), 2.72 (2H, td, J = 12,4, 2.4 Hz), 3.76 (2H, d, J = 12.0 Hz), 3.82 (3H, s), 4.40 (2H, d, J = 6.0 Hz), 6.29 (1H, brs), 6.50 (1H, dd, J = 8.8, 2.8 Hz), 6.64 (1H, d, J = 2.4 Hz), 7.16 (1H, d, J = 8.8 Hz), 7.63 (1H, t, J = 7.6 Hz), 7.72 (1H, d, J = 8.4 Hz), 8.55 (1H, t, J = 6.0 Hz), 12.95 (1H, brs) | 552(M⁺) |
| 94 | 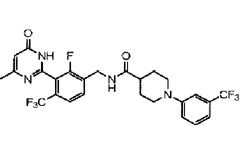 | DMSO-d₆,δ:1.67 (2H, qd, J = 12.4, 3.6 Hz), 1.84 (2H, d, J = 12.8 Hz), 2.23 (3H, s), 2.44 (1H, tt, J = 11.6, 4.0 Hz), 2.79 (2H, td, J = 12.4, 2.4 Hz), 3.84 (2H, d, J = 12.8 Hz), 4.40 (2H, d, J = 6.0 Hz), 6.29 (1H, brs), 7.03 (1H, d, J = 8.0 Hz), 7.16 (1H, s), 7.23 (1H, dd, J = 8.4, 2.0 Hz), 7.40 (1H, t, J = 8.4 Hz), 7.63 (1H, t, J = 7.6 Hz), 7.72 (1H, d, J = 8.4 Hz), 8.57 (1H, t, J = 6.0 Hz), 12.95 (1H, brs) | 556(M⁺) |
| 95 | 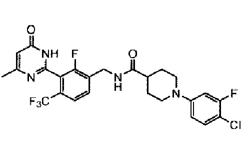 | DMSO-d₆,δ:1.63 (2H, qd, J = 12.4, 3.2 Hz), 1.80 (2H, dd, J = 12.8, 2.4 Hz), 2.23 (3H, s), 2.43 (1H, tt, J = 12.0, 4.0 Hz), 2.76 (2H, td, J = 12.4, 2.4 Hz), 3.78 (2H, d, J = 12.4 Hz), 4.39 (2H, d, J = 5.6 Hz), 6.29 (1H, brs), 6.78 (1H, dd, J = 8.8, 2.8 Hz), 5.95 (1H, dd, J = 9.2, 2.8 Hz), 7.31 (1H, t, J = 9.2 Hz), 7.62 (1H, t, J = 8.0 Hz), 7.72 (1H, d, J = 8.4 Hz), 8.55 (1H, **t,** J = 6.0 Hz), 12.94 (1H, brs) | 540(M⁺) |
| 96 | 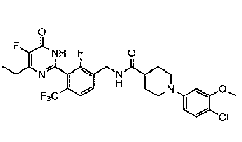 | DMSO-d₆,δ:1.14 (3H, t, J = 7.6 Hz), 1.67 (2H, qd, J = 12.4, 4.0 Hz), 1.82 (2H, dd, J = 12.4, 2.0 Hz), 2.41 (1H, tt, J = 11.6, 4.0 Hz), 2.56-2.64 (2H, m), 2.72 (2H, td, J = 12.4, 2.0 Hz), 3.76 (2H, d, J = 12.4 Hz), 3.82 (3H, s), 4.40 (2H, d, J = 6.0 Hz), 6.50 (1H, dd, J = 8.8, 2.4 Hz), 6.64 (1H, d, J = 2.4 Hz), 7.16 (1H, d, J = 8.8 Hz), 7.64 (1H, t, J = 8.0 Hz), 7.72 (1H, d, J = 8.4 Hz), 3.56 (1H, t, J = 6.0 Hz), 13.61 (1H, brs) | 584(M⁺) |

**[Table 25]**

| **Example No.** | **Chemical Structure** | **NMR (¹H-NMR)** | **MS (m/z)** |
|---|---|---|---|
| 97 | 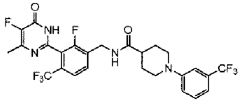 | DMSO-d₆,δ:1.67 (2H, qd, J = 12.4, 3.6 Hz), 1.79-1.88 (2H, m), 2.26 (3H, d, J = 3.6 Hz), 2.39-2.54 (1H, m), 2.79 (2H, td, J = 12.4, 2.4 Hz), 3.84 (2H, d, J = 12.8 Hz), 4.40 (2H, d, J = 5.6 Hz), 7.04 (1H, d, J = 7.2 Hz), 7.16 (1H, s), 7.21-7.26 (1H, m), 7.40 (1H, t, J = 8,0 Hz), 7.65 (1H, t, J = 7.6 Hz), 7.74 (1H, d, J = 8.0 Hz), 8.57 (1H, t, J = 6.0 Hz), 13.61 (1H, brs) | 574(M⁺) |
| 98 | 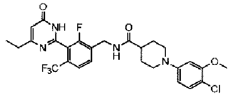 | DMSO-d_{θ},δ:1.13 (3H, t, J = 7.6 Hz), 1.68 (2H, qd, J = 12.4, 3.6 Hz), 1.82 (2H, dd, J = 13.2, 2.4 Hz), 2.36-2.54 (3H, m), 2.72 (2H, td, J = 12.0, 2.0 Hz), 3.76 (2H, d, J = 12.0 Hz), 3.82 (3H, s), 4.40 (2H, d, J = 6.0 Hz), 6.25 (1H, brs), 6.50 (1H, dd, J = 8.8, 2.4 Hz), 6.64 (1H, d, J = 2.4 Hz), 7.17 (1H, d, J = 8.8 Hz), 7.63 (1H, t, J = 8.0 Hz), 7.72 (1H, d, J = 8.4 Hz), 8.55 (1H, t, J = 6.0 Hz), 12.94 (1H, brs) | 566(M⁺) |
| 99 | 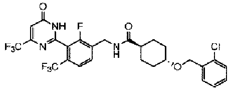 | DMSO-d₆,δ:1.18-1.29 (2H, m), 1.43 (2H, qd, J = 12.0, 2.8 Hz), 1.84 (2H, d, J = 11.2 Hz), 2,11 (2H, dd, J = 12.4, 3.2 Hz), 2.21 (1H, tt, J = 11.6, 3.2 Hz), 3.32-3.42 (1H, m), 4.39 (2H, d, J = 5.6 Hz), 4.58 (2H, s), 7.05 (1H, s), 7.29-7.38 (2H, m), 7.43 (1H, dd, J = 7.6, 1.6 Hz), 7.50 (1H, dd, J = 7.6, 1.6 Hz), 7.66 (1H, t, J = 8.0 Hz), 7.77 (1H, d, J = 8.4 Hz), 8.49 (1H, t, J = 6.0 Hz), 13.89 (1H, brs) | 605(M⁺) |
| 100 | 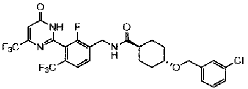 | DMSO-d₆,δ:1.22 (2H, qd, J = 13.2, 2.8 Hz), 1.41 (2H, qd, J = 12.8, 2.4 Hz), 1.84 (2H, dd, J = 13.6, 2.4 Hz), 2.04-2.12 (2H, m), 2.20 (1H, td, J = 11.6, 3.6 Hz), 3.29-3.36 (1H, m), 4.39 (2H, d, J = 5.2 Hz), 4.52 (2H, s), 7.03 (1H, brs), 7.26-7.40 (4H, m), 7.66 (1H, t, J = 8.0 Hz), 7.77 (1H, d, J = 8.4 Hz), 8.49 (1H, t, J = 5.6 Hz), 13.88 (1H, brs) | 605(M⁺) |
| 101 | 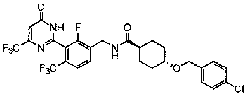 | DMSO-d₆,δ:1.15-1.26 (2H, m), 1.40 (2H, qd, J = 12.4, 2.4 Hz), 1.83 (2H, d, J = 11.6 Hz), 2.08 (2H, dd, J = 12.4, 3.6 Hz), 2.20 (1H, tt, J = 12.0, 3.6 Hz), 3.26-3.34 (1H, m), 4.38 (2H, d, J = 5.6 Hz), 4.50 (2H, s), 7.05 (1H, s), 7.34 (2H, d, J = 8.4 Hz), 7.40 (2H, d, J = 8.4 Hz), 7.66 (1H, t, J = 8.0 Hz), 7.77 (1H, d, J = 7.6 Hz), 8.49 (1H, t, J = 6.0 Hz), 13.88 (1H, brs) | 605(M⁺) |
| 102 | 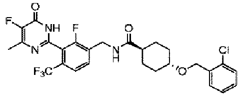 | DMSO-d₆,δ:1.24 (2H, qd, J = 13.2, 2.8 Hz), 1.43 (2H, qd, J = 12.0, 2.4 Hz), 1.84 (2H, d, J = 11.2 Hz), 2.11 (2H, dd, J = 12.8, 2.8 Hz), 2.16-2.26 (1H, m), 2.26 (3H, d, J = 3.6 Hz), 3.32-3.42 (1H, m), 4.37 (2H, d, J = 5.6 Hz), 4.58 (2H, s), 7.29-7.38 (2H, m), 7.43 (1H, dd, J = 7.6, 2.0 Hz), 7.50 (1H, dd, J = 7.6, 2.4 Hz), 7.62 (1H, t, J = 8.0 Hz), 7.73 (1H, d, J = 8.4 Hz), 8.48 (1H, t, J = 5.6 Hz), 13.61 (1H, brs) | 569(M⁺) |

**[Table 26]**

| **Example No.** | **Chemical Structure** | **NMR (¹H-NMR)** | **MS (m/z)** |
|---|---|---|---|
| 103 | 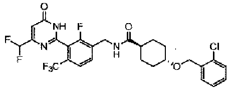 | DMSO-d₆,δ:1.24 (2H, qd, J = 13.2, 3.6 Hz), 1.43 (2H, qd, J = 12.8, 2.8 Hz), 1.84 (2H, d, J = 11.2 Hz), 2.11 (2H, dd, J = 12.8, 2.8 Hz), 2.21 (1H, tt, J = 11.6, 3.6 Hz), 3.33-3.42 (1H, m), 4.38 (2H, d, J = 5.6 Hz), 4.58 (2H, s), 8.73 (1H, brs), 6.79 (1H, t, J = 54.4 Hz), 7.29-7.38 (2H, m), 7.43 (1H, dd, J = 7.6, 2.0 Hz), 7.50 (1H, dd, J = 7.6, 2.4 Hz), 7.65 (1H, t, J = 8.0 Hz), 7.75 (1H, d, J = 8.4 Hz), 8.49 (1H, t, J = 6.0 Hz), 13.56 (1H, brs) | 587(M⁺) |
| 104 | 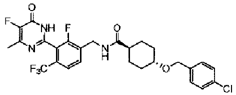 | DMSO-d₆,δ:1.20 (2H, qd, J = 12.8, 3.6 Hz), 1.40 (2H, qd, J = 12.8, 2.4 Hz), 1.82 (2H, d, J = 11.2 Hz), 2.08 (2H, dd, J = 12.0, 2.8 Hz), 2.15-2.24 (1H, m), 2.26 (3H, d, J = 3.6 Hz), 3.27-3.35 (1H, m), 4.37 (2H, d, J = 6.0 Hz), 4.50 (2H, s), 7.34 (2H, d, J = 8.4 Hz), 7.40 (2H, d, J = 8.4 Hz), 7.61 (1H, t, J = 8.0 Hz), 7.73 (1H, d, J = 8.4 Hz), 8.48 (1H, t, J = 6.0 Hz), 13,61 (1H, brs) | 569(M⁺) |
| 105 | 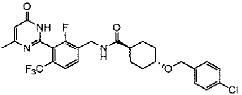 | DMSO-d₆,δ:1.21 (2H, qd, J = 13.2, 3.6 Hz), 1.40 (2H, qd, J = 12.8, 2.8 Hz), 1.82 (2H, d, J = 11.2 Hz), 2.08 (2H, dd, J = 13.2, 2.8 Hz), 2.16-2.24 (1H, m), 2.22 (3H, s), 3.26-3.36 (1H, m), 436 (2H, d, J = 6.0 Hz), 4.50 (2H, s), 6.29 (1H, brs), 7.34 (2H, d, J = 8.4 Hz), 7.39 (2H, d, J = 8.4 Hz), 7.59 (1H, t, J = 7.6 Hz), 7.71 (1H, d, J = 8.4 Hz), 8.47 (1H, t, J = 6.0 Hz), 12.94 (1H, brs) | 551(M⁺) |
| 106 | 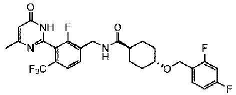 | DMSO-d₆,δ:1.19 (2H, qd, J = 13.2, 3.2 Hz), 1.41 (2H, qd, J = 12.4, 2.8 Hz), 1.82 (2H, d, J = 11.6 Hz), 2.07 (2H, dd, J = 12.8, 3.2 Hz), 2.15-2.24 (1H, m), 2.22 (3H, s), 3.29-3.37 (1H, m), 4.37 (2H, d, J = 6.0 Hz), 4.52 (2H, s), 8.28 (1H, brs), 7.07 (1H, tdd, J = 8.8, 2.4, 1.2 Hz), 7.21 (1H, td, J = 9.2, 2.8 Hz), 7.48 (1H, td, J = 8.8, 6,8 Hz), 7.59 (1H, t, J = 8.0 Hz), 7.71 (1H, d, J = 8.4 Hz), 8.47 (1 H, t, J = 6.0 Hz), 12.96 (1H, brs) | 553(M⁺) |
| 107 | 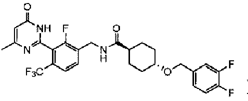 | DMSO-d₆,δ:1.21 (2H, qd, J = 13.2, 3.2 Hz), 1.41 (2H, qd, J = 12.4, 2.8 Hz), 1.82 (2H, d, J = 12.0 Hz), 2.08 (2H, dd, J = 13.2, 2.8 Hz), 2.15-2.25 (1H, m), 2.23 (3H, s), 3.27-3.36 (1H, m), 4.37 (2H, d, J = 6.0 Hz), 4.50 (2H, s), 6.28 (1H, brs), 7.15-7.19 (1H, m), 7.32-7.43 (2H, m), 7.60 (1H, t, J = 8.0 Hz), 7.71 (1H, d, J = 8.4 Hz), 8.47 (1H, t, J = 6.0 Hz), 12.95 (1H, brs) | 553(M⁺) |
| 108 | 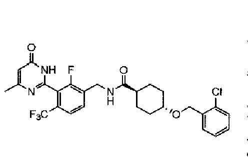 | DMSO-d₆,δ:1.24 (2H, qd, J = 12.4, 2.8 Hz), 1.42 (2H, qd, J = 12.0, 2.8 Hz), 1.84 (2H, d, J = 11.2 Hz), 2.11 (2H, dd, J = 12.8, 3.2 Hz), 2.17-2.25 (1H, m), 2.23 (3H, s), 3.33-3.42 (1H, m), 4.37 (2H, d, J = 5.6 Hz), 4.58 (2H, s), 6.28 (1H, brs), 7.29-7.38 (2H, m), 7.43 (1H, dd, J = 7.6, 2.0 Hz), 7.50 (1H, dd, J = 7.6, 2.0 Hz), 7.60 (1H, t, J = 8.0 Hz), 7.71 (1H, d, J = 8.0 Hz), 8.48 (1H, t, J = 5.6 Hz), 12.95 (1H, brs) | 551(M⁺) |

**[Table 27]**

| **Example No.** | **Chemical Structure** | **NMR (¹H-NMR)** | **MS (m/z)** |
|---|---|---|---|
| 109 | 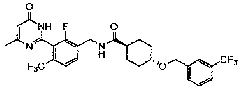 | DMSO-d₆,δ: 1.24 (2H, qd, J = 13.2, 3.6 Hz), 1.42 (2H, qd, J = 12.8, 2.8 Hz), 1.83 (2H, d, J = 12.0 Hz), 2.10 (2H, dd, J = 12.4, 3.2 Hz), 2.16-2.24 (1H, m), 2.22 (3H, s), 3.30-3.30 (1H, m), 4.37 (2H, d, J = 5.2 Hz), 4.02 (2H, s), 8.28 (1H, brs), 7.55-7.66 (5H, m), 7.71 (1H, d, J = 8.0 Hz), 8.48 (1H, t, J = 6.0 Hz), 12.95 (1H, brs) | 585(M⁺) |
| 110 | 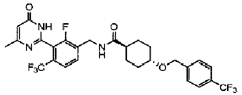 | DMSO-d₆,δ: 1.23 (2H, qd, J = 12.4, 3.2 Hz), 1.42 (2H, qd, J = 13.2, 2.8 Hz), 1.83 (2H, d, J = 11.2 Hz), 2.11 (2H, dd, J = 12.8, 3.2 Hz), 2.16-2.25 (1H, m), 2.22 (3H, s), 3.30-3.38 (1H, m), 4.37 (2H, d, J = 6.0 Hz), 4.62 (2H, s), 6.28 (1H, brs), 7.54 (2H, d, J = 8.4 Hz), 7.60 (1H, t, J = 8.0 Hz), 7.69-7.73 (3H, m), 8.48 (1H, t, J = 6.0 Hz), 12.95 (1H, brs) | 585(M⁺) |
| 111 | 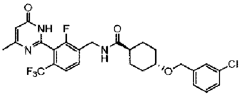 | DMSO-d₆,δ:1.20-1.27 (2H, m), 1.41 (2H, qd, J = 13.2, 2.8 Hz), 1.83 (2H, d, J = 11.2 Hz), 2.05-2.13 (2H, m), 2.15-2.28 (4H, m), 3.27-3.37 (1H, m), 4.37 (2H, d, J = 6.0 Hz), 4.52 (2H, s), 6.27 (1H, brs), 7.26-7.40 (4H, m), 7.60 (1H, t, J = 8.0 Hz), 7.72 (1H, d, J = 8.4 Hz), 8.47 (1H, t, J = 6.0 Hz), 12.97 (1H, brs) | 551(M⁺) |
| 112 | 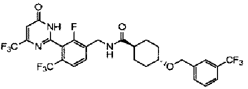 | CDCl₃,δ: 1.23-1.36 (2H, m), 1.38-1.53 (2H, m), 1.82-1.94 (2H, m), 2.04-2,12 (1H, m), 2.16 (2H, dd, J = 13.2, 3.7 Hz), 3.28-3.39 (1H, m), 4.44 (2H, d, J = 6.1 Hz), 4.59 (2H, s), 6.15 (1H, t, J = 6.0 Hz), 6.82 (1H, s), 7.41-7.56 (5H, m), 7.60 (1H, s) | 640 [M+H]⁺ |
| 113 | 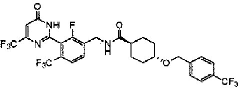 | CDCl₃,δ: 1.20-1.37 (2H, m), 1.39-1.54 (2H, m), 1.79-1.97 (2H, m), 2.02-2.24 (3H, m), 3.19-3.45 (1H, m), 4.45 (2H, d, J = 6.1 Hz), 4.60 (2H, s), 6.11 (1H, t, J = 6.1 Hz), 6.82 (1H, s), 7.44 (2H, d, J = 8.1 Hz), 7.50-7.57 (2H, m), 7.59 (2H, d, J = 8.3 Hz) | 640 [M,H]⁺ |
| 114 | 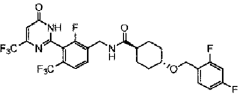 | CDCl₃,δ : 1.18-1.35 (2H, m), 1.38-1.54 (2H. m), 1.81-1.94 (2H, m), 2.03-2.20 (3H, m), 3.25-3.38 (1H, m), 4.44 (2H, d, J = 6.1 Hz), 4.55 (2H, s), 6.18 (1H, t, J = 6.0 Hz), 6.75-6.82 (2H, m), 6.82-6.91 (1H, m), 7.34-7.44 (1H, m), 7.54 (2H, s) | 608 [M+H]⁺ |

**[Table 28]**

| **Example No.** | **Chemical Structure** | **NMR (¹H-NMR)** | **MS (m/z)** |
|---|---|---|---|
| 115 | 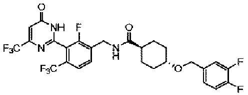 | CDCl₃,δ: 1.18-1.35(2H, m), 1.37-1.54 (2H, m), 1.80-1.94 (2H, m), 2.03-2.18 (3H, m), 3.25-3.37 (1H, m), 4.44 (2H, d, J = 5.9 Hz), 4.48 (2H, s), 6.16 (1H, br t, J = 5.7 Hz), 6.81 (1H, s), 6.88-7.05 (1H, m), 7.06-7.20 (2H, m), 7.53 (2H, s) | 608 [M+H]⁺ |
| 116 | 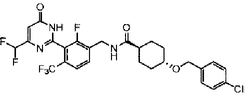 | CDCl₃,δ : 1.17-1.35 (2H, m), 1.36-1.53 (2H, m), 1.80-1.93 (2H, m), 2.03-2.18 (3H, m), 3.30 (1H, tt, J = 10.6, 4.2 Hz), 4.45 (2H, d, J = 5.9 Hz), 4.50 (2H, s), 6.21 (1H, t, J = 6.1 Hz), 6.38 (1H, t, J = 54.7 Hz), 6.68 (1H, s), 7.21-7.33 (4H, m), 7.49-7.59 (2H, m) | 588 [M+H]⁺ |
| 117 | 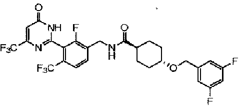 | CDCl₃,δ: 1.22-1.38 (2H, m), 1.39-1.55 (2H, m), 1.82-1.95 (2H, m), 2.04-2.20 (3H, m), 3.32 (1H, tt, J = 10.6, 4.3 Hz), 4.44 (2H, d, J = 5.9 Hz), 4.52 (2H, s), 6.13 (1H, t, J = 6.0 Hz), 6.70 (1H, tt. J = 9.0. 2.4 Hz), 6.80 (1H, s), 6.82-6.91 (2H, m), 7.54 (2H, d, J = 2.0 Hz) | 608 [M+H]⁺ |
| 118 | 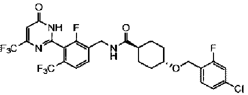 | DMSO-d₆,δ :1.11-1.27 (2H, m), 1.34-1.50 (2H, m), 1.71-1.89 (2H, m), 2.01-2.12 (2H, m), 2.14-2.26 (1H, m), 3.33-3.38 (1H, m), 4.38 (2H, d, J = 5.4 Hz), 4.54 (2H, s), 6.94-7.02 (1H, m), 7.29 (1H, dd, J = 8.2, 2.1 Hz), 7.40 (1H, dd, J = 10.0, 2.0 Hz), 7.47 (1H, t, J = 8.2 Hz), 7.60-7.70 (1H, m), 7.75 (1H, d, J = 8.1 Hz), 8.46 (1H, t, J = 5.7 Hz), 13.88 (1H, brs) | 624 [M+H]⁺ |
| 119 | 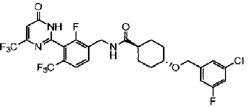 | CDCl₃,δ : 1.22-1.35 (2H, m), 1.39-1.54 (2H, m), 1.83-1.93 (2H, m), 2.05-2.19 (3H, m), 3.25-3.36 (1H, m), 4.43 (2H, d, J = 6.1 Hz), 4.50 (2H, s), 6.28 (1H, t, J = 6.0 Hz), 6.76 (1H, s), 6.92-7.02 (2H, m), 7.11 (1H, d, J = 1.2 Hz), 7.45-7.57 (2H, m) | 624 [M+H]⁺ |
| 120 | 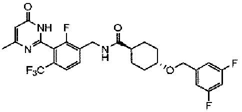 | DMSO-d₆,δ: 1.22 (2H, qd, J = 13.2, 3.6 Hz), 1.41 (2H, qd, J = 12.4, 3.2 Hz), 1.83 (2H, d, J = 11.2 Hz), 2.08 (2H, dd, J = 12.4, 2.8 Hz), 2.16-2.25 (1H, m), 2.22 (3H, s), 3.28-3.36 (1H, m), 4.37 (2H, d, J = 6.0 Hz), 4.54 (2H, s), 8.28 (1H, brs), 7.03 (2H, dd, J = 8.4, 2.4 Hz), 7.12 (1H, tt, J = 9.2, 2.4 Hz), 7.60 (1H, t, J = 8.0 Hz), 7.71 (1H, d, J = 8.4 Hz), 8.47 (1H, t, J = 5.6 Hz), 12.96 (1H, brs) | 553(M⁺) |

**[Table 29]**

| **Example No.** | **Chemical Structure** | **NMR (¹H-NMR)** | **MS (m/z)** |
|---|---|---|---|
| 121 | 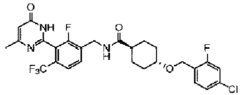 | DMSO-d₆,δ : 1.20 (2H, qd, J = 13.2, 3.2 Hz), 1.41 (2H, qd, J = 13.2, 2.8 Hz), 1.82 (2H, d, J = 11.2 Hz), 2.07 (2H, dd, J = 12.4, 3.6 Hz), 2.15-2.24 (1H, m), 2.22 (3H, s), 3.29-3.37 (1H, m), 4.37 (2H, d, J = 6.0 Hz), 4.54 (2H, s), 6.28 (1H, brs), 7.29 (1H, dd, J = 8.0, 1.6 Hz), 7.41 (1H, dd, J = 10.4, 2.0 Hz), 7.47 (1H, t, J = 8.4 Hz), 7.60 (1H, t, J = 8.0 Hz), 7.71 (1H, d, J = 8.4 Hz), 8.47 (1H, t, J = 5.6 Hz), 12.95 (1H, brs) | 569(M⁺) |
| 122 | 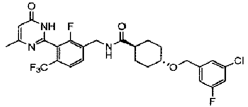 | DMSO-d₆,δ:1.22 (2H, qd, J = 12.8, 2.8 Hz), 1.41 (2H, qd, J = 12.4, 2.8 Hz), 1.83 (2H, d, J = 12.0 Hz), 2.08 (2H, dd, J = 12.8, 3.6 Hz), 2.16-2.25 (1H, m), 2.23 (3H, s), 3.28-3.36 (1H, m), 4.37 (2H, d, J = 5.2 Hz), 4.54 (2H, s), 6.28 (1H, brs), 7.15 (1H, dd, J = 8.4, 0.8 Hz), 7.25 (1H, s), 7.32 (1H, dt, J = 8.8, 2.4 Hz), 7.60 (1H, t, J = 7.8 Hz), 7.71 (1H, d, J = 8.4 Hz), 8.47 (1H, t, J = 6.0 Hz), 12.95 (1H, brs) | 569(M⁺) |
| 123 | 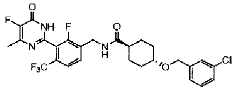 | DMSO-d₆,δ: 1.16-1.27 (2H, m), 1.41 (2H, qd, J = 13.2, 2.8 Hz), 1.83 (2H, d, J = 11.2 Hz), 2.09 (2H, dd, J = 12.4, 2.8 Hz), 2.15-2.26 (1H, m), 2.26 (3H, d, J = 3.6 Hz), 3.26-3.37 (1H, m), 4.37 (2H, d, J = 5.2 Hz), 4.52 (2H, s), 7.26-7.40 (4H, m), 7.62 (1H, t, J = 7.6 Hz), 7.73 (1H, d, J = 8.4 Hz), 8.48 (1H, t, J = 6.0 Hz),13.61 (1H, brs) | 569(M⁺) |
| 124 | 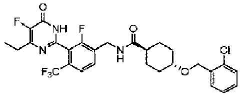 | DMSO-d₆,δ : 1.14 (3H, t, J = 8.0 Hz), 1.24 (2H, qd, J = 10.8, 2.8 Hz), 1.43 (2H, qd, J = 11.6, 2.8 Hz), 1.84 (2H, d, J = 11.2 Hz), 2.11 (2H, dd, J = 12.0, 3.2 Hz), 2.17-2.25 (1H, m), 2.56-2.64 (2H, m), 3.37 (1H, tt, J = 10.8, 4.0 Hz), 4.37 (2H, d, J = 6.0 Hz), 4.58 (2H, s), 7.29-7.38 (2H, m), 7.43 (1H, dd, J = 7.6, 2.0 Hz), 7.50 (1H, dd, J = 7.6, 2.4 Hz), 7.62 (1H, t, J = 7.6 Hz), 7.73 (1H, d, J = 8.4 Hz), 8.48 (1H, t, J = 5.6 Hz), 13.60 (1H, brs) | 583(M⁺) |
| 125 | 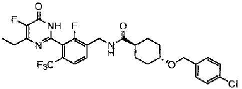 | DMSO-d₆,δ: 1.14 (3H, t, J = 8.0 Hz), 1.21 (2H, qd, J = 12.8, 2.8 Hz), 1.40 (2H, qd, J = 12.8, 2.8 Hz), 1.82 (2H, d, J = 12.0 Hz), 2.08 (2H, dd, J = 12.0, 2.8 Hz), 2.20 (1H, tt, J = 12.4, 4.0 Hz), 2.56-2.64 (2H, m), 3.26-3.35 (1H, m), 4.37 (2H, d, J = 6.0 Hz), 4.50 (2H. s), 7.34 (2H, d, J = 8.8 Hz), 7.40 (2H, d, J = 8.8 Hz), 7.61 (1H, t, J = 8.0 Hz), 7.73 (1H, d, J = 8.4 Hz), 8.48 (1H, t, J = 5.6 Hz), 13.60 (1H, brs) | 583(M⁺) |
| 126 | 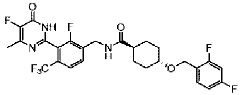 | DMSO-d₆,δ: 1.20 (2H, qd, J = 12.4, 3.2 Hz), 1.41 (2H, qd, J = 12.8, 2.8 Hz), 1.82 (2H, d, J = 11.2 Hz), 2.07 (2H, dd, J = 12.8, 2.8 Hz), 2.15-2.24 (1H, m), 2.26 (3H, d, J = 3.6 Hz), 3.29-3.37 (1H, m), 4.37 (2H, d, J = 6.0 Hz), 4.52 (2H, s), 7.07 (1H, tdd, J = 8.4, 2.4, 1.2 Hz), 7.22 (1H, td, J = 9.6, 2.4 Hz), 7.48 (1H, td, J = 8.8, 6.8 Hz), 7.61 (1H, t, J = 8.0 Hz), 7.73 (1H, d, J = 8.4 Hz), 8.48 (1H, t, J = 6.0 Hz), 13.60 (1H, brs) | 571(M⁺) |

**[Table 30]**

| **Example No.** | **Chemical Structure** | **NMR (¹H-NMR)** | **MS (m/z)** |
|---|---|---|---|
| 127 | 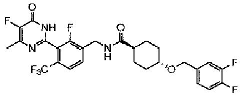 | DMSO-d₆,δ: 1.21 (2H, qd, J = 13.2, 3.2 Hz), 1.41 (2H, qd, J = 12.4, 2.8 Hz), 1.82 (2H, d, J = 11.6 Hz), 2.07 (2H, dd, J = 12.8, 3.6 Hz), 2.15-2.25 (1H, m), 2.26 (3H, d, J = 3.6 Hz), 3.27-3.36 (1H, m), 4.37 (2H, d, J = 5.6 Hz), 4.50 (2H, s), 7.15-7.19 (1H, m), 7.32-7.43 (2H, m), 7.61 (1H, t, J = 8.0 Hz), 7.73 (1H, d, J = 8.4 Hz), 8.48 (1H, t, J = 5.6 Hz), 13.60 (1H, brs) | 571(M⁺) |
| 128 | 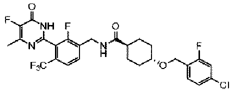 | DMSO-d₆,δ: 1.20 (2H, qd, J = 13.2, 2.8 Hz), 1.41 (2H, qd, J = 12.4, 2.8 Hz), 1.82 (2H, d, J = 11.6 Hz), 2.07 (2H, dd, J = 12.8, 3.6 Hz), 2.15-2,25 (1H, m), 2.26 (3H, d, J = 3.6 Hz), 3.28-3.36 (1H, m), 4.37 (2H, d, J = 5.6 Hz), 4.54 (2H, s), 7.29 (1H, dd, J = 8.0, 1.6 Hz), 7.41 (1H, dd, J = 10.0, 2.0 Hz), 7.47 (1H, t, J = 8.4 Hz), 7.61 (1H, t, J = 8.0 Hz), 7.73 (1H, d, J = 8.4 Hz), B.48 (1H, t, J = 5.6 Hz), 13.61 (1H, brs) | 587(M⁺) |
| 129 | 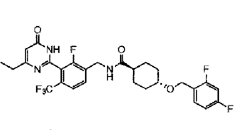 | DMSO-d₆,δ: 1.15 (3H, t, J = 8.0 Hz), 1.14-1.26 (2H, m), 1.41 (2H, qd, J = 13.2, 2.8 Hz), 1.82 (2H, d, J = 12.0 Hz), 2.03-2.12 (2H, m), 2.14-2.24 (1H, m), 2.46-2.56 (2H, m), 3.27-3.38 (1H, m), 4.37 (2H, d, J = 5.2 Hz), 4.52 (2H, s), 6.25 (1H, brs), 7.04-7.11 (1H, m), 7.22 (1H, td, J = 10.0, 2.4 Hz), 7.44-7.52 (1H, m), 7.60 (1H, t, J = 8.0 Hz), 7.72 (1H, d, J = 8.4 Hz), 8.47 (1H, t, J = 6.0 Hz), 12.93 (1H, brs) | 567(M⁺) |
| 130 | 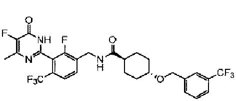 | DMSO-d₆,δ: 1.24 (2H, qd, J = 12.8, 2.8 Hz), 1.42 (2H, qd, J = 12.4, 3.2 Hz), 1.83 (2H, d, J = 11.6 Hz), 2.10 (2H, dd, J = 12.4, 2.4 Hz), 2.16-2.26 (1H, m), 2.26 (3H, d, J = 3.6 Hz), 3.31-3.39 (1H, m), 4.37 (2H, d, J = 6.0 Hz), 4.62 (2H, s), 7.55-7.65 (5H, m), 7.73 (1H, d, J = 8.4 Hz), 8.48 (1H, t, J = 6.0 Hz), 13.61 (1H, brs) | 603(M⁺) |
| 131 | 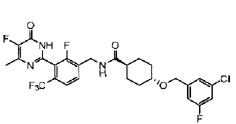 | DMSO-d₆,δ : 1.22 (2H, qd, J = 13.2, 3.2 Hz), 1.41 (2H, qd, J = 13.2, 2.8 Hz), 1.83 (2H, d, J = 11.2 Hz), 2.08 (2H, dd, J = 12.8, 3.6 Hz), 2.16-2.25 (1H, m), 2.26 (3H, d, J = 4.0 Hz), 3.28-3.36 (1H, m), 4.37 (2H, d, J = 5.6 Hz), 4.54 (2H, s), 7.15 (1H, id, J = 9.2, 1.2 Hz), 7.25 (1H, s), 7.32 (1H, dt, J = 8.8, 2.4 Hz), 7.62 (1H, t, J = 7.6 Hz), 7.73 (1H, d, J = 8.0 Hz), 8.48 (1H, t. J = 6.0 Hz), 13.60 (1 H, brs) | 587(M⁺) |
| 132 | 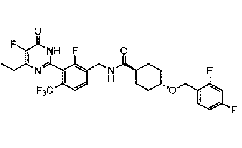 | DMSO-d₆,δ:1.14 (3H, t, J = 7.6 Hz), 1.20 (2H, qd, J = 13.2, 3.6 Hz), 1.41 (2H, qd, J = 12.8, 2.8 Hz), 1.82 (2H, d, J = 11.6 Hz), 2.07 (2H, dd, J = 12.8, 2.8 Hz), 2.15-2.24 (1H, m), 2.58-2.62 (2H, m), 3.28-3.36 (1H, m), 4.37 (2H, d, J = 8.0 Hz), 4.52 (2H, s), 7.07 (1H, tdd, J = 8.4, 2.4, 1.2 Hz), 7.21 (1H, td, J = 9.6, 2.4 Hz), 7.48 (1H, dd, J = 15.6, 8.8 Hz), 7.62 (1H, t, J = 7.6 Hz), 7.73 (1H, d, J = 8.4 Hz), 8.48 (1H, t, J = 6.0 Hz), 13,60 (1H, brs) | 585(M⁺) |

**[Table 31]**

| **Example No.** | **Chemical Structure** | **NMR (¹H-NMR)** | **MS (m/z)** |
|---|---|---|---|
| 133 | 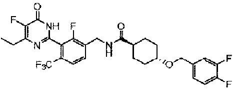 | DMSO-d₆,δ:1.14 (3H, t, J = 8.0 Hz), 1.19-1.27 (2H, m), 1.41 (2H, qd, J = 12.0, 2.8 Hz), 1.83 (2H, d, J = 11.8 Hz), 2.06-2.10 (2H, m), 2.20 (1H, tt, J = 12.4, 4.0 Hz), 2.58-2.61 (2H, m), 3.27-3.36 (1H, m), 4.37 (2H, d. J = 6.0 Hz), 4.50 (2H, s), 7.15-7.19 (1H, m), 7.32-7.43 (2H, m), 7.61 (1H, t, J = 8.0 Hz), 7.73 (1H, d, J = 7.6 Hz), 8.48 (1H, t, J = 6.0 Hz), 13.60 (1H, brs) | 585(M⁺) |
| 134 | 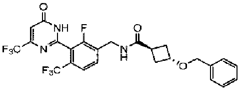 | DMSO-d₆,δ : 2.10-2.19 (2H, m), 2.30-2.37 (2H, m), 2.96-3.04 (1H, m), 4.19 (1H, quin, J = 6.8 Hz), 4.36 (2H, s), 4.40 (2H, d, J = 5.6 Hz), 6.94 (1H, brs), 7.25-7.37 (5H, m), 7.64 (1H, t, J = 7.6 Hz), 7.75 (1H, d, J = 8.4 Hz), 8.51 (1H, t, J = 5.6 Hz), 13.90 (1H, brs) | 543(M⁺) |
| 135 | 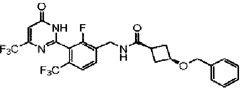 | DMSO-d₆,δ : 1.98-2.08 (2H, m), 2.30-2.39 (2H, m), 2.54-2.64 (1H, m), 3.93 (1H, quin, J = 7.2 Hz), 4.37 (2H, s), 4.39 (2H, d, J = 6.0 Hz), 8.93 (1H, brs), 7.25-7.37 (5H, m), 7.63 (1H, t, J = 8.0 Hz), 7.74 (1H, d, J = 8.4 Hz), 8.49 (1H, t, J = 6.0 Hz), 13.91 (1H, brs) | |
| 136 | 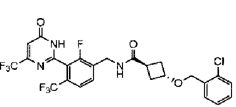 | CDCl₃,δ:2.21-2.32 (2H, m), 2.39-2.48 (2H, m), 2.87-2.98 (1H, m), 4.30 (1H, quin, J = 6.4 Hz), 4.43-4.54 (4H, m), 6.26 (1H, t, J = 6.0 Hz), 6.85 (1H, s), 7.18-7.28 (2H, m), 7.32-7.38 (1H, m), 7.44 (1H, dd, J = 7.3, 2.0 Hz), 7.51-7.64 (2H, m) | 578 [M+H]⁺ |
| 137 | 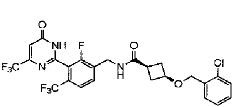 | CDCl₃,δ: 2.09-2.22 (2H, m), 2.39-2.58 (3H, m), 3.94-4.04 (1H, m), 4.44 (2H, d, J = 5.9 Hz), 4.50 (2H, s), 6.48 (1H, t, J = 6.1 Hz), 5.85 (1H, s), 7.19-7.28 (2H, m), 7.32-7.36 (1H, m), 7.41-7.45 (1H, m), 7.49-7.59 (2H, m) | 578 [M+H]⁺ |
| 138 | 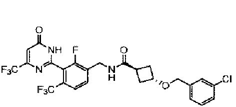 | DMSO-d₆,δ: 2.07-2.22 (2H, m), 2.28-2.40 (2H, m), 2.92-3.09 (1H, m), 4.19 (1H, quin, J = 6.7 Hz), 4.38 (2H, s), 4.40 (2H, d, J = 5.9 Hz), 7.02 (1H, brs), 7.24-7.31 (1H, m), 7.31-7.42 (3H, m), 7.62-7.70 (1H, m), 7.76 (1H, d, J = 8.3 Hz), 8.50 (1H, t, J = 5.9 Hz), 13.87 (1H, brs) | 578 [M+H]⁺ |

**[Table 32]**

| **Example No.** | **Chemical Structure** | **NMR (¹H-NMR)** | **MS (m/z)** |
|---|---|---|---|
| 139 | 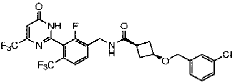 | DMSO-d₆,δ: 1.95-2.12 (2H, m), 2.28-2.42 (2H, m), 2.54-2.66 (1H, m), 3.94 (1H, quin, J = 7.3 Hz), 4.34-4.44 (4H, m), 6.99 (1H, brs), 7.25-7.31 (1H, m), 7.32-7.40 (3H, m), 7.61-7.69 (1H, m), 7.76 (1H, d, J = 8.3 Hz), 8.47 (1H, t, J = 5.9 Hz), 13.86 (1H, brs) | 578 [M+H]⁺ |
| 140 | 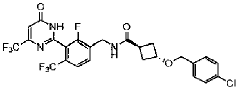 | DMSO-d₆,δ: 2.10-2.21 (2H, m), 2.29-2.37 (2H, m), 2.96-3.05 (1H, m), 4.15-4.23 (1H, m), 4.33-4.36 (2H, m), 4.40 (2H, d, J = 5.6 Hz), 7.01 (1H, s), 7.31-7.37 (2H, m), 7.38-7.43 (2H, m), 7.62-7.71 (1H, m), 7.77 (1H, d, J = 8.3 Hz), 8.53 (1H, t, J = 5.9 Hz), 13.89 (1H, brs) | 578 [M+H]⁺ |
| 141 | 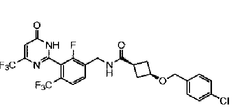 | DMSO-d₆,δ: 1.91-2.11 (2H, m), 2.23-2.40 (2H, m), 2.54-2.66 (1H, m), 3.79-4.01 (1H, m), 4.37 (2H, s), 4.39 (2H, d, J = 5.9 Hz), 7.01 (1H, s), 7.29-7.44 (4H. m), 7.59-7.69 (1H, m), 7.76 (1H, d, J = 8.3 Hz), 8.47 (1H, t, J = 5.7 Hz), 13.86 (1H, brs) | 578 [M+H]⁺ |
| 142 | 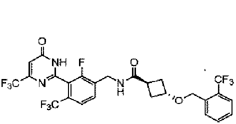 | DMSO-d₆,δ:2.11-2.22 (2H, m), 2.33-2.40 (2H, m), 2.97-3.07 (1H, m), 4.23 (1H, quin, J = 6.5 Hz), 4.40 (2H, d, J = 5.9 Hz), 4.53 (2H, s), 6.99 (1H, s), 7.48-7.57 (1H, m), 7.63-7.80 (5H, m), 8.52 (1H, t, J = 6.0 Hz), 13.88 (1H, brs) | 612 [M+H]⁺ |
| 143 | 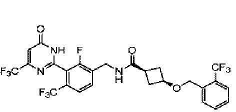 | DMSO-d₆,δ:1.96-2.13 (2H, m), 2.33-2.42 (2H, m), 2.55-2.69 (1H, m), 3.90-4.07 (1H, m), 4.40 (2H, d, J = 5.9 Hz), 4.53 (2H, s), 7.01 (1H, s), 7.48-7.57 (1H, m), 7.62-7.79 (5H, m), 8.50 (1H, t, J = 5.9 Hz), 13.88 (1H, brs) | 612 [M+H]⁺ |
| 144 | 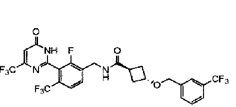 | DMSO-d₆,δ : 2.13-2.23 (2H, m), 2.29-2,42 (2H, m), 2.97-3.07 (1H, m), 4.22 (1H, quin, J = 6.6 Hz), 4.41 (2H, d, J = 5.9 Hz), 4.47 2H, s), 7.02 (1H, s), 7.55-7.70 (5H, m), 7.74-7.80 (1H, m), 8.53 (1H, t, J = 5.9 Hz), 13.88 (1H, brs) | 612 [M+H]⁺ |

**[Table 33]**

| **Example No.** | **Chemical Structure** | **NMR (¹H-NMR)** | **MS (m/z)** |
|---|---|---|---|
| 145 | 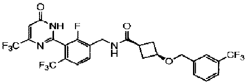 | DMSO-d₆,δ :2.00-2.12 (2H, m), 2.31-2.41 (2H, m), 2.56-2.88 (1H, m), 3.93-4.02 (1H, m), 4.40 (2H, d, J = 5.6 Hz), 4.48 (2H, s), 7.02 (1H, s), 7.53-7.89 (5H, m), 7.76 (1H, d, J = 8.3 Hz), 8.50 (1H, t, J = 5.9 Hz), 13.88 (1H, brs) | 612 [M+H]⁺ |
| 146 | 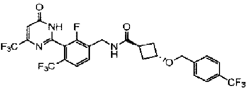 | DMSO-d₆,δ:2.12-2.22 (2H, m), 2.28-2.41 (2H, m), 2.96-3.08 (1H, m), 4.22 (1H, quin, J = 6.6 Hz), 4.40 (2H, d, J = 5.9 Hz), 4.48 (2H, s), 7.02 (1H, s), 7.55 (2H, d, J = 7.8 Hz), 7.63-7.69 (1H, m), 7.71 (2H, d, J = 7.8 Hz), 7.77 (1H, d, J = 8.3 Hz), 8.54 (1H, t, J = 5.9 Hz), 13.89 (1H, brs) | 612 [M+H]⁺ |
| 147 | 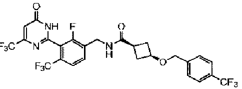 | DMSO-d₆,δ : 1.97-2.11 (2H, m), 2.30-2.43 (2H, m), 2.55-2.69 (1H, m), 3.89-4.02 (1H, m), 4.40 (2H, d, J = 5.6 Hz), 4.48 (2H, s), 7.01 (1H, brs), 7.55 (2H, d, J = 8.1 Hz), 7.62-7.69 (1H, m), 7.71 (2H, d, J = 8.1 Hz), 7.76 (1H, d, J = 8.3 Hz), 8.51 (1H, t, J = 5.9 Hz), 13.89 (1H, brs) | 612 [M+H]⁺ |
| 148 | 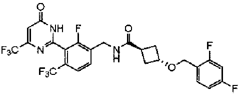 | DMSO-d₆, δ: 2.09-2.17 (2H, m), 2.28-2.40 (2H, m), 2.93-3.06 (1H, m), 4.19 (1H, quin, J = 6.8 Hz), 4.35-4.43 (4H, m), 6.92 (1H, brs), 7.03-7.13 (1H, m), 7.16-7.29 (1H, m), 7.48 (1H, td, J = 8.5, 6.8 Hz), 7.60-7.68 (1H, m), 7.70-7.78 (1H, m), 8.50 (1H, t, J = 5.7 Hz), 13.88 (1H, brs) | 580 [M+H]⁺ |
| 149 | 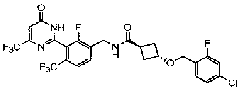 | DMSO-d₆,δ : 2.09-2.19 (2H, m), 2.28-2.38 (2H, m), 2.94-3.05 (1H, m), 4.20 (1H, quin, J = 6.8 Hz), 4.34-4.44 (4H, m), 6.87 (1H, brs), 7.29 (1H, dd, J = 8.1, 2.1 Hz), 7.38-7.51 (2H, m), 7.59-7.68 (1H, m), 7.70-7.78 (1H, m), 8.50 (1H, t, J = 5.9 Hz) | 596 [M+H]⁺ |
| 150 | 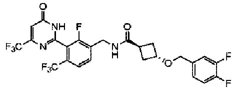 | DMSO-d₆,δ :2.08-2.22 (2H, m), 2,27-2,39 (2H, m), 2.94-3.06 (1H, m), 4.19 (1H, quin, J =6.6 Hz), 4.35 (2H, s), 4.40 (2H, d, J = 5.7 Hz), 6.90 (1H, brs), 7.13-7.22 (1H, m), 7.29-7.46 (2H, m), 7.59-7.68 (1H, m), 7.70-7.78 (1H, m), 8.50 (1H, t, J = 5.9 Hz), 13.91 (1H, brs) | 580 [M+H]⁺ |

**[Table 34]**

| **Example No.** | **Chemical Structure** | **NMR (¹H-NMR)** | **MS (m/z)** |
|---|---|---|---|
| 151 | 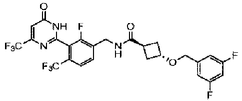 | DMSO-d₆,δ :2.10-2.22 (2H, m), 2.28-2.40 (2H, m), 2.95-3.07 (1H, m), 4.20 (1H, quin, J = 6.8 Hz), 4.40 (4H, s), 6.91 (1H, brs), 7.00-7.07 (2H, m), 7.13 (1H, tt, J = 9.5, 2.4 Hz), 7.60-7.69 (1H, m), 7.70-7.79 (1H, m), 8.51 (1H, t, J = 5.7 Hz), 13.89 (1H, brs) | 580 [M+H]⁺ |
| 152 | 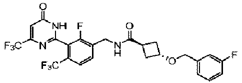 | DMSO-d₆,δ:2.09-2.22 (2H, m), 2.29-2.39 (2H, m), 2.96-3.06 (1H, m), 4.20 (1H, quin, J = 6.6 Hz), 4.37-4.43 (4H, m), 6.90 (1H, brs), 7.05-7.19 (3H, m), 7.33-7.45 (1H, m), 7.59-7.68 (1H, m), 7.70-7.76 (1H, m), 8.50 (1H, t, J = 5.9 Hz), 13.95 (1H, brs) | 562 [M+H]⁺ |
| 153 | 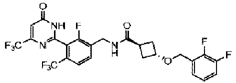 | DMSO-d₆,δ:2.08-2.20 (2H, m), 2.29-2.39 (2H, m), 2.94-3.06 (1H, m), 4.21 (1H, quin, J = 6.6 Hz), 4.40 (2H, d, J = 5.7 Hz), 4.46 (2H, d, J = 1.1 Hz), 6.98 (1H, s), 7.15-7.30 (2H, m), 7.32-7.45 (1H, m), 7.61-7.70 (1H, m), 7.73-7.79 (1H, m), 8.51 (1H, t, J = 5.9 Hz) | 580 [M+H]⁺ |
| 154 | 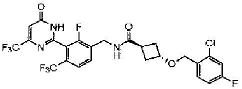 | DMSO-d₆,δ:2.12-2.22 (2H, m), 2.32-2.40 (2H, m), 2.96-3.07 (1H, m), 4.23 (1H, quin, J = 6.8 Hz), 4.38-4.43 (4H, m), 6.90 (1H, brs), 7.23 (1H, td, J = 8.7. 2.6 Hz), 7.44 (1H, dd, J = 9.1, 2.6 Hz), 7.53 (1H, dd, J = 8.5, 6.6 Hz), 7.59-7.68 (1H, m), 7.71-7.77 (1H, m), 8.50 (1H, t, J = 5.9 Hz), 13.96 (1H, brs) | 596 [M+H]⁺ |
| 155 | 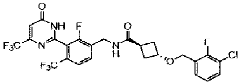 | DMSO-d₆, δ:2.10-2.20 (2H, m), 2.29-2.39 (2H, m), 2.95-3.06 (1H, m), 4.21 (1H, quin, J = 6.8 Hz), 4.40 (2H, d, J = 5.3 Hz), 4.45 (2H, s), 6.90 (1H, brs), 7.19-7.28 (1H, m), 7.37-7.46 (1H, m), 7.50-7.58 (1H, m), 7.60-7.68 (1H, m), 7.71-7.78 (1H, m), 8.50 (1H, t, J = 5.7 Hz), 13.85 (1H, brs) | 596 [M+H]⁺ |
| 156 | 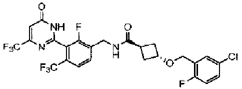 | DMSO-d₆,δ: 2.11-2.21 (2H, m), 2.29-2.40 (2H, m), 2.95-3.07 (1H, m). 4.21 (1H, quin, J = 6.8 Hz), 4.38-4.43 (4H, m), 6.93 (1H, brs), 7.22-7.29 (1H, m), 7.39-7.46 (1H, m), 7.49 (1H, dd, J = 6.0, 2.6 Hz), 7.60-7.69 (1H, m), 7.72-7.78 (1H, m), 8.51 (1H, t, J = 5.9 Hz), 13.89 (1H, brs) | 596 [M+H]⁺ |

**[Table 35]**

| **Example No.** | **Chemical Structure** | **NMR (¹H-NMR)** | **MS (m/z)** |
|---|---|---|---|
| 157 | 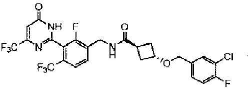 | DMSO-d₆,δ: 2.11-2.20 (2H, m), 2.28-2.36 (2H, m), 2.96-3.06 (1H, m), 4.19 (1H, quin, J = 6.5 Hz), 4.36 (2H, s), 4.40 (2H, d, J = 5.6 Hz), 7.01 (1H, s), 7.30-7.43 (2H, m), 7.53 (1H, dd, J = 7.3, 2.2 Hz), 7.62-7.71 (1H, m), 7.76 (1H, d, J = 8.1 Hz), 8.50 (1H, t, J = 5.7 Hz), 13.87 (1H, brs) | 596 [M+H]⁺ |
| 158 | 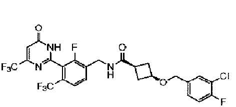 | DMSO-d₆,δ: 1.97-2.09 (2H, m), 2.29-2.39 (2H, m), 2.55-2.65 (1H, m), 3.89-3.98 (1H, m), 4.36 (2H, s), 4.39 (2H, d, J = 6.1 Hz), 6.99 (1H, s), 7.30-7,42 (2H, m), 7.53 (1H, dd, J = 7.2, 1.8 Hz), 7.65 (1H, t, J = 7.5 Hz), 7.76 (1H, d, J = 8.3 Hz), 8.48 (1H, t, J = 6.0 Hz), 13.87 (1H, brs) | 596 [M+H]⁺ |
| 159 | 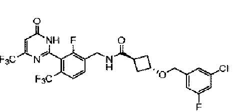 | DMSO-d₆,δ: 2.09-2.25 (2H. m), 2.29-2.41 (2H, m), 2.96-3.07 (1 H, m), 4.20 (1H, quin, J = 6.6 Hz), 4.35-4.46 (4H, m), 6.89 (1H, brs), 7.14-7.19 (1H, m), 7.25 (1H, s), 7.34 (1H, dt, J = 8.8, 2.2 Hz), 7.63 (1H, t, J = 7.2 Hz), 7.73 (1H, d, J = 7.9 Hz), 8.51 (1H, t, J = 6.0 Hz) | 596 [M+H]⁺ |
| 160 | 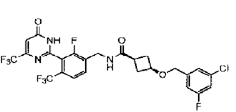 | CDC_{I3},δ : 2.10-2.26 (2H, m), 2.37-2.58 (3H, m), 3.88-4.00 (1H, m), 4.37 (2H, s), 4.46 (2H, d, J = 5.9 Hz), 6.35 (1H, t, J = 6.0 Hz), 6.84 (1H, s), 6.90-6.95 (1H, m), 7.00 (1H, dt, J = 8.4, 2.2 Hz), 7.09 (1H, s), 7.47-7.61 (2H, m) | 596 [M+H]⁺ |
| 161 | 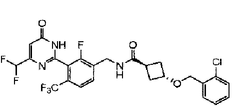 | DMSO-d₆,δ: 2.11-2.25 (2H, m), 2.30-2.43 (2H, m), 2.97-3.09 (1H, m), 4.24 (1H, quin, J = 6.6 Hz), 4.40 (2H, d, J = 5.7 Hz), 4.44 (2H, s), 8.70 (1H, s), 6.77 (1H, t, J = 54.0 Hz), 7.30-7.39 2H, m), 7.41-7.47 (1H, m), 7.47-7.53 (1H, m), 7.65(1H, t, J = 7.2 Hz), 7.75 (1H, d, J = 8.3 Hz), 8.51 (1H, t, J = 5.9 Hz), 13.56 (1H, brs) | 560 [M+H]⁺ |
| 162 | 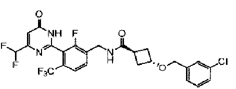 | DMSO-d₆, δ :2.09-2.23(2H, m), 2.29-2.40 (2H, m), 2.94-3.09 (1H, m), 4.19 (1H, quin. J = 6.6 Hz), 4.35-4.46 (4H, m), 6.69 (1H, s), 6.77(1H, J = 54.2 Hz), 7.25-7.43 (4H, m), 7.64 (1H, t, J = 7.6 Hz), 7.74 (1H, d, J = 8.3 Hz), 8.51 (1H, J = 5.9 Hz), 13.55 (1H, brs) | 560 [M+H]⁺ |

**[Table 36]**

| **Example No.** | **Chemical Structure** | **NMR (¹H-NMR)** | **MS (m/z)** |
|---|---|---|---|
| 163 | 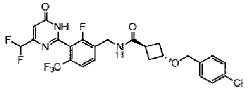 | DMSO-d₆,δ:2.08-2.21 (2H, m), 2.28-2.39 (2H, m), 2.94-3.05 (1H, m), 4.18 (1H, quin, J = 6.6 Hz), 4.38 (2H, s), 4.39 (2H, d, J = 5.7 Hz), 6.65 (1H, brs), 6.75 (1H, t, J = 54.2 Hz), 7.31-7.37 (2H, m), 7.37-7.43 (2H, m), 7.63 (1H, t, J = 7.9 Hz), 7.73 (1H, d, J = 8.3 Hz), 8.50 (1H, t, J = 5.9 Hz), 13.54 (1H, brs) | 560 [M+H]⁺ |
| 164 | 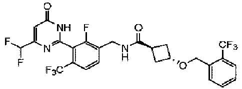 | DMSO-d₆,δ:2.08-2.24 (2H, m), 2.29-2.44 (2H, m), 2.96-3.09 (1H, m), 4.23 (1H, quin, J = 6.6 Hz), 4.40 (2H, d, J = 5.7 Hz), 4.52 (2H, s), 6.70 (1H, s), 8.77 (1H, t, J = 54.0 Hz), 7.47-7.57 (1H, m), 7.58-7.82 (5H, m), 8.51 (1H, 1, J = 5.9 Hz), 13.54 (1H brs) | 594 [M+H]⁺ |
| 165 | 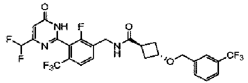 | DMSO-d₆,δ:2.10-2.24 (2H, m), 2.29-2.41 (2H, m), 2.95-3.08 (1H, m), 4.22 (1H, quin, J = 6.6 Hz), 4.40 (2H, d, J = 6.0 Hz), 4.47 (2H, s), 6.66 (1H, brs), 6.75 (1H, t, J = 54.0 Hz), 7.51-7.81 (6H, m), 8.51 (1H, t, J = 6.0 Hz), 13.58 (1H, brs) | 594 [M+H]⁺ |
| 166 | 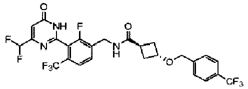 | DMSO-d₆,δ:2.09-2.25 (2H, m), 2.29-2,42 (2H, m), 2.95-3.07 (1H, m), 4.22 (1H, quin, J = 6.6 Hz), 4.39 (2H, d, J = 6.0 Hz), 4.47 (2H, s), 6.67 (1H, brs), 6.76 (1H, t, J = 54.2 Hz), 7.54 (2H, d, J = 7.9 Hz), 7.63 (1H, t, J = 7.6 Hz), 7.68-7.79 (3H, m), 8.51 (1H, t, J = 5.9 Hz), 13.50 (1H, brs) | 594 [M+H]⁺ |
| 167 | 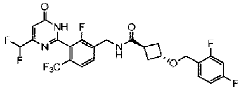 | DMSO-d₆,δ :2.08-2.18 (2H. m), 2.28-2.38 (2H, m), 2.95-3.05 (1H, m), 4.19 (1H, quin, J = 6.5 Hz), 4.35-4.43 (4H, m), 6.75 (1H, brs), 8.80 (1H, t, J = 53.8 Hz), 7.08 (1H, tdd, J = 8.6. 2.4, 1.0 Hz), 7.18-7.28 (1H, m), 7.43-7.54 (1H, m), 7.66 (1H, t, J = 7.6 Hz). 7.77 (1H, d, J = 8.3 Hz), 8,53 (1H, t, J = 6.0 Hz), 13.59 (1H. brs) | 562 [M+H]⁺ |
| 168 | 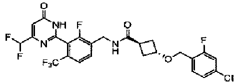 | DMSO-d₆,δ:2.04-2.22 (2H, m), 2.28-2.42 (2H, m), 2.93-3.06 (1H, m), 4.20 (1H, quin, J = 6.5 Hz), 4.34-4.50 (4H, m), 6.67 (1H, brs), 6.75 (1H, t, J = 54.0 Hz), 7.29 (1H, dd, J = 7.7, 2.1 Hz), 7.38-7.52 (2H, m), 7.63 (1H, t, J = 7.6 Hz), 7.74 (1H, d, J = 7.9 Hz), 8.50 (1H, t, J = 6.0 Hz), 13.55 (1H, brs) | 578 [M+H]⁺ |

**[Table 37]**

| **Example No.** | **Chemical Structure** | **NMR (¹H-NMR)** | **MS (m/z)** |
|---|---|---|---|
| 169 | 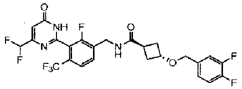 | DMSO-d₆,δ.2.08-2.23 (2H, m), 2.25-2.41 (2H, m), 2.95-3.09 (1H, m), 4.19 (1H, quin, J = 6.6 Hz), 4.35 (2H, s), 4.40 (2H, d, J = 5.7 Hz), 6.71 (1H, s), 6.77 (1H, t, J = 54.0 Hz), 7.11-7.22 (1H, m), 7.32-7.48 (2H, m), 7.64 (1H, t, J = 7.2 Hz), 7.75 (1H, d, J = 8.3 Hz), 8.51 (1H, t, J = 6.0 Hz), 13.56 (1H, brs) | 562 [M+H]⁺ |
| 170 | 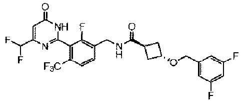 | DMSO-d₆. δ:2.09-2.22 (2H, m), 2.29-2,40 (2H, m), 2.95-3.07 (1H, m), 4.20 (1H, quin, J = 6.5 Hz), 4.36-4.45 (4H, m), 6.65 (1H. brs), 6.75 (1H, t, J = 54.0 Hz), 7.04 (2H, dd, J = 8.3, 2.3 Hz), 7.13 (1H, tt, J = 9.4, 2.5 Hz), 7.63 (1H, t, J = 7.9 Hz), 7.73 (1H, d, J = 8.3 Hz), 8.51 (1H, t, J = 5.7 Hz), 13.49 (1H, brs) | 562 [M+H]⁺ |
| 171 | 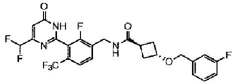 | DMSO-d₆,δ:2.09-2.23 (2H, m), 2.29-2.41 (2H, m), 2.95-3.07 (1H, m), 4.19 (1H, quin, J = 6.8 Hz), 4.34-4.44 (4H, m), 6.65 (1H, brs), 6.75 (1H, t, J = 54.2 Hz), 7.05-7.19 (3H, m), 7.39 (1H, td, J = 7.9, 6.0 Hz), 7.63 (1H, t, J = 7.9 Hz), 7.73 (1H, d, J = 7.9 Hz), 8.50 (1 H, 1, J = 5.9 Hz), 13.53 (1H, brs) | 544 [M+H]⁺ |
| 172 | 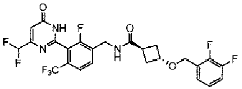 | DMSO-d₆,δ : 2.08-2.20 (2H, m), 2.28-2.40 (2H, m), 2.95-3.08 (1H, m), 4.21 (1H, quin, J = 6.5 Hz), 4.39 (2H, d, J = 5.3 Hz), 4.46 (2H, d, J = 1.1 Hz), 6.68 (1H, brs), 6.76 (1H, t, J = 54.0 Hz), 7.15-7.30 (2H, m), 7.32-7.46 (1H, m), 7.64 (1H, t, J = 7.2 Hz), 7.74 (1H, d, J = 8.3 Hz), 8.50 (1H, t, J = 6.0 Hz). 13.56 (1H, brs) | 562 [M+H]⁺ |
| 173 | 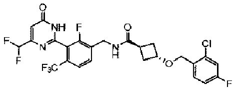 | DMSO-d₆,δ:2.10-2.24 (2H, m), 2.30-2.42 (2H, m), 2.95-3.08 (1H, m), 4.23 (1H, quin, J = 6.5 Hz), 4.35-4.47 (4H, m), 6.66 (1H, brs), 6.75 (1H, t, J = 54.2 Hz), 7.23 (1H, td, J = 8.7. 2.6 Hz), 7.44 (1H, dd, J = 8.9, 2.5 Hz), 7.53 (1H, dd, J = 8.7, 6.4 Hz), 7.63 (1H, t, J = 7.6 Hz), 7.73 (1 H, d, J = 8.3 Hz), 8.50 (1H, t. J = 5.9 Hz), 13.58 (1H, brs) | 578 [M+H]⁺ |
| 174 | 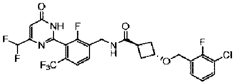 | DMSO-d₆,δ: 2.08-2.24 (2H, m), 2.27-2.41 (2H, m), 2.94-3.10 (1H, m), 4.21 (1H, quin, J = 6.4 Hz), 4.40 (2H, d, J = 5.7 Hz), 4.45 (2H, s), 6.71 (1H, s), 6.77 (1H, t, J = 54.0 Hz). 7.23 (1H, td, J = 7.9, 0.8 Hz), 7.39-7.43 {1H, m), 7.49-7.58 (1H, m), 7.65 (1H, t, J = 7.2 Hz). 7.75 (1H, d, J = 8.3 Hz), 8.51 (1H, t, J = 5.9 Hz), 13.54 (1H, brs) | 578 [M+H]⁺ |

**[Table 38]**

| **Example No.** | **Chemical Structure** | **NMR (¹H-NMR)** | **MS (m/z)** |
|---|---|---|---|
| 175 | 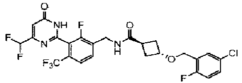 | DMSO-d₆,δ: 2.08-2.22 (2H, m), 2.29-2.42 (2H, m), 2.95-3.08 (1H, m), 4.21 (1H, quin, J = 6.7 Hz), 4.33-4.48 (4H, m), 6.70 (1H, s), 6.77 (1H, t. J = 54.0 Hz), 7.23-7.29 (1H, m), 7.39-7.46 (1H, m), 7.49 (1H, dd, J = 6.2. 2.8 Hz), 7.65 (1H, t, J = 7.6 Hz), 7.75 (1H, d, J = 7.9 Hz), 8.51 (1H, t, J = 5.9 Hz), 13.55 (1H, brs) | 578 [M+H]⁺ |
| 176 | 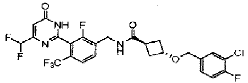 | DMSO-d₆,δ:2.09-2.23 (2H, m), 2.27-2.43 (2H, m), 2.95-3.07 (1H, m), 4.19 (1H, quin, J = 6.7 Hz), 4.36 (2H, s), 4.39 (2H, d, J = 5.7 Hz), 6.66 (1H, brs), 6.75 (1H, t, J = 54.0 Hz), 7.26-7.43 (2H, m), 7.53 (1H, dd, J = 7.4, 1.7 Hz), 7.63 (1H, t, J = 7.2 Hz), 7.73 (1H, d, J = 8.3 Hz), 8.50 (1H, t, J = 5.9 Hz), 13.56 (1H, brs) | 578 [M+H]⁺ |
| 177 | 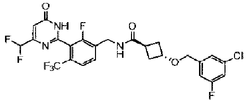 | DMSO-d₆,δ: 2.08-2.23 (2H, m), 2.28-2.44 (2H, m), 2.95-3.09 (1H, m), 4.20 (1H, quin, J = 6.6 Hz), 4.33-4.48 (4H, m), 6.71 (1H, s), 6.77 (1H, t, J = 54.2 Hz), 7.14-7.18 (1H, m), 7.25 (1H, s), 7.34 (1H, dt, J = 9.0, 2.1 Hz), 7.65 (1H, t, J = 7.6 Hz), 7.75 (1H, d, J = 8.3 Hz), 8.51 (1H, t, J = 5.9 Hz), 13.55 (1H, brs) | 578 [M+H]⁺ |
| 178 | 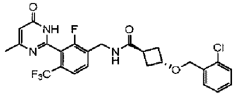 | DMSO-d₆,δ:2.08-2.26 (2H, m), 2.22 (3H, s), 2.30-2.42 (2H, m), 2.95-3.08 (1H, m), 4.24 (1H, quin, J = 6.7 Hz), 4.39 (2H, d, J = 5.7 Hz), 4.44 (2H, s), 6.26 (1H, s), 7,31-7,39 (2H, m). 7.41-7.53 (2H, m), 7.61 (1H, t, J = 7.6 Hz), 7.71 (1H, d, J = 8.3 Hz). 8.50 (1H, t, J = 5.9 Hz), 12.92 (1H, brs) | 524 [M+H]⁺ |
| 179 | 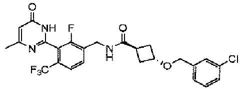 | DMSO-d₆,δ: 2.09-2.25 (2H, m), 2.22 (3H. s), 2.28-2.39 (2H, m), 2.95-3.06 (1H, m), 4.19 (1H, quin, J = 6.7 Hz), 4.34-4.42 (4H, m), 6.26 (1H, s), 7.25-7.31 (1H, m), 7.31-7.42 (3H, m), 7.60 (1H, t, J = 7.9 Hz), 7.71 (1H, d, J = 8.3 Hz), 8.50 (1H, t, J = 5.9 Hz), 12.85 (1H, brs) | 524 [M+H]⁺ |
| 180 | 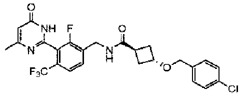 | DMSO-d₆,δ:2.07-2.19 (2H, m), 2.22 (3H, s), 2.27-2.39 (2H, m), 2.94-3.06 (1H, m), 4.18 (1H, quin, J = 6.7 Hz), 4.31-4.45 (4H, m), 6.26 (1H, brs), 7.29-7.37 (2H, m), 7.37-7.43 (2H, m), 760 (1H, t, J= 7.6 Hz), 7.71 (1H, d, J = 8.3 Hz), 8.49 (1H, t, J = 5.9 Hz), 12.91 (1H, brs) | 524 [M+H]⁺ |

**[Table 39]**

| **Example No.** | **Chemical Structure** | **NMR (¹H-NMR)** | **MS (m/z)** |
|---|---|---|---|
| 181 | 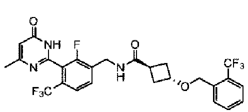 | DMSO-d₆,δ: 2.09-2.20 (2H, m), 2.22 (3H, s), 2.30-2.42 (2H, m), 2.95-3.08 (1H, m), 4.23 (1H, quin, J = 6.7 Hz), 4.39 (2H, d, J = 6.0 Hz), 4.53 (2H, s), 6,28 (1H, brs), 7.48-7.56 (1H, m), 7.57-7.65 (1H, m), 7.67-7.75 (4H, m), 8.50 1H, t, J = 6.0 Hz), 12.91 (1H, brs) | 558 [M+H]⁺ |
| 182 | 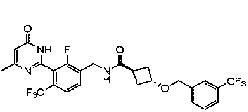 | DMSO-d₆,δ:2.10-2.26 (2H, m), 2.22 (3H, s), 2.28-2.41 (2H, m), 2.95-3.07 (1H, m), 4.22 1H, quin. J = 6.5 Hz), 4.39 (2H, d, J = 5.7 Hz), 4.47 (2H, s), 6.27 (1H, s), 7.31-7.96 (6H. m), 8.50 (1H, t, J = 5.9 Hz), 12.88 (1H, brs) | 558 [M+H]⁺ |
| 183 | 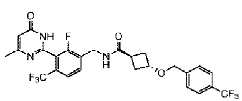 | DMSO-d₆,δ:2.10-2.25 (2H, m), 2.22 (3H, s), 2.30-2.41 (2H, m), 2.94-3.09 (1H, m), 4.22 1H, quin, J = 6.7 Hz), 4.38 (2H, d, J = 6.0 Hz), 4.48 (2H, s). 6.27 (1H. s). 7.49-7.65 (3H, m), 7.65-7.77 (3H, m), 8.50 (1H, 1, J = 5.9 Hz), 12.89 (1H, brs) | 558 [M+H]⁺ |
| 184 | 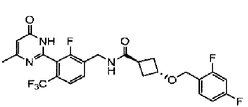 | DMSO-d₆,δ:2.05-2.18 (2H, m), 2.22 (3H, s), 2.27-2.39 (2H, m), 2.93-3.08 (1H, m), 4.19 (1H, quin, J = 6.6 Hz), 4.31-4.45 (4H, m), 6.27 1H, s), 7.08 (1 H, tdd, J = 8.5, 2.5, 1.1 Hz), 7.18-7.29 (1H, m), 7.48 (1H, td, J = 8.5, 6.8 Hz), 7.60 (1H, t, J = 7.6 Hz), 7.71 (1H, d, J = 8.3 Hz), 8.49 (1H, t, J = 5.7 Hz), 12.87 (1H, brs) | 526 [M+H]⁺ |
| 185 | 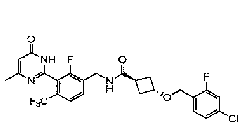 | DMSO-d₆,δ:2.05-2.18 (2H, m), 2.22 (3H. s), 2.27-2.41 (2H, m), 2.94-3.05 (1H, m), 4.20 (1H, quin, J = 6.5 Hz), 4.34-4.44 (4H, m), 6.27 (1H, s), 7.29 (1H, dd, J = 8.3, 2.3 Hz), 7.38-7.52 (2H, m), 7.60 (1H, t, J = 7.6 Hz), 7.71 1H, d, J = 8.3 Hz), 8.49 (1H, t, J = 5.9 Hz), 2.89 (1H, brs) | 542 [M+H]⁺ |
| 186 | 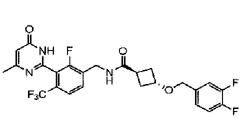 | DMSO-d₆,δ:2.08-2.20 (2H, m), 2.22 (3H, s), 2.28-2.38 (2H, m), 2.94-3.06 (1H, m), 4.19 (1H, quin, J = 6.6 Hz), 4.35 (2H, s), 4.38 (2H, d, J = 5.7 Hz), 6.28 (1H, s), 7.13-7.21 (1H, m), 7.32-7.45 (2H, m), 7.60 (1H, t. J = 7.6 Hz), 7.71 (1H, d, J = 8.3 Hz), 8.49 (1H, t, J = 6.0 Hz), 12.88 (1H, brs) | 526 [M+H]⁺ |

**[Table 40]**

| **Example No.** | **Chemical Structure** | **NMR (¹H-NMR)** | **MS (m/z)** |
|---|---|---|---|
| 187 | 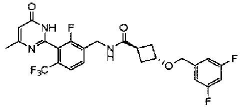 | DMSO-d₆,δ:2.08-2.25 (2H, m), 2.22 (3H, s), 2.29-2.40 (2H, m), 2.95-3.10 (1H, m), 4.20 (1H, quin, J = 6.5 Hz), 4.30-4.45 (4H, m), 6.27 (1H, s), 6.98-7.08 (2H, m), 7.13 (1H, tt, J = 9.4, 2.3 Hz), 7.61 (1H, t, J = 7.6 Hz), 7.71 (1H, d, J = 8.3 Hz), 8.50 (1H, t, J = 5.9 Hz), 12,92 (1H, brs) | 526 [M+H]⁺ |
| 188 | 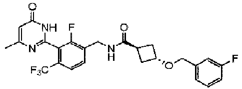 | DMSO-d₆,δ:2.08-2.25 (2H, m), 2.22 (3H, s), 2.29-2.40 (2H, m), 2.94-3.06 (1H, m), 4.19 (1H, quin, J = 6.6 Hz), 4.32-4.45 (4H, m), 6.28 (1H, s). 7.04-7.23 (3H, m), 7.39 (1H, td, J = 7.9, 6.0 Hz), 7.61 (1H, t, J = 7.8 Hz), 7.71 (1H, d, J = 8.3 Hz), 8.50 (1H, t, J = 5.9 Hz), 12.85 (1H, brs) | 508 [M+H]⁺ |
| 189 | 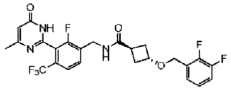 | DMSO-d₆,δ:2,06-2.20 (2H, m), 2.22 (3H, s), 2.28-2.42 (2H, m), 2.93-3.06 (1H, m), 4.21 (1H, quin, J = 6.6 Hz), 4.38 (2H, d, J = 5.7 Hz), 4.48 (2H, d, J = 1.1 Hz), 6.28 (1H, s), 7.13-7.30 (2H, m), 7.30-7.44 (1H, m), 7.61 (1H, t, J = 7.6 Hz), 7.71 (1H, d, J = 8.3 Hz), 8.50 (1H, t, J = 5.9 Hz), 12,89 (1H, brs) | 526 [M+H]⁺ |
| 190 | 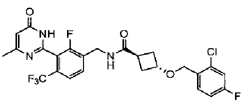 | DMSO-d₆,δ.2.09-2.24 (2H, m), 2.22 (3H, s), 2.29-2.42 (2H, m), 2.95-3.08 (1H, m), 4.23 (1H. quin, J = 6.4 Hz), 4.39 (2H, d, J = 5.7 Hz), 4.41 (2H, s), 6.26 (1H. s), 7.23 (1H, td, J = 8.7, 2.6 Hz). 7.44 (1H, dd, J = 9.1, 2.6 Hz), 7.53 (1H, dd, J = 8.7, 8.4 Hz). 7.61 (1H, t, J = 7.6 Hz), 7.71 (1H, d, J = 7.9 Hz), 8.50 (1H, t, J = 5.9 Hz), 12.91 (1H, brs) | 542 [M+H]⁺ |
| 191 | 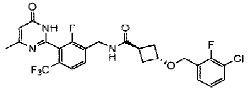 | DMSO-d₆,δ:2.08-2.19 (2H, m), 2.22 (3H, s), 2.29-2.39 (2H, m), 2.95-3.06 (1H, m), 4.21 (1H, quin, J = 6.6 Hz), 4.38 (2H, d, J = 5.7 Hz), 4.45 (2H, d, J = 0.8 Hz), 6.27 (1H, s), 7.18-7.27 (1H, m), 7.38-7.45 (1H, m), 7.50-7.85 (2H, m), 7.68-7.75 (1H, m), 8.50 (1H, t, J = 5.9 Hz), 12.93 (1H, brs) | 542 [M+H]⁺ |
| 192 | 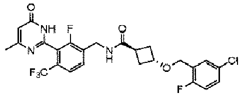 | DMSO-d₆,δ: 2.10-2.19 (2H, m), 2.22 (3H, s), 2.30-2.38 (2H, m), 2.97-3.05 (1H, m), 4.21 (1H, quin, J = 6.6 Hz), 4.35-4.43 (4H, m), 626 (1H, s), 7.26 (1H, t, J = 9.2 Hz), 7.39-7.46 (1H, m), 7.48-7.51 (1H, m), 7.57-7.64 (1H, m), 7.71 (1H, d, J = 8.3 Hz), 8.52 (1H, t, J = 5.9 Hz), 12.94 (1H, brs) | 542 [M+H]⁺ |

**[Table 41]**

| **Example No.** | **Chemical Structure** | **NMR (¹H-NMR)** | **MS (m/z)** |
|---|---|---|---|
| 193 | 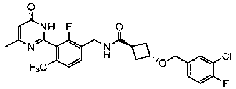 | DMSO-d₆,δ:2.10-2.19 (2H, m), 2.22 (3H, s), 2.29-2.37 (2H, m), 2.96-3.05 (1H, m), 4.19 (1H, quin, J = 6.5 Hz), 4.36 (2H, s), 4.38 (2H, d, J = 5.6 Hz), 6.26 (1H, s), 7.31-7.36 (1H, m), 7.36-7.42 (1H, m), 7.53 (1H, dd, J = 7.3, 2.0 Hz), 7.57-7.64 (1H, m), 7.71 (1H, d, J = 8.3 Hz), 8.52 (1H, t, J = 5.9 Hz), 12.94 (1H, brs) | 542 [M+H]⁺ |
| 194 | 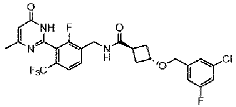 | DMSO-d₆,δ:2.12-2.21 (2H, m), 2.22 (3H, s), 2.30-2.38 (2H, m), 2.97-3.06 (1H, m), 4.20 (1H, quin, J = 6.7 Hz), 4.36-4.41 (4H, m), 6.28 (1H, s), 7.13-7.20 (1H, m), 7.26 (1H, s), 7.35 (1H. dt, J = 8.8, 2.2 Hz), 7.57-7.65 (1H, m), 7.72 (1H, d, J = 8.3 Hz), 8.52 (1H, t, J = 5.9 Hz), 12.97 (1H, brs) | 542 [M+H]⁺ |
| 195 | 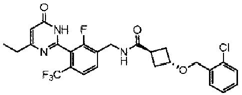 | DMSO-d₆,δ: 1.13 (3H, t, J = 7.5 Hz), 2.13-2.22 (2H, m), 2.32-2.40 (2H, m), 2.45-2.49 (2H, m), 2.97-3.06 (1H, m), 4.24 (1H, quin, J = 6.6 Hz), 4.39 (2H, d, J = 5.9 Hz), 4.44 (2H, s), 6.25 (1H, brs), 7.31-7.38 (2H, m), 7.43-7.48 (1H, m), 7.48-7.51 (1H, m), 7.61 (1H, t, J = 7.5 Hz), 7.72 (1H, d, J = 8.3 Hz), 8.52 (1H, t, J = 5.9 Hz), 12.95 (1H, brs) | 538 [M+H]⁺ |
| 196 | 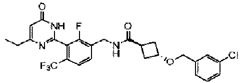 | DMSO-d₆,δ:1.13 (3H, t, J = 7.5 Hz), 2.11-2.20 (2H, m), 2.30-2.38 (2H, m), 2.44-2.49 (2H, m), 2.96-3.05 (1H, m), 4.19 (1H, quin, J = 6.6 Hz), 4.36-4.41 (4H, m), 6.25 (1H, brs), 7.26-7.31 (1H, m), 7.32-7.42 (3H, m), 7.57-7.65 (1H, m), 7.72 (1H, d, J = 8.1 Hz), 8.52 (1H, t, J = 6.0 Hz), 12.95 (1H, brs) | 538 [M+H]⁺ |
| 197 | 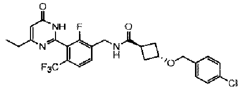 | DMSO-d₆,δ: 1.13 (3H, t, J = 7.6 Hz), 2.10-2.19 (2H, m), 2.29-2.37 (2H, m), 2.45-2.49 (2H, m), 2.95-3.05 (1H, m), 4.18 (1H, quin. J = 6.6 Hz), 4.36 (2H, s), 4.38 (2H, d, J = 5.9 Hz), 6.25 (1H, brs), 7.32-7.36 (2H, m), 7.38-7.43 (2H, m), 7.57-7.64 (1H, m), 7.72 (1H, d, J = 8.1 Hz), 8.51 (1H, t, J = 5.9 Hz), 12.96 (1H, brs) | 538 [M+H]⁺ |
| 198 | 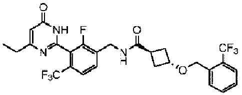 | DMSO-d₆,δ:1.13 (3H, t. J = 7.5 Hz), 2.12-2,21 (2H, m), 2.32-2.39 (2H, m), 2.45-2.49 (2H, m), 2.98-3.06 (1H, m), 4.23 (1H, quin, J = 6.5 Hz), 4.39 (2H, d, J = 5.9 Hz), 4.53 (2H, s), 6.24 (1H, brs), 7.49-7.57 (1H, m), 7.58-7.65 (1H, m), 7.66-7.75 (4H, m), 8.53 (1H, t, J = 5.9 Hz), 12.96 (1H, brs) | 572 [M+H]⁺ |

**[Table 42]**

| **Example No.** | **Chemical Structure** | **NMR (¹H-NMR)** | **MS (m/z)** |
|---|---|---|---|
| 199 | 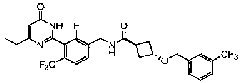 | DMSO-d₆,δ:1.13 (3H, t, J = 7.5 Hz), 2.11-2.24 (2H, m), 2.29-2.39 (2H, m), 2.42-2.56 (2H, m), 2.97-3.05 (1H, m), 4.22 (1H, quin. J = 6.5 Hz), 4.39 (2H, d, J = 5.9 Hz), 4.47 (2H, s), 6.25 (1H, brs), 7.56-7.68 (5H, m), 7.72 (1H, d, J = 8.3 Hz), 8,53 (1H, t, J = 5,7 Hz), 12.96 (1H, brs) | 572 [M+H]⁺ |
| 200 | 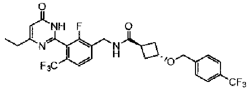 | DMSO-d₆,δ:1.13 (3H, t, J = 7.5 Hz), 2.11-2.22 (2H, m), 2.30-2.40 (2H, m), 2.43-2.49 (2H, m), 2.97-3.06 (1H, m), 4.22 (1H, quin, J = 6.6 Hz), 4.39 (2H, d, J = 5.9 Hz), 4.48 (2H, s), 6.25 (1H, brs), 7.55 (2H, d, J = 8.1 Hz), 7.57-7.66 (1H, m), 7.71 (3H, d, J = 8.1 Hz), 8.52 (1H, t, J = 5.9 Hz), 12.96 (1H, brs) | 572 [M+H]⁺ |
| 201 | 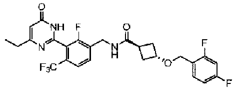 | DMSO-d₆,δ:1.13 (3H, t, J = 7.5 Hz), 2.06-2.19 (2H, m), 2.27-2.39 (2H, m), 2.45-2.57 (2H, m), 2.93-3.06 (1H, m), 4.19 (1H, quin, J = 6.5 Hz), 4.36-4.41 (4H, m), 6.25 (1H, brs), 7.08 (1H, tdd, J = 8.5, 2.6, 0.9 Hz), 7.19-7.29 (1H, m), 7.48 (1H, td, J = 8.6, 6.8 Hz), 7.57-7.66 (1H, m), 7.72 (1H, d, J = 8.3 Hz), 8.51 (1H, t, J = 5.9 Hz), 12.93 (1H, brs) | 540 [M+H]⁺ |
| 202 | 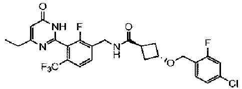 | DMSO-d₆,δ: 1.09-1.16 (3H, m), 2.07-2.18 (2H, m), 2.28-2.38 (2H, m), 2.43-2.49 (2H, m), 2.93-3.06 (1H, m), 4.20 (1H, quin, J = 6.5 Hz). 4.36-4.42 (4H, m), 6.24 (1H, brs), 7.30 (1H, dd, J = 8.3, 2.0 Hz), 7.40-7.51 (2H, m), 7.56-7.65 (1H, m), 7.72 (1H, d, J = 8.3 Hz), 8.51 (1H, t, J = 5.9 Hz), 12.97 (1H, brs) | 556 [M+H]⁺ |
| 203 | 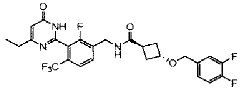 | DMSO-d₆,δ: 1.13 (3H, t, J = 7.5 Hz), 2.10-2.20 (2H, m), 2.28-2.37 (2H, m), 2.44-2.49 (2H, m), 2.95-3.05 (1H, m), 4.19 (1H, quin, J = 6.7 Hz), 4.35 (2H, s), 4.39 (2H, d, J = 5.9 Hz), 6.25 (1H, brs), 7.14-7.22 (1H, m), 7.33-7.45 (2H, m), 7.57-7.84 (1H, m), 7.72 (1H, d, J = 8.1 Hz), 8.52 (1H, t, J = 5.9 Hz), 12.95 (1H, brs) | 540 [M+H]⁺ |
| 204 | 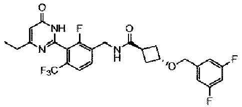 | DMSO-d₆,δ: 1.13 (3H, t, J = 7.5 Hz), 2.12-2.23 (2H, m), 2.29-2.39 (2H, m), 2.45-2.49 (2H, m), 2,96-3.07 (1H, m), 4.20 (1H, quin, J = 6.5 Hz), 4.37-4.42 (4H, m), 6.25 (1H, brs), 7.04 (2H, dd, J = 8.4, 2.3 Hz), 7.14 (1H, tt. J = 9.4, 2.4 Hz), 7.58-7.66 (1H, m), 7.72 (1H, d, J = 8.3 Hz), 8,53 (1H, t, J = 6.0 Hz), 12.97 (1H, brs) | 540 [M+H]⁺ |

**[Table 43]**

| **Example No.** | **Chemical Structure** | **NMR (¹H-NMR)** | **MS (m/z)** |
|---|---|---|---|
| 205 | 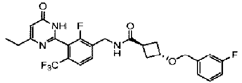 | DMSO-d₆,δ: 1.13 (3H, t, J = 7.5 Hz). 2.11-2.22 (2H, m), 2.28-2.39 (2H, m), 2.43-2.49 (2H, m), 2.96-3.06 (1H, m), 4.19 (1H, quin. J = 6.6 Hz), 4.39 (4H, s), 6.25 (1H, brs), 7.06-7.19 (3H, m), 7.39 (1H, td, J = 7.9, 6.1 Hz), 7.61 (1H, t, J = 7.8 Hz), 7.72 (1H, d, J = 8.1 Hz), 8.52 (1H, t, J = 6.0 Hz), 12.92 (1H, brs) | 522 [M+H]⁺ |
| 206 | 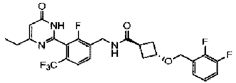 | DMSO-d₆,δ: 1.13 (3H, t, J = 7.5 Hz), 2.09-2.19 (2H, m), 2.29-2.39 (2H, m), 2.45-2.56 (2H, m), 2.96-3.06 (1H, m), 4.21 (1H, quin, J = 6.7 Hz), 4.39 (2H, d, J = 5.6 Hz), 4.46 (2H, s), 6.25 (1H, brs), 7.16-7.31 (2H, m), 7.33-7.46 (1H, m), 7.56-7.67 (1H, m), 7.72 (1H, d, J = 8.3 Hz), 8.52 (1H, t, J = 5.9 Hz), 12.96 (1H, brs) | 540 [M+H]⁺ |
| 207 | 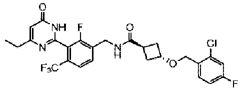 | DMSO-d₆,δ: 1.13 (3H, t, J = 7.5 Hz), 2.11-2.22 (2H, m), 2.35 (2H, ddd, J = 12.8, 6.8, 3.8 Hz), 2.44-2.49 (2H, m), 2.97-3.06 (1H, m), 4.23 (1H, quin, J = 6.5 Hz), 4.37-4.43 (4H, m), 6.25 (1H, brs), 7.23 (1H, td, J = 8.6, 2.7 Hz), 7.45 (1H, dd, J = 8.9, 2.6 Hz), 7.53 (1H. dd, J = 8.7, 6.5 Hz), 7.58-7.66 (1H, m), 7.72 (1H, d, J = 8.1 Hz), 8.52 (1H, t, J = 5.9 Hz), 12.95 (1H, brs) | 556 [M+H]⁺ |
| 208 | 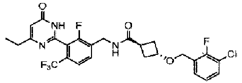 | DMSO-d₆,δ: 1.13 (3H, 1, J = 7.5 Hz), 2.09-2.19 (2H, m), 2.29-2.39 (2H, m), 2.45-2.55 (2H, m), 2.95-3.06 (1H, m), 4.21 (1H, quin, J = 6.6 Hz). 4.36-4.43 (2H, m), 4.45 (2H, s), 6.26 (1H, brs), 7.23 (1H, td, J = 7.9, 1.0 Hz), 7.38-7.45 (1H, m), 7.52-7.58 (1H, m), 7.58-7.65 (1H, m), 7.72 (1H, d, J = 8.3 Hz), 8.52 (1H, t, J = 5.9 Hz), 12.97 (1H, brs) | 556 [M+H]⁺ |
| 209 | 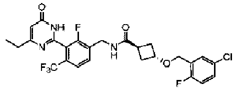 | DMSO-d₆,δ: 1.13 (3H, t, J = 7.5 Hz), 2.10-2.21 (2H, m), 2.34 (2H, qd, J = 6.6, 3.9 Hz), 2.43-2.49 (2H, m), 2.96-3.06 (1H, m), 4.21 (1H, quin, J = 6.5 Hz), 4.37-4.43 (4H, m), 6.25 (1H, brs), 7.22-7.31 (1H, m), 7.43 (1H, ddd, J = 8.8, 4.5, 2.8 Hz), 7.49 (1H, dd, J = 6.2, 2.8 Hz), 7.58-7.66 (1H, m), 7.72 (1H, d, J = 8.3 Hz), 8.52 (1H, t, J = 6.0 Hz), 12.94 (1H, brs) | 556 [M+H]⁺ |
| 210 | 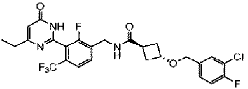 | DMSO-d₆,δ: 1.13 (3H, t, J = 7.6 Hz), 2.10-2.20 (2H, m), 2.29-2.37 (2H, m), 2.45-2.49 (2H, m), 2.96-3.05 (1H, m), 4.19 (1H, quin, J = 6.5 Hz), 4.36 (2H, s), 4.39 (2H, d, J = 5.9 Hz), 8.25 (1H, brs), 7.31-7.42 (2H, m), 7.53 (1H, dd, J = 7.3, 2.0 Hz), 7.57-7.66 (1H, m), 7.72 (1H, d, J = 8.3 Hz), 8.52 (1H, t, J = 6.0 Hz), 12.95 (1H, brs) | 556 [M+H]⁺ |

**[Table 44]**

| **Example No.** | **Chemical Structure** | **NMR (¹H-NMR)** | **MS (m/z)** |
|---|---|---|---|
| 211 | 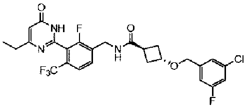 | DMSO-d₆,δ:1.13 (3H, t, J = 7.5 Hz), 2.12-2.22 (2H, m), 2.28-2.38 (2H, m), 2.46-2.49 (2H, m), 2.96-3.06 (1H, m), 4.20 (1H, quin, J = 6.5 Hz), 4.36-4.42 (4H, m), 6.25 (1H, brs), 7.17 (1H, d, J = 9.3 Hz), 7.26 (1H, s), 7.35 (1H, dt, J = 8.7, 2.1 Hz), 7.58-7.65 (1H, m), 7.70-7.75 (1H, m), 8.53 (1H, t, J = 5.9 Hz), 12.96 (1H, brs) | 556 [M+H]⁺ |
| 212 | 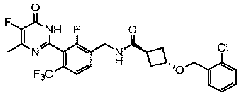 | DMSO-d₆,δ:2.12-2.21 (2H, m), 2.26 (3H, d, J = 3.7 Hz), 2.32-2.41 (2H, m), 2.97-3.06 (1H, m), 4.24 (1H, quin, J = 6.5 Hz), 4.39 (2H, d, J = 5.6 Hz), 4.44 (2H, s), 7.31-7.38 (2H, m), 7.42-7.47 (1H, m), 7.47-7.52 (1H, m), 7.60-7.67 (1H, m), 7.74 (1H, d, J = 8.3 Hz), 8.53 (1H, t, J = 5.7 Hz), 13,62 (1H, brs) | 542 [M+H]⁺ |
| 213 | 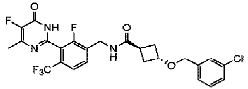 | DMSO-d₆,δ:2.12-2.19 (2H, m), 2.26 (3H, d, J = 4.0 Hz), 2.30-2.36 (2H, m), 2.97-3.04 (1H, m), 4.19 (1H, quin, J = 6.4 Hz), 4.38-4.39 (4H, m), 7.28 (1H, dd, J = 7.6, 1.2 Hz), 7.32-7.41 (3H, m), 7.63 (1H, t, J = 8.0 Hz), 7.73 (1H, d, J = 8.4 Hz), 8.52 (1H, t, J = 6.0 Hz), 13.61 (1H, brs) | 542 [M+H]⁺ |
| 214 | 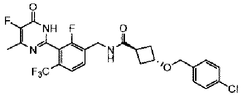 | DMSO-d₆,δ:2.09-2.19 (2H, m), 2.26 (3H, d, J = 3.7 Hz), 2.29-2.37 (2H, m), 2.95-3.05 (1H, m), 4.18 (1H, quin, J = 6.7 Hz), 4.36 (2H, s), 4.38 (2H, d, J = 5.9 Hz), 7.32-7.36 (2H, m), 7.38-7,42 (2H, m), 7.58-7.65 (1H, m), 7.71-7.75 (1H, m), 8.52 (1H, t, J = 6.0 Hz), 13.63 (1H, brs) | 542 [M+H]⁺ |
| 215 | 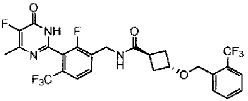 | DMSO-d₆,δ:2.12-2.21 (2H, m), 2.26 (3H, d, J = 3.4 Hz), 2.31-2.40 (2H, m), 2.97-3.06 (1H, m), 4.23 (1H, quin, J = 6.5 Hz), 4.39 (2H, d, J = 5.6 Hz), 4.53 (2H, s), 7.49-7.56 (1H, m), 7.59-7.66 (1H, m), 7.67-7.70 (2H, m), 7.71-7.75 (2H, m), 8.53 (1H, t, J = 6.0 Hz), 13.63 (1H, brs) | 576 [M+H]⁺ |
| 216 | 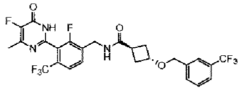 | DMSO-d₆,δ:2.12-2.23 (2H, m), 2.26 (3H, d, J = 3.8 Hz), 2.30-2.40 (2H, m), 2.96-3.07 (1H, m), 4.22 (1H, quin, J = 6.7 Hz), 4.39 (2H, d, J = 5.7 Hz), 4.47 (2H, s), 7.55-7.88 (5H, m), 7.70-7.76 (1H, m), 8.51 (1H, t, J = 5.9 Hz), 13.59 (1H, brs) | 576 [M+H]⁺ |

**[Table 45]**

| **Example No.** | **Chemical Structure** | **NMR (¹H-NMR)** | **MS (m/z)** |
|---|---|---|---|
| 217 | 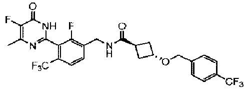 | DMSO-d₆,δ:2.10-2.23 (2H, m), 2.25 (3H, d, J = 3.4 Hz), 2.35 (2H, qd, J = 6.5, 3.8 Hz), 2.96-3.07 (1H, m), 4.22 (1H, quin, J = 6.7 Hz), 4.39 (2H, d, J = 5.7 Hz), 4.48 (2H. s), 7.55 (2H, d, J = 7.9 Hz), 7.59-7.67 (1H, m), 7.68-7.78 (3H, m), 8.51 (1H, t, J = 5.9 Hz), 13.60 (1H, brs) | 576 [M+H]⁺ |
| 218 | 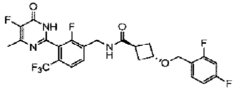 | DMSO-d₆,δ:2.07-2.17 (2H, m), 2.26 (3H, d, J = 3.7 Hz), 2.29-2.38 (2H, m), 2.92-3.08 (1H, m), 4.19 (1H, quin, J = 6.6 Hz), 4.34-4.42 (4H, m), 7.08 (1H, t, J = 8.3 Hz), 7.23 (1H, t, J = 9.9 Hz), 7.48 (1H, td, J = 8.5, 7.0 Hz), 7.63 (1H, t, J = 7.7 Hz), 7.74 (1H, d, J = 8.1 Hz), 8.52 (1H, t, J = 5.9 Hz), 13.64 (1H, brs) | 544 [M+H]⁺ |
| 219 | 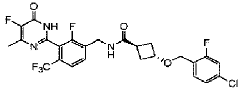 | DMSO-d₆,δ:2.05-2.20 (2H, m), 2.26 (3H, d, J = 3.8 Hz), 2.33 (2H, qd, J = 6.5, 3.8 Hz), 2.93-3.06 (1H, m), 4.20 (1H, quin, J = 6.6 Hz), 4.35-4.43 (4H, m), 7.29 (1H, dd, J = 8.3, 1.5 Hz), 7.39-7.53 (2H, m), 7.58-7.67 (1H, m), 7.69-7.77 (1H, m), 8.50 (1H, t, J = 5.9 Hz), 13.59 (1H, brs) | 560 [M+H]⁺ |
| 220 | 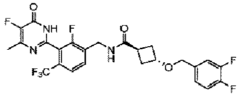 | DMSO-d₆,δ:2.08-2.21 (2H, m), 2.26 (3H, d, J = 3.8 Hz), 2.28-2.38 (2H, m), 2.94-3.08 (1H, m), 4.19 (1H, quin, J = 6.6 Hz), 4.32-4.45 (4H, m), 7.10-7.23 (1H, m), 7.32-7.47 (2H, m), 7.56-7.68 (1H, m), 7.69-7.78 (1H, m), 8.50 (1H, t, J = 5.9 Hz), 13.60 (1H, brs) | 544 [M+H]⁺ |
| 221 | 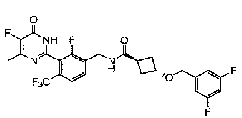 | DMSO-d₆,δ:2.10-2.22 (2H, m), 2.26 (3H, d, J = 3.4 Hz), 2.34 (2H, qd, J = 6.5, 3.8 Hz), 2.93-3.08 (1H, m), 4.20 (1H, quin, J = 6.5 Hz), 4.33-4.47 (4H, m), 6.97-7.22 (3H, m), 7.57-7.67 (1H, m), 7.70-7.78 (1H, m), 8.51 (1H, t, J = 5.9 Hz), 13.59 (1H, brs) | 544 [M+H]⁺ |
| 222 | 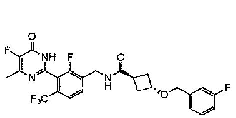 | DMSO-d₆,δ:2.10-2.22 (2H, m), 2.26 (3H, d, J = 3.8 Hz), 2.29-2.39 (2H, m), 2.95-3.07 (1H, m), 4.19 (1H, quin, J = 6.7 Hz), 4.34-4.43 (4H, m), 7.05-7.19 (3H, m), 7.39 (1H, td, J = 7.8, 6.2 Hz), 7.58-7.67 (1H, m), 7.70-7.78 (1H, m), 8.50 (1H, t, J = 5.9 Hz), 13.60 (1H, brs) | 526 [M+H]⁺ |

**[Table 46]**

| **Example No.** | **Chemical Structure** | **NMR (¹H-NMR)** | **MS (m/z)** |
|---|---|---|---|
| 223 | 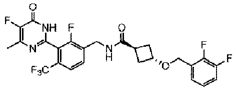 | DMSO-d₆,δ:2.06-2.20 (2H, m), 2.26 (3H, d, J = 3.4 Hz), 2.34 (2H, qd, J = 6.5, 3.8 Hz), 2.94-3.07 (1H, m), 4.21 (1H, quin, J = 6.7 Hz), 4.39 (2H, d, J = 5.7 Hz), 4.46 (2H, d, J = 1.1 Hz), 7.16-7.30 (2H, m), 7.33-7.45 (1H, m), 7.59-7.67 (1H, m), 7.70-7.76 (1H, m), 8.50 (1H, t, J = 5.9 Hz), 13.59 (1H, brs) | 544 [M+H]⁺ |
| 224 | 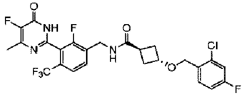 | DMSO-d₆,δ:2.10-2.22 (2H, m), 2.26 (3H, d, J = 3.8 Hz), 2.30-2.41 (2H, m), 2.96-3.07 (1H, m), 4.22 (1H, quin, J = 6.6 Hz), 4.36-4.43 (4H, m), 7.23 (1H, td, J = 8.5, 2.8 Hz), 7.44 (1H, dd, J=8.9, 2.5 Hz), 7.53 (1H, dd, J = 8.3, 6.4 Hz), 7.59-7.68 (1H, m), 7.70-7.78 (1H, m), 8.50 (1H, t, J = 5.9 Hz), 13.60 (1H, brs) | 560 [M+H]⁺ |
| 225 | 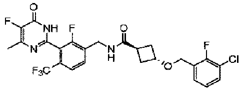 | DMSO-d₆,δ:2.08-2.20 (2H, m), 2.26 (3H, d, J = 3.8 Hz), 2.29-2.40 (2H, m), 2.94-3.07 (1H, m), 4.21 (1H, quin, J = 6.5 Hz), 4.39 (2H, d, J = 6.0 Hz), 4.45 (2H, s). 7.18-7.27 (1H, m), 7.37-7.46 (1H, m), 7.54 (1H, td, J = 7.6, 1.7 Hz), 7.59-7.68 (1H, m), 7.70-7.77 (1H, m), 8.50 (1H. t, J = 5.9 Hz), 13.59 (1H, brs) | 560 [M+H]⁺ |
| 226 | 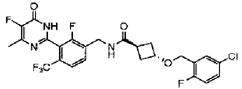 | DMSO-d₆,δ:2.09-2.21 (2H, m), 2.26 (3H, d, J = 3.8 Hz), 2.29-2.41 (2H, m), 2.95-3.07 (1H, m), 4.21 (1H, quin, J = 6.7 Hz), 4.36-4.44 (4H, m), 7.20-7.31 (1H, m), 7.39-7.46 (1H, m), 7.49 (1H, dd, J = 6.2, 2.8 Hz), 7.58-7.68 (1H, m), 7.70-7.77 (1H, m), 8.50 (1H, t, J = 5.9 Hz), 13.60 (1H, brs) | 560 [M+H]⁺ |
| 227 | 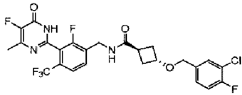 | DMSO-d₆,δ:2.08-2.21 (2H, m), 2.26 (3H, d, J = 3.8 Hz), 2.33 (2H, qd, J = 6.5, 3.8 Hz), 2.95-3.07 (1H, m), 4.19 (1H, quin, J = 6.6 Hz), 4.33-4.42 (4H, m), 7.30-7.44 (2H, m), 7.53 (1H, dd, J = 7.2, 1.9 Hz), 7.59-7.67 (1H, m), 7.70-7.76 (1H, m), 8.50 (1H, t, J = 5.9 Hz), 13.59 (1H, brs) | 560 [M+H]⁺ |
| 228 | 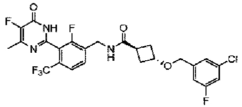 | DMSO-d₆,δ:2.10-2.22 (2H, m), 2.25 (3H. d, J = 3.8 Hz), 2.29-2.41 (2H, m), 2.95-3.09 (1H, m), 4.20 (1H, quin, J = 6.5 Hz), 4.35.4.43 (4H, m), 7.13-7.20 (1H, m), 7.25 (1H, s), 7.34 (1H, dt, J = 9.0, 2.1 Hz), 7.58-7.66 (1H, m), 7.68-7.77 (1H, m), 8.50 (1H, t, J = 5.9 Hz), 13.58 (1H, brs) | 560 [M+H]⁺ |

**[Table 47]**

| **Example No.** | **Chemical Structure** | **NMR (¹H-NMR)** | **MS (m/z)** |
|---|---|---|---|
| 229 | 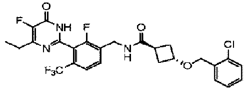 | DMSO-d₆,δ:1.13 (3H, t, J = 7.6 Hz), 2.10-2.24 (2H, m), 2.36 (2H, ddd, J = 12.7, 6.9, 4.2 Hz), 2.55-2.64 (2H, m), 2.94-3.10 (1H, m), 4.24 (1H, quin, J = 6.6 Hz), 4.39 (2H, d, J = 5.7 Hz), 4.44 (2H, s), 7.31-7.39 (2H, m), 7.42-7.54 (2H, m), 7.58-7.67 (1H, m), 7.69-7.76 (1H, m), 8.51 (1H, t, J = 6.0 Hz), 13.60 (1H, brs) | 556 [M+H]⁺ |
| 230 | 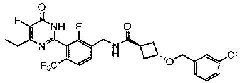 | DMSO-d₆,δ:1.14 (3H, t, J = 7.6 Hz), 2.12-2.19 (2H, m), 2.31-2.37 (2H, m), 2.58-2.61 (2H, m), 2.98-3.04 (1H, m), 4.19 (1H, quin, J = 6.4 Hz), 4.38-4.40 (4H, m), 7.27-7.30 (1H, m), 7.32-7.41 (3H, m), 7.63 (1H, t, J = 8.0 Hz), 7.74 (1H, d, J = 8.0 Hz), 8.52 (1H, 1, J = 6.0 Hz), 13.60 (1H, brs) | 556 [M+H]⁺ |
| 231 | 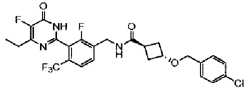 | DMSO-d₆,δ:1.13 (3H, t, J = 7.6 Hz), 2.06-2.21 (2H, m), 2.27-2.39 (2H, m), 2.55-2.64 (2H, m), 2.94-3.07 (1H, m), 4.18 (1H, quin, J = 6.8 Hz), 4.34-4.42 (4H, m), 7.30-7.43 (4H, m), 7.57-7.66 (1H, m), 7.68-7.76 (1H, m), 8.49 (1H, t, J = 5.9 Hz), 13.56 (1H, brs) | 556 [M+H]⁺ |
| 232 | 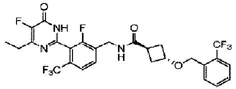 | DMSO-d₆,δ:1.13 (3H, t, J = 7.6 Hz), 2.10-2.23 (2H, m), 2.30-2.42 (2H, m), 2.60 (2H, qd, J = 7.5, 3.2 Hz), 2.97-3.08 (1H, m), 4.23 (1H, quin, J = 6.5 Hz), 4.39 (2H, d, J = 5.7 Hz), 4.52 (2H, s), 7.48-7.57 (1H, m), 7.59-7.76 (5H, m), 8.51 (1H, t, J = 5.9 Hz), 13.59 (1H, brs) | 590 [M+H]⁺ |
| 233 | 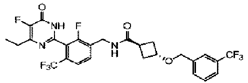 | DMSO-d₆,δ:1,13 (3H, t, J = 7.6 Hz), 2.11-2.24 (2H, m), 2.30-2.41 (2H, m), 2.60 (2H, qd, J = 7.4, 3.8 Hz), 2.95-3.07 (1H, m), 4.22 (1H, quin, J = 6.6 Hz), 4.39 (2H, d, J = 5.7 Hz), 4.47 (2H, s), 7,55-7.69 (5H, m), 7.70-7.77 (1H, m), 8.51 (1H, t, J = 5.9 Hz), 13.59 (1H, brs) | 590 [M+H]⁺ |
| 234 | 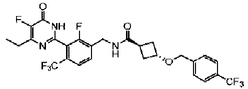 | DMSO-d₆,δ:1.13 (3H, t, J = 7.6 Hz), 2.10-2.24 (2H, m), 2.29-2.41 (2H, m), 2.54-2.65 (2H, m), 2.96-3.09 (1H, m), 4.22 (1H, quin, J = 6.8 Hz), 4.39 (2H, d, J = 5.7 Hz), 4.47 (2H, s), 7.54 (2H, d, J = 7.9 Hz), 7.58-7.66 (1H, m), 7.68-7.76 (3H, m), 8.51 (1H, t, J = 5.9 Hz), 13.60 (1H, brs) | 590 [M+H]⁺ |

**[Table 48]**

| **Example No.** | **Chemical Structure** | **NMR (¹H-NMR)** | **MS (m/z)** |
|---|---|---|---|
| 235 | 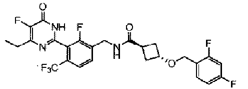 | DMSO-d₆,δ:1.13 (3H, t, J = 7.6 Hz), 2.04-2.19 (2H, m), 2.28-2.41 (2H, m), 2.55-2.64 (2H, m), 2.94-3.07 (1H, m), 4.19 (1H, quin, J = 6.5 Hz), 4.38 (4H, s), 7.08 (1H, tdd, J = 8.5, 2.6, 1.1 Hz), 7.17-7.28 (1H, m), 7.48 (1H, td, J = 8.5, 8.8 Hz), 7.56-7.66 (1H, m), 7.69-7.76 (1H, m), 8.49 (1H, t, J = 5.9 Hz), 13.58 (1H, brs) | 558 [M+H]⁺ |
| 236 | 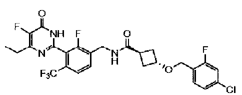 | DMSO-d₆,δ:1.13 (3H, t, J = 7.6 Hz), 2.07-2.19 (2H, m), 2.28-2.39 (2H, m), 2.59 (2H, qd, J = 7.6, 3.0 Hz), 2.94-3.07 (1H, m), 4.20 (1H, quin, J = 6.6 Hz), 4.37-4.43 (4H, m), 7.29 (1H, dd, J = 8.5, 2.1 Hz), 7.38-7.52 (2H, m), 7.56-7.66 (1H, m), 7.68-7.76 (1H, m), 8.50 (1H, t, J = 5.9 Hz), 13.60 (1H, brs) | 574 [M+H]⁺ |
| 237 | 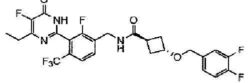 | DMSO-d₆,δ:1.10-1.16 (3H, m), 2.08-2.22 (2H, m), 2.25-2.40 (2H, m), 2.55-2.65 (2H, m), 2.95-3.07 (1H, m), 4.19 (1H, quin, J = 6.7 Hz), 4.33-4.42 (4H, m), 7.12-7.22 (1H, m), 7.32-7.43 (2H, m), 7.56-7.66 (1H, m), 7.68-7.76 (1H, m), 8.50 (1H, t, J = 5.7 Hz), 13.60 (1H, brs) | 558 [M+H]⁺ |
| 238 | 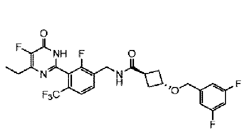 | DMSO-d₆,δ:1.13 (3H, t, J = 7.6 Hz), 2.11-2.25 (2H, m), 2.29-2.41 (2H, m), 2.59 (2H, qd, J = 7.4, 3.0 Hz), 2.95-3.09 (1H, m), 4.20 (1H, quin, J = 6.7 Hz), 4.36-4.43 (4H, m), 6.99-7.08 (2H, m), 7.13 (1H, tt, J = 9.4, 2.5 Hz), 7.57-7.67 (1H, m), 7.69-7.77 (1H, m), 8.51 (1H, t, J = 5.9 Hz), 13.59 (1H, brs) | 558 [M+H]⁺ |
| 239 | 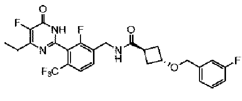 | DMSO-d₆,δ:1.13 (3H, t, J = 7.6 Hz), 2.08-2.22 (2H, m), 2.29-2.39 (2H, m), 2.59 (2H, qd, J = 7.5, 2.8 Hz), 2.94-3.08 (1H, m), 1.19 (1H, quin, J = 6.7 Hz), 4.39 (4H, s), 1.06-7.19 (3H, m), 7.39 (1H, td, J = 7.7, 6.0 Hz), 7.57-7.66 (1H, m), 7.68-7.76 (1H, m), 8.50 (1H, t, J = 5.9 Hz), 13.59 (1H, brs) | 540 [M+H]⁺ |
| 240 | 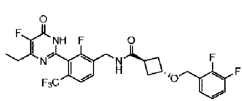 | DMSO-d₆,δ:1.13 (3H, t, J = 7.6 Hz), 2.06-2.21 (2H, m), 2.29-2.41 (2H, m), 2.59 (2H, qd, J = 7.4, 3.0 Hz), 2.95-3.09 (1H, m), 1.21 (1H, quin, J = 6.6 Hz), 4.39 (2H, d, J = 6.0 Hz), 4.46 (2H, d, J = 1.5 Hz), 7.14-7.30 (2H, m), 7.32-7.47 (1H, m), 7.57-7.67 1H, m), 7.69-7.79 (1H, m), 8.50 (1H, t, J = 6.0 Hz), 13.58 (1H, brs) | 558 [M+H]⁺ |

**[Table 49]**

| **Example No.** | **Chemical Structure** | **NMR (¹H-NMR)** | **MS (m/z)** |
|---|---|---|---|
| 241 | 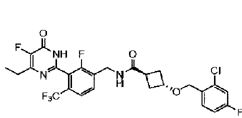 | DMSO-d₆,δ:1.13 (3H, t, J = 7.6 Hz), 2.10-2.24 (2H, m), 2.30-2.42 (2H, m), 2.56-2.65 2H, m), 2.95-3.08 (1H, m), 4.23 (1H, quin, J = 6.5 Hz), 4.36-4.44 (4H, m), 7.23 (1H, d, J = 8.7, 2.6 Hz), 7.44 (1H, dd, J = 9.1, 2.6 Hz), 7.53 (1H, dd, J = 8.5, 6.2 Hz), 7.58-7.67 (1H, m), 7.69-7.77 (1H, m), 8.50 (1H, t, J = 5.9 Hz), 13.59 (1H, brs) | 574 [M+H]⁺ |
| 242 | 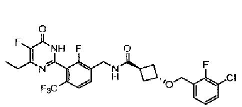 | DMSO-d₆,δ:1.13 (3H, t, J = 7.6 Hz), 2.07-2.22 (2H, m), 2.28-2.41 (2H, m), 2.55-2.65 2H, m), 2.94-3.07 (1H, m), 4.21 (1H, quin, J = 6.7 Hz), 4.39 (2H, d, J = 5.7 Hz), 4.45 2H, d, J = 0.8 Hz), 7.23 (1H, td, J = 7.8, 1.9 Hz), 7.37-7.47 (1H, m), 7.49-7.68 (2H, n), 7.69-7.77 (1H, m), 8.50 (1H, t, J = 5.9 Hz), 13.60 (1H, brs) | 574 [M+H]⁺ |
| 243 | 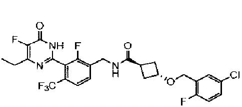 | DMSO-d₆,δ:1.13 (3H, t, J = 7.6 Hz), 2.07-2.23 (2H, m), 2.28-2.41 (2H, m), 2.56-2.64 2H, m), 2.95-3.08 (1H, m), 4.21 (1H, quin, J = 6.5 Hz), 4.36-4.44 (4H, m), 7.21-7.30 1H, m), 7.39-7.46 (1H, m), 7.49 (1H, dd, J = 6.2, 2.8 Hz), 7.58-7.67 (1H, m), 7.69-7.76 (1H, m), 8.50 (1H, t, J = 5.9 Hz), 3.64 (1H, brs) | 574 [M+H]⁺ |
| 244 | 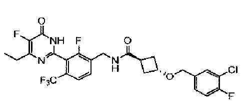 | DMSO-d₆,δ:1.10-1.16 (3H, m), 2.09-2.22 2H, m), 2.28-2.39 (2H, m), 2.56-2.65 (2H, n), 2.94-3.07 (1H, m), 4.13-4.25 (1H, m), 4.36 (2H, s), 4.39 (2H, d, J = 6.0 Hz), '.29-7.43 (2H, m), 7.53 (1H, dd, J = 7.2, .9 Hz), 7.57-7.66 (1H, m), 7.69-7.76 (1H, n), 8.50 (1H, t, J = 5.9 Hz), 13.59 (1H, brs) | 574 [M+H]⁺ |
| 245 | 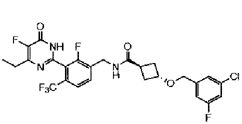 | DMSO-d₆,δ:1.10-1.16 (3H, m), 2.10-2.23 2H, m), 2.29-2.41 (2H, m), 2.54-2.66 (2H, n), 2.96-3.06 (1H, m), 4.20 (1H, quin, J = 6.7 Hz), 4.37-4.42 (4H, m), 7.16 (1H, dd, J = 8.7, 1.5 Hz), 7.25 (1H, s), 7.34 (1H, dt, J = 8.8, 2.2 Hz), 7.57-7.66 (1H, m), 7.69-7.75 (1H, m), 8.51 (1H, t, J = 6.0 Hz), 3.61 (1H, brs) | 574 [M+H]⁺ |
| 246 | 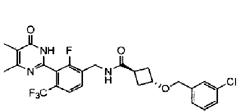 | DMSO-d₆,δ:1.98 (3H, s), 2.12-2.19 (2H, n), 2.24 (3H, s), 2.31-2.36 (2H, m), 2.97-3.04 (1H, m), 4.19 (1H, quin, J = 6.4 Hz), 4.38 (4H, brs), 7.28 (1H, d, J = 7.6 Hz), 7.32-7.41 (3H, m), 7.60 (1H, t, J = 7.6 Hz), 7.70 (1H, d, J = 8.0 Hz), 8.51 (1H, t, J = 6.0 Hz), 12.89 (1H, brs) | 537(M⁺) |

**[Table 50]**

| **Example No.** | **Chemical Structure** | **NMR (¹H-NMR)** | **MS (m/z)** |
|---|---|---|---|
| 247 | 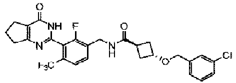 | DMSO-d₆,δ:2.02 (2H, quin, J = 6.0 Hz), 2.11-2.20 (2H, m), 2.30-2.37 (2H, m), 2.70 (2H, t, J = 7.6 Hz), 2.80 (2H, t, J = 8.0 Hz), 2.97-3.05 (1H, m), 4.19 (1H, quin, J = 6.4 Hz), 4.38-4.40 (4H, m), 7.28 (1H, d, J = 7.6 Hz), 7.32-7.41 (3H, m), 7.61 (1H, t, J = 8.0 Hz), 7.71 (1H, d, J = 8.4 Hz), 8.51 (1H, t, J = 6.0 Hz), 12.90 (1H, brs) | 549(M⁺) |
| 248 | 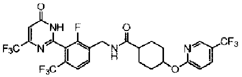 | DMSO-d₆,δ:1.37-2.04 (7H, m), 2.10-2.40 (2H, m), 4.40 (2H, d, J = 5.6 Hz), 4.91-5.36 (1H, m), 6.82-7.09 (2H, m), 7.54-7.86 (2H, m), 8.04 (1H, dd, J = 8.8, 2.7 Hz), 8.45-8.88 (2H, m), 13.91 (1H, brs) | 627 [M+H]⁺ |
| 249 | 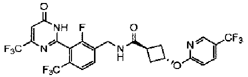 | DMSO-d₆,δ:2.29-2.42 (2H, m), 2.54-2.67 (2H, m), 3.10-3.22 (1H, m), 4.30-4.55 (2H, m), 5.11-5.47 (1H, m), 6.80-7.09 (2H, m), 7.59-7.85 (2H, m), 8.07 (1H, dd, J = 8.8, 2.2 Hz), 8.52-8.67 (2H, m), 13.91 (1H, brs) | 599 [M+H]⁺ |
| 250 | 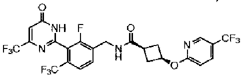 | DMSO-d₆,δ:2.17-2.30 (2H, m), 2.55-2.69 (2H, m), 2.76-2.87 (1H, m), 4.30-4.50 (2H, m), 5.09-5.46 (1H, m), 6.94 (1H, s), 6.97-7.03 (1H, m), 7.60-7.71 (1H, m), 7.76 (1H, d, J = 8.3 Hz), 8.07 (1H, dd, J = 8.8, 2.2 Hz), 8.48-8.70 (2H, m), 13.89 (1H, brs) | 599 [M+H]⁺ |
| 251 | 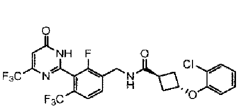 | CDCl₃,δ:2.37-2.55 (2H, m), 2.58-2.70 (2H, m), 3.06 (1H, tt, J = 9.4, 4.6 Hz), 4.51 (2H, d, J = 5.9 Hz), 4.87 (1H, quin, J = 6.4 Hz), 6.16 (1H, t, J = 6.0 Hz), 6.71 (1H, dd, J = 8.3, 1.2 Hz), 6.80 (1H, s), 6.89 (1H, td, J = 7.7, 1.2 Hz), 7.12-7.21 (1H, m), 7.36 (1H, dd, J = 7.8, 1.7 Hz), 7.52-7.67 (2H, m) | **564** [M+H]⁺ |
| 252 | 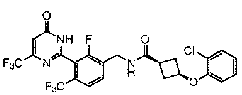 | CDC]₃,δ:2.25-2.45 (2H, m), 2.61-2.74 (3H, m), 4.47 (2H, d, J = 6.1 Hz), 4.53-4.65 (1H, m), 6.41 (1H, t, J = 6.0 Hz), 6.73 (1H, dd, J = 8.3, 1.2 Hz), 6.79 (1H, s), 6.90 (1H, td, J = 7.7, 1.2 Hz), 7.17 (1H, td, J = 7.8, 1.5 Hz), 7.35 (1H, dd, J = 7.9, 1.6 Hz), 7.45-7.60 (2H, m) | 564 [M+H]⁺ |

**[Table 51]**

| **Example No.** | **Chemical Structure** | **NMR (¹H-NMR)** | **MS (m/z)** |
|---|---|---|---|
| 253 | 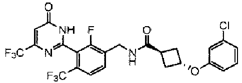 | CDCl₃,δ:2.31-2.48 (2H, m), 2.57-2.74 (2H, m), 2.96-3.08 (1H, m), 4.52 (2H, d, J = 6.1 Hz), 4.82 (1H, quin, J = 6.4 Hz), 6.12 (1H, t, J = 5.9 Hz), 6.66 (1H, ddd, J = 8.4, 2.4, 1.0 Hz), 6.73-6.81 (2H, m), 6.92 (1H, ddd, J = 7.9, 2.0, 0.9 Hz), 7.11-7,22 (1H, m), 7.53-7.69 (2H, m) | 564 [M+H]⁺ |
| 254 | 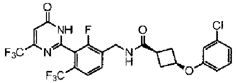 | CDCl₃,δ:2.19-2.40 (2H, m), 2.53-2.72 (3H, m), 4.46 (2H, d, J = 5.9 Hz), 4.48-4.59 (1H, m), 6.30 (1H, t, J = 5.5 Hz), 6.67 (1H, ddd, J = 8.3, 2.4, 0.7 Hz), 6.73-6.83 (2H, m), 6.88-6.98 (1H, m), 7.17 (1H, t, J = 8.2 Hz), 7.44-7.61 (2H, m) | 564 [M+H]⁺ |
| 255 | 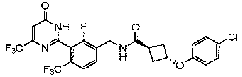 | CDCl₃,δ:2.28-2.45 (2H, m), 2.56-2.68 (2H, m), 2.97-3.09 (1H, m), 4.48 (2H, d, J = 5.9 Hz), 4.79 (1H, quin, J = 6.4 Hz), 6.24 (1H, t, J = 5.6 Hz), 6.61-6.76 (3H, m), 7.17-7.24 (2H, m), 7.49-7.64 (2H, m) | 564 [M+H]⁺ |
| 256 | 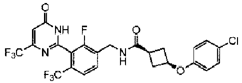 | CDCl₃,δ:2.19-2.35 (2H, m), 2.56-2.68 (3H, m), 4.40-4.55 (3H, m), 6.29 (1H, t, J = 5.9 Hz), 6.67-6.72 (2H, m), 6.74 (1H, s), 7.17-7.23 (2H, m), 7.45-7.57 (2H, m) | 564 [M+H]⁺ |
| 257 | 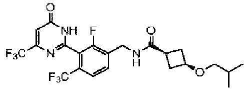 | DMSO-d₆,δ:0.84 (6H, d, J = 6.8 Hz), 1.64-1.78 (1H, m), 1.91-2.04 (2H, m), 2.24-2.42 (2H, m), 2.53-2.64 (1H, m), 3.03 (2H, d, J = 6.6 Hz), 3.71-3.89 (1H, m), 4.39 (2H, d, J = 5.6 Hz), 6.95 (1H, s), 7.59-7.69 (1H, m), 7.76 (1H, d, J = 8.3 Hz), 8.49 (1H, t, J = 5.7 Hz), 13.87 (1H, brs) | 510 [M+H]⁺ |
| 258 | 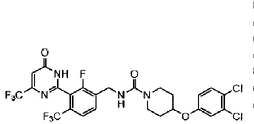 | DMSO-d₆,δ:1.44-1.61 (2H, m), 1.84-2.00 (2H, m), 3.11-3.23 (2H, m), 3.66-3.79 (2H, m), 4.37 (2H, d, J = 5.1 Hz), 4.55-4.70 (1H, m), 6.95 (1H, brs), 7.02 (1H, dd, J = 8.9, 2.8 Hz), 7.27-7.35 (2H, m), 7.52 (1H, d, J = 9.0 Hz), 7.63-7.80 (2H, m), 13.88 (1H, brs) | 627 [M+H]⁺ |

**[Table 52]**

| **Example No.** | **Chemical Structure** | **NMR (¹H-NMR)** | **MS (m/z)** |
|---|---|---|---|
| 259 | 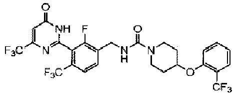 | CDCl₃,δ:1.72-1.91 (4H, m), 3.25-3.37 (2H, m), 3.42-3.51 (2H, m), 4.37 (2H, d, J = 5.1 Hz), 4.88 (1H, d, J = 2.9 Hz), 5.66 (1H, t, J = 5.0 Hz), 6.86 (1H, s), 6.95 (1H, d, J = 8.3 Hz), 7.02 (1H, t, J = 7.7 Hz), 7.43-7.51 (1H, m), 7.51-7.57 (2H, m), 7.59 (1H, d, J = 6.6 Hz) | 627 [M+H]⁺ |
| 260 | 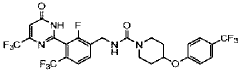 | CDCl₃,δ:1.73-1.85 (2H, m), 1.87-1.97 (2H, m), 3.27-3.41 (2H, m), 3.48-3.59 (2H, m), 4.38 (2H, d, J = 5.4 Hz), 4.52-4.63 (1H, m), 5.32 (1H. t, J = 5.5 Hz), 8.83 (1H, s), 6.95 (2H, d, J = 8.6 Hz), 7.50-7.63 (4H, m) | 627 [M+H]⁺ |
| 261 | 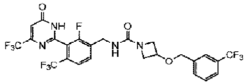 | CDCl₃,δ:3.83 (2H, dd, J = 8.8, 4.2 Hz), 4.02-4.09 (2H, m), 4.26 (2H, d, J = 5.6 Hz), 4.31-4.42 (1H, m), 4.49 (2H, s), 4.79-4.95 (1H, m), 6.79 (1H, s), 7.44-7.53 (4H, m), 7.55-7.61 (2H, m) | 613 [M+H]⁺ |
| 262 | 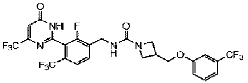 | CDCl₃,δ:2.91-3.16 (1H, m), 3.80 (2H, dd, J = 8.1, 5.4 Hz), 4.06 (2H, t, J = 8.3 Hz), 4.11 (2H, d, J = 6.4 Hz), 4.30 (2H, d, J = 5.9 Hz), 4.79 (1H, t, J = 5.3 Hz), 6.77 (1H, s), 7.06 (1H, dd, J = 8.3, 2.4 Hz), 7.12 (1H, s), 7.24 (1H, d, J = 7.8 Hz), 7.34-7.43 (1H, m), 7.47-7.55 (2H, m) | 613 [M+H]⁺ |
| 264 | 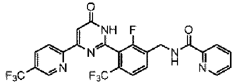 | DMSO-d₆,δ:4.67 (2H, d, J = 6.1 Hz), 7.37 (1H, brs), 7.50-7.87 (3H, m), 7.96-8.13 (2H, m), 8.26-8.44 (2H, m), 8.60-8.77 (1H, m), 9.13 (1H, s), 9.43-9.65 (1H, m), 13.54 (1H, brs) | 538 [M+H]⁺ |
| 265 | 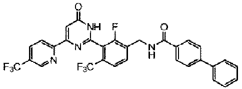 | DMSO-d₆,δ:4.61-4.73 (2H, m), 7.30-7.58 (4H, m), 7.68-7.88 (6H, m), 7.97-8.12 (2H, m), 8.37 (2H, s), 9.13 (1H, s), 9.22-9.38 (1H, m), 13.47 (1H, brs) | 613 [M+H]⁺ |

**[Table 53]**

| **Example No.** | **Chemical Structure** | **NMR (¹H-NMR)** | **MS (m/z)** |
|---|---|---|---|
| 270 | 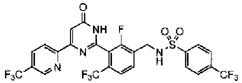 | DMSO-d₆,δ:4.12-4.35 (2H, m), 7.37 (1H, s), 7.61-7.78 (2H, m), 7.87-8.08 (4H, m), 8.22-8.50 (2H, m), 8.63-8.89 (1H, m), 9.02-9.27 (1H, m), 13.44 (1H, brs) | 641 [M+H]⁺ |
| 271 | 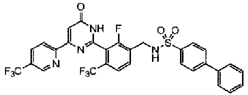 | DMSO-d₆,δ:4.16-4.28 (2H, m), 7.20-7.35 (1H, m), 7.38-7.56 (3H, m), 7.88-7.80 (4H, m), 7.84-7.97 (4H, m), 8.23-8.36 (2H, m), 8.42-8.60 (1H, *m),* 0.10 (1H, s), 13.45 (1H, brs) | 649 [M+H]⁺ |
| 272 | 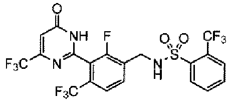 | DMSO-d₆,δ:4.32 (2H, d, J = 6.4 Hz), 6.99 (1H, brs), 7.74-7.88 (4H, m), 7.97 (1H, dd, J = 7.6, 2.0 Hz), 8.07 (1H, dd, J = 7.2, 1.6 Hz), 8.77 (1H, t, J = 6.0 Hz), 13.88 (1H, brs) | 563(M⁺) |
| 273 | 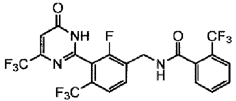 | DMSO-d₆,δ:4.60 (2H, d, J = 6.0 Hz), 7.04 (1H, brs), 7.52-7.83 (6H, m), 9.25 (1H, t, J = 5.6 Hz), 13.92 (1H, brs) | 527(M⁺) |
| 274 | 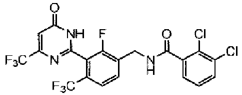 | DMSO-d₆,δ:4.61 (2H, d, J = 5.6 Hz), 7.05 (1H, s), 7.43-7.50 (2H, m), 7.74 (1H, dd, J = 7.6, 2.4 Hz), 7.62-7.90 (2H, m), 9.29 (1H, t, J = 6.0 Hz), 13.92 (1H, brs) | 527(M⁺) |
| 275 | 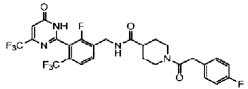 | DMSO-d₆,δ:1.32-1.42 (2H, m), 1.68-1.77 (2H, m), 2.43-2.54 (1H, m), 2.58-2.65 (1H, m), 2.99-3.07 (1H, m), 3.70 (2H, s), 3.95-4.00 (1H, m), 4.35-4.41 (3H, m), 7.05 (1H, s), 7.09-7.16 (2H, m), 7.22-7.28 (2H, m), 7.66 (1H, t, J = 7.6 Hz), 7.77 (1H, d, J = 8.4 Hz), 8.55 (1H, t, J = 5.6 Hz), 13.89 (1H, brs) | 602(M⁺) |

**[Table 54]**

| **Example No.** | **Chemical Structure** | **NMR (¹H-NMR)** | **MS (m/z)** |
|---|---|---|---|
| 279 | 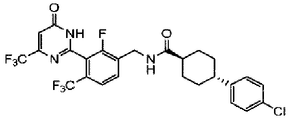 | DMSO-d₆,δ:1.38-1.60 (4H, m), 1.87 (4H, qd, J = 11.2, 2.0 Hz), 2.29 (1H, tt, J = 12.0, 3.6 Hz), 2.46-2.55 (1H, m), 4.41 (2H, d, J = 5.6 Hz), 7.06 (1H, brs), 7.27 (2H, dt, J = 8.0, 2.0 Hz), 7.34 (2H, dt, J = 8.4, 2.0 Hz), 7.68 (1H, t, J = 8.0 Hz), 7.79 (1H, d, J = 8.4 Hz), 8.52 (1H, t, J = 6.0 Hz), 13.89 (1H, brs) | 575(M⁺) |
| 280 | 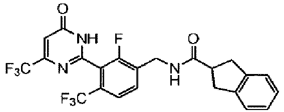 | DMSO-d₆,δ:3.04-3.16 (4H, m), 3.25-3.36 (1H, m), 4.46 (2H, d, J = 5.6 Hz), 7.05 (1H, s), 7.11-7.15 (2H, m), 7.18-7.22 (2H, m), 7.73 (1H, t, J = 8.0 Hz), 7.79 (1H, d, J = 8.4 Hz), 8.69 (1H, t, J = 6.0 Hz), 13.89 (1H, brs) | 499(M⁺) |
| 281 | 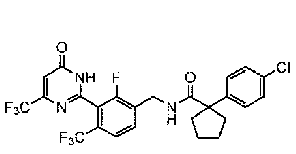 | DMSO-d₆,δ:1.59-1.65 (4H, m), 1.80-1.86 (2H, m), 2.46-2.55 (2H, m), 4.32 (2H, d, J = 6.0 Hz), 7.03 (1H, s), 7.30-7.42 (5H, m), 7.66 (1H, d, J = 8.4 Hz), 8.25 (1H, t, J = 6.0 Hz), 13.86 (1H, brs) | 561(M⁺) |
| 282 | 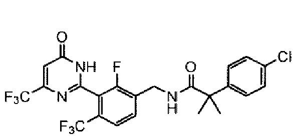 | DMSO-d₆,δ:1.46 (6H, s), 4.36 (2H, d, J = 5.6 Hz), 7.05 (1H, s), 7.31-7.36 (2H, m), 7.37-7.42 (2H, m), 7.51 (1H, t, J = 8.0 Hz), 7.74 (1H, d, J = 8.4 Hz), 8.13 (1H, t, J = 3.0 Hz), 13.88 (1H, brs) | 535(M⁺) |
| 283 | 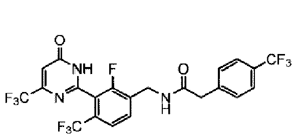 | DMSO-d₆,δ:3.65 (2H, s), 4.43 (2H, d, J = 5.6 Hz), 7.05 (1H, s), 7.50 (2H, d, J = 8.0 Hz), 7.66-7.72 (3H, m), 7.76 (1H, d, J = 3.4 Hz), 8.83 (1H, t, J = 6.0 Hz), 13.89 (1H, brs) | 541(M⁺) |

### Test Example 1: Test for mPGES-1 inhibitory activity

Microsomes were prepared from COS-1 cells transiently transfected with a plasmid containing human mPGES-1 cDNA, and used as mPGES-1 enzyme. The mPGES-1 enzyme was diluted with a sodium phosphate buffer (pH 7.2) containing reduced glutathione (2.5 mM) and EDTA (1 mM), DMSO or a DMSO solution of a test compound (final concentration of DMSO was 2%) was added to the enzyme, and the mixture was preincubated at 4°C for 15 minutes. Then, PGH2 (Prostaglandin H2) as the substrate was added at a final concentration of 1 µM to start the enzymatic reaction, and after incubation at 4°C for 4 minutes, a solution of ferric chloride (25 mM) and citric acid (50 mM) was added to terminate the enzymatic reaction. Generated PGE2 (Prostaglandin E2) was measured by using Prostaglandin E2 Express EIA Kit (Cayman Chemical). IC₅₀ values were determined by using a standard method. The results are shown in the tables mentioned below.

**[Table 55]**

| **Example No.** | **h mPGES1 IC₅₀ (nM)** | **Example No.** | **h mPGES1 IC₅₀ (nM)** | **Example No.** | **h mPGES1 IC₅₀ (nM)** | **Example No.** | **h mPGES1 IC₅₀ (nM)** |
|---|---|---|---|---|---|---|---|
| 1 | 37 | 36 | 14 | 72 | < 1 | 111 | 19 |
| 2 | 28.7 | 37 | 18 | 73 | 4.4 | 112 | 29 |
| 3 | < 1 | 38 | 7.8 | 74 | < 1 | 114 | 5.2 |
| 4 | < 1 | 39 | < 1 | 75 | < 1 | 115 | 21 |
| 5 | < 10 | 40 | 15 | 76 | 31 | 116 | 10 |
| 6 | < 10 | 41 | 26 | 77 | 16 | 117 | <1 |
| 7 | < 1 | 42 | < 1 | 79 | 88 | 118 | 41 |
| 8 | 0.64 | 44 | < 1 | 80 | 22 | 119 | < 1 |
| 9 | < 10 | 45 | 15 | 81 | 8 | 120 | 11 |
| 10 | < 1 | 46 | 49 | 82 | 8.1 | 121 | < 1 |
| 11 | < 1 | 48 | < 1 | 83 | 7.1 | 122 | < 1 |
| 12 | 2.1 | 49 | 21 | 84 | 6.2 | 123 | < 1 |
| 13 | 2.4 | 50 | < 1 | 85 | 0.61 | 124 | < 1 |
| 14 | < 1 | 51 | < 1 | 86 | < 1 | 125 | 19 |
| 15 | < 1 | 52 | 4.9 | 87 | 39 | 126 | < 1 |
| 17 | < 10 | 53 | < 1 | 88 | < 1 | 127 | < 1 |
| 19 | < 1 | 55 | 30 | 89 | < 1 | 128 | 13 |
| 20 | < 1 | 56 | 79 | 90 | 1.6 | 129 | 25 |
| 21 | < 1 | 57 | 5 | 93 | 1.4 | 130 | < 1 |
| 22 | 4.9 | 58 | < 1 | 94 | 13 | 131 | < 1 |
| 23 | 4.8 | 59 | 4.2 | 95 | < 1 | 132 | 21 |
| 24 | 76 | 60 | < 1 | 97 | < 1 | 133 | 27 |
| 25 | < 1 | 61 | 0.87 | 98 | 19 | 134 | < 10 |
| 26 | 4.1 | 62 | < 1 | 99 | < 1 | 135 | 6.4 |
| 27 | < 1 | 63 | 1.1 | 100 | < 1 | 136 | 2 |
| 28 | < 1 | 64 | < 1 | 101 | < 1 | 137 | 2.9 |
| 29 | 11 | 65 | < 1 | 102 | < 1 | 138 | < 1 |
| 30 | < 1 | 66 | < 1 | 103 | < 1 | 139 | < 1 |
| 31 | < 1 | 67 | < 1 | 104 | 1.2 | 140 | 1 |
| 32 | < 1 | 68 | < 1 | 105 | 23 | 141 | < 1 |
| 33 | 14 | 69 | 8.4 | 107 | 13 | 142 | < 1 |
| 34 | < 1 | 70 | 10 | 108 | 38 | 143 | 28 |
| 35 | 11 | 71 | 10 | 109 | 10 | 144 | < 1 |

**[Table 56]**

| **Example No.** | **h mPGES1 IC₅₀ (nM)** | **Example No.** | **h mPGES1 IC₅₀ (nM)** | **Example No.** | **h mPGES1 IC₅₀ (nM)** | **Example No.** | **h mPGES1 IC₅₀ (nM)** |
|---|---|---|---|---|---|---|---|
| 145 | 98 | 178 | 10 | 211 | 7.9 | 244 | < 1 |
| 146 | 2.1 | 179 | < 1 | 212 | 9.3 | 245 | < 1 |
| 147 | < 1 | 180 | < 1 | 213 | < 1 | 246 | < 1 |
| 148 | < 1 | 181 | 1.7 | 214 | 11 | 247 | < 1 |
| 149 | < 1 | 182 | 19 | 215 | 8.6 | 248 | < 1 |
| 150 | < 1 | 183 | 12 | 216 | 6.9 | 249 | < 1 |
| 151 | < 1 | 184 | 10 | 217 | 16 | 250 | < 1 |
| 152 | < 1 | 185 | < 1 | 218 | 16 | 251 | < 1 |
| 153 | < 1 | 186 | 1.2 | 219 | 13 | 253 | 1 |
| 154 | < 1 | 187 | 3.2 | 220 | 15 | 254 | 4.6 |
| 155 | < 1 | 188 | 6.4 | 221 | 15 | 255 | 2.8 |
| 156 | < 1 | 189 | 10 | 222 | 14 | 257 | 6.8 |
| 157 | < 1 | 190 | 13 | 223 | 15 | 258 | < 1 |
| 158 | < 1 | 191 | < 1 | 224 | < 1 | 260 | < 1 |
| 159 | < 1 | 192 | < 1 | 225 | < 1 | 266 | < 1 |
| 160 | 12 | 193 | < 1 | 226 | < 1 | 267 | < 1 |
| 161 | < 1 | 194 | < 1 | 227 | < 1 | 269 | 7.6 |
| 162 | < 1 | 195 | 3.6 | 228 | < 1 | 271 | 76 |
| 163 | < 1 | 196 | < 1 | 229 | < 1 | 272 | 33 |
| 164 | < 1 | 197 | < 1 | 230 | < 1 | 273 | 8.4 |
| 165 | < 1 | 198 | 3.7 | 231 | < 1 | 274 | < 1 |
| 166 | < 1 | 199 | 1.4 | 232 | < 1 | 275 | 0.94 |
| 167 | 8.8 | 200 | 1.1 | 233 | < 1 | 276 | 24 |
| 168 | < 1 | 201 | 6.5 | 234 | < 1 | 278 | 35 |
| 169 | < 1 | 202 | < 1 | 235 | 1.1 | 279 | < 1 |
| 170 . | < 1 | 203 | < 1 | 236 | < 1 | 280 | < 1 |
| 171 | 1.3 | 204 | < 1 | 237 | < 1 | 281 | 45 |
| 172 | < 1 | 205 | 13 | 238 | < 1 | 283 | 1.5 |
| 173 | < 1 | 206 | 14 | 239 | < 1 | | |
| 174 | < 1 | 207 | 15 | 240 | 3.2 | | |
| 175 | < 1 | 208 | 9 | 241 | < 1 | | |
| 176 | < 1 | 209 | 8.8 | 242 | < 1 | | |
| 177 | < 1 | 210 | 8.8 | 243 | < 1 | | |

## Claims

1. A compound represented by the following general formula (1): 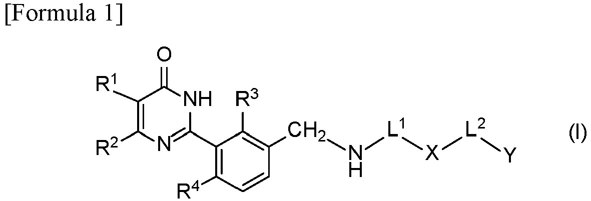
(in the formula, R¹ and R² each independently represent a hydrogen atom, a halogen atom, a C₁₋₆ alkyl group which may have a substituent, or an aromatic heterocyclic group having 1 or 2 or more ring-constituting heteroatoms which may have a substituent, and when R¹ and R² both represent an alkyl group, they may be bonded to each other to form a C₃₋₆ alkylene group which may have a substituent;
R³ and R⁴ each independently represent a hydrogen atom, a halogen atom, or a C₁₋₆ alkyl group which may have a substituent;
L¹ represents -CO-, -SO₂-, or -CO-C(R⁵)(R⁶)- (R⁵ and R⁶ each independently represent a hydrogen atom or a C₁₋₆ alkyl group which may have a substituent, and when R⁵ and R⁶ both represent an alkyl group, they may be bonded to each other to form a C₂₋₅ alkylene group which may have a substituent);
X represents a cyclic hydrocarbon group which may have a substituent or a heterocyclic group having 1 or 2 or more ring-constituting heteroatoms which may have a substituent;
L² represents -A¹-R⁷-A²- (A¹ represents a single bond, -CO-, -NH-, or -O-; R⁷ represents a single bond or a C₁₋₆ alkylene group; and A² represents a single bond, -CO-, -NH-, or -O-); and
Y represents a hydrogen atom, a cyclic hydrocarbon group which may have a substituent, or a heterocyclic group having 1 or 2 or more ring-constituting heteroatoms which may have a substituent)
or a salt thereof.

2. The compound or a salt thereof according to claim 1,
wherein R¹ and R² each independently is a hydrogen atom, a fluorine atom, a C₁₋₆ alkyl group (this alkyl group may be substituted with 1 or 2 or more halogen atoms), or an aromatic heterocyclic group having 1 or 2 or more ring-constituting heteroatoms which may have a substituent, and when R¹ and R² both represent alkyl groups, they may be bonded to each other to form a C₃₋₅ alkylene group;
R³ and R⁴ each independently is a hydrogen atom, a fluorine atom, a chlorine atom, or a C₁₋₆ alkyl group (this alkyl group may be substituted with 1 or 2 or more fluorine atoms);
L¹ is -CO-, -SO₂-, or -CO-C(R⁵)(R⁶)- (R⁵ and R⁶ each independently represent a hydrogen atom or a C₁₋₆ alkyl group (when R⁵ and R⁶ both represent an alkyl group, they may be bonded to each other to form a C₃₋₅ alkylene group);
X is an aromatic hydrocarbon group which may have a substituent, a saturated or partially saturated cyclic hydrocarbon group which may have a substituent, an aromatic heterocyclic group having 1 or 2 or more ring-constituting heteroatoms which may have a substituent, or a saturated or partially saturated heterocyclic group having 1 or 2 or more ring-constituting heteroatoms which may have a substituent;
in -A¹-R⁷-A²- represented by L², A¹, R⁷, and A² are all single bonds; A¹ is a single bond, R⁷ is -CO-, -NH-, or -O-, and A² is a single bond or a C₁₋₆ alkylene group; or A¹ is a C₁₋₆ alkylene group, R⁷ is -CO-, -NH-, or -O-, and A² is a single bond); and
Y is a hydrogen atom, an aromatic hydrocarbon group which may have a substituent, a saturated or partially saturated cyclic hydrocarbon group which may have a substituent, an aromatic heterocyclic group having 1 or 2 or more ring-constituting heteroatoms which may have a substituent, or a saturated or partially saturated heterocyclic group having 1 or 2 or more ring-constituting heteroatoms which may have a substituent.

3. The compound or a salt thereof according to claim 1,
wherein R¹ is a hydrogen atom, a fluorine atom, or a C₁₋₆ alkyl group (this alkyl group may be substituted with 1 or 2 or more fluorine atoms);
R² is a fluorine atom, a C₁₋₆ alkyl group (this alkyl group may be substituted with 1 or 2 or more halogen atoms), or an aromatic heterocyclic group having 1 or 2 or more ring-constituting heteroatoms which may have a substituent, and when R¹ and R² both represent alkyl groups, they may be bonded to each other to form a C₃₋₅ alkylene group;
R³ and R⁴ each independently is a hydrogen atom, a fluorine atom, a chlorine atom, or a C₁₋₆ alkyl group (this alkyl group may be substituted with 1 or 2 or more fluorine atoms);
L¹ is -CO-, -SO₂-, or -CO-C(R⁵)(R⁶)- (R⁵ and R⁶ each independently is a hydrogen atom or a C₁₋₆ alkyl group (this alkyl group may be bonded to each other to form a C₃₋₄ alkylene group);
X is a monocyclic or bicyclic aromatic hydrocarbon group which may have a substituent, a monocyclic or bicyclic, saturated or partially saturated cyclic hydrocarbon group which may have a substituent, a monocyclic or bicyclic aromatic heterocyclic group having 1 or 2 or more ring-constituting heteroatoms which may have a substituent, or a monocyclic or bicyclic, saturated or partially saturated heterocyclic group having 1 or 2 or more ring-constituting heteroatoms which may have a substituent;
in -A¹-R⁷-A²- represented by L², A¹, R⁷, and A² are all single bonds; A¹ is a single bond, R⁷ is -CO-, -NH-, or -O-, and A² is a single bond or a C₁₋₄ alkylene group; or A¹ is a C₁₋₄ alkylene group, R⁷ is -CO-, -NH-, or -O-, and A² is a single bond); and
Y is a hydrogen atom, a monocyclic or bicyclic aromatic hydrocarbon group which may have a substituent, a monocyclic or bicyclic, saturated or partially saturated cyclic hydrocarbon group which may have a substituent, a monocyclic or bicyclic aromatic heterocyclic group having 1 or 2 or more ring-constituting heteroatoms which may have a substituent, or a monocyclic or bicyclic, saturated or partially saturated heterocyclic group having 1 or 2 or more ring-constituting heteroatoms which may have a substituent.

4. The compound or a salt thereof according to claim 1,
wherein R¹ represents a hydrogen atom, a fluorine atom, or a C₁₋₆ alkyl group (this alkyl group may be substituted with 1 or 2 or more fluorine atoms);
R² is a fluorine atom or a C₁₋₆ alkyl group (this alkyl group may be substituted with 1 or 2 or more fluorine atoms), or a monocyclic aromatic heterocyclic group containing 1 or 2 or more ring-constituting nitrogen atoms which may have a substituent;
R³ and R⁴ each independently is a hydrogen atom, a fluorine atom, a chlorine atom, or a C₁₋₆ alkyl group (this alkyl group may be substituted with 1 or 2 or more fluorine atoms);
L¹ is -CO- or -SO₂-;
X is a phenyl group which may have a substituent, a naphthyl group which may have a substituent, a monocyclic or bicyclic, 3- to 12-membered, saturated or partially saturated cyclic hydrocarbon group which may have a substituent, a monocyclic or bicyclic aromatic heterocyclic group containing 1 or 2 or more ring-constituting nitrogen atoms which may have a substituent, or a monocyclic or bicyclic, saturated or partially saturated heterocyclic group containing 1 or 2 or more ring-constituting nitrogen atoms which may have a substituent;
in -A¹-R⁷-A²- represented by L², A¹, R⁷, and A² are all single bonds; A¹ is a single bond, R⁷ is -O-, and A² is a single bond or a C₁₋₄ alkylene group; or A¹ is a C₁₋₄ alkylene group, R⁷ is -O-, and A² is a single bond; and
Y is a hydrogen atom, a monocyclic or bicyclic aromatic hydrocarbon group which may have a substituent, a monocyclic or bicyclic, 3- to 12-membered, saturated or partially saturated cyclic hydrocarbon group which may have a substituent, a monocyclic or bicyclic aromatic heterocyclic group containing 1 or 2 or more ring-constituting nitrogen atoms which may have a substituent, or a monocyclic or bicyclic, saturated or partially saturated heterocyclic group containing 1 or 2 or more ring-constituting nitrogen atoms which may have a substituent.

5. The compound or a salt thereof according to claim 1,
wherein R¹ is a hydrogen atom, a fluorine atom, or a methyl group;
R² is a hydrogen atom, a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a pyridyl group, a pyridazinyl group, or a pyrazinyl group (provided that the aforementioned pyridyl group, pyridazinyl group, or pyrazinyl group may have 1 or 2 or more substituents selected from the following group: a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group);
R³ and R⁴ each independently is a hydrogen atom, a fluorine atom, a chlorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
L¹ is -CO-;
X is a phenyl group which may have a substituent, a naphthyl group which may have a substituent, a monocyclic saturated hydrocarbon group which may have a substituent, a monocyclic aromatic heterocyclic group containing 1 or 2 or more ring-constituting nitrogen atoms which may have a substituent, or a monocyclic saturated or partially saturated heterocyclic group containing 1 or 2 or more ring-constituting nitrogen atoms which may have a substituent;
in -A¹-R⁷-A²- represented by L², A¹, R⁷, and A² are all single bonds; A¹ is a single bond, R⁷ is -O-, and A² is a single bond, a methylene group, or an ethylene group; or A¹ is a methylene group or an ethylene group, R⁷ is -O-, and A² is a single bond; and
Y is a hydrogen atom, a monocyclic or bicyclic aromatic hydrocarbon group which may have a substituent, a saturated, monocyclic, 3- to 7-membered cyclic hydrocarbon group which may have a substituent, a monocyclic or bicyclic aromatic heterocyclic group containing 1 or 2 or more ring-constituting nitrogen atoms which may have a substituent, or a saturated or partially saturated, monocyclic or bicyclic heterocyclic group containing 1 or 2 or more ring-constituting nitrogen atoms which may have a substituent.

6. The compound or a salt thereof according to claim 1,
wherein R¹ is a hydrogen atom or a fluorine atom;
R² is a hydrogen atom, a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a 2-pyridyl group, a 3-pyridyl group, a 4-pyridyl group, a 3-pyridazinyl group, a 4-pyridazinyl group, or a 2-pyrazinyl group (provided that the aforementioned 2-pyridyl group, 3-pyridyl group, 4-pyridyl group, 3-pyridazinyl group, 4-pyridazinyl group, or 2-pyrazinyl group may have 1 or 2 or more substituents selected from the following group: a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group);
R³ is a hydrogen atom or a fluorine atom;
R⁴ is a fluorine atom, a chlorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
L¹ is -CO-;
X is a monocyclic saturated hydrocarbon group which may have a substituent or a monocyclic, saturated or partially saturated heterocyclic group containing 1 or 2 or more ring-constituting nitrogen atoms which may have a substituent;
L² is a single bond, -O-, -CH₂-O-, or -O-CH₂-; and
Y is a hydrogen atom, a phenyl group which may have a substituent, a naphthyl group which may have a substituent, or a monocyclic or bicyclic aromatic heterocyclic group containing 1 or 2 or more ring-constituting nitrogen atoms which may have a substituent.

7. The compound or a salt thereof according to claim 1,
wherein R¹ is a hydrogen atom or a fluorine atom;
R² is a fluorine atom, a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, a trifluoromethyl group, a 2-pyridyl group, a 3-pyridyl group, a 4-pyridyl group, a 3-pyridazinyl group, a 4-pyridazinyl group, or a 2-pyrazinyl group (provided that the aforementioned 2-pyridyl group, 3-pyridyl group, 4-pyridyl group, 3-pyridazinyl group, 4-pyridazinyl group, or 2-pyrazinyl group may have 1 or 2 or more substituents selected from the following group: a methyl group, an ethyl group, a methoxy group, an ethoxy group, a fluorine atom, a chlorine atom, a difluoromethyl group, a trifluoromethyl group, a difluoromethyloxy group, a trifluoromethyloxy group, and a cyano group);
R³ is a fluorine atom;
R⁴ is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
L¹ is -CO-;
X is a monocyclic saturated hydrocarbon group which may have a substituent or a monocyclic saturated heterocyclic group containing 1 or 2 or more ring-constituting nitrogen atoms which may have a substituent;
L² is a single bond, -O-, or -O-CH₂-; and
Y is a hydrogen atom, a phenyl group which may have a substituent, a naphthyl group which may have a substituent, a pyridyl group which may have a substituent, or a quinolyl group which may have a substituent.

8. The compound or a salt thereof according to claim 1,
wherein R¹ is a hydrogen atom or a fluorine atom;
R² is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
R³ is a fluorine atom;
R⁴ is a trifluoromethyl group;
L¹ is -CO-;
X is a 4- to 6-membered saturated hydrocarbon group which may have a substituent or a 4- to 6-membered saturated heterocyclic group containing one ring-constituting nitrogen atom which may have a substituent;
L² is a single bond, -O-, or -O-CH₂-; and
Y is a hydrogen atom, a phenyl group which may have a substituent, a naphthyl group which may have a substituent, a pyridyl group which may have a substituent, or a quinolyl group which may have a substituent.

9. The compound or a salt thereof according to claim 1,
wherein R¹ is a hydrogen atom or a fluorine atom;
R² is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
R³ is a fluorine atom;
R⁴ is a trifluoromethyl group;
L¹ is -CO-;
X is a cyclobutanediyl group, a cyclopentanediyl group, a cyclohexanediyl group, an azetidinediyl group, a pyrrolidinediyl group, or a piperidinediyl group;
L² is a single bond or -O-CH₂-; and
Y is a hydrogen atom, a phenyl group, a naphthyl group, a pyridyl group, or a quinolyl group (provided that the aforementioned phenyl group, naphthyl group, pyridyl group, or quinolyl group may have 1 or 2 or more substituents selected from the following group: a C₁₋₆ alkyl group, a C₁₋₆ alkoxy group, a halogen atom, a C₁₋₆ alkyl group substituted with 1 or 2 or more fluorine atoms, a C₁₋₆ alkoxy group substituted with 1 or 2 or more fluorine atoms, and a cyano group).

10. The compound or a salt thereof according to claim 1,
wherein R¹ is a hydrogen atom or a fluorine atom;
R² is a methyl group, an ethyl group, a propyl group, an isopropyl group, a 1-methyl-1-methoxyethyl group, a cyclopropyl group, a cyclopentyl group, a tert-butyl group, a fluoromethyl group, a difluoromethyl group, or a trifluoromethyl group;
R³ is a fluorine atom;
R⁴ is a trifluoromethyl group;
L¹ is -CO-;
X is a 1,3-cyclobutanediyl group, a 1,4-cyclohexanediyl group, a 1,3-azetidinediyl group, or a 1,4-piperidinediyl group;
L² is a single bond or -O-CH₂-; and
Y is a hydrogen atom, a phenyl group, a naphthyl group, a pyridyl group, or a quinolyl group (provided that the aforementioned phenyl group, naphthyl group, pyridyl group, or quinolyl group may have 1 or 2 or more substituents selected from the following group: a methyl group, an ethyl group, a methoxy group, an ethoxy group, a fluorine atom, a chlorine atom, a difluoromethyl group, a trifluoromethyl group, a difluoromethyloxy group, a trifluoromethyloxy group, and a cyano group).

11. An mPGES-1 inhibitor containing the compound represented by the general formula (I), or a salt thereof according to claim 1.

12. A PGE2 biosynthesis inhibitor containing the compound represented by the general formula (I), or a salt thereof according to claim 1.

13. A medicament containing the compound represented by the general formula (I), or a physiologically acceptable salt thereof according to claim 1 as an active ingredient.

14. A medicament containing the compound represented by the general formula (I), or a physiologically acceptable salt thereof according to claim 1 as an active ingredient, which is for use in prophylactic and/or therapeutic treatment of inflammation, pain, rheumatism, osteoarthritis, pyrexia, Alzheimer's disease, multiple sclerosis, arteriosclerosis, ocular hypertension, ischemic retinopathy, systemic scleroderma, malignant tumors overactive bladder, bladder outlet obstruction associated with benign prostatic hyperplasia, nocturia, urinary incontinence, neurogenic bladder, interstitial cystitis, bladder pain syndrome, urinary calculus, benign prostatic hyperplasia, or a disease for which suppression of the PGE2 production exhibits efficacy.
